(19) Europäisches Patentamt
European Patent Office
Office européen des brevets

(11) EP 1 184 036 A2

(12) **EUROPEAN PATENT APPLICATION**

(43) Date of publication:
**06.03.2002 Bulletin 2002/10**

(21) Application number: **01127194.7**

(22) Date of filing: **18.03.1999**

(51) Int Cl.[7]: **A61K 31/551**, A61K 31/4375, A61P 25/24, A61P 25/22, A61P 25/18, A61P 13/00, A61P 11/06, A61P 19/02, A61P 29/00, A61P 1/08, A61P 1/00, A61P 11/14, A61P 43/00

(84) Designated Contracting States:
**AT BE CH CY DE DK ES FI FR GB GR IE IT LI LU MC NL PT SE**

(30) Priority: **19.03.1998 JP 6999998**

(62) Document number(s) of the earlier application(s) in accordance with Art. 76 EPC:
**99909233.1 / 1 061 926**

(71) Applicant: **Takeda Chemical Industries, Ltd.**
**Osaka-shi, Osaka 541-0045 (JP)**

(72) Inventors:
- **Natsugari, Hideaki**
  **Ashiya-shi, Hyogo 659-0042 (JP)**
- **Doi, Takayuki**
  **Izumi-shi, Osaka 549-0013 (JP)**
- **Ikeura, Yoshinori**
  **Kashiba-shi, Nara 639-0251 (JP)**

(74) Representative:
**von Kreisler, Alek, Dipl.-Chem. et al**
**Patentanwälte, von Kreisler-Selting-Werner,**
**Bahnhofsvorplatz 1 (Deichmannhaus)**
**50667 Köln (DE)**

Remarks:
This application was filed on 16 - 11 - 2001 as a divisional application to the application mentioned under INID code 62.

# (54) Heterocyclic compounds, their production and use as tachykinin receptor antagonists

(57) A compound represented by the formula:

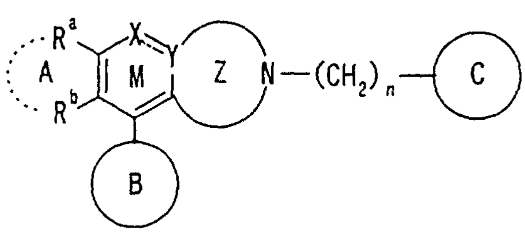

wherein ring M is a heterocyclic ring wherein

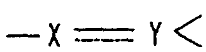

is one of -N=C<, -CO-N< or -CS-N<;

$R^a$ and $R^b$ are bonded to each other to form Ring A, or they are the same or different and represent, independently, a hydrogen atom or a substituent on the Ring M;
Ring A and Ring B represent, independently, an optionally substituted homocyclic or heterocyclic ring, with the proviso that at least one of them is an optionally substituted heterocyclic ring;
Ring C is an optionally substituted homocyclic or heterocyclic ring;
Ring Z is an optionally substituted nitrogen-containing heterocyclic ring; and
n is an integer from 1 to 6, or a salt thereof has a tachykinin receptor antagonistic activity in vitro, and is useful for preventing or treating depression, anxiety, manic-depressive illness or psychopathy.

EP 1 184 036 A2

## Description

Technical Field

The present invention relates to a novel utility of heterocyclic compounds having a tachykinin receptor antagonistic action, and novel heterocyclic compounds having such action, a method for producing them, as well as a pharmaceutical composition comprising the foregoing heterocyclic compounds.

Background

EP-A-0733632 describes that a heterocyclic compound represented by the formula:

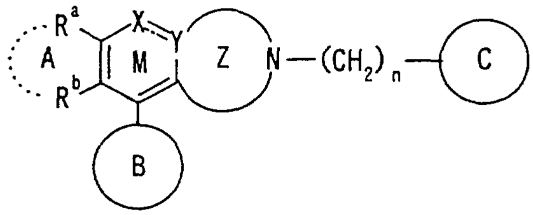

wherein ring M is a heterocyclic ring wherein

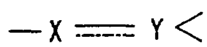

is one of -N=C<, -CO-N< or -CS-N<; $R^a$ and $R^b$ are bonded to each other to form Ring A, or they are the same or different and represent, independently, a hydrogen atom or a substituent on the Ring M; Ring A and Ring B represent, independently, an optionally substituted homocyclic or heterocyclic ring, with the proviso that at least one of them is an optionally substituted heterocyclic ring; Ring C is an optionally substituted homocyclic or heterocyclic ring; Ring Z is an optionally substituted nitrogen-containing heterocyclic ring; and n is an integer from 1 to 6, and a method for producing the compound, and furthermore the compound has a tachykinin receptor antagonistic action, a substance P receptor antagonistic action and a neurokinin A receptor antagonistic action.

The present invention aims to provide a novel utility of the heterocyclic compound (I) or a salt thereof.

Disclosure of Invention

The present inventors have assiduously studied in consideration of the above-mentioned situation and, as a result, have found that the heterocyclic compound (I) shows a treating effect against to depression, anxiety, manic-depressive illness or psychopathy. On the basis of these findings, the present inventors have completed the present invention.

Specifically, the present invention relates to:

(I) A pharmaceutical composition for preventing or treating depression, anxiety, manic-depressive illness or psychopathy which comprises a compound represented by the formula:

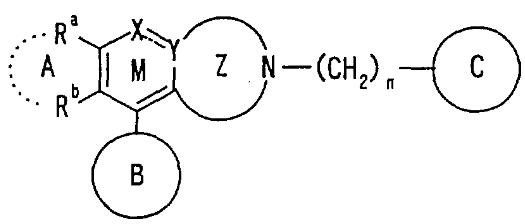

(I)

wherein ring M is a heterocyclic ring wherein

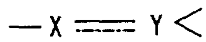

is one of -N=C<, -CO-N< or -CS-N<;
$R^a$ and $R^b$ are bonded to each other to form Ring A, or they are the same or different and represent, independently, a hydrogen atom or a substituent on the Ring M;
Ring A and Ring B represent, independently, an optionally substituted homocyclic or heterocyclic ring, with the proviso that at least one of them is an optionally substituted heterocyclic ring;
Ring C is an optionally substituted homocyclic or heterocyclic ring;
Ring Z is an optionally substituted nitrogen-containing heterocyclic ring; and
n is an integer from 1 to 6, or a salt thereof,
(II) The pharmaceutical composition as defined in (I),
wherein $R^a$ and $R^b$ are the same or different and represent, independently, a hydrogen atom or a substituent selected from the group consisting of

(1) a halogen atom,
(2) a $C_{1-6}$ alkyl group optionally having from 1 to 5 substituents selected from the group consisting of

(a) a hydroxy group,
(b) a $C_{1-6}$ alkoxy group,
(c) a $C_{1-6}$ alkylthio group,
(d) an amino group,
(e) a $C_{1-7}$ acylamino group,
(f) a carboxyl group,
(g) a nitro group,
(h) a mono- or di-$C_{1-6}$ alkylamino group,
(i) a mono- or di-$C_{3-8}$ cycloalkylamino group,
(j) a $C_{6-10}$ arylamino group,
(k) a 5-membered to 9-membered cyclicamino group which may have 1 to 3 hetero atoms selected from the group consisting of nitrogen, oxygen and sulfur atoms in addition to the nitrogen atom in the amino group and which may be substituted by $C_{1-6}$ alkyl group,
(l) a 5-membered or 6-membered aromatic heterocyclic group having from 1 to 3 hetero atoms selected from the group consisting of nitrogen, oxygen and sulfur atoms in addition to carbon atoms,
(m) a 5-membered to 9-membered non-aromatic heterocyclic ring having from 1 to 3 hetero atoms selected from the group consisting of nitrogen, oxygen and sulfur atoms in addition to carbon atoms,
(n) a $C_{1-4}$ alkylsulfonylamino group,
(o) a $C_{1-6}$ alkyl-carbonyloxy group and
(p) a halogen atom,

(3) an optionally halogenated $C_{1-6}$ alkoxy group,
(4) an optionally halogenated $C_{1-6}$ alkylthio group,
(5) a $C_{3-10}$ cycloalkyl group,
(6) a $C_{6-10}$ aryl group,
(7) a $C_{1-7}$ acylamino group,
(8) a $C_{1-3}$ acyloxy group,
(9) a hydroxy group,
(10) a nitro group,
(11) a cyano group,
(12) an amino group,
(13) a mono- or di-$C_{1-6}$ alkylamino group,
(14) a 5-membered to 9-membered cyclicamino group Which may have 1 to 3 hetero atoms selected from the group consisting of nitrogen, oxygen and sulfur atoms in addition to the nitrogen atom in the amino group and which may be substituted by $C_{1-6}$ alkyl group,
(15) a $C_{1-6}$ alkyl-carbonylamino group,
(16) a $C_{1-6}$ alkyl-sulfonylamino group,
(17) a $C_{1-6}$ alkox-ycarbonyl group,
(18) a carboxyl group,
(19) a $C_{1-6}$ alkylcarbonyl group,

(20) a carbamoyl group,
(21) a mono- or di-$C_{1-6}$ alkylcarbamoyl group and
(22) $C_{1-6}$ alkylsulfonyl group, or

$R^a$ and $R^b$ are bonded to each other to form Ring A, and the Ring A is

(i) a 5-membered to 9-membered aromatic heterocycle having 1 to 3 of hetero atoms selected from the group consisting of nitrogen, oxygen and sulfur atoms in addition to carbon atoms,
(ii) a 5-membered to 9-membered non-aromatic heterocycle having 1 to 3 hetero atoms selected from the group consisting of nitrogen, oxygen and sulfur atoms in addition to carbon atoms, or
(iii) a 3-membered to 10-membered cyclic hydrocarbon each of which may have 1 to 4 substituents selected from

(1) a halogen atom,
(2) a $C_{1-6}$ alkyl group optionally having from 1 to 5 substituents selected from the group consisting of

(a) a hydroxy group,
(b) an amino group,
(c) a carboxyl group,
(d) a nitro group,
(e) a mono- or di-$C_{1-6}$ alkylamino group,
(f) a $C_{1-6}$ alkyl-carbonyloxy group and
(g) a halogen atom,

(3) an optionally halogenated $C_{1-6}$ alkoxy group,
(4) an optionally halogenated $C_{1-6}$ alkylthio group,
(5) a $C_{6-10}$ aryl group,
(6) a $C_{1-7}$ acylamino group,
(7) a $C_{1-3}$ acyloxy group,
(8) a hydroxy group,
(9) a nitro group,
(10) a cyano group,
(11) an amino group,
(12) a mono- or di-$C_{1-6}$ alkylamino group,
(13) a 5-membered to 9-membered cyclicamino group which may have 1 to 3 hetero atoms selected from the group consisting of nitrogen, oxygen and sulfur atoms in addition to the nitrogen atom in the amino group,
(14) a $C_{1-6}$ alkyl-carbonylamino group,
(15) a $C_{1-6}$ alkyl-sulfonylamino group,
(16) a $C_{1-6}$ alkoxy-carbonyl group,
(17) a carboxyl group,
(18) a $C_{1-6}$ alkylcarbonyl group,
(19) a carbamoyl group,
(20) a mono- or di-$C_{1-6}$ alkylcarbamoyl group,
(21) a $C_{1-6}$ alkylsulfonyl group, or
(22) an oxo group;

the Ring B is a

(i) 5-membered to 9-membered aromatic heterocycle having 1 to 3 hetero atoms selected from the group consisting of nitrogen, oxygen and sulfur atoms in addition to carbon atoms,
(ii) a 5-membered to 9-membered non-aromatic heterocycle having 1 to 3 hetero atoms selected from the group consisting of nitrogen, oxygen and sulfur atoms in addition to carbon atoms, or
(iii) a 3-membered to 10-membered cyclic hydrocarbon each of which may have 1 to 4 substituents selected from the group consisting of

(1) a halogen atom,
(2) a $C_{1-6}$ alkyl group optionally having from 1 to 5 substituents selected from the group consisting of

(a) a hydroxy group,
(b) an amino group,
(c) a carboxyl group,
(d) a nitro group,
(e) a mono- or di-$C_{1-6}$ alkylamino group,
(f) a $C_{1-6}$ alkyl-carbonyloxy group and
(g) a halogen atom,

(3) an optionally halogenated $C_{1-6}$ alkoxy group,
(4) an optionally halogenated $C_{1-6}$ alkylthio group,
(5) a $C_{6-10}$ aryl group,
(6) a $C_{1-7}$ acylamino group,
(7) a $C_{1-3}$ acyloxy group,
(8) a hydroxy group,
(9) a nitro group,
(10) a cyano group,
(11) an amino group,
(12) a mono- or di-$C_{1-6}$ alkylamino group,
(13) a 5-membered to 9-membered cyclicamino group which may have 1 to 3 hetero atoms selected from the group consisting of nitrogen, oxygen and sulfur atoms in addition to the nitrogen atom in the amino group,
(14) a $C_{1-6}$ alkyl-carbonylamino group,
(15) a $C_{1-6}$ alkyl-sulfonylamino group,
(16) a $C_{1-6}$ alkoxy-carbonyl group,
(17) a carboxyl group,
(18) a $C_{1-6}$ alkylcarbonyl group,
(19) a carbamoyl group,
(20) a mono- or di-$C_{1-6}$ alkylcarbamoyl group,
(21) a $C_{1-6}$ alkylsulfonyl group, and
(22) an oxo group;

the Ring C is

(i) a 5-membered to 9-membered heterocycle which may have 1 to 3 hetero atoms selected from the group consisting of nitrogen, oxygen and sulfur atoms which optionally having 1 to 5 substituents selected from the group consisting of

(1) a halogen atom,
(2) an optionally halogenated $C_{1-10}$ alkyl group,
(3) an amino-substituted $C_{1-4}$ alkyl group,
(4) a mono- or di-$C_{1-4}$ alkylamino-substituted $C_{1-4}$ alkyl group,
(5) a carboxyl-substituted $C_{1-4}$ alkyl group,
(6) a $C_{1-4}$ alkoxy-carbonyl-substituted $C_{1-4}$ alkyl group,
(7) a hydroxy-substituted $C_{1-4}$ alkyl group,
(8) a $C_{3-10}$ cycloalkyl group,
(9) a nitro group,
(10) a cyano group,
(11) a hydroxy group,
(12) an optionally-halogenated $C_{1-10}$ alkoxy group,
(13) an optionally-halogenated $C_{1-4}$ alkylthio group,
(14) an amino group,
(15) a mono- or di-$C_{1-4}$ alkylamino group,
(16) a 5-membered to 9-membered cyclic amino group optionally having 1 to 3 hetero atoms selected from the group consisting of nitrogen, oxygen and sulfur atoms in addition to the nitrogen atom in the amino group,
(17) a $C_{1-4}$ alkyl-carbonylamino group,
(18) an aminocarbonyloxy group,
(19) a mono- or di-$C_{1-4}$ alkylaminocarbonyloxy group,

(20) a $C_{1-4}$ alkylsulfonylamino group,
(21) a $C_{1-4}$ alkoxy-carbonyl group,
(22) an aralkyloxycarbonyl group,
(23) a carboxyl group,
(24) a $C_{1-6}$ alkylcarbonyl group,
(25) a $C_{3-6}$ cycloalkyl-carbonyl group,
(26) a carbamoyl group,
(27) a mono- or di-$C_{1-4}$ alkylcarbamoyl group,
(28) a $C_{1-6}$ alkylsulfonyl group and
(29) a 5-membered or 6-membered aromatic monocyclic heterocyclic group having 1 to 4 hetero atoms selected from the group consisting of nitrogen, oxygen and sulfur atoms in addition to carbon atoms, which may be substituted by 1 to 3 optionally halogenated $C_{1-4}$ alkyls;, or

(ii) a 3-membered to 10-membered cyclic hydrocarbon, optionally having 1 to 5 substituents selected from the group consisting of

(1) a halogen atom,
(2) an optionally halogenated $C_{1-10}$ alkyl group,
(3) an amino-substituted $C_{1-4}$ alkyl group,
(4) a mono- or di-$C_{1-4}$ alkylamino-substituted $C_{1-4}$ alkyl group,
(5) a carboxyl-substituted $C_{1-4}$ alkyl group,
(6) a hydroxy-substituted $C_{1-4}$ alkyl group,
(7) a $C_{1-4}$ alkoxy-carbonyl-substituted $C_{1-4}$ alkyl group,
(8) a $C_{3-10}$ cycloalkyl group,
(9) a nitro group,
(10) a cyano group,
(11) a hydroxy group,
(12) an optionally-halogenated $C_{1-10}$ alkoxy group,
(13) an optionally-halogenated $C_{1-4}$ alkylthio group,
(14) an amino group,
(15) a mono- or di-$C_{1-4}$ alkylamino group,
(16) a 5-membered to 9-membered cyclic amino group optionally having 1 to 3 hetero atoms selected from the group consisting of nitrogen, oxygen and sulfur atoms in addition to the nitrogen atom in the amino group,
(17) a $C_{1-4}$ alkyl-carbonylamino group,
(18) an aminocarbonyloxy group,
(19) a mono- or di-$C_{1-4}$ alkylaminocarbonyloxy group,
(20) a $C_{1-4}$ alkylsulfonylamino group,
(21) a $C_{1-4}$ alkoxy-carbonyl group,
(22) an aralkyloxycarbonyl group,
(23) a carboxyl group,
(24) a $C_{1-6}$ alkylcarbonyl group,
(25) a $C_{3-6}$ cycloalkyl-carbonyl group,
(26) a carbamoyl group,
(27) a mono- or di-$C_{1-4}$ alkylcarbamoyl group,
(28) a $C_{1-6}$ alkylsulfonyl group and
(29) a 5-membered or 6-membered aromatic monocyclic heterocyclic group having 1 to 4 hetero atoms selected from the group consisting of nitrogen, oxygen and sulfur atoms in addition to carbon atoms, which may be substituted by 1 to 3 optionally halogenated $C_{1-4}$ alkyls;
the Ring Z is a 5-membered to 12-membered heterocycle optionally having at least one hetero atom selected from the group consisting of nitrogen, oxygen and sulfur atoms in addition to Y and the nitrogen atom, and optionally having 1 to 5 substituents selected from the group consisting of

(1) a $C_{1-6}$ alkyl group,
(2) a $C_{2-6}$ alkenyl group,
(3) a $C_{2-6}$ alkynyl group,
(4) a $C_{3-8}$ cycloalkyl group,
(5) a $C_{3-8}$ cycloalkyl-$C_{1-4}$ alkyl group,

(6) a $C_{6-14}$ aryl group,
(7) a nitro group,
(8) a cyano group,
(9) a hydroxy group,
(10) a $C_{1-4}$ alkoxy group,
(11) a $C_{1-4}$ alkylthio group,
(12) a amino group,
(13) a mono- or di-$C_{1-4}$ alkylamino group,
(14) a 5-membered to 9-membered cyclic amino group optionally having 1 to 3 hetero atoms selected from the group consisting of nitrogen, oxygen and sulfur atoms in addition to the nitrogen atom in the amino group,
(15) a $C_{1-4}$ alkyl-carbonylamino group,
(16) a $C_{1-4}$ alkylsulfonylamino group,
(17) a $C_{1-4}$ alkoxy-carbonyl group,
(18) a carboxyl group,
(19) a $C_{1-6}$ alkylcarbonyl group,
(20) a carbamoyl group,
(21) a mono- or di-$C_{1-4}$ alkylcarbamoyl group,
(22) a $C_{1-6}$ alkylsulfonyl group,
(23) an oxo group, and
(24) a thioxo group,

(III) The pharmaceutical composition as defined in (I), wherein $R^a$ and $R^b$ are the same or different and represent, independently, a hydrogen atom or a substituent selected from the group consisting of

(1) a halogen atom,
(2) a $C_{1-6}$ alkyl group optionally having from 1 to 5 substituents selected from the group consisting of

(a) a hydroxy group,
(b) a $C_{1-6}$ alkoxy group,
(c) a $C_{1-6}$ alkylthio group,
(d) an amino group,
(e) a $C_{1-7}$ acylamino group,
(f) a mono- or di-$C_{1-6}$ alkylamino group,
(g) a mono- or di-$C_{3-8}$ cycloalkylamino group,
(h) a 5-membered to 9-membered cyclicamino group which may have 1 to 3 hetero atoms selected from the group consisting of nitrogen, oxygen and sulfur atoms in addition to the nitrogen atom in the amino group and,
(i) a $C_{1-4}$ alkylsulfonylamino group,
(j) a $C_{1-6}$ alkyl-carbonyloxy group and
(k) a halogen atom,

(3) a 5-membered to 9-membered (preferably 6-membered) cyclicamino group which may have 1 to 3 hetero atoms (preferably 1 or 2) selected from the group consisting of nitrogen, oxygen and sulfur atoms, in addition to the nitrogen atom in the amino group and which may be substituted by $C_{1-6}$ alkyl group,
(4) a carboxyl group,
(5) a carbamoyl group,
(6) a mono- or di-$C_{1-6}$ alkylcarbamoyl group; or

$R^a$ and $R^b$ are bonded to each other to form Ring A, and the Ring A is a 5-membered to 9-membered aromatic heterocyclic ring having 1 to 3 hetero atoms selected from the group consisting of nitrogen, oxygen and sulfur atoms in addition to carbon atoms (preferably pyridine ring), which may be substituted by $C_{1-6}$ alkyl group;
the Ring B is a $C_{6-14}$ aryl group (preferably benzene ring) which may be substituted by substituents selected from the group consisting of (i) a $C_{1-6}$ alkyl group optionally substituted by a hydroxy group, (ii) a $C_{1-6}$ alkylcarbonyl group (including formyl) and (iii) a carboxyl group;
the Ring C is a $C_{6-14}$ aryl group (preferably benzene ring) which may be substituted by 1 to 3 substituents selected from the group consisting of (i) a halogen atom, (ii) an optionally halogenated $C_{1-10}$ alkyl group

and (iii) a $C_{1-10}$ alkoxy group;

the Ring Z is a 5-membered to 12-membered heterocyclic ring optionally having at least one hetero atom selected from the group consisting of nitrogen, oxygen and sulfur atoms in addition to Y and the nitrogen atom, which may be substituted by 1 to 3 substituents selected from the group consisting of (i) a $C_{1-6}$ alkyl group, (ii) a hydroxy group and (iii) an oxo group;

(IV) The pharmaceutical composition as defined in (I), which comprises a compound represented by the formula:

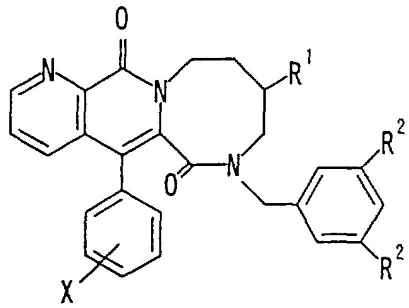

(I')

wherein $R^1$ is a $C_{1-6}$ alkyl group, $R^2$ is a $C_{1-6}$ alkoxy group, optionally halogenated $C_{1-6}$ alkyl group, a halogen atom, a hydroxy group or $C_{7-15}$ aralkyloxy group, X is 1 to 5 groups selected from the group consisting of a hydrogen atom, a $C_{1-6}$ alkyl group and a halogen atom, provided that (1) when $R^1$ is a methyl group and $R^2$ is s trifluoromethyl group, X is a halogen atom, and (2) when $R^1$ is a methyl group and $R^2$ is a methoxy group, X is a hydrogen atom or a halogen atom,

(V) The pharmaceutical composition as defined in (I), which comprises (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine or a salt thereof,

(VI) The pharmaceutical composition as defined in (I), which comprises (9R)-7-(3,5-dimethoxybenzyl)-6,7,8,9,10,11-hexahydro-9-methyl-5-(4-methylphenyl)-6,13-dioxo-13H-[1,4]diazocino[2,1-g][1,7]naphthyridine or a salt thereof,

(VII) The pharmaceutical composition as defined in (I), which comprises (9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine or a salt thereof,

(VIII) (9S)-7-[3,5-bis(trifluoromethyl)benzyl]-6,7,8,9,10,12-hexahydro-9-methyl-5-(4-methyl)phenyl-6,12-dioxo-[1,4]diazepino[2,1-g][1,7]naphthyridine or a salt thereof,

(IX) A compound represented by the formula:

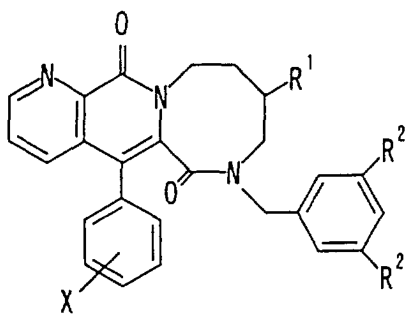

(I')

wherein $R^1$ is a $C_{1-6}$ alkyl group, $R^2$ is a $C_{1-6}$ alkoxy group, optionally halogenated $C_{1-6}$ alkyl group, a halogen atom, a hydroxy group or a $C_{7-15}$ aralkyloxy group, X is 1 to 5 groups selected from the group consisting of a hydrogen atom, a $C_{1-6}$ alkyl group and a halogen atom, provided that (1) when $R^1$ is a methyl group and $R^2$ is s trifluoromethyl group, X is a halogen atom and (2) when $R^1$ is a methyl group and $R^2$ is a methoxy group, X is a hydrogen atom or a halogen atom, or a salt thereof,

(X) A compound as defined in (IX), wherein $R^1$ is a $C_{1-3}$ alkyl group, $R^2$ is a $C_{1-3}$ alkoxy group, optionally halogenated $C_{1-3}$ alkyl group, a halogen atom, a hydroxy group or a benzyloxy group, X is a hydrogen atom, or 1 or 2 groups selected from the group consisting of a $C_{1-3}$ alkyl group and a halogen atom,

(XI) A compound as defined in (IX), wherein $R^1$ is a methyl group group, $R^2$ is a methoxy group, an ethoxy group,

an isopropoxy group, a methyl group, a trifluoromethyl group, a chlorine atom, a hydroxy group or a benzyloxy group, X is a hydrogen atom, a methyl group, or 1 or 2 chlorine or fluorine atoms,
(XII) A compound as defined in (IX) which is (9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine,
(XIII) A pro-drug of a compound as defined in (IX),
(XIV) A method for producing a compound as defined in (IX), characterized by cyclizing a compound of a formula:

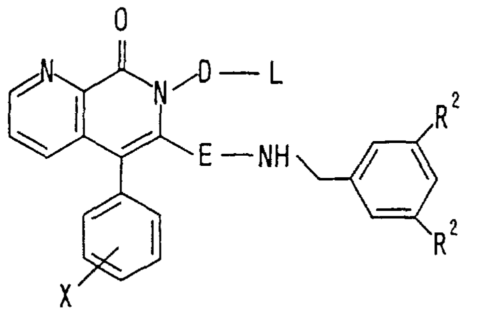

(II)

wherein D and E represent groups from which a group represented by the formula:

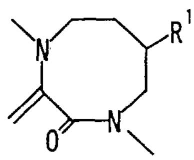

is formed together with the nitrogen atom adjacent to E, L represents a leaving group, and the other symbols are the same meanings as those in claim (IX), or a salt thereof,
(XV) A pharmaceutical composition which comprises a compound as defined in (IX),
(XVI) A composition for antagonizing a tachykinin receptor which comprises a compound as defined in (IX),
(XVII) A composition for antagonizing a Substance P receptor which comprises a compound as defined in (IX),
(XVIII) A composition for antagonizing a neurokinin A receptor which comprises a compound as defined in (IX),
(XIX) A pharmaceutical composition for preventing or treating disorders of micturition which comprises a compound as defined in (IX),
(XX) A pharmaceutical composition for preventing or treating disorders of asthma, rheumatoid arthritis, osteoarthritis, pain, cough, irritable bowel syndrome or emesis, which comprises a compound as defined in (IX),
(XXI) Use of a compound represented by the formula:

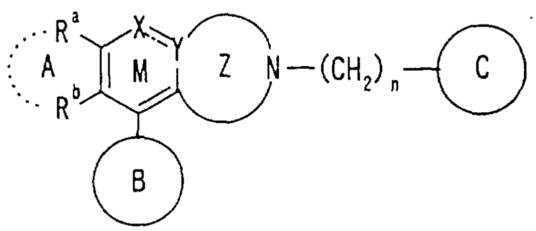

(I)

wherein ring M is a heterocyclic ring wherein

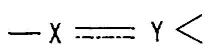

is one of -N=C<, -CO-N< or -CS-N<;

$R^a$ and $R^b$ are bonded to each other to form Ring A, or they are the same or different and represent, independently, a hydrogen atom or a substituent on the Ring M;

Ring A and Ring B represent, independently, an optionally substituted homocyclic or heterocyclic ring, with the proviso that at least one of them is an optionally substituted heterocyclic ring;
Ring C is an optionally substituted homocyclic or heterocyclic ring;
Ring Z is an optionally substituted nitrogen-containing heterocyclic ring; and
n is an integer from 1 to 6, or a salt thereof for manufacturing a composition for preventing or treating depression, anxiety, manic-depressive illness or psychopathy,

(XXII) A method for preventing or treating disorders of micturition in mammals which comprises administrating to a subject in need an effective amount of a compound represented by the formula:

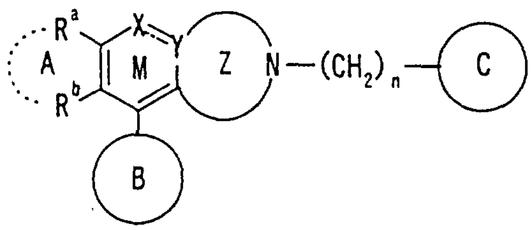

(I)

wherein ring M is a heterocyclic ring wherein

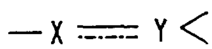

is one of -N=C<, -CO-N< or -CS-N<;

$R^a$ and $R^b$ are bonded to each other to form Ring A, or they are the same or different and represent, independently, a hydrogen atom or a substituent on the Ring M;
Ring A and Ring B represent, independently, an optionally substituted homocyclic or heterocyclic ring, with the proviso that at least one of them is an optionally substituted heterocyclic ring;
Ring C is an optionally substituted homocyclic or heterocyclic ring;
Ring Z is an optionally substituted nitrogen-containing heterocyclic ring; and
n is an integer from 1 to 6, or a salt thereof,

(XXIII) Use of a compound as defined in (IX) for manufacturing a composition for antagonizing a tachykinin receptor,
(XXIV) Use of a compound as defined in (IX) for manufacturing a pharmaceutical composition for antagonizing a Substance P receptor,
(XXV) Use of a compound as defined in (IX) for manufacturing a pharmaceutical composition for antagonizing a neurokinin A receptor,
(XXVI) Use of a compound as defined in (IX) for manufacturing a pharmaceutical composition for treating disorders of micturition,
(XXVII) Use of a compound as defined in (IX) for manufacturing a pharmaceutical composition for treating disorders of asthma, rheumatoid arthritis, osteoarthritis, pain, cough, irritable bowel syndrome or emesis, which comprises a compound as defined in (IX),
(XXVIII) A method for antagonizing a tachykinin receptor in mammals which comprises administrating to a subject in need, an effective amount of a compound as defined in (IX),
(XXIX) A method for antagonizing a Substance P receptor in mammals which comprises administrating to a subject in need an effective amount of a compound as defined in (IX),
(XXX) A method for antagonizing a neurokinin A receptor in mammals which comprises administrating to a subject in need an effective amount of a compound as defined in (IX),
(XXXI) A method for preventing or treating disorders of micturition in mammals which comprises administrating to a subject in need an effective amount of a compound as defined in (IX), and
(XXXII) A method for preventing or treating disorders of asthma, rheumatoid arthritis, osteoarthritis, pain, cough, irritable bowel syndrome or emesis in mammals which comprises administrating to a subject in need an effective amount of a compound as defined in (IX).

A compound as defined in the above-mentioned (I) includes a compound of a formula (Ia):

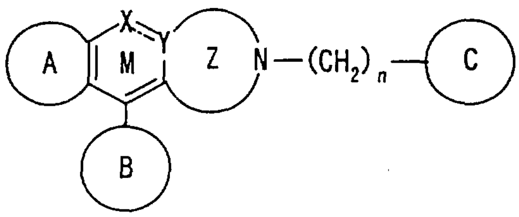

(Ia)

wherein ring A is formed by $R^a$ and $R^b$ are bonded to each other to form Ring A, and the other symbols have the same meanings as mentioned above, or a salt thereof.

The heterocyclic compounds (I) used for the composition of the present invention are compounds disclosed in EP-A-0733632.

In the above-mentioned formulae (I) and (Ia), Ring M is a heterocyclic ring having -N=C<, -CO-N< or -CS-N< as the partial structure:

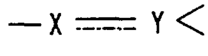

.

Preferably, Ring M has -CO-N< or -N=C- as the partial structure:

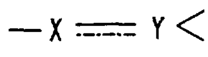

.

In the above-mentioned formulae (I) and (Ia), $R^a$ and $R^b$ are bonded to each other to form Ring A, or these are the same or different and represent, independently, a hydrogen atom or a substituent on the Ring M.

The substituents $R^a$ and $R^b$ on the Ring M include, for example, a halogen atom, an optionally substituted alkyl group, an optionally halogenated alkoxy group, an optionally halogenated alkylthio group, a cycloalkyl group, an aryl group, an acylamino group, an acyloxy group, a hydroxy group, a nitro group, a cyano group, an amino group, a mono- or di-alkylamino group, a cyclic amino group (e.g., a cyclic amino group optionally containing hetero atom(s) of oxygen atom, sulfur atom, etc., in addition to nitrogen atom), an alkylcarbonylamino group, an alkylsulfonylamino group, an alkoxycarbonyl group, a carboxyl group, an alkylcarbonyl group, a carbamoyl group, a mono- or di-alkylcarbamoyl group, an alkylsulfonyl group, an oxo group, etc.

The above-mentioned "halogen atom" includes, for example, fluorine, chlorine, bromine and iodine atoms. Preferably, the halogen atom includes, for example, fluorine, chlorine and bromine atoms.

The "optionally substituted alkyl group" includes, for example, $C_{1-6}$ alkyl groups (e.g., methyl, ethyl, propyl, isopropyl, butyl, isobutyl, sec-butyl and tert-butyl groups, etc.) optionally having from 1 to 5 substituents selected from a hydroxy group, a $C_{1-6}$ alkoxy group (e.g., methoxy, ethoxy, propoxy, butoxy, isobutoxy, sec-butoxy, tert-butoxy, etc.), a $C_{1-6}$ alkylthio group (e.g., methylthio, ethylthio, propylthio, butylthio, isobutylthio, sec-butyltio, tert-butyltio, etc.), an amino group, a $C_{1-7}$ acylamino group (e.g. formylamino, acethyl amino, propyonyl amino, butylyl amino, benzoyl amino, etc.), an N-alkylamino group, a carboxyl group, a nitro group, a mono- or di-$C_{1-6}$ alkylamino group (e.g., methylamino, ethylamino, dimethylamino and diethylamino groups, etc.), an optionally substituted N-substituted amino group substituted by one or two homocyclic groups (e.g., mono- or di- $C_{3-8}$ cycloalkylamino groups, for example, cyclopropylamino, cyclobutylamino, cyclohexylamino; $C_{6-10}$ arylamino groups, for example, phenylamino, etc.), an optionally substituted heterocyclic groups [e.g., 5-membered to 9-membered cycloamino groups which may have 1 to 3 hetero atoms selected from nitrogen, oxygen and sulfur atoms (e.g., 5-membered or 6-membered non-aromatic cycloamino groups, for example, piperidyno, 4-methylpiperodyno, morpholino, thiomorpholino, piperadinyl, 4-methylpiperadinyl, 4-ethylpiperadinyl, pyrrolidinyl, imidazolydinyl, pyrazolydinyl; 5-membered or 6-membered aromatic cycloamino groups, for example, pyridyl, pyradyl, pyrimidinyl, pyridazinyl, pyrrolyl, imidazolyl, pyrazulyl, etc.), aromatic heterocyclic rings (e.g., thiophenyl, furanyl, thiazolyl, isothiazolyl, oxazolyl, etc.), non-aromatic heterocyclic rings (e.g., tetrohydropyridyl, dihydropyridyl, tetrahydropyradyl, tetrahydropyrimidinyl, tetrahydropyridazinyl, dihydropyranyl, dihydropyrrolyl, dyhydroimidazolyl, dihydropyrazolyl, dihydrothiophenyl, dihydrofuranyl, dihydrooxazolyl, dihydroisooxazolyl, hexahydropyrimidinyl, hexahydropyridazinyl, tetrahydropyranyl, pyrazolydinyl, tetrahydrothiophenyl, tetrahydrofuranyl, tetrahydrothiazolyl, tetrahydroisothiazolyl, tetrahydrooxazolyl, tetrahydroisooxazolyl, etc.)], an alkylsulfonylamino groups (e.g. $C_{1-4}$ alkylsulfonylamino groups, for example, methylsulfonylamino, ethylsulfonylamino, etc.), a $C_{1-6}$ alkyl-carbonyloxy group (e.g., acetoxy and ethylcarbonyloxy groups, etc.) and a halogen atom (e.g., fluorine, chlorine and bromine atoms,

etc.), etc..

Preferably, the "optionally substituted alkyl group" includes $C_{1-6}$ alkyl groups optionally substituted by from 1 to 4 or so halogen atoms, especially optionally halogenated $C_{1-4}$ alkyl groups (e.g., $C_{1-4}$ alkyl groups and $C_{1-4}$ alkyl groups substituted by from 1 to 3 or so halogen atoms, etc., such as methyl, chloromethyl, fluoromethyl, difluoromethyl, trichloromethyl, trifluoromethyl, ethyl, 2-bromoethyl, 2,2,2-trichloroethyl, 2,2,2-trifluoroethyl, pentafluoroethyl, propyl, 3,3,3-trifluoropropyl, isopropyl, 1-(trifluoromethyl)ethyl, butyl, 4,4,4-trifluorobutyl, isobutyl, sec-butyl and tert-butyl groups, etc.), $C_{1-6}$ alkoxy-$C_{1-6}$ alkyl groups (e.g. $C_{1-4}$ alkoxy-$C_{1-4}$ alkyl groups, for example, methoxymethyl, ethoxymethyl, isopropoxymethyl, butoxymethyl, methoxyethyl, ethoxyethyl, etc.), $C_{1-6}$ alkyltho-$C_{1-6}$ alkyl groups (e.g. $C_{1-4}$ alkylthio-$C_{1-4}$ alkyl groups, for example, methylthiomethyl, ethylthiomethyl, butylthiomethyl, methylthioethyl, ethylthioethyl, etc.), amino-$C_{1-6}$ alkyl groups (preferably, amino-$C_{1-4}$ alkyl groups), for example, aminomethyl, 2-aminoethyl, 2-aminopropyl, 3-aminopropyl, 2-aminobutyl, 3-aminobutyl and 4-aminobutyl groups, $C_{1-7}$ acylamino-$C_{1-6}$ alkyl groups (e.g. $C_{1-7}$ acylamino-$C_{1-4}$ alkyl groups, for example, formylaminomethyl, acetylaminomethyl, propionylaminomethyl, butylylaminoethyl, benzoylaminomethyl, etc.), mono-$C_{1-4}$ alkylamino-$C_{1-6}$ alkyl groups (e.g. mono-$C_{1-4}$ alkylamino-$C_{1-4}$ alkyl groups, for example, methylaminomethyl, ethylaminomethyl, butylaminomethyl, dimethylaminomethyl, diethylaminomethyl, 2-(N-methylamino)ethyl, 2-(N-ethylamino)ethyl, 2-(N-methylamino)propyl, 3-(N-methylamino)propyl, 3-(N-methylamino)butyl, 4-(N-methylamino)butyl, 2-(N-dimethylamino)ethyl, 2-(N-dimethylamino)ethyl groups, $C_{3-10}$ cycloalkylamino-$C_{1-6}$ alkyl groups (e.g. $C_{3-10}$ cycloalkylamino-$C_{1-4}$ alkyl groups, for example, cyclopropylaminomethyl, cyclobutylaminomethyl, cyclohexylaminomethyl, cyclopropylaminomethyl, cyclobutylaminomethyl, cyclohexylaminomethyl, phenylaminomethyl, etc.), 5-membered or 6-membered non-aromatic cycloamino optionally having 1 to 3 hetero atoms selected from nitrogen, oxygen and sulfur atoms in addtion to carbon atoms-$C_{1-6}$ alkyl groups (e.g. 5-membered or 6-membered non-aromatic cycloamino-$C_{1-4}$ alkyl groups, for example, piperidinomethyl, 4-methylpiperidinomethyl, morpholinomethyl, thiomorpholinomethyl, piperadinylmethyl, 4-methylpiperadinylmethyl, piperidinoethyl, morpholinoethyl, piperadinylethyl; 5-membered or 6-membered aromatic cycloamino-$C_{1-4}$ alkyl groups, for example, pyridylmethyl, pyrimidinylmethyl, imidazolylmethyl, pyridylethyl, etc.), $C_{1-6}$ alkylsulfonylamino-$C_{1-6}$ alkyl groups (e.g. $C_{1-6}$ alkylsulfonylamino-$C_{1-4}$ alkyl groups, for example, methylsulfonylaminomethyl, ethylsulfonylaminomethyl, methylsulfonylaminobutyl, ethylsulfonylaminoethyl, etc.), $C_{1-6}$ alkyl-carbonyloxy-$C_{1-6}$ alkyl groups (e.g. $C_{1-4}$ alkyl-carbonyloxy-$C_{1-4}$ alkyl groups, for example, methylcarbonyloxymethyl, ethylcarbonyloxymethyl, butylcarbonyloxymethyl, methylcarbonyloxyethyl, ethylcarbonyloxyethyl, etc.), etc.

The "optionally halogenated alkoxy group" includes, for example, $C_{1-6}$ alkoxy groups or $C_{1-6}$ alkoxy groups substituted by from 1 to 5 or so halogen atoms, etc. Such alkoxy groups or halogenated alkoxy groups include, for example, methoxy, fluoromethoxy, difluoromethoxy, trifluoromethoxy, trichloromethoxy, ethoxy, 2,2,2-trifluoroethoxy, 2,2,2-trichloroethoxy, pentafluoroethoxy, propoxy, isopropoxy, butoxy, 4,4,4-trifluorobutoxy, isobutoxy, sec-butoxy, pentoxy and hexyloxy groups, etc. Preferably, the "optionally-halogenated alkoxy group" includes $C_{1-4}$ alkoxy groups or $C_{1-4}$ alkoxy group substituted by from 1 to 3 or so halogen atoms, for example, methoxy, difluoromethoxy, trifluoromethoxy, ethoxy, 2,2,2-trifluoroethoxy, propoxy, isopropoxy, butoxy, 4,4,4-trifluorobutoxy, isobutoxy and sec-butoxy groups, etc.

The "optionally halogenated alkylthio group" includes, for example, $C_{1-6}$ alkylthio groups, and $C_{1-6}$ alkylthio groups having from 1 to 5 or so halogen atoms, etc. Such alkylthio groups and halogenated alkylthio groups include, for example, methylthio, difluoromethylthio, trifluoromethylthio, ethylthio, propylthio, isopropylthio, butylthio, 4,4,4-trifluorobutylthio, pentylthio and hexylthio groups, etc. Preferably, the "optionally halogenated alkylthio group" includes $C_{1-4}$ alkylthlo groups, or $C_{1-4}$ alkylthio groups substituted by from 1 to 3 or so halogen atoms, for example, methylthio, difluoromethylthio, trifluoromethylthio, ethylthio, propylthio, isopropylthio, butylthio and 4,4,4-trifluorobutylthio groups, etc.

Furtheremore, the "cycloalkyl group" includes $C_{3-10}$ cycloalkyl groups (e.g., cyclopropyl, cyclobutyl, cyclopentyl, cyclohexyl and cyclooctyl groups, etc.); the "aryl group" includes $C_{6-10}$ aryl groups (e.g., phenyl group, etc.); the "acylamino group" includes, for example, $C_{1-7}$ acylamino groups (e.g., formylamino, acetylamino, propionylamino, butyrylamino and benzoylamino groups, etc.), etc. The "acyloxy group" includes, for example, $C_{1-3}$ acyloxy groups (e.g., formyloxy, acetoxy and propionyloxy groups, etc.), etc. The "mono- or di-alkylamino group" includes, for example, mono- or di-$C_{1-4}$ alkylamino groups (e.g., methylamino, ethylamino, propylamino, dimethylamino and diethylamino groups, etc.), etc. The "cyclic amino group" includes, for example, 5-membered to 9-membered cyclic amino groups optionally having from 1 to 3 hetero atoms, such as oxygen atom, sulfur atom, etc., in addition to nitrogen atom (e.g., pyrrolidino, piperidino, morpholino and thiomorpholino groups, etc.), etc. The "alkylcarbonylamino group" includes, for example, $C_{1-4}$ alkyl-carbonylamino groups (e.g., acetylamino, propionylamino and butyrylamino groups, etc.); the "alkylsulfonylamino group" includes, for example, $C_{1-4}$ alkylsulfonylamino groups (e.g., methylsulfonylamino and ethylsulfonylamino groups, etc.); the "alkoxycarbonyl group" includes, for example, $C_{1-4}$ alkoxy-carbonyl groups (e.g., methoxycarbonyl, ethoxycarbonyl, propoxycarbonyl and butoxycarbonyl groups, etc.); the "alkylcarbonyl group" includes, for example, $C_{1-6}$ alkylcarbonyl groups (e.g., formyl, methylcarbonyl, ethylcarbonyl and propylcarbonyl groups, etc.); the "mono- or di-alkylcarbamoyl group" includes for example, mono- or di-$C_{1-4}$ alkylcarbamoyl groups (e.g., methylcarbamoyl, ethylcarbamoyl, dimethylcarbamoyl and diethylcarbamoyl groups, etc.); the "alkylsulfonyl group" includes, for example, $C_{1-6}$ alkylsulfonyl groups (e.g., methylsulfonyl, ethylsulfonyl and propylsulfonyl groups, etc.), etc.

In the above-mentioned formulae (I) and (Ia), Ring A and Ring B represent, independently, an optionally substituted homocyclic or heterocyclic ring, and at least one of these is an optionally substituted heterocyclic ring.

The "homocyclic or heterocyclic ring" includes, for example, (i) an aromatic heterocyclic ring or non-aromatic heterocyclic ring having the same one or different hetero atoms selected from nitrogen, sulfur and oxygen atoms, preferably from 1 to 3 such hetero atoms, in addition to carbon atoms, or (ii) a cyclic hydrocarbon ring (homocyclic ring) consisting of carbon atoms, etc.

The "aromatic heterocyclic ring" includes, for example, 5-membered or 6-membered aromatic heterocyclic rings having 1 to 3 hetero atoms selected from nitrogen, oxygen and sulfur atoms, in addition to carbon atoms (e.g., pyridine, pyrazine, pyrimidine, pyridazine, pyrrole, imidazole, pyrazole, triazole, thiophene, furan, thiazole, isothiazole, oxazole and isoxazole rings, etc.), etc. Preferably, the aromatic heterocyclic ring includes, for example, pyridine, pyrazine and thiophene rings, etc., as well as pyrrole and thiazole rings, etc. Especially preferred are (i) 6-membered, nitrogen-containing heterocyclic rings having one or two nitrogen atoms in addition to carbon atoms (e.g., pyridine and pyrazine rings, etc.) or (ii) 5-membered aromatic heterocyclic rings having one sulfur atom in addition to carbon atoms (e.g., thiophene ring, etc.), etc.

The "non-aromatic heterocyclic ring" includes, for example, 5-membered to 9-membered, non-aromatic heterocyclic rings, preferably 5-membered or 6-membered, non-aromatic heterocyclic rings, having from 1 to 3 hetero atoms selected from nitrogen, oxygen and sulfur atoms in addition to carbon atoms, etc.

For example, Ring A includes tetrahydropyridine, dihydropyridine, tetrahydropyrazine, tetrahydropyrimidine, tetrahydropyridazine, dihydropyran, dihydropyrrole, dihydroimidazole, dihydropyrazole, dihydrothiophene, dihydrofuran, dihydrothiazole, dihydroisothiazole, dihydroxazole and dihydroisoxazole rings, etc.; and Ring B includes, in addition to the above mentioned rings, piperidine, piperazine, hexahydropyrimidine, hexahydropyridazine, tetrahydropyran, morpholine, pyrrolidine, imidazolidine, pyrazolidine, tetrahydrothiophene, tetrahydrofuran, tetrahydrothiazole, tetrahydroisothiazole, tetrahydroxazole and tetrahydroisoxazole rings, etc. Preferably, Ring A includes, for example, 6-membered, non-aromatic heterocyclic rings having one or two nitrogen atoms in addition to carbon atoms (e.g., tetrahydropyridine, tetrahydropyrimidine and tetrahydropyridazine rings, etc.), etc., and is especially preferably a tetrahydropyridine ring, etc. Preferably, Ring B includes, for example, 6-membered, non-aromatic heterocyclic rings having one or 2 nitrogen atoms in addition to carbon atoms (e.g., piperidine and piperazine rings, etc.), etc., and is especially preferably a piperazine ring, etc.

The "cyclic hydrocarbon ring (homocyclic ring)" includes, for example, 3-membered to 10-membered (for example, 5-membered to 9-membered) cyclic hydrocarbon rings, preferably 5-membered or 6-membered cyclic hydrocarbon rings, etc. For example, Ring A includes benzene, $C_{3-10}$ cycloalkenes (e.g., cyclobutene, cyclopentene, cyclohexene, cycloheptene, cyclooctene, etc.), etc. The cycloalkenes are preferably $C_{5-6}$ cycloalkenes (e.g., cyclopentene, cyclohexene, etc.), etc. Ring B includes, in addition to the above mentioned rings, $C_{3-10}$ cycloalkanes (e.g., cyclobutane, cyclopentane, cyclohexane, cycloheptane, cyclooctane, etc.), etc. The cycloalkanes are preferably $C_{5-6}$ cycloalkanes (e.g., cyclohexane, cyclopentane, etc.), etc. Preferably, Ring A includes, for example, 6-membered homocyclic rings such as benzene and cyclohexene rings, etc. Especially preferred are a benzene ring, etc. Ring B preferably includes, for example, 6-membered homocyclic rings such as benzene and cyclohexane rings, etc. Especially preferred is a benzene ring.

At least one of Ring A and Ring B is an optionally-substituted heterocyclic ring. Both of Ring A and Ring B may be optionally substituted heterocyclic rings. Preferably, one of Ring A and Ring B is 1) an optionally substituted aromatic ring and the other is 2) an optionally substituted aromatic heterocyclic ring (preferably, aromatic heterocyclic ring).

The above-mentioned 1) "aromatic ring" includes, for example, (i) the above-mentioned "aromatic heterocyclic rings", namely, optionally substituted, 5-membered or 6-membered, aromatic heterocyclic rings having the same one or different two hetero atoms selected from nitrogen, sulfur and oxygen atoms, preferably from 1 to 3 such hetero atoms, in addition to carbon atoms (e.g., pyridine, pyrazine, pyrimidine, pyridazine, pyrrole, imidazole, pyrazole, triazole, thiophene, furan, thiazole, isothiazole, oxazole and isoxazole rings, etc.), or (ii) optionally substituted benzene rings.

For the substituents for the above-mentioned 1) "optionally substituted aromatic ring", for example, referred to are the same substituents as those for Ring A and Ring B which are mentioned hereinunder. The "aromatic heterocyclic ring" of the above-mentioned 2) "optionally substituted aromatic heterocyclic ring" includes, for example, the same aromatic heterocyclic rings as those in the above-mentioned "5-membered or 6-membered, aromatic heterocyclic ring". For the substituents for the above-mentioned 2) "optionally substituted aromatic heterocyclic ring", for example, referred to are the same substituents as those for Ring A and Ring B which are mentioned hereinunder. The "5-membered or 6-membered, aromatic heterocyclic ring" preferably includes the same heterocyclic rings as those referred to hereinabove for the foregoing "aromatic heterocyclic ring".

More preferably, one of Ring A and Ring B is an optionally substituted aromatic heterocyclic ring (e.g., a 5-membered or 6-membered aromatic heterocyclic ring) and the other is an optionally substituted benzene ring.

.The substituents for the optionally substituted "homocyclic or heterocyclic ring", "aromatic heterocyclic ring", "non-aromatic heterocyclic ring", "cyclic hydrocarbon ring", "aromatic ring" and "benzene ring" to be represented by Ring A and Ring B include, for example, a halogen atom, an optionally substituted alkyl group, an optionally halogenated alkoxy group, an optionally halogenated alkylthio group, an aryl group, an acylamino group, an acyloxy group, a hydroxy group, a nitro group, a cyano group, an amino group, a mono- or di-alkylamino group, a cyclic amino group (e.g., a cyclic amino group optionally having hetero atom selected from oxygen atom, sulfur atom, etc., in addition to nitrogen atom), an alkylcarbonylamino group, an alkylsulfonylamino group, an alkoxycarbonyl group, a carboxyl group, an alkylcarbonyl group, a carbamoyl group, a mono- or di-alkylcarbamoyl group, an alkylsulfonyl group, an oxo group, etc.

The "halogen atom", which Ring A and Ring B may have, includes, for example, fluorine, chlorine, bromine and iodine atoms. Preferably, the halogen atom includes, for example, fluorine, chlorine and bromine atoms (especially, fluorine and chlorine atoms, etc.).

The "optionally substituted alkyl group", which Ring A and Ring B may have, includes, for example, $C_{1-6}$ alkyl groups (e.g., methyl, ethyl, propyl, isopropyl, butyl, isobutyl, sec-butyl and tert-butyl groups, etc.) optionally having from 1 to 5 substituents selected from a hydroxy group, an amino group, a carboxyl group, a nitro group, a mono- or di-$C_{1-6}$ alkylamino group (e.g., methylamino, ethylamino, dimethylamino and diethylamino groups, etc.), a $C_{1-6}$ alkyl-carbonyloxy group (e.g., acetoxy and ethylcarbonyloxy groups, etc.) and a halogen atom (e.g., fluorine, chlorine and bromine atoms, etc.), etc. Especially preferred are optionally-halogenated alkyl groups, for example, $C_{1-6}$ alkyl groups, and $C_{1-6}$ alkyl groups substituted by from 1 to 4 or so halogen atoms, etc. Such alkyl groups and halogenated alkyl groups include, for example, methyl, chloromethyl, fluoromethyl, difluoromethyl, trichloromethyl, trifluoromethyl, ethyl, 2-bromoethyl, 2,2,2-trichloroethyl, 2,2,2-trifluoroethyl, pentafluoroethyl, propyl, 3,3,3-trifluoropropyl, isopropyl, 1-(trifluoromethyl)ethyl, butyl, 4,4,4-trifluorobutyl, isobutyl, sec-butyl, tert-butyl, pentyl, isopentyl, neopentyl, 5,5,5-trifluoropentyl, 4-trifluoromethylbutyl, hexyl, 6,6,6-trifluorohexyl and 5-trifluoromethylpentyl groups, etc.

More preferably, the "optionally substituted alkyl group" includes optionally halogenated $C_{1-4}$ alkyl groups, for example, $C_{1-4}$ alkyl groups and $C_{1-4}$ alkyl groups substituted by from 1 to 3 or so halogen atoms, etc., such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, 2-trifluoromethylethyl, butyl, 4,4,4-trifluorobutyl, isobutyl, sec-butyl and tert-butyl groups, etc.

The "optionally halogenated alkoxy group", which Ring A and Ring B may have, includes, for example, $C_{1-6}$ alkoxy groups or $C_{1-6}$ alkoxy groups substituted by from 1 to 5 or so halogen atoms such as those mentioned hereinabove, etc. Such alkoxy groups or halogenated alkoxy groups include, for example, methoxy, difluoromethoxy, trifluoromethoxy, trichloromethoxy, ethoxy, 2,2,2-trifluoroethoxy, 2,2,2-trichloroethoxy, pentafluoroethoxy, propoxy, isopropoxy, butoxy, 4,4,4-trifluorobutoxy, isobutoxy, sec-butoxy, pentoxy and hexyloxy groups, etc. Preferably, the "optionally halogenated alkoxy group" includes $C_{1-4}$ alkoxy groups or $C_{1-4}$ alkoxy group substituted by from 1 to 3 or so halogen atoms, for example, methoxy, difluoromethoxy, trifluoromethoxy, ethoxy, 2,2,2-trifluoroethoxy, propoxy, isopropoxy, butoxy, 4,4,4-trifluorobutoxy, isobutoxy and sec-butoxy groups, etc.

The "optionally halogenated alkylthio group", which Ring A and Ring B may have, includes, for example, $C_{1-6}$ alkylthio groups, and $C_{1-6}$ alkylthio groups having from 1 to 5 or so halogen atoms such as those mentioned hereinabove, etc. Such alkylthio groups and halogenated alkylthio groups include, for example, methylthio, difluoromethylthio, trifluoromethylthio, ethylthio, propylthio, isopropylthio, butylthio, 4,4,4-trifluorobutylthio, pentylthio and hexylthio groups, etc. Preferably, the "optionally halogenated alkylthio group" includes $C_{1-4}$ alkylthio groups, or $C_{1-4}$ alkylthio groups substituted by from 1 to 3 or so halogen atoms, for example, methylthio, difluoromethylthio, trifluoromethylthio, ethylthio, propylthio, isopropylthio, butylthio and 4,4,4-trifluorobutylthio groups, etc.

The aryl group as the substituent includes $C_{6-10}$ aryl groups (e.g., phenyl group, etc.); the acylamino group includes, for example, $C_{1-7}$ acylamino groups (e.g., formylamino, acetylamino, propionylamino, butyrylamino and benzoylamino groups, etc.), etc. The acyloxy group includes, for example, $C_{1-3}$ acyloxy groups (e.g., formyloxy, acetoxy and propionyloxy groups, etc.), etc. The mono- or di-alkylamino group includes, for example, mono- or di-$C_{1-4}$ alkylamino groups (e.g., methylamino, ethylamino, propylamino, dimethylamino and diethylamino groups, etc.), etc. The cyclic amino group includes, for example, 5-membered to 9-membered cyclic amino groups optionally having from 1 to 3 hetero atoms, such as oxygen atom, sulfur atom, etc., in addition to nitrogen atom (e.g., pyrrolidino, piperidino and morpholino groups, etc.), etc. The alkylcarbonylamino group includes, for example, $C_{1-4}$ alkyl-carbonylamino groups (e.g., acetylamino, propionylamino and butyrylamino groups, etc.); the alkylsulfonylamino group includes, for example, $C_{1-4}$ alkylsulfonylamino groups (e.g., methylsulfonylamino and ethylsulfonylamino groups, etc.); the alkoxycarbonyl group includes, for example, $C_{1-4}$ alkoxy-carbonyl groups (e.g., methoxycarbonyl, ethoxycarbonyl, propoxycarbonyl and butoxycarbonyl groups, etc.); the alkylcarbonyl group includes, for example, $C_{1-6}$ alkylcarbonyl groups (e.g., formyl, methylcarbonyl, ethylcarbonyl and propylcarbonyl groups, etc.); the mono- or di-alkylcarbamoyl group includes, for example, mono- or di-$C_{1-4}$ alkylcarbamoyl groups (e.g., methylcarbamoyl, ethylcarbamoyl, dimethylcarbamoyl and

diethylcarbamoyl groups, etc.); the alkylsulfonyl group includes, for example, $C_{1-6}$ alkylsulfonyl groups (e.g., methylsulfonyl, ethylsulfonyl and propylsulfonyl groups, etc.), etc.

The terminology "optionally halogenated" as referred to herein means that the number of halogen atoms, if substituted, is from 1 to 5, preferably from 1 to 3 or so.

Preferred substituents for the optionally substituted Ring A and Ring B include a halogen atom, an optionally halogenated $C_{1-4}$ alkyl group, an optionally halogenated $C_{1-4}$ alkoxy group, an optionally halogenated $C_{1-4}$ alkylthio group, a $C_{1-3}$ acyloxy group, a hydroxy group, an amino group, a mono- or di-$C_{1-4}$ alkylamino group, a carboxyl group, a $C_{1-4}$ alkoxy-carbonyl group, an oxo group, etc.

More preferred substituents for the optionally substituted Ring A and Ring B include a halogen atom, an optionally halogenated $C_{1-4}$ alkyl group, an optionally halogenated $C_{1-4}$ alkoxy group, a hydroxy group, an amino group, a mono- or di-$C_{1-4}$ alkylamino group, a $C_{1-3}$ acyloxy group, an oxo group, etc. Especially preferred are a halogen atom, an optionally halogenated $C_{1-4}$ alkyl group, an optionally halogenated $C_{1-4}$ alkoxy group, etc.

The substituents for Ring A and Ring B, if any, may be at any substitutable position. If the rings are substituted by two or more substituents, the substituents may be the same or different. The number of the substituents may be from 1 to 4 or so, preferably from 1 to 3 or so.

If the Ring A and/or the Ring B has nitrogen atom(s), the ring may form a quaternary salt. For example, it may form a salt with halide ion(s) (e.g., $Cl^-$, $Br^-$, $I^-$, etc.) or other anion(s) such as sulfato ion, hydroxy ion, etc. Regarding "Ring A":

Preferred homocyclic rings for Ring A are optionally substituted homocyclic rings composed of carbon atoms, for example, including those of a formula (A-1):

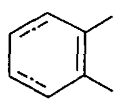 , 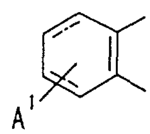 (A-1)

wherein ---- indicates a single bond or a double bound and the same shall apply hereinunder; and $A^1$ represents a halogen atom (e.g., fluorine and chlorine atoms, etc.), an optionally-halogenated $C_{1-4}$ alkyl group (e.g., methyl, isopropyl, trifluoromethyl, trichloromethyl, ethyl, 2,2,2-trifluoroethyl and pentafluoroethyl groups, etc.), or an optionally halogenated $C_{1-4}$ alkoxy group (e.g., methoxy, trifluoromethoxy, trichloromethoxy, ethoxy, 2,2,2-trifluoroethoxy and pentafluoroethoxy groups, etc.); or those of a formula (A-2):

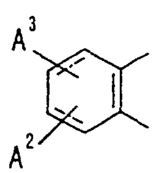

wherein $A^2$ and $A^3$ are the same or different and represent, independently, a halogen atom (e.g., fluorine and chlorine atoms, etc.), an optionally halogenated $C_{1-4}$ alkyl group (e.g., methyl, isopropyl, trifluoromethyl, trichloromethyl, ethyl, 2,2,2-trifluoroethyl and pentafluoroethyl groups, etc.), or an optionally halogenated $C_{1-4}$ alkoxy group (e.g., methoxy, trifluoromethoxy, trichloromethoxy, ethoxy, 2,2,2-trifluroroethoxy and pentafluoroethoxy groups, etc.).

More preferred homocyclic rings include, for example, benzene rings of a formula (A-3):

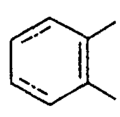 or 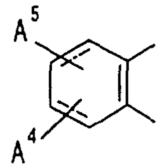 (A-3)

wherein $A^4$ and $A^5$ are the same or different and represent, independently, a halogen atom (e.g., fluorine and chlorine atoms, etc.), or an optionally-halogenated $C_{1-4}$ alkyl group (e.g., methyl, trifluoromethyl, trichloromethyl, ethyl, 2,2,2-trifluoroethyl, pentafluoroethyl and isopropyl groups, etc.).

Also preferred are optionally substituted benzene rings of a formula (A-4):

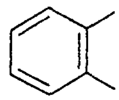, 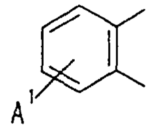 or 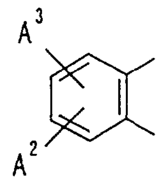

particularly,

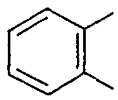 or 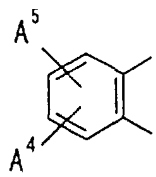 (A-4)

wherein the symbols have the same meanings as mentioned above.

Of the homocyclic rings of the above-mentioned formulae, especially preferred are those as substituted by the following substituent(s):

(1) Homocyclic rings where $A^1$ is a halogen atom (e.g., fluorine and chlorine atoms, etc.), or an optionally-substituted $C_{1-4}$ alkyl group (e.g., methyl, trifluoromethyl, ethyl and isopropyl groups, etc.).
(2) Homocyclic rings where $A^2$ and $A^3$ are the same or different and represent, independently, an optionally-halogenated $C_{1-4}$ alkyl group (e.g., methyl, trifluoromethyl, ethyl and isopropyl groups, etc.), or an optionally-halogenated $C_{1-4}$ alkoxy group (e.g., methoxy, trifluoromethoxy and ethoxy groups, etc.).
(3) Homocyclic rings where $A^4$ and $A^5$ are the same or different and represent, independently, a $C_{1-4}$ alkyl group (e.g., methyl, ethyl and isopropyl groups, etc.).
(4) Homocyclic rings where $A^1$ is a halogen atom (e.g., fluorine and chlorine atoms, etc.).
(5) Homocyclic rings where $A^2$ and $A^3$ are the same or different and represent, independently, a $C_{1-4}$ alkoxy group (e.g., methoxy and ethoxy groups, etc.).

Preferred aromatic heterocyclic or non-aromatic heterocyclic rings for Ring A are 5-membered or 6-membered, aromatic heterocyclic or non-aromatic heterocyclic rings including, for example, pyridine, pyrazine, thiophene, tetrahydropyridine, pyrrole and thiazole rings, etc. Concretely, for example, preferred are heterocyclic rings of a formula (A-5):

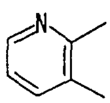, 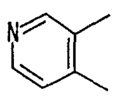, 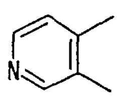, 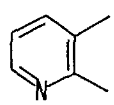, 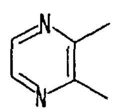, 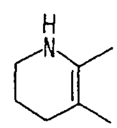,
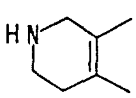, 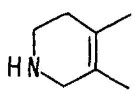, 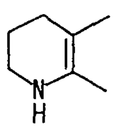, 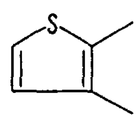, 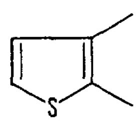, 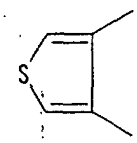
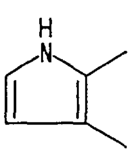, 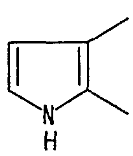, 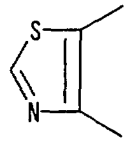, 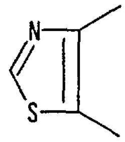 (A-5)

As preferred examples of optionally substituted aromatic or non-aromatic heterocyclic rings for Ring A, mentioned are pyridine, pyrazine, thiophene, tetrahydropyridine, pyrrole and thiazole rings, etc. optionally having one or two substituents selected from an oxo group, an optionally substituted alkyl group (this has the same meaning as the substituent for the optionally substituted Ring A and Ring B), a $C_{6-10}$ aryl group (e.g., phenyl group, etc.) and a halogen atom (e.g., fluorine, chlorine and bromine atoms, etc.). Concretely, for example, preferred are aromatic or non-aromatic heterocyclic rings of a formula (A-6):

(i) 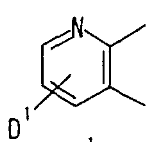, 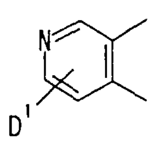, 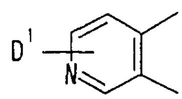, 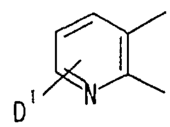, 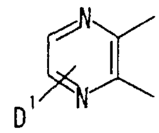;

(ii) 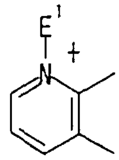, 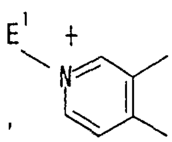, 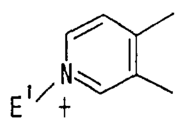, 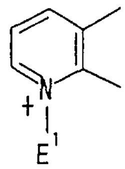;

(iii) 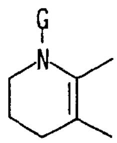, 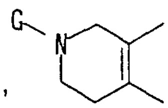, 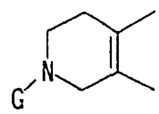, 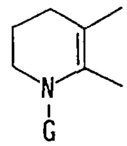;

(iv) 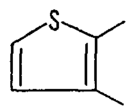, 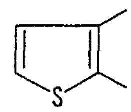, 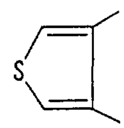;

(v) 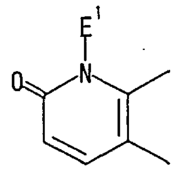;

(vi) 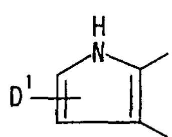, 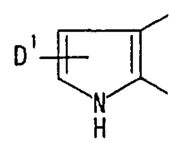;

(vii) 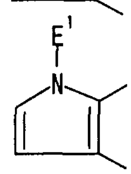, 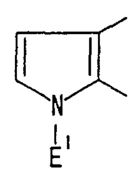;

or

(viii) 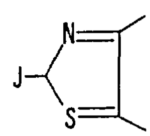, 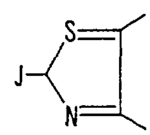 (A-6)

wherein $D^1$ represents a hydrogen atom, a halogen atom (e.g., fluorine, chlorine and bromine atoms, etc.); $E^1$ represents a $C_{1-4}$ alkyl group (e.g., methyl, ethyl, propyl and isopropyl groups, etc.); the compounds having the partial

structure of (ii) form quaternary ammonium salts along with a halide ion (e.g., $Cl^-$, $Br^-$, $I^-$, etc.), a sulfato ion, a hydroxy ion or the like; G represents a hydrogen atom or a $C_{1-4}$ alkyl group (e.g., methyl, ethyl, propyl and isopropyl groups, etc.); J represents a hydrogen atom, a $C_{1-4}$ alkyl group (e.g., methyl, ethyl, propyl and isopropyl groups, etc.) or a $C_{6-10}$ aryl group (e.g., phenyl group, etc.).

Ring A is preferably a 5-membered or a 6-membered, nitrogen-containing heterocyclic ring, for example, (i) a 6-membered, aromatic, nitrogen-containing heterocyclic ring having one or two nitrogen atoms in addition to carbon atoms (e.g., pyridine and pyrazine rings, etc.), (ii) a 6-membered, non-aromatic heterocyclic ring having one or two nitrogen atoms in addition to carbon atoms (e.g., tetrahydropyridine, tetrahydropyrimidine and tetrahydropyridazine rings, etc.), or the like. Especially preferably, Ring A is an aromatic, nitrogen-containing heterocyclic ring, particularly, a pyridine ring or the like.

More preferably, ring A is a pyridine ring which may be substituted by 1 to 3 substituents selected from a halogen atom or $C_{1-4}$ alkyl group.

Preferred homocyclic rings for Ring B are optionally substituted homocyclic rings consisting of carbon atoms, for example, including those of a formula (B-1):

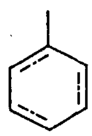 , 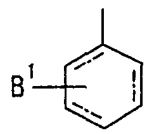 (B-1)

wherein $B^1$ represents a halogen atom, a $C_{1-4}$ alkyl group optionally substituted by hydroxy or optionally halogenated , an optionally halogenated $C_{1-4}$ alkoxy group, a $C_{1-6}$ alkylcarbonyl group or a carboxyl group; those of a formula (B-2):

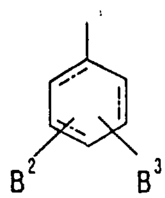 (B-2)

wherein $B^2$ and $B^3$ are the same or different and represent, independently, a halogen atom, an optionally halogenated $C_{1-4}$ alkyl group or an optionally halogenated $C_{1-4}$ alkoxy group; and those of a formula (B-3):

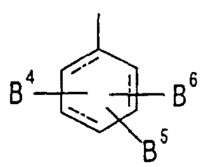 (B-3)

wherein $B^4$, $B^5$ and $B^6$ are the same or different and represent, independently, a halogen atom, an optionally halogenated $C_{1-4}$ alkyl group or an optionally halogenated $C_{1-4}$ alkoxy group.

More preferred are homocyclic rings of a formula (B-4):

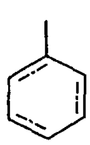 , 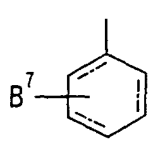 or 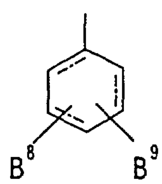 (B-4)

wherein $B^7$, $B^8$ and $B^9$ are the same or different and represent, independently, a halogen atom, an optionally halogenated $C_{1-4}$ alkyl group or an optionally halogenated $C_{1-4}$ alkoxy group.

Even more preferred are homocyclic rings of a formula (B-5):

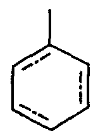 or 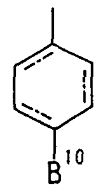 (B-5)

wherein $B^{10}$ represents, a halogen atom, a $C_{1-4}$ alkyl group optionally substituted by hydroxy or optionally halogenated , an optionally halogenated $C_{1-4}$ alkoxy group, a $C_{1-6}$ alkylcarbonyl group or a carboxyl group.

In the above-mentioned formulae, the halogen atom for any of $B^1$ to $B^{10}$ includes, for example, fluorine, chlorine and bromine atoms, etc.; the optionally-halogenated $C_{1-4}$ alkyl group includes, for example, methyl, trifluoromethyl, trichloromethyl, ethyl, 2,2,2-trifluoroethyl, 2,2,2-trichloroethyl, 1,1,2,2-tetrafluoroethyl, pentafluoroethyl, propyl, 2,2,3,3-tetrafluoropropyl and isopropyl groups, etc.; and the optionally-halogenated $C_{1-4}$ alkoxy group includes, for example, methoxy, trifluoromethoxy, trichloromethoxy, ethoxy, 2,2,2-trifluoroethoxy, 2,2,2-trichloroethoxy, 1,1,2,2-tetrafluoroethoxy, pentafluoroethoxy, propoxy, 2,2,3,3-tetrafluoropropoxy and isopropoxy groups, etc..

In the above mentioned formulae, the $C_{1-6}$ alkylcarbonyl group includes, for example, formyl, acetyl.

Ring B is also preferably an optionally substituted benzene ring, which includes, for example, benzene rings of a formula (B-6):

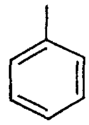 , 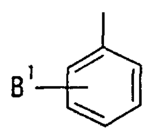 , 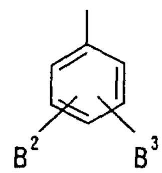 or 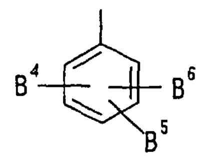 (B-6)

More preferred are benzene rings of a formula (B-7):

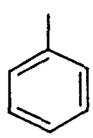 , 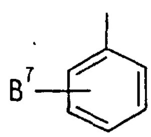 or 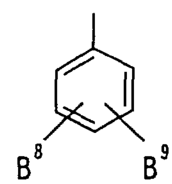 (B-7)

Especially preferred are benzene rings of a formula (B-8):

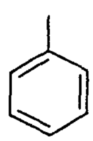 or 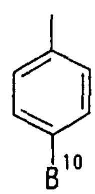 (B-8)

In these formulae, the symbols have the same meanings as mentioned above.

Of the substituents in the above-mentioned formulae, for example, especially preferred are the following:

(1) $B^1$, $B^2$, $B^3$, $B^4$, $B^5$ and $B^6$ are the same or different and represent, independently, a halogen atom (e.g., fluorine and chlorine atoms, etc.) or an optionally halogenated $C_{1-4}$ alkyl group (e.g., methyl, trifluoromethyl, ethyl and isopropyl groups, etc.).

(2) $B^1$, $B^2$, $B^3$, $B^4$, $B^5$ and $B^6$ are the same or different and represent, independently, an optionally-halogenated $C_{1-4}$ alkoxy group (e.g., methoxy, trifluoromethoxy and ethoxy groups, etc.).

(3) $B^7$, $B^8$ and $B^9$ represent halogen atoms (e.g., fluorine and chlorine atoms, etc.).

(4) $B^{10}$ represents a fluorine atom.

(5) $B^{10}$ represents a $C_{1-4}$ alkyl group (e.g., methyl group, etc.).

(6) B1 or B10 represents a $C_{1-6}$ alkyl group which may be substituted by hydroxy (e,g, hydroxymethyl, etc.), a $C_{1-6}$

alkylcarbonyl group (e.g., formyl, acetyl, etc.), a carboxyl group.

More preferred optionally substituted benzene rings are phenyl groups of a formula (B-9):

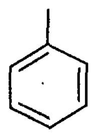 , 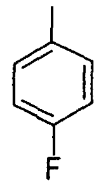 or 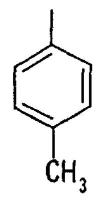 (B-9)

As preferred examples of aromatic heterocyclic rings or non-aromatic heterocyclic rings for Ring B, mentioned are 5-membered or 6-membered aromatic heterocyclic rings or non-aromatic heterocyclic rings such as pyridine, thiophene and piperidine rings, etc. These rings may optionally be substituted by substituents such as those mentioned hereinabove as preferred substituents for Ring A.

Where Ring B is an aromatic heterocyclic ring or a non-aromatic heterocyclic ring, it especially preferably includes, for example, heterocyclic rings of a formula (B-10):

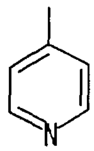 , 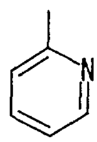 or 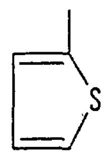 (B-10)

Where one or both of Ring A and Ring B is/are heterocyclic ring(s), the ring(s) is/are also preferably unsubstituted one(s).

Preferred combinations of Ring A and Ring B (1) are as follows:

(1) One of Ring A and Ring B is a 5-membered or 6-membered heterocyclic ring having one or two hetero atoms selected from nitrogen and sulfur atoms in addition to carbon atoms (e.g., pyridine, pyrazine, thiophene, tetrahydropyridine, piperidine and piperazine rings, etc.) which may be optionally substituted by $C_{1-4}$ alkyl group(s) (e.g., methyl, ethyl and isopropyl groups, etc.).

The other of Ring A and Ring B is a benzene ring optionally substituted by from 1 to 3 substituents selected from a halogen atom (e.g., fluorine, chlorine and bromine atoms, etc.), an optionally halogenated $C_{1-4}$ alkyl group (e.g., methyl, trifluoromethyl, trichloromethyl, ethyl, 2,2,2-trifluoroethyl, pentafluoroethyl, 2,2,2-trichloroethyl, propyl and isopropyl groups, etc.) and an optionally halogenated $C_{1-4}$ alkoxy group (e.g., methoxy, trifluoromethoxy, trichloromethoxy, ethoxy, 2,2,2-trifluoroethoxy, pentafluoroethoxy, 2,2,2-trichloroethoxy, propoxy and isopropoxy groups, etc.).

More preferred combinations of Ring A and Ring B (2) are as follows:

(2) One of Ring A and Ring B is a 5-membered or 6-membered aromatic heterocyclic ring having one or two hetero atoms selected from nitrogen and sulfur atoms in addition to carbon atoms (e.g., pyridine, pyrazine and thiophene rings, etc.).

The other of Ring A and Ring B is a benzene ring optionally substituted by from 1 to 3 substituents selected from a halogen atom (e.g., fluorine, chlorine and bromine atoms, etc.), an optionally halogenated $C_{1-4}$ alkyl group (e. g., methyl, trifluoromethyl, trichloromethyl, ethyl, 2,2,2-trifluoroethyl, pentafluoroethyl, 2,2,2-trichloroethyl, propyl and isopropyl groups, etc.) and an optionally halogenated $C_{1-4}$ alkoxy group (e.g., methoxy, trifluoromethoxy, trichloromethoxy, ethoxy, 2,2,2-trifluoroethoxy,pentafluoroethoxy,2,2,2-trichloroethoxy, propoxy and isopropoxy groups, etc.).

Especially preferably, Ring A is an optionally substituted aromatic heterocyclic ring such as mentioned above (e.g., an optionally substituted, 5-membered or 6-membered aromatic heterocyclic ring, especially pyridine ring, etc.), while Ring B is an optionally substituted benzene ring.

In the above-mentioned formulae (I) and (Ia), Ring C represents an optionally substituted homocyclic ring or an optionally substituted heterocyclic ring. The homocyclic ring or the heterocyclic ring may have from 1 to 5 or so, preferably from 1 to 3 or so substituents, which may be the same or different. The substituents may be positioned at any position of the homocyclic or heterocyclic-ring.

The homocyclic ring includes "cyclic hydrocarbon (homocyclic) rings" such as those as referred to hereinabove

for "Ring A and Ring B", for example, from 3-membered to 10-membered cyclic hydrocarbon rings such as benzene, $C_{3-10}$ cycloalkenes (e.g., cyclobutene, cyclopentene, cyclohexene, cycloheptene, cyclooctene, etc.), $C_{3-10}$ cycloalkanes (e.g., cyclobutane, cyclopentane, cyclohexane, cycloheptane, cyclooctane, etc.), etc., preferably 5-membered or 6-membered cyclic hydrocarbon rings. Of these, preferred are 6-membered homocyclic rings, such as benzene, cyclohexene and cyclohexane rings, etc. Especially preferred is benzene ring.

The substituents for the homocyclic rings such as the above-mentioned benzene ring include, for example, a halogen atom (e.g., fluorine, chlorine, bromine and iodine atoms), an optionally-halogenated $C_{1-10}$ alkyl group (e.g., methyl, chloromethyl, difluoromethyl trichloromethyl, trifluoromethyl, ethyl, 2-bromoethyl, 2,2,2-trifluoroethyl, perfluoroethyl, propyl, isopropyl, 3,3,3-trifluoropropyl, butyl, isobutyl, t-butyl, perfluorobutyl, pentyl, hexyl, octyl and decyl groups, etc.), an amino-substituted $C_{1-4}$ alkyl group (e.g., aminomethyl and 2-aminoethyl groups, etc.), a mono- or di-$C_{1-4}$ alkylamino-substituted $C_{1-4}$ alkyl group (e.g., methylaminomethyl, dimethylaminomethyl, 2-aminoethyl and 2-dimethylaminoethyl groups, etc.), a carboxyl-substituted $C_{1-4}$ alkyl group (e.g., carboxymethyl and carboxyethyl groups, etc.), a $C_{1-4}$ alkoxy-carbonyl-substituted $C_{1-4}$ alkyl group (e.g., methoxycarbonylethyl and ethoxycarbonylethyl groups, etc.), a hydroxy-substituted $C_{1-4}$ alkyl group (e.g., hydroxymethyl and hydroxyethyl groups, etc.), a $C_{1-4}$ alkoxy-carbonyl-substituted $C_{1-4}$ alkyl group (e.g., methoxymethyl, ethoxyethyl and ethoxyethyl groups, etc.), a $C_{3-10}$ cycloalkyl group (e.g., cyclopropyl, cyclobutyl, cyclopentyl, cyclohexyl, cycloheptyl and cyclooctyl groups, etc.), a nitro group, a cyano group, a hydroxy group, an optionally-halogenated $C_{1-10}$ alkoxy group (e.g., methoxy, difluoromethoxy, trichloromethoxy, trifluoromethoxy, ethoxy, 2,2,2-trifluoroethoxy, perfluoroethoxy, propoxy, isopropoxy, butoxy, isobutoxy, t-butoxy, perfluorobutoxy, pentyloxy, hexyloxy, octyloxy and decyloxy groups, etc.), an optionally-halogenated $C_{1-4}$ alkylthio group (e.g., methylthio, difluoromethylthio, trifluoromethylthio, ethylthio, propylthio, isopropylthio and butylthio groups, etc.), an amino group, a mono- or di-$C_{1-4}$ alkylamino group (e.g., methylamino, ethylamino, propylamino, dimethylamino and diethylamino groups, etc.), a cyclic amino group (e.g., a 5-membered to 9-membered cyclic amino group optionally having from 1 to 3 hetero atoms such as oxygen and sulfur atoms, etc., in addition to nitrogen atoms, concretely for example, pyrrolidino, piperidino and morpholino groups, etc.), a $C_{1-4}$ alkyl-carbonylamino group (e.g., acetylamino, propionylamino and butyrylamino groups, etc.), an aminocarbonyloxy group, a mono- or di-$C_{1-4}$ alkylaminocarbonyloxy group (e,g., methylaminocarbonyloxy, ethylaminocarbonyloxy, dimethylaminocarbonyloxy and diethylaminocarbonyloxy groups, etc.), a $C_{1-4}$ alkylsulfonylamino group (e.g., methylsulfonylamino, ethylsulfonylamino and propylsulfonylamino groups, etc.), a $C_{1-4}$ alkoxy-carbonyl group (e.g., methoxycarbonyl, ethoxycarbonyl, propoxycarbonyl, butoxycarbonyl and isobutoxycarbonyl groups, etc.), an aralkyloxycarbonyl group (e.g., benzyloxycarbonyl group, etc.), a carboxyl group, a $C_{1-6}$ alkyl-carbonyl group (e.g., methylcarbonyl, ethylcarbonyl and butylcarbonyl groups, etc.), a $C_{3-6}$ cycloalkyl-carbonyl group (e.g., cyclohexylcarbonyl group, etc.), a carbamoyl group, a mono- or di-$C_{1-4}$ alkylcarbamoyl group (e.g., methylcarbamoyl, ethylcarbamoyl, propylcarbamoyl, butylcarbamoyl, diethylcarbamoyl and dibutylcarbamoyl groups, etc.), a $C_{1-6}$ alkylsulfonyl group (e.g., methylsulfonyl, ethylsulfonyl and propylsulfonyl groups, etc.), etc.

The homocyclic Ring C may optionally be substituted, for example, by one 5-membered or 6-membered, aromatic monocyclic heterocyclic group (e.g., furyl, thienyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl groups, etc.), etc., and the aromatic monocyclic heterocyclic group may optionally be substituted by from 1 to 3 or so optionally halogenated $C_{1-4}$ alkyl groups (e.g., methyl, chloromethyl, difluoromethyl, trichloromethyl, trifluoromethyl, ethyl and isopropyl groups, etc.), etc.

As preferred substituents for the homocyclic Ring C (e.g., benzene ring, etc.), for example, mentioned are a halogen atom (e.g., fluorine, chlorine and bromine atoms, etc.), an optionally halogenated $C_{1-6}$ alkyl group (e.g., methyl, chloromethyl, difluoromethyl, trichloromethyl, trifluoromethyl, ethyl, 2-bromoethyl, 2,2,2-trifluoroethyl, perfluoroethyl, propyl, isopropyl, 3,3,3-trifluoropropyl, butyl, s-butyl, t-butyl and perfluorobutyl groups , etc.), a nitro group, a hydroxy group, an optionally halogenated $C_{1-6}$ alkoxy group (e.g., methoxy, difluoromethoxy, trifluoromethoxy, ethoxy, 2,2,2-trifluoroethoxy, perfluoroethoxy, propoxy, isopropoxy, 3,3,3-trifluoropropoxy and butoxy groups, etc.), an amino group, a mono- or di-$C_{1-4}$ alkylamino-substituted $C_{1-4}$ alkyl group (e.g., methylaminomethyl, dimethylaminomethyl, 2-methylaminoethyl and 2-dimethylaminoethyl groups, etc.), a mono- or di-$C_{1-4}$ alklamino group (e.g., methylamino, ethylamino, dimethylamino and diethylamino groups, etc.), a $C_{1-4}$ alkoxy-carbonyl group (e.g., methoxycarbonyl and ethoxycarbonyl groups, etc.), a carboxyl group, a carbamoyl group, etc.

More preferred are a halogen atom (e.g., fluorine, chlorine and bromine atoms, etc.), an optionally halogenated $C_{1-4}$ alkyl group (e.g., methyl, chloromethyl, difluoromethyl, trichloromethyl, trifluoromethyl, ethyl, 2-bromoethyl, 2,2,2-trichloroethyl, 2,2,2-trifluoroethyl, perfluoroethyl, propyl, isopropyl and t-butyl groups, etc.), an optionally halogenated $C_{1-4}$ alkoxy group (e.g., methoxy, trifluoromethoxy, ethoxy, 2,2,2-trichloroethoxy, 2,2,2-trifluoroethoxy, perfluoroethoxy and propoxy groups , etc.), a di-$C_{1-4}$ alkylamino group (e.g., dimethylamino and diethylamino groups, etc.), a $C_{1-3}$ acyloxy group (e.g., acetoxy group, etc.), a hydroxy group, etc. Preferably, the number of the substituents is, for example, from 1 to 3 or so.

Especially, preferred are a halogen atom (e.g., fluorine, chlorine and bromine atoms, etc.), an optionally halogenated $C_{1-4}$ alkyl group (e.g., methyl, chloromethyl, difluoromethyl, trichloromethyl, trifluoromethyl, ethyl, 2-bromoethyl, 2,2,2-trichloroethyl, 2,2,2-trifluoroethyl, perfluoroethyl, propyl, isopropyl and t-butyl groups, etc.), an optionally halogenated $C_{1-4}$ alkoxy group (e.g., methoxy, trifluoromethoxy, ethoxy, 2,2,2-trichloroethoxy, 2,2,2-trifluoroethoxy, perfluoroethoxy and propoxy groups, etc.)

The "heterocyclic ring" of the "optionally substituted heterocyclic ring" includes, for example, from 5-membered to 10-membered heterocyclic rings having 1 to 4 hetero atoms of the same type or different two types, such as nitrogen, oxygen, sulfur atoms, etc., in addition to carbon atoms, etc. Concretely, the heterocyclic ring includes, for example;

(1) 5-membered or 6-membered, aromatic monocyclic heterocyclic rings, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.;
(2) 9-membered or 10-membered, aromatic, condensed heterocyclic rings, such as benzofuranyl, isobenzofuranyl, benzo[b]thienyl, indoliyl, isoindolyl, 1H-indazolyl, benzimidazolyl, benzoxazolyl, 1,2-benzoisoxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1.5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.;
(3) 5-membered to 10-membered, non-aromatic heterocyclic rings, such as oxiranyl, azetidinyl, oxetanyl, thietanyl, pyrrolidinyl, tetrahydrofuryl, piperidyl, tetrahydrofuranyl, morpholinyl, thiomorpholinyl, pyrazinyl, etc.

Of the above-mentioned heterocyclic rings (1) to (3), for example, 5-membered or 6-membered heterocyclic rings having from 1 to 3 hetero atoms, such as nitrogen, oxygen and sulfur atoms, etc., in addition to carbon atoms, are widely utilized. Such heterocyclic rings include, for example, furyl, thienyl, pyrrolyl, oxazolyl, isoxazolyl, imidazolyl, pyrazolyl, pyridyl, pyridazinyl, quinolyl, isoquinolyl, thiazolyl, thiadiazolyl, thiophenyl, etc.

As the substituents for the optionally substituted heterocyclic rings, mentioned are substituents such as those as referred to hereinabove for the foregoing "optionally substituted homocyclic rings".

More preferably, Ring C includes optionally substituted benzene rings (especially, substituted benzene rings), for example, benzene rings optionally substituted by 1 to 3 substituents selected from a halogen atom, an optionally halogenated $C_{1-4}$ alkyl group, an optionally substituted $C_{1-4}$ alkoxy group, a di-$C_{1-4}$ alkylamino group, a $C_{1-3}$ acyloxy group and a hydroxy group (especially, benzene rings substituted by such substituent(s)). Concretely, the preferred Ring C includes, for example, optionally substituted benzene rings of a formula (C-1):

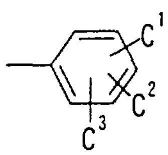

(C-1)

wherein $C^1$, $C^2$ and $C^3$ are the same or different and represent, independently, a hydrogen atom, a halogen atom, an optionally halogenated $C_{1-4}$ alkyl group, an optionally halogenated $C_{1-4}$ alkoxy group, a mono- or di-$C_{1-4}$ alkylamino group, a $C_{1-3}$ acyloxy group or a hydroxy group; and optionally substituted benzene rings of a formula (C-2):

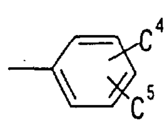

(C-2)

wherein $C^4$ and $C^5$ are the same or different and represent, independently, a hydrogen atom, a halogen atom, an optionally halogenated $C_{1-4}$ alkyl group or an optionally halogenated $C_{1-4}$ alkoxy group.

The halogen atom, the optionally halogenated $C_{1-4}$ alkyl group, the optionally halogenated $C_{1-4}$ alkoxy group and the mono- or di-$C_{1-4}$ alkylamino group to be represented by any of $C^1$, $C^2$, $C^3$, $C^4$ and $C^5$ may be the same as the above-mentioned halogen atom, optionally halogenated $C_{1-4}$ alkyl group, optionally halogenated $C_{1-4}$ alkoxy group and mono- or di-$C_{1-4}$ alkylamino group, respectively.

Even more preferably, Ring C includes, for example, benzene rings of the above-mentioned formulae (C-1) and (C-2) where $C^1$ to $C^5$ are as follows:

(1) $C^1$, $C^2$ and $C^3$ are the same or different and represent, independently, a halogen atom, an optionally halogenated $C_{1-4}$ alkyl group or an optionally halogenated $C_{1-4}$ alkoxy group;
(2) $C^1$, $C^2$ and $C^3$ are the same or different and represent, independently, a halogen atom or an optionally halogenated $C_{1-4}$ alkyl group;
(3) $C^1$, $C^2$ and $C^3$ are the same or different and represent, independently, a halogen atom;
(4) $C^1$, $C^2$ and $C^3$ are the same or different and represent, independently, an optionally halogenated $C_{1-4}$ alkyl group;
(5) $C^1$, $C^2$ and $C^3$ are the same or different and represent, independently, an optionally halogenated $C_{1-4}$ alkoxy group;
(6) $C^4$ and $C^5$ are the same or different and represent, independently, a halogen atom;
(7) $C^4$ and $C^5$ are the same or different and represent, independently, an optionally halogenated $C_{1-4}$ alkyl group; or
(8) $C^4$ and $C^5$ are the same or different and represent, independently, an optionally halogenated $C_{1-4}$ alkoxy group.

As examples of the "optionally halogenated $C_{1-4}$ alkyl group", the "optionally halogenated $C_{1-4}$ alkoxy group" and the "halogen atom" in the above-mentioned embodiments (1) to (8), referred to are the same ones as those mentioned hereinabove.

Further more preferably, Ring C includes, for example, benzene rings of the above-mentioned formula (C-2) where $C^4$ and $C^5$ are as follows:

(a) one of $C^4$ and $C^5$ is a hydrogen atom and the other is a methoxy group;
(b) $C^4$ and $C^5$ are both chlorine atoms;
(c) one of $C^4$ and $C^5$ is a methoxy group and the other is an isopropyl group;
(d) one of $C^4$ and $C^5$ is a methoxy group and the other is a 1-methoxy-1-methylethyl group; or
(e) $C^4$ and $C^5$ are both trifluoromethyl groups.

In the above-mentioned formulae, Ring Z represents an optionally-substituted nitrogen containing heterocyclic ring. Various substituents are referred to as substituents for Ring Z, which include, for example, an alkyl group (e.g., a linear or branched alkyl group having from 1 to 6 carbon atoms, preferably a linear or branched alkyl group having from 1 to 4 carbon atoms, such as methyl, ethyl, propyl, isopropyl, butyl, isobutyl, sec-butyl and tert-butyl groups, etc.), an alkenyl group (e.g., an alkenyl group having from 2 to 6 carbon atoms, preferably an alkenyl group having from 2 to 4 carbon atoms, such as ethenyl, propenyl, isopropenyl, butenyl, isobutenyl sec-butenyl groups, etc.), an alkynyl group (e.g., an alkynyl group having from 2 to 6 carbon atoms, preferably an alkynyl group having from 2 to 4 carbon atoms, such as ethynyl, propynyl, isopropynyl, butynyl, isobutynyl and sec-butynyl groups, etc.), a cycloalkyl group (e. g., a $C_{3-8}$ cycloalkyl group, preferably a $C_{3-6}$ cycloalkyl group, such as cyclopropyl, cyclobutyl, cyclopentyl and cyclohexyl groups, etc.), a cycloalkyl-alkyl group (e.g., a $C_{3-6}$ cycloalkyl-$C_{1-4}$ alkyl group, such as cyclopropylmethyl, cyclopropylethyl and cyclohexylmethyl groups, etc.), an aryl group (e.g., an aryl group having from 6 to 14 carbon atoms, preferably an aryl group having from 6 to 10 carbon atoms, such as phenyl, 1-naphthyl, 2-naphthyl, anthryl and phenanthryl groups, etc., especially, phenyl group), a nitro group, a cyano group, a hydroxy group, a $C_{1-4}$ alkoxy group (e.g., methoxy, ethoxy, propoxy, isopropoxy and butoxy groups, etc.), a $C_{1-4}$ alkylthio group (e.g., methylthio, ethylthio and propylthio groups, etc.), an amino group, a mono- or di-$C_{1-4}$ alkylamino group (e.g., methylamino, ethylamino, propylamino, dimethylamino and diethylamino groups, etc.), a cyclic amino group (e.g., a 5-membered to 9-membered cyclic amino group optionally having from 1 to 3 hetero atoms, such as oxygen and sulfur atoms, etc., in addition to nitrogen atom, concretely, for example, pyrrolidino, piperidino, morpholino and thiomorpholino groups, etc.), a $C_{1-4}$ alkyl-carbonylamino group (e.g., acetylamino, propionylamino and butyrylamino groups, etc.), a $C_{1-4}$ alkylsulfonylamino group (e.g., methylsulfonylamino and ethylsulfonylamino groups, etc.), a $C_{1-4}$ alkoxy-carbonyl group (e.g., methoxycarbonyl, ethoxycarbonyl and propoxycarbonyl groups, etc.), a carboxyl group, a $C_{1-6}$ alkyl-carbonyl group (e.g., methylcarbonyl, ethylcarbonyl and propylcarbonyl groups, etc.), a carbamoyl group, a mono- or di-$C_{1-4}$ alkylcarbamoyl group (e.g., methylcarbamoyl and ethylcarbamoyl groups, etc.), a $C_{1-6}$ alkylsulfonyl group (e.g., methylsulfonyl, ethylsulfonyl and propylsulfonyl groups, etc.), an oxo group, a thioxo group, etc. The number of the substituents is, for example, from 1 to 5 or so, preferably 1, 2 or so, depending on the size of Ring Z.

Ring Z may be a heterocyclic ring optionally having at least one hetero atom selected from nitrogen, oxygen and sulfur atoms, in addition to Y and the nitrogen atom N, and is preferably an optionally oxoated ring.

Ring Z includes rings of a formula (Z-1):

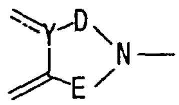

(Z-1)

wherein D and E represent groups from which Ring Z as mentioned above is formed together with the nitrogen atom adjacent to E.

Preferably, D and E which form Ring Z represent, independently, an alkylene group optionally having an oxo group, oxyalkylene group, or iminoalkylene group. The alkylene groups optionally having an oxo group to be represented by D and E preferably have carbon atoms from which Ring Z is formed to be a 5-membered to 12-membered ring, preferably a 5-membered to 9-membered ring. The numbers of the carbon atoms that constitute the alkylene groups of D and E may be the same or different.

Preferably, D includes, for example, $C_{1-7}$ alkylene group optionally having an oxo group, especially $C_{1-5}$ alkylene group optionally having an oxo group $C_{1-7}$ oxyalkylene groups, especially $C_{1-5}$ oxyalkylene groups, $C_{1-7}$ iminoalkylene groups, especially $C_{1-5}$ imminoalkylene groups. More preferably, D includes an alkylene group of a formula $-(CH_2)_m-$ (where m is from 1 to 7), an oxyalkylene group of a formula $-O-(CH_2)p$ (where p is from 1 to 7), iminoalkylene group of a formula $-NH-(CH_2)q$ (where q is from 1 to 7. In these formula, m is preferably from 1 to 5, more preferably from 2 to 5.

Preferably, E includes, for example, $C_{1-3}$ alkylene group optionally having an oxo group, more preferably an alkylene group optionally having an oxo group having one or two carbon atoms, even more preferably a methylene group optionally having an oxo group.

The number of the oxo groups that are substitutable in Ring Z is not specifically limited but may be selected from 1 to 3 or so depending on the size of Ring Z. Where Ring Z is a 5-membered to 10-membered ring, the number of the substitutable oxo groups is 1, 2 or so. Oxo group(s) may be substituted at at least either one of D and/or E. Preferably, oxo group(s) is/are substituted at E in Ring Z.

Preferably, in Ring Z, D is an alkylene group or oxyalkylene group having from 1 to 5 carbon atoms, more preferably from 2 to 5 carbon atoms especially, an alkylene group having from 2 to 5 carbon atoms, while E is an alkylene group having an oxo group having 1 or 2 carbon atoms, especially >C=O. Especially preferably, Ring Z includes, for example, from 5-membered to 9-membered rings of a formula (Z-2):

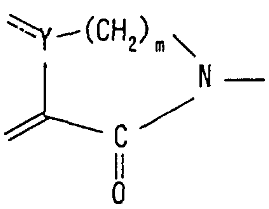

or

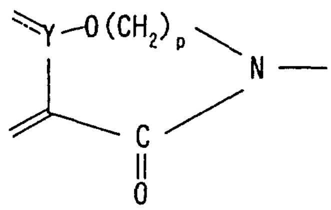

(Z-2)

wherein each m and p, independently, represents an integer of from 1 to 5.

In the above-mentioned formulae, n represents an integer of from 1 to 6, preferably an integer of from 1 to 3, especially preferably 1 or 2. More preferably, n is 1.

In compounds represented by the above-mentioned general formulae (I) and (Ia), the combination of "Ring M",

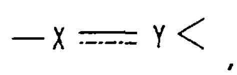

"$R^a$", "$R^b$", "Ring A", "Ring B", "Ring C", "Ring Z" and "n" is not specifically limited. These may be combined suitably to construct the compounds (I) and (Ia). Preferred compounds (I) and (Ia) are constructed by combining the above-mentioned preferred embodiments of "Ring M",

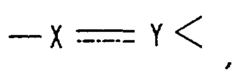

"$R^a$", "$R^b$", "Ring A", "Ring B", "Ring C", "Ring Z" and "n".

Of compounds of the above-mentioned general formula (I), especially those of the above-mentioned general

formula (Ia), preferred are (1) the following compounds or pharmaceutically-acceptable salts thereof.

Compounds of formula (I) or (Ia) wherein;

one of Ring A and Ring B is a 5-membered or 6-membered heterocyclic ring having one or two hetero atoms selected from nitrogen and oxygen atoms, in addition to carbon atoms, while the other is a benzene ring, and the Rings A and B may optionally have one or two substituents selected from a halogen atom and an optionally halogenated $C_{1-4}$ alkyl group;

Ring C is a benzene ring optionally having from 1 to 3 substituents selected from a halogen atom, an optionally halogenated $C_{1-6}$ alkyl group (preferably, $C_{1-4}$ alkyl group) and an optionally halogenated $C_{1-6}$ alkoxy group (preferably, $C_{1-4}$ alkoxy group);

D that constitutes Ring Z is $-(CH_2)_m-$ (where m is an integer of from 1 to 7) or $-O-(CH_2)p-$ (where p is an integer of from 1 to 7);

E that constitutes Ring Z is >C=O;

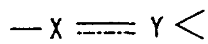

is -CO-N< or -N=C<;

n is 1,

or pharmaceutically-acceptable salts thereof.

The above-mentioned "5-membered or 6-membered heterocyclic ring" includes, for example, pyridine, pyrazine, pyrrole, thiophene, thiazole, tetrahydropyrazine, piperidine, etc. Concretely, Ring A includes heterocyclic rings of the above-mentioned formula (A-5), etc., and Ring B includes benzene rings of the above-mentioned formulae (B-7) and (B-8), especially the above-mentioned formula (B-10), etc.

The above-mentioned "halogen atom" includes, for example, fluorine, chlorine and bromine atoms, etc.; the "optionally-halogenated $C_{1-4}$ alkyl group" includes, for example, methyl, chloromethyl, difluoromethyl, trichloromethyl, trifluoromethyl, ethyl, 2-bromoethyl, 2,2,2-trifluoroethyl, perfluoroethyl, propyl, 3,3,3-trifluoropropyl, isopropyl, 2-trifluoromethylethyl, butyl, 4,4,4-trifluorobutyl, isobutyl, sec-butyl and tert-butyl groups, etc.; the "optionally halogenated $C_{1-6}$ alkyl group" includes pentyl and hexyl groups, etc., in addition to the above-mentioned alkyl groups and halogenated alkyl groups.

The "optionally halogenated $C_{1-4}$ alkoxy group" includes, for example, methoxy, difluoromethoxy, trifluoromethoxy, ethoxy, 2,2,2-trifluoroethoxy, perfluoroethoxy, propoxy, isopropoxy, butoxy, 4,4,4-trifluorobutoxy, isobutoxy, sec-butoxy and tert-butoxy groups, etc.; and the "optionally halogenated $C_{1-6}$ alkoxy group" includes pentyloxy and hexyloxy groups, etc., in addition to the above-mentioned alkoxy groups and halogenated alkoxy groups.

Of compounds of the above-mentioned general formula (I), especially those of the above-mentioned general formula (Ia), also preferred are (2) the following compounds or pharmaceutically-acceptable salts thereof.

Compounds of formula (I) or (Ia) wherein; Ring A is a 5-membered or 6-membered heterocyclic ring having one nitrogen atom or one sulfur atom, in addition to carbon atoms, for example, a heterocyclic ring of a formula (A-7):

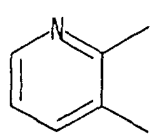 , 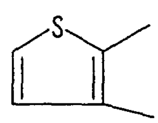 (A-7)

Ring B is a benzene ring optionally having 1 to 3 substituents selected from a halogen atom and an optionally halogenated $C_{1-4}$ alkyl group;

Ring C is a benzene ring optionally having 1 to 3 substituents selected from a halogen atom, an optionally halogenated $C_{1-4}$ alkyl group and an optionally halogenated $C_{1-4}$ alkoxy group;

D that constitutes Ring Z is $-(CH_2)_m-$ (where m is an integer of from 1 to 7) or -O-(CH2)p- (where p is integer of from 1 to 7);

E that constitutes Ring Z is >C=O;

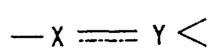

is -CO-N<;

n is 1,

or pharmaceutically acceptable salts thereof.

As examples of the "halogen atom", the "optionally halogenated $C_{1-4}$ alkyl group" and the "optionally halogenated $C_{1-4}$ alkoxy group", mentioned are those as referred to hereinabove for the foregoing compounds (1).

More preferably, compounds of formula (I) or (Ia) wherein; $R^a$ and $R^b$ are the same or different and represent, independently, a hydrogen atom, optionally halogenated $C_{1-4}$ alkyl groups, $C_{1-6}$ alkoxy-$C_{1-6}$ alkyl groups, $C_{1-6}$ alkylthio-$C_{1-6}$ alkyl groups, amino-$C_{1-6}$ alkyl groups, $C_{1-7}$ acylamino-$C_{1-6}$ alkyl groups, mono- or di-$C_{1-6}$ alkylamino-$C_{1-4}$ alkyl groups, $C_{3-10}$ cycloalkylamino-$C_{1-6}$ alkyl groups, $C_{1-6}$ alkyl groups having 5-membered or 6-membered cyclicamino which optionally substituted by $C_{1-6}$ alkyl, $C_{1-6}$ alkylsulfonylamino-$C_{1-6}$ alkyl groups or $C_{1-6}$ alkylcarbonyloxy-$C_{1-6}$ alkyl groups; or

$R^a$ and $R^b$ are bonded to each other to form pyridine ring which is optionally substituted by 1 to 3 substituents selected from a halogen atom and a $C_{1-4}$ alkyl group;

Ring B is a benzene ring optionally having 1 to 3 substituents selected from a halogen atom, an optionally halogenated $C_{1-4}$ alkyl group and an optionally halogenated $C_{1-4}$ alkoxy group; Ring C is a benzene ring optionally having 1 to 3 substituents selected from a halogen atom, an optionally halogenated $C_{1-4}$ alkyl group, an optionally halogenated $C_{1-4}$ alkoxy group, an amino group optionally substituted by $C_{1-4}$ alkyl group, a $C_{1-3}$ acyloxy group and a hydroxy group;

Ring Z is a 5-membered to 10-membered nitrogen containing heterocyclic ring optionally having an oxo group and optionally substituted $C_{1-4}$ alkyl group or a hydroxy group;

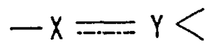

is -CO-N< or -N=C< and n is an integer of 1;

and n is 1, or salts thereof.

Preferred compounds of formulae (I) and (Ia) include, for example, compounds of the following general formula:

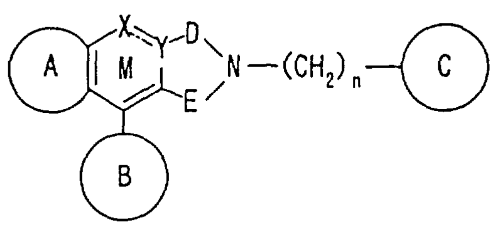

wherein D and E represent alkylene groups optionally having an oxo group and the other symbols have the same meanings as above, or salts thereof.

Preferably, D and E represent, independently, a $C_{1-3}$ alkylene group optionally substituted by one oxo group.

More preferred compounds of formulae (I) and (Ia) include, for example, compounds of the following general formula:

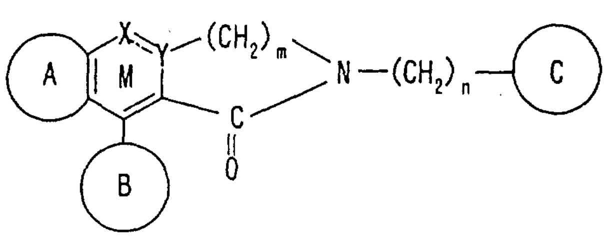

wherein m represents an integer of from 1 to 7, and the other symbols have the same meanings as above or salts thereof.

m is preferably an integer of from 2 to 5.

In the above mentioned formulae, preferably $R^a$ and $R^b$ are the same or different and represent, independently, a hydrogen atom or a substituent selected from the group consisting of

(1) a halogen atom,
(2) a $C_{1-6}$ alkyl group optionally having from 1 to 5 substituents selected from the group consisting of

(a) a hydroxy group,
(b) a $C_{1-6}$ alkoxy group,
(c) a $C_{1-6}$ alkylthio group,
(d) an amino group,
(e) a $C_{1-7}$ acylamino group,
(f) a mono- or di-$C_{1-6}$ alkylamino group,
(g) a mono- or di-$C_{3-8}$ cycloalkylamino group,
(h) a 5-membered to 9-membered cyclicamino group which may have 1 to 3 hetero atoms selected from the group consisting nitrogen, oxygen and sulfur atoms in addition to the nitrogen atom in the amino group and,
(i) a $C_{1-4}$ alkylsulfonylamino group,
(j) a $C_{1-6}$ alkyl-carbonyloxy group and
(k) a halogen atom,

(3) a 5-membered to 9-membered (preferably 6-membered) cyclicamino group which may have 1 to 3 hetero atoms (preferably 1 or 2) selected from the group consisting of nitrogen, oxygen and sulfur atoms, in addition to the nitrogen atom in the amino group and which may be substituted by $C_{1-6}$ alkyl group,
(4) a carboxyl group,
(5) a mono- or di-$C_{1-6}$ alkylcarbamoyl group; or
$R^a$ and $R^b$ are bonded to each other to form Ring A, and the Ring A is a 5-membered to 9-membered aromatic heterocyclic ring having from 1 to 3 hetero atoms selected from the group consisting of nitrogen, oxygen and sulfur atoms, in addition to carbon atoms (preferably pyridine ring), which may be substituted by $C_{1-6}$ alkyl group;
the Ring B is a $C_{6-14}$ aryl group (preferably benzene ring) which may be substituted by substituents selected from the group consisting of (i) a $C_{1-6}$ alkyl group optionally substituted by a hydroxy group, (ii) a carboxyl group and (iii) a $C_{1-6}$ alkylcarbonyl group (including formyl);
the Ring C is a $C_{6-14}$ aryl group (preferably benzene ring) which may be substituted by 1 to 3 substituents selected from the group consisting of (i) a halogen atom, (ii) optionally halogenated $C_{1-10}$ alkyl group and (iii) $C_{1-10}$ alkoxy group;
the Ring Z is a 5-membered to 12-membered heterocyclic ring optionally having at least one hetero atom selected from the group consisting of nitrogen, oxygen and sulfur atoms, in addition to Y and the nitrogen atom already on Ring Z, which may be substituted by 1 to 3 substituents selected from the group consisting of (i) a $C_{1-6}$ alkyl group, (ii) a hydroxy group and (iii) oxo group.

Particularly, (i) (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine, (ii) (9R)-7-(3,5-dimethoxybenzyl)-6,7,8,9,10,11-hexahydro-9-methyl-5-(4-methylphenyl)-6,13-dioxo-13H-[1,4]diazocino[2,1-g][1,7]naphthyridine, (iii) (9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine, (iv) (9S)-7-[3,5-bis(trifluoromethyl)benzyl]-6,7,8,9,10,12-hexahydro-9-methyl-5-(4-methyl)phenyl-6,12-dioxo-[1,4]diazepino[2,1-g][1,7]naphthyridine, etc. are preferred.
Furthermore, in the compounds represent by the above mentioned formulae, a compound represented by the formula:

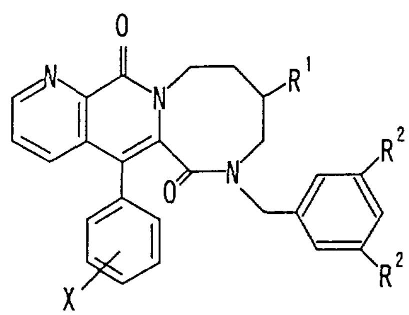

(I')

wherein $R^1$ is a $C_{1-6}$ alkyl group, $R^2$ is a $C_{1-6}$ alkoxy group, optionally halogenated $C_{1-6}$ alkyl group, a halogen atom, a hydroxy group or a $C_{7-15}$ aralkyloxy group, X is 1 to 5 groups selected from the group consisting of a hydrogen atom, a $C_{1-6}$ alkyl group and a halogen atom, provided that (1) when $R^1$ is a methyl group and $R^2$ is s trifluoromethyl group, X is a halogen atom and (2) when $R^1$ is a methyl group and $R^2$ is a methoxy group, X is a hydrogen atom or a halogen atom, is a novel compound.

The "$C_{1-6}$ alkyl group represented by $R^1$, $R^2$ and X includes, for example, methyl, ethyl, propyl, isopropyl, butyl, isobutyl, sec-butyl, tert-butyl groups and so on, and preferably a $C_{1-3}$ alkyl group such as methyl, ethyl and so on, and more preferably methyl.

The "halogen atom" which are substituents of the $C_{1-6}$ alkyl group" represented by $R^2$ includes , for example, fluorine, chlorine, bromine and iodine atoms. Preferably, the halogen atom includes, for example, chlorine atom.

The "optionally halogenated $C_{1-6}$ alkyl group" includes, for example, trichloromethyl, trifluoromethyl and so on, and preferably the "optionally halogenated $C_{1-6}$ alkyl group" includes, for example, trifluoromethyl.

The "$C_{7-15}$ aralkyloxy group" represented by $R^2$ includes, for example, benzyloxy, phenylethyloxy and so on, and preferably the "$C_{7-15}$ aralkyloxy group" includes, for example, phenylethyloxy.

The "halogen atom" represented by $R^2$ or X includes , for example, fluorine, chlorine, bromine and iodine atoms. Preferably, the halogen atom includes, for example, chlorine atom.

The "$C_{1-6}$ alkoxy group" represented by $R^2$ includes, for example, methoxy, ethoxy, propoxy, isopropoxy, butoxy, isobutoxy, sec-butoxy, tert-butoxy, etc., preferably the "$C_{1-6}$ alkoxy group" includes, for example, methoxy, ethoxy, isopropoxy, etc., and more preferably the "$C_{1-6}$ alkoxy group" includes, for example, methoxy.

The number of X which substitutes a phenyl group is 1 to 5. When X is a $C_{1-6}$ alkyl group or a halogen atom, the number of them are usually 1 to 3, preferably 1 or 2. Although the position of substitution of X is not limited when X is is a $C_{1-6}$ alkyl group or a halogen atom, for example, when the number is 1, 4-position of phenyl is preferred, and when the number is 2, 3- or 4-position of phenyl is preferred. Preferably, when the number is 2, preferable examples of X are a halogen atom (particularly, a chlorine atom) and so on.

In the above mentioned, preferable examples of $R^1$ are a $C_{1-3}$ alkyl group such as methyl, ethyl, propyl, isopropyl, etc., and more preferable examples are methyl, etc.. preferable examples of $R^2$ are a $C_{1-3}$ alkoxy group such as methoxy, ethoxy, isopropoxy, etc., an optionally halogenated $C_{1-3}$ alkyl group such as methyl, trifluoromethyl, etc., a halogen atom such as a chlorine atom, etc., a hydroxy group or a benzyloxy group. Preferable examples of X are a hydrogen atom, a $C_{1-3}$ alkyl group such as methyl, etc. or 1 or 2 halogen atoms such as a chlorine atom, a fluoro arom, etc.. Particularly,(9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine is preferred.

Where the heterocyclic compounds (I) form salts and used in medicines, it is preferable that the salts are pharmaceutically-acceptable salts.

Examples of such pharmaceutically-acceptable salts include salts with inorganic acids, such as hydrochloric acid, sulfuric acid, phosphoric acid, diphosphoric acid, hydrobromic acid, nitric acid, etc., or salts with organic acids, such as acetic acid, malic acid, maleic acid, fumaric acid, tartaric acid, succinic acid, citric acid, lactic acid, methanesulfonic acid, p-toluenesulfonic acid, palmitic acid, salicylic acid, stearic acid, etc.

Heterocyclic compounds (I) or salts thereof include stereoisomers such as cis- and trans-isomers, etc., racemates, as well as optically-active forms such as R-forms, S-forms, etc. Depending on the size of Ring Z, the heterocyclic compounds (I) or salts thereof may include conformation-dependent isomers. All such isomers are within the scope of the heterocyclic compounds (I) or salts thereof. And, hydrate and non-hydrate are within the scope of the heterocyclic compounds (I) or salts thereof.

The pro-drugs of the compounds (I) or salts thereof means heterocyclic compounds which are converted to the heterocyclic compounds (I) or salts thereof under the physiological condition or with a reaction due to an enzyme, an gastric acid, etc. in the living body, that is, (i) compounds which are converted to the compounds (I) or salts thereof with oxidation, reduction, heterocyclic hydrolysis, etc. according to an enzyme; (ii) compounds which are converted to the heterocyclic compounds (I) or salts thereof with gastric acid, etc..

Examples of the pro-drug of the heterocyclic compounds (I) or salts thereof include compounds wherein amino groups of the heterocyclic compounds (I) are substituted with acyl, alkyl, phosphoric acid, etc. (e.g. compounds wherein amino groups of the the compounds (I) are substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl, etc.); compounds wherein hydroxy groups of the heterocyclic compound (I) are substituted with acyl, alkyl, phosphoric acid, boric acid, etc. (e.g. compounds wherein hydroxy groups of the heterocyclic compounds (I) are substituted with acetyl, palmitoyl, propanoyl, pivaloyl, succinyl, fumaryl, alanyl, dimethylaminomethylcarbonyl, etc.); compounds wherein carboxyl groups of the heterocyclic compound (I) are modified with ester, amide, etc. (e.g. compounds wherein carboxyl groups of the compound (I) are modified with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); etc. These pro-drug can be produced by per se known method from the heterocyclic compounds (I) or salts thereof.

The pro-drugs of the heterocyclic compounds (I) or salts thereof may be compounds which are converted into the heterocyclic compounds (I) under the physiological conditions as described in "Pharmaceutical Research and Development", Vol. 7 (Drug Design), pages 163-198 published in 1990 by Hirokawa Publishing Co. (Tokyo, Japan).

The heterocyclic compound (I), the heterocyclic compound (I') or a salt thereof can be produced according

to the method disclosed in EP-A-0733632, particularly working examples thereof, or an analogous method thereof.

Specifically, the heterocyclic compound (I') or salts thereof can be produced, for example, by cyclizing a compound of the following general formula (II):

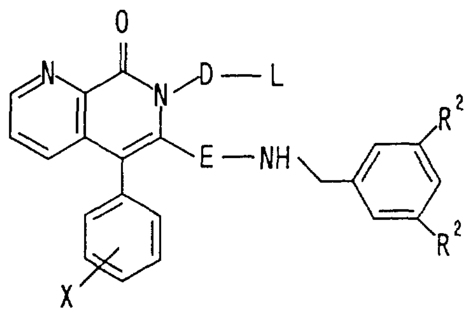

wherein D and E represent groups from which a group represented by the formula:

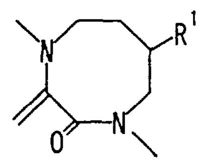

is formed together with the nitrogen atom adjacent to E, L represents a leaving group, and the other symbols are the same meanings as defined above, or a salt thereof.

D, E and $R^1$ are the same meanings as defined above. The leaving group L in compound (II) includes, for example, a halogen atom (e.g., chlorine, bromine and iodine atoms, etc.), a substituted sulfonyloxy group (e.g., methanesulfonyloxy, ethanesulfonyloxy, benzenesulfonyloxy and p-toluenesulfonyloxy groups, etc.), etc.

The compound (II) can be applied to the reaction as a free compound but may also be applied thereto as its salt (for example, as an alkali metal salt, such as lithium, sodium, potassium or the like salt, of the compound). In general, the reaction is conducted in a solvent that is inert to the reaction. As the solvent, for example, preferably used is any of halogenated hydrocarbons such as dichloromethane, chloroform, etc., nitriles such as acetonitrile, etc., ethers such as dimethoxyethane, tetrahydrofuran, etc., aprotic polar solvents such as dimethylformamide, dimethylsulfoxide, hexamethylphosphoramide, etc.

Addition of a base to the reaction system advantageously promotes the reaction. As the base, for example, advantageously employed is any of inorganic bases (alkali metal hydroxides such as sodium hydroxide, potassium hydroxide, etc.; alkali metal hydrogencarbonates such as sodium hydrogencarbonate, potassium hydrogencarbonate, etc.; alkali metal carbonates such as sodium carbonate, potassium carbonate, etc.; alkali metal hydrides such as sodium hydride, potassium hydride, etc.; sodium amide; alkoxides such as sodium methoxide, sodium ethoxide, etc.), and organic bases (amines such as trimethylamine, triethylamine, diisopropylethylamine, etc.; cyclic amines such as pyridine, etc.).

In the above-mentioned cyclization, it is also possible to convert the compound (II) into its salt with a base (for example, any of the above-mentioned alkali metal salts, alkaline earth metal salts, etc.) prior to the reaction, in place of using the base. The amount of the base, if used, varies, depending on the kind of the compound (II) and the solvent to be used and on the other reaction conditions, and is, in general, from 1 to 10 mols or so, preferably from 1 to 5 mols or so, per mol of the compound (II) used.

The reaction temperature falls, for example, within the range between about -50°C and about 200°C, preferably between about -20°C and about 150°C. The reaction time varies, depending on the kind of the compound (II) used or the kind of its salt used and also on the reaction temperature, etc., and is, for example, from 1 to 72 hours or so, preferably from 1 to 24 hours or so.

The starting compounds (II) can be produced according to the methods disclosed in EP-A-0733632.

The heterocyclic compounds (I) or salts thereof have tachykinin receptor (especially SP and/or NKA receptor (s)) antagonistic activity in vitro, and have the function of inhibiting the tracheal plasma extravasation induced by capsaicin (in vivo). Capsaicin (a main ingredient of the burning taste of red pepper) is known as a substance that liberates endogenous neuropeptides, such as SP, NKA and calcitonin gene-related peptide(CGRP) by stimulating C-fiber primary sensory nerve that contains such neuropeptides. Thus, the inhibitory action of the plasma extravasation of the

heterocyclic compounds (I) or salt thereof is considered to be based on the antagonistic activity toward tachykinin receptor. In addition, the heterocyclic compounds (I) or salts thereof are safe as having low toxicity.

Therefore, the heterocyclic compounds (I) or salts thereof, thus having such an excellent tachykinin receptor antagonistic effect, are usable as safe medicines for preventing and treating depression, anxiety, manic-depressive illness or psychopathy in mammals (e.g., mice, rats, hamsters, rabbits, cats, dogs, bovines, sheep, monkeys, man, etc.). And, the heterocyclic compounds (I) or salts thereof are also usable as safe medicines for preventing and treating Down's syndrome, amyotrophic lateral aclerosis (ALS; Lou Gehring's disease), diabetic neuropathy, peripheral neuropathy, bronchoconvulsion, chronic obstructive plumonary disease (COPD), adverse immunological reactions such as rejection of transplanted tissues, plasma extravation induced by cytokines in chemotherapy, disturbances of blood flow by vasodilation and angina, fibrotissue inflammation, vernal conjunctitis, stimulation-indeced miosis, dry eye syndrome, proliferative vitreoretinopathy, eczema, urticaria, sunburn, atopic dermatitis, addiction disorders such as alcoholism, foods ataxia(inhibition of uptaking foods), stress-related somatic disorders, reflex sympathetic dystrophy such as shoulder/hand syndrome, systemic lupus erythematosus, fibrosis and collagen disease such as scleroderma and eosinophilic fasciola hepatica disease, small cell carcinomas such as small cell lung cancer, Sjogren's sindrome, hyperlipoproteinemias IV and V, hemocromatosis, sarcoidosis, amyloidosis, bladder detrusor hyper-reflexia and incotience, artherosclerosis, irritative symptoms of benign prostatic hypertrophy, cancer-associated pain, chronic lower back pain, cluster headaches, herpes neuralgia, plantom limb pain, dental pain, neutropathic pain, opioid-resostant pain, visceral pain, surgical pain, bone injury pain, pain during labor and delivery, pain resulting from burns, post partum pain, angina pain, genitourinary tract-related pain including cystitis, spinal cord trauma, stroke, cerebral apoplexy, renal failure, Huntington's chorea and so on.

The heterocyclic compound (I') are also usable as safe medicines for preventing and treating inflammations or allergic disorders (e.g., atopy, dermatitis, herpes, proriasis, asthma, bronchitis, expectoration, rhinitis, rheumatoid arthritis, osteoarthritis, osteoporosis, multiple sclerosis, conjunctivitis, cystitis, etc.), pain, migraine, neuralgia, pruritus, cough, and additionally disorders of central nervous systems [e.g., schizophrenia, Parkinson's disease, psychosomatic disorders, dementia (e.g., Alzheimer's disease, etc.), etc.], digestive diseases (for example, irritable bowel syndrome (e.g., disorders of dejection such as diarrhea, constipation and mucous diarrhea; bellyache, distention of abdomen, etc.), ulcerative colitis, Crohn's disease, diseases caused by a spiral urease-positive gram-negative bacterium such as Helicobacter pylori, etc.), emesis (e.g., nausea, vomiturition, acute vomiting, delayed vomiting, retching,epidemic vomiting, fecal vomiting, morning vomiting, pernicious vomiting, vomiting of pregnancy, projectile vomiting, psychogenic vomiting, retention vomiting, etc.), disorders of micturition (for example, pollakisuria, urinary incontinence etc.), disturbances of circulation (for example, angina pectories, hypertension, cardiac insufficiency, thrombosis, etc.) and immunopathy, etc.. More particularly, the compounds (I') or salts thereof are usable as a tachykinin receptor antagonist and as an ameliorative preparation for disorders of micturition such as pollakisuria urimary incontinence, etc., and even as medicines for treating such disorders of micturition.

Pharmaceutical preparations comprising heterocyclic compounds (I) or salts thereof of the present invention may be in any solid forms of powders, granules, tablets, capsules, etc., and in any liquid forms of syrups, emulsions, injections, etc.

The preventive and remedial compositions of the present invention can be produced by any conventional methods of, for example, blending, kneading, granulation, tabletting, coating, sterilization, emulsification, etc., in accordance with the forms of the preparations to be produced. For the production of such pharmaceutical preparations, for example, referred to are the particular items in the general remarks for pharmaceutical preparations in the Japanese Pharmacopeia.

In the pharmaceutical compositions of the present invention, the content of the compounds or salts thereof is, though varying depending on the forms of the preparations, generally from 0.01 to 100 % by weight or so, preferably from 0.1 to 50 % by weight or so, more preferably from 0.5 to 20 % by weight or so, relative to the total weight of each preparation.

Where the heterocyclic compounds (I) or salts thereof of the present invention are used in medicines such as those mentioned above, they are, either directly or after having been mixed with suitable, pharmaceutically-acceptable carriers, for example, vehicles (e.g., starch, lactose, sucrose, calcium carbonate, calcium phosphate, etc.), binders (e.g., starch, arabic gum, carboxymethyl cellulose, hydroxypropyl cellulose, crystalline cellulose, alginic acid, gelatin, polyvinyl pyrrolidone, etc.), lubricants (e.g., stearic acid, magnesium stearate, calcium stearate, talc, etc.), disintegrators (e.g., calcium carboxymethyl cellulose, talc, etc.), diluents (e,g., water for injection, physiological saline, etc.) and optionally with additives (e.g., stabilizer, preservative, colorant, fragrance, dissolution aid, emulsifier, buffer, isotonic agent, etc.), etc., by ordinary methods, formulated into solid preparations such as powders, fine granules, granules, tablets, capsules, etc., or into liquid preparations such as injections, etc., for peroral or parenteral administration. The dose of the pharmaceutical composition of the present invention varies, depending on the kind of the heterocyclic compounds (I) or pharmaceutically-acceptable salts thereof, the administration route, the condition and the age of patients, etc. For example, the dose for oral administration of the pharmaceutical preparation to an adult patient suffering from depression is, in general, from about 0.005 to 50 mg/kg/day, preferably from about 0.05 to 10 mg/kg/day, more preferably from about 0.2 to 4 mg/kg/day, in terms of the heterocyclic compound (I) or its salt, which may be administered once a day or in two or three portions a day.

Other anti-depression agent, anti-anxiety agent, anti-manic-depressive illness, anti-psychopathy agent can be added into the pharmaceutical compositions of the present invention or can be used together with the heterocyclic compounds (I) or salts thereof.

Examples of the anti-depression agent are fluoxetine, sertraline, paroxetine and so on.

Examples of the anti-anxiety agent are diazepam, buspirone, lorazepam, alprazolam and so on.

Examples of the anti-manic-depressive illness are lithium carbonate, sultopride, carbamazepine, levomepromazine and so on.

Examples of the anti-psychopathy agent are risperidone, olanzapine, quetiapine and so on.

The heterocyclic compounds (I) or salts thereof may be optionally blended with any desired amounts of any other pharmaceutically-active ingredients to formulate pharmaceutical compositions. Such active ingredients include, for example, drugs for central nervous systems (e.g., imipramine, etc.), anti-cholinergic drugs (e.g., oxybutynin, etc.), $\alpha_1$-receptor-blocking drugs (e.g., tamsulosin, etc.), muscle relaxants (e.g., baclofen, etc.), potassium channel-opening drugs (e.g., nicorandil, etc.), calcium channel-blocking drugs (e.g., nifedipine, etc.), etc.

In the pharmaceutical compositions of the present invention, the content of drugs other than the heterocyclic compounds (I) or salts thereof is, though varying depending on the forms of the preparations, generally from 0.01 to 80 % by weight or so, preferably from 0.1 to 50 % by weight or so, more preferably from 0.5 to 20 % by weight or so, relative to the total weight of each preparation.

Best Mode for Carrying out the Invention

The present invention will be described in more detail hereinunder, with reference to Examples and Reference Examples. However, the present invention is not restricted by these examples , and changes and modifications can be made within the range which does not deviate the scope of the present invention.

Elution in the column chromatography in the following Reference Examples and Examples was conducted under observation by TLC (thin layer chromatography), unless otherwise specifically indicated. In the TLC observation, $60F_{254}$ produced by Merck Co. was used as the TLC plate, and the solvent employed in the column chromatography was used as the developing eluent. For the detection, a UV detector was used. As silica gel for the column chromatography, Silica Gel 60 (70-230 mesh) produced by Merck Co. was used. Room temperature as referred to hereinunder generally means temperatures falling between about 10° C and about 35° C. For dring the extract solutions, sodium sulfate or magnesium sulfate was used.

The meanings of the abbreviations as used in the following Examples and Reference Examples are as follows:

NMR: Nuclear magnetic resonance spectrum
EI-MS: Electron impact mass spectrum
SI-MS: Secondary ionization mass spectrum
DMF: Dimethylformamide
THF: Tetrahydrofuran
DMSO: Dimethylsulfoxide
Hz: Herz
J: coupling constant
m: multiplet
q: quartet
t: triplet
d: doublet
s: singlet
b: broad
like: approximate

Working Example

Reference Example 1:

N-[3,5-Bis(trifluoromethyl)benzyl]-7,8-dihydro-7-(2-hydroxyethyl)-5-(4-methylphenyl)-8-oxo-6-pyrido[3,4-b]pyridinecarboxamide

(Step 1)

Iodine (catalytic amount) was added to a THF (30 ml) suspension of magnesium (2.4 g) in a nitrogen atmosphere at room temperature with stirring, and then a THF (20 ml) solution of 4-bromotoluene (17.1 g) was dropwise added thereto and stirred for 1 hour. The resulting mixture was added to a THF (50 ml) solution of 2,3-pyridinedicarboxylic acid anhydride (12.7 g) at 0 - 5°C with stirring, and this was stirred for additional 30 minutes as it was and then for 1 hour at room temperature. The solvent was removed from the reaction mixture by distillation, and water (30 ml) was added to the residue, which was then adjusted the pH to 1.0 with hydrochloric acid. The mixture was extracted with dichloromethane, washed with water and dried. Then, the solvent was removed by distillation. Dichloromethane (about 10 ml) was added to the residue, and then isopropyl ether (about 70 ml) was added thereto. This was stirred for 16 hours at room temperature, and 3-(4-methylbenzoyl)-2-pyridinecarboxylic acid was obtained as colorless crystals (5.0 g).
m.p. 168-170° C (recrystallized from dichloromethane-ethyl acetate)
NMR(200MHz, $CDCl_3$) ppm: 2.41(3H,s), 7.24(2H,d,J=8.4Hz), 7.62(2H,d,J=8.4Hz), 7.70(1H,dd,J=8.0,4.8Hz), 7.85(1H,dd,J=8.0,1.5Hz), 8.77(1H,dd,J=4.8,1.5Hz).

(Step 2)

A mixture of the compound (6.0 g) as obtained in Step 1, DMF (catalytic amount), thionyl chloride (10 ml), THF (50 ml) and dichloroethane (50 ml) was refluxed for 3 hours. After the solvent was removed by distillation, the residue was dissolved in dichloromethane (100 ml).
Iminodiacetonitrile (3.0 g) and triethylamine (10 ml) were added to the resulting solution and stirred for 16 hours at room temperature. Then, the reaction mixture was washed with water, diluted hydrochloric acid, aqueous sodium hydrogencarbonate and water in that order, and dried. After the solvent was removed by distillation, obtained was N,N-bis(cyanomethyl)-3-(4-methylbenzoyl)-2-pyridinecarboxamide as pale brown crystals (4.3 g).
m.p. 166-168°C (recrystallized from ethyl acetate-ethyl ether)
NMR(200MHz, $CDCl_3$) ppm: 2.44(3H,s), 4.55(2H,s), 4.69(2H,s), 7.31(2H,d,J=8.1Hz), 7.56(1H,dd,J=7.9,4.9Hz), 7.69(2H,d,J=8.1Hz), 7.94(1H,dd,J=7.9,1.6Hz), 8.78(1H,dd,J=4.9,1.6Hz)

| Elemental Analysis for $C_{18}H_{14}N_4O_2$: | | | |
|---|---|---|---|
| Calcd.(%) | C, 67.92; | H, 4.43; | N, 17.60 |
| Found (%) | C, 67.76; | H, 4.54; | N, 17.62 |

(Step 3)

A mixture of the compound (0.86 g) as obtained in Step 2, 1,8-diazabicyclo[5.4.0]-7-undecene (DBU) (1 ml) and toluene (40 ml) was heated under reflux for 1 hour. The reaction mixture was diluted with ethyl acetate, then washed with water, diluted hydrochloric acid, aqueous sodium hydrogencarbonate and water in that order, and dried. After the solvent was removed by distillation, obtained was 7-cyanomethyl-7,8-dihydro-5-(4-methylphenyl)-8-oxo-6-pyrido[3,4-b]pyridinecarbonitrile as pale brown crystals (765 mg).
m.p. 229-231°C (recrystallized from ethyl acetate)
NMR(200MHz, $CDCl_3$) ppm: 2.48(3H,s), 5.28(2H,s), 7.31(2H,d,J=8.2Hz), 7.40(2H,d,J=8.2Hz), 7.64(1H,dd,J=8.2,4.4Hz), 7.80(1H,dd,J=8.2,1.4Hz), 9.06(1H,dd,J=4.4,1.4Hz)

| Elemental Analysis for $C_{18}H_{12}N_4O\cdot0.2H_2O$: | | | |
|---|---|---|---|
| Calcd.(%) | C, 71.14; | H, 4.11; | N, 18.43 |
| Found (%) | C, 71.20; | H, 4.26; | N, 18.20 |

(Step 4)

A mixture of the compound (2.35 g) as obtained in Step 3, hydrochloric acid (25 ml) and acetic acid (25 ml) was heated under reflux for 1.5 hours. After the solvent was removed by distillation, water was added to the residue, and the resulting mixture was extracted with ethyl acetate. The resulting extract was washed with a saturated saline solution and then dried. After solvent was removed by distillation, obtained was 7-carboxymethyl-7,8-dihydro-5-(4-methylphenyl)-8-oxo-6-pyrido[3,4-b]pyridinecarbonitrile as colorless crystals (1.62 g).
m.p. 253-254°C (recrystallized from ethyl acetate)
NMR(200MHz, $CDCl_3$) ppm: 2.46(3H,s), 5.22(2H,s), 6.64(1H,bs,-$CO_2H$), 7.32(2H,d,J=8.2Hz), 7.37(2H,d,J=8.2Hz), 7.62(1H,dd,J=8.4,4.4Hz), 7.82(1H,d,J=8.4Hz), 9.09(1H,d,J=4.4Hz)

| Elemental Analysis for $C_{18}H_{13}N_3O_3 \cdot 0.1H_2O$: | | | |
|---|---|---|---|
| Calcd.(%) | C, 67.33; | H, 4.14; | N, 13.09 |
| Found (%) | C, 67.28; | H, 4.19; | N, 13.00 |

(Step 5)

Hydroxybenzotriazole (770 mg) and 1,3-dicyclohexylcarbodiimide (1.23 g) were added to a THF (50 ml) solution of the compound (1.54 g) as obtained in Step 4, and stirred for 3 hours at room temperature. Next, sodium borohydride (550 mg) was added to the reaction mixture and stirred for 20 minutes at room temperature. The resulting reaction mixture was diluted with ethyl acetate, then washed with water and dried, and the solvent was removed by distillation. Dichloromethane was added to the residue, the insoluble substances were removed by filtration, and the solvent was removed by distillation. Hydrochloric acid (50 ml) was added to the residue and heated under reflux for 16 hours. The solvent was removed by distillation, and water with ice was added to the residue. Then, this was made alkaline with aqueous potassium carbonate, and thereafter extracted with ethyl acetate. The extract was washed with water and dried, and the solvent was removed by distillation. Thus, obtained was 6,8,9,11-tetrahydro-5-(4-methylphenyl)-6,11-dioxo[1,4]oxazino[3,4-g][1,7]naphthyridine as colorless crystals (0.86 g).
m.p. 247-249°C (recrystallized from ethyl acetate)
NMR(200MHz, $CDCl_3$) ppm: 2.45(3H,s), 4.48-4.72(4H,m), 7.12(2H,d,J=8.0Hz), 7.32(2H,d,J=8.0Hz), 7.55(1H,dd, J=8.4,4.4Hz), 7.68(1H,dd,J=8.4,1.6Hz), 9.01(1H,dd,J=4.4,1.6Hz)

| Elemental Analysis for $C_{18}H_{14}N_2O_3 \cdot 0.2H_2O$: | | | |
|---|---|---|---|
| Calcd.(%) | C, 69.76; | H, 4.68; | N, 9.04 |
| Found (%) | C, 69.64; | H, 4.86; | N, 8.95 |

(Step 6)

A mixture of the compound (410 mg) as obtained in Step 5 and 3,5-bis(trifluoromethyl)benzylamine (1.2 g) was heated in an argon atmosphere at 150° C for 2.5 hours. After this was cooled to room temperature, isopropyl ether was added thereto and the entitled compound was obtained as colorless crystals (441 mg).
m.p. 123-125°C (recrystallized from ethyl acetate)
NMR(200MHz, $CDCl_3$) ppm: 2.28(3H,s), 3.71(2H,m), 3.97(2H,m), 4.46(2H,d,J=5.2Hz), 7.00-7.20(4H,m), 7.37(1H,dd, J=8.4,4.2Hz), 7.52(1H,dd,J=8.4,1.6Hz), 7.66(2H,s), 7.76(1H,s), 8.51(1H,bs), 8.61(1H,dd,J=4.2,1.6Hz)

| Elemental Analysis for $C_{27}H_{21}N_3O_3F_6$: | | | |
|---|---|---|---|
| Calcd.(%) | C, 59.02; | H, 3.85; | N, 7.65 |
| Found (%) | C, 58.95; | H, 3.95; | N, 7.52 |

Reference Example 2:

N-[3,5-Bis(trifluoromethyl)benzyl]-7-(3-chloropropyl)-7,8-dihydro-5-(4-methylphenyl)-8-oxo-6-pyrido[3,4-b] pyridinecarboxamide

(Step 1)

A mixture of 3-(4-methylbenzoyl)-2-pyridinecarboxylic acid (13.9 g), diethyl bromomalonate (15.4 g), triethylamine (9.1 ml) and THF (120 ml) was heated under reflux for 6 hours. The solvent was removed by distillation, and ethyl acetate was added to the residue, which was washed with water, diluted hydrochloric acid and a saturated saline solution in that order and then dried. After the solvent was removed by distillation, the oily residue (20.5 g) was dissolved in THF (120 ml). DBU (4.2 ml) was added to the solution at -78°C. The mixture was stirred at 0°C for 15 minutes, and then the solvent was concentrated. The resulting concentrate was poured into 2 N-HCl and then adjusted the pH to about 10 with sodium hydrogencarbonate. Then, this was extracted with ethyl acetate. The extract was washed with water and dried, and then the solvent was removed by distillation. Thus, obtained was diethyl 5,6-dihydro-5-hydroxy-5-(4-methylphenyl)-8-oxo-8H-pyrano[3, 4-b]pyridine-6,6-dicarboxylate as colorless crystals (14.1 g).
m.p. 148-149°C (recrystallized from ethyl acetate-isopropyl ether)
NMR(200MHz, $CDCl_3$) ppm: 1.06(3H,t,J=7.1Hz), 1.21(3H,t,J=7.1Hz), 2.31(3H,s), 3.95-4.30(4H,m), 4.65(1H,s), 7.15 (2H,d,J=8.3Hz), 7.55(1H,dd,J=8.0,4.8Hz), 7.65(2H,d,J=8.3Hz), 8.47(1H,dd,J=8.0,1.4Hz), 8.86(1H,dd,J=4.8,1.4Hz)

| Elemental Analysis for $C_{21}H_{21}NO_7$: | | | |
|---|---|---|---|
| Calcd.(%) | C, 63.15; | H, 5.30; | N, 3.51 |
| Found (%) | C, 63.09; | H, 5.16; | N, 3.47 |

The same compound was alternatively obtained by the method described below.
A mixture of 3-(4-methylbenzoyl)-2-pyridinecarboxylic acid (3.0 g), DMF (1 drop), thionyl chloride (4.5 ml) and THF (30 ml) was heated under reflux for 2 hours. The solvent was evaporated, and the crystalline residue was dissolved in THF (50 ml). To the solution was added diethyl hydroxymalonate (4.1 g), and then added portionwise sodium hydride (60% oily) (646 mg) with stirring and cooling at - 10°C. After being stirred for 30 minutes at -10°C, the reaction mixture was added to a solution of ethyl acetate (100 ml) - water (100 ml). The organic layer was separated, and the aqueous layer was extracted with ethyl acetate. The organic layer and the extract were combined, washed with water and aqueous sodium chloride solution, dried and evaporated to give the above described compound as colorless crystals (4.0 g).

(Step 2)

A mixture of the compound (14.1 g) as obtained in Step 1, acetic acid (100 ml) and hydrochloric acid (100 ml) was heated under reflux for 3 hours. The solvent was removed by distillation, and water was added to the residue. Thus, obtained was 5-(4-methylphenyl)-8-oxo-8H-pyrano[3,4-b]pyridine-6-carboxylic acid as colorless crystals (8.45 g).
m.p. 274-277°C (browned at about 240° C) (recrystallized from THF-isopropyl ether)
NMR(200MHz, $CDCl_3$-DMSO-$d_6$) ppm: 2.43(3H,s), 6.10(1H,bs,-$CO_2H$), 7.16(2H,d,J=8.0Hz), 7.29(2H,d,J=8.0Hz), 7.50-7.70(2H,m), 8.94(1H,m)

| Elemental Analysis for $C_{16}H_{11}NO_4 \cdot 0.1H_2O$: | | | |
|---|---|---|---|
| Calcd.(%) | C, 67.89; | H, 3.99; | N, 4.95 |
| Found (%) | C, 67.70; | H, 4.06; | N, 4.83 |

(Step 3)

A THF (5 ml) solution of 5-(4-methylphenyl)-8-oxo-8H-pyrano[3,4-b]pyridine-6-carboxylic acid (150 mg) was dropwise added to a mixture of 3-bromopropylamine hydrobromide (1.5 g), triethylamine (2.0 ml) and methanol (5 ml), and stirred at room temperature for 2 hours. Then, the solvent was removed by distillation. Hydrochloric acid (10 ml) was added to the residue, stirred for 14 hours at room temperature and then concentrated. The resulting concentrate was adjusted the pH to 1 with 1 N-NaOH. The crystals thus precipitated were taken out by filtration and washed with water to obtain 7-(3-bromopropyl)-7,8-dihydro-5-(4-methylphenyl)-8-oxo-6-pyrido[3,4-b]pyridinecarboxylic acid as

colorless crystals (131 mg).
m.p. 194-196°C (recrystallized from THF-isopropyl ether)
NMR(200MHz, $CDCl_3$-DMSO-$d_6$) ppm: 2.30-2.60(2H,m), 2.42(3H,s), 3.54(2H,t,J=6.8Hz), 4.29(2H,t,J=7.2Hz), 5.42 (1H,bs,-$CO_2$H), 7.20-7.40(4H,m), 7.50(1H,m), 7.64(1H,d,J=8.0Hz), 8.88(1H,m).

(Step 4)

A mixture of the compound (110 mg) as obtained in Step 3, DMF (catalytic amount), thionyl chloride (0.3 ml), 1,2-dichloroethane (3 ml) and THF (3 ml) was heated under reflux for 40 minutes, and then the solvent was removed by distillation. A mixture of THF (5 ml), 3,5-bis(trifluoromethyl)benzylamine (82 mg), triethylamine (0.12 ml) and THF (2 ml) was added to the residue and stirred for 2 hours at room temperature. Ethyl acetate was added to the reaction mixture, which was washed with water, diluted hydrochloric acid, aqueous sodium hydrogencarbonate and water in that order, and then dried. After the solvent was removed by distillation, the entitled compound was obtained as colorless crystals (79 mg).
m.p. 227-229°C (recrystallized from ethyl acetate-isopropyl ether)
NMR(200MHz, $CDCl_3$) ppm: 2.10-2.40(2H,m), 2.27(3H,s), 3.4-3.7(4H,m), 4.49(2H,d,J=5.8Hz), 7.07(2H,d,J=7.6Hz), 7.24(2H,d,J=7.6Hz), 7.40(1H,dd,J=8.4,4.2Hz), 7.54(1H,dd,J=8.4,1.4Hz), 7.67(2H,s), 7.78(1H,s), 8.06(1H,bt), 8.70 (1H,dd,J=4.2,1.4Hz)

| Elemental Analysis for $C_{28}H_{22}N_3O_2ClF_6 \cdot 0.2H_2O$: | | | |
|---|---|---|---|
| Calcd.(%) | C, 57.43; | H, 3.86; | N, 7.18 |
| Found (%) | C, 57.29; | H, 3.98; | N, 7.07 |

Reference Example 3:

N-[3,5-Bis(trifluoromethyl)benzyl]-5-(4-methylphenyl)-8-oxo-8H-pyrano[3,4-b]pyridine-6-carboxamide

The compound as obtained in Step 2 in Reference Example 2 was reacted with 3,5-(bistrifluoromethyl)benzylamine and treated in the same manner as in Step 4 in Reference Example 2, to obtain the entitled compound as colorless crystals. m.p. 182-183°C (recrystallized from ethyl acetate-ether) NMR(200MHz, $CDCl_3$) ppm: 2.44(3H,s), 4.63(2H,d,J=6.4Hz), 7.17(2H,d,J=8.1Hz), 7.34(2H,d,J=8.1Hz), 7.50-7.65(3H,m), 7.73(2H,s), 7.80(1H,s), 8.96(1H,m)

| Elemental Analysis for $C_{25}H_{16}N_2O_3F_6$: | | | |
|---|---|---|---|
| Calcd.(%) | C, 59.30; | H, 3.18; | N, 5.53 |
| Found (%) | C, 59.42; | H, 3.30; | N, 5.45 |

Reference Example 4:

N-(2-methoxybenzyl)-5-(4-methylphenyl)-8-oxo-8H-pyrano[3,4-b]pyridine-6-carboxamide

The compound as obtained in Step 2 in Reference Example 2 was reacted with 2-methoxybenzylamine and treated in the same manner as in Step 4 in Reference Example 2, to obtain the entitled compound as colorless crystals.
m.p. 189-190°C (recrystallized from ethyl acetate)
NMR(200MHz, $CDCl_3$) ppm: 2.44(3H,s), 3.90(3H,s), 4.48(2H,d,J=5.8Hz), 6.85-6.95(2H,m), 7.10-7.35(6H,m), 7.43(1H, bt), 7.50-7.63(2H,m), 8.93(1H,m).

Reference Example 5:

N-[3,5-Bis(trifluoromethyl)benzyl]-7,8-dihydro-7-(4-hydroxybutyl)-5-(4-methylphenyl)-8-oxo-6-pyrido[3,4-b] pyridinecarb oxamide

4-Amino-1-butanol (0.5 ml) was added to a THF (5 ml)-methanol (10 ml) solution of the compound (200 mg) as obtained in Reference Example 3, at 0° C, and stirred at room temperature for 1 hour. The solvent was removed from the reaction mixture by distillation, and hydrochloric acid (15 ml) was added to the residue and stirred for 14 hours at room temperature. The reaction mixture was poured into aqueous potassium carbonate with ice and then extracted with ethyl acetate. The extract was washed with water and dried, and the solvent was removed by distillation. Thus,

the entitled compound was obtained as colorless crystals (144 mg).
m.p. 187-188°C (recrystallized from ethyl acetate-isopropyl ether)
NMR(200MHz, $CDCl_3$) ppm: 1.3-1.5(2H,m), 1.6-1.9(2H,m), 2.29(3H,s), 2.82(1H,bs), 3.55(2H,t,J=5.7Hz), 3.69(2H,m), 4.48(2H,d,J=5.8Hz), 7.08(2H,d,J=8.1Hz), 7.21(2H,d,J=8.1Hz), 7.29(1H,dd,J=8.4,4.2Hz), 7.52(1H,dd,J=8.4,1.4Hz), 7.68(2H,s), 7.78(1H,s), 8.39(1H,bt), 8.61(1H,dd,J=4.2,1.4Hz).

Reference Example 6:

7,8-Dihydro-7-(3-hydroxypropyl)-N-(2-methoxybenzyl)-5-(4-methylphenyl)-8-oxo-6-pyrido[3.4-b]pyridinecarboxamide

The compound as obtained in Reference Example 4 was reacted with 3-amino-1-propanol and treated in the same manner as in Reference Example 5, to obtain the entitled compound. This compound was used in Example 4 without being purified.

Reference Example 7:

7,8-Dihydro-7-(4-hydroxybutyl)-N-(2-methoxybenzyl)-5-(4-methylphenyl)-8-oxo-6-pyrido[3,4-b]pyridinecarboxamide

The compound as obtained in Reference Example 4 was reacted with 4-amino-1-butanol and treated in the same manner as in Reference Example 5, to obtain the entitled compound as colorless crystals.
m.p. 205-206°C (recrystallized from methanol-ethyl ether)
NMR(200MHz, $CDCl_3$) ppm: 1.57(2H,m), 1.95(2H,m), 2.33(3H,s), 2.71(1H,bs), 3.66(2H,t,J=6.0Hz), 3.75(3H,s), 4.00-4.15(2H,m), 4.29(2H,d,J=6.2Hz), 6.59(1H,bt), 6.71-6.92(3H,m), 7.04-7.30(5H,m), 7.41(1H,dd,J=8.4,4.4Hz), 7.62 (1H,dd,J=8.4,1.4Hz), 8.82(1H,dd,J=4.4,1.4Hz).

Reference Example 8:

N-[3,5-Bis(trifluoromethyl)benzyl]-7,8-dihydro-7-(5-hydroxypentyl)-5-(4-methylphenyl)-8-oxo-6-pyrido[3,4-b] pyridinecarboxamide

The compound as obtained in Reference Example 3 was reacted with 5-amino-1-pentanol and treated in the same manner as in Reference Example 5, to obtain the entitled compound as colorless crystals.
m.p. 136-137°C (recrystallized from ethyl acetate-ethyl ether)
NMR(200MHz, $CDCl_3$) ppm: 1.10-1.35(2H,m), 1.35-1.55(2H,m), 1.6-1.9(2H,m), 2.28(3H,s), 3.50-3.70(4H,m), 4.47(2H, d,J=5.8Hz), 7.06(2H,d,J=8.0Hz), 7.19(2H,d,J=8.0Hz), 7.35(1H,dd,J=8.3,4.4Hz), 7.50(1H,d,J=8.3,1.4Hz), 7.69(2H,s), 7.78(1H,s), 8.29(1H,bt), 8.64(1H,dd,J=4.4,1.4Hz).

Reference Example 9:

N-[3,5-Bis(trifluoromethyl)benzyl]-7,8-dihydro-7-(3-hydroxypropyl)-8-oxo-5-phenyl-6-pyrido[3,4-b] pyridinecarboxamide

(Step 1)

3-Benzoyl-2-pyridinecarboxylic acid was used in place of 3-(4-methylbenzoyl)-2-pyridinecarboxylic acid in Step 1 in Reference Example 2, reacted and treated in the same manner as in Step 1 in Reference Example 2, to obtain diethyl 5,6-dihydro-5-hydroxy-8-oxo-5-phenyl-8H-pyrano[3,4-b]pyridine-6,6-dicarboxylate as colorless crystals.
m.p. 146-147°C (recrystallized from ethyl acetate-isopropyl ether)
NMR(200MHz, $CDCl_3$) ppm: 1.07(3H,t,J=7.2Hz), 1.18(3H,t,J=7.2Hz), 4.00-4.25(4H,m), 4.70(1H,s), 7.30-7.40(3H,m), 7.56(1H,dd,J=8.0,4.8Hz), 7.74-7.85(2H,m), 8.48(1H,dd,J=8,0,1.5Hz), 8.87(1H,dd,J=4.8,1.5Hz).

(Step 2)

The compound as obtained in Step 1 was reacted with acetic acid and hydrochloric acid and treated in the same manner as in Step 2 in Reference Example 2, to obtain 8-oxo-5-phenyl-8H-pyrano[3,4-b]pyridine-6-carboxylic acid as colorless crystals.
m.p. 288-290°C (recrystallized from THF-methanol-ether)
NMR(200MHz, DMSO-$d_6$) ppm: 7.28-7.60(6H,m), 7.81(1H,dd,J=8.2,4.4Hz), 8.95(1H,dd,J=4.4,1.6Hz).

(Step 3)

The compound as obtained in Step 2 was reacted with 3,5-bis(trifluoromethyl)benzylamine and treated in the same manner as in Reference Example 3, to obtain N-[3,5-bis(trifluoromethyl)benzyl]-8-oxo-5-phenyl-8H-pyrano [3,4-b]pyridine-6-carboxamide as colorless crystals.
m.p. 182-183°C (recrystallized from ethyl acetate-isopropyl ether)
NMR(200MHz, $CDCl_3$) ppm: 4.61(2H,t,J=6.2Hz), 7.24-7.34(2H,m), 7.49-7.67(6H,m), 7.72(2H,s), 7.79(17,s), 8.96(1H, dd, J=4.2,1.6Hz).

(Step 4)

The compound as obtained in Step 3 was reacted with 3-amino-1-propanol and treated in the same manner as in Reference Example 5, to obtain the entitled compound as colorless crystals.
m.p. 129-130°C (recrystallized from ethyl acetate-ethyl ether)
NMR(200MHz, $CDCl_3$) ppm: 1.91(2H,m), 3.45(2H,t,J=5.4Hz), 3.70(2H,m), 4.46(2H,d,J=6.0Hz), 7.2-7.4(5H,m), 7.44 (1H,dd,J=8.4,4.4Hz), 7.59(1H,dd,J=8.4,1.6Hz), 7.61(2H,s), 7.78(1H,s), 8.25(1H,bt), 8.70(1H,dd,J=4.4,1.6Hz).

Reference Example 10:

N-[3,5-Bis(trifluoromethyl)benzyl]-7,8-dihydro-7-(4-hydroxybutyl)-8-oxo-5-phenyl-6-pyrido[3,4-b]pyridinecarboxamide

The compound as obtained in Step 3 in Reference Example 9 was reacted with 4-amino-1-butanol and treated in the same manner as in Reference Example 5, to obtain the entitled compound as colorless crystals.
m.p. 155-157°C (recrystallized from ethyl acetate-isopropyl ether)
NMR(200MHz, $CDCl_3$) ppm: 1.45(2H,m), 1.83(2H,m), 3.58(2H,t,J=5.8Hz), 3.73(2H,m), 4.44(2H,d,J=5.8Hz), 7.2-7.4 (6H,m), 7.54(1H,dd,J=8.1,1.1Hz), 7.60(2H,s), 7.77(1H,s), 8.05(1H,bt), 8.66(1H,dd,J=4.1,1.1Hz).

Reference Example 11:

N-[3,5-Bis(trifluoromethyl)benzyl]-1,2-dihydro-2-(4-hydroxybutyl)-4-(4-methylphenyl)-1-oxo-3-pyrido[3,4-c] pyridinecarboxamide

(Step 1)

4-(4-Methylbenzoyl)-3-pyridinecarboxylic acid was used in place of 3-(4-methylbenzoyl)-2-pyridinecarboxylic acid in Step 1 in Reference Example 2, reacted and treated in the same manner as in Step 1 in Reference Example 2, to obtain diethyl 3,4-dihydro-4-hydroxy-4-(4-methylphenyl)-1-oxo-1H-pyrano[3,4-c]pyridine-3,3-dicarboxylate as an yellow oil.
NMR(200MHz, $CDCl_3$) ppm: 1.08(3H,t,J=7.1Hz), 1.21(3H,t,J=7.2Hz), 2.31(3H,s), 4.00-4.40(4H,m), 4.72(1H,bs), 7.14 (2H,d,J=8.4Hz), 7.64(2H,d,J=8.4Hz), 8.05(1H,d,J=5.3Hz), 8.85(1H,d,J=5.3Hz), 9.12(1H,s).

(Step 2)

The compound as obtained in Step 1 was reacted with acetic acid and hydrochloric acid and treated in the same manner as in Step 2 in Reference Example 2, to obtain 4-(4-methylphenyl)-1-oxo-lH-pyrano[3,4-c]pyridine-3-carboxylic acid as colorless crystals.
m.p. 254-256°C (recrystallized from THF-isopropyl ether)
NMR(200MHz, $CDCl_3$+$d_6$-DMSO) ppm: 2.43(3H,s), 5.31(1H,bs,COOH), 7.04(1H,d,J=5.5Hz), 7.16(2H,d,J=7.8Hz), 7.29(2H,d,J=7.8Hz), 8.81(1H,d,J=5.5Hz), 9.54(1H,s).

(Step 3)

The compound as obtained in Step 2 was reacted with 3,5-bis(trifluoromethyl)benzylamine and treated in the same manner as in Reference Example 3, to obtain N-[3,5-bis(trifluoromethyl)benzyl]-4-(4-methylphenyl)-1-oxo-1H-pyrano[3,4-c]pyridine-3-carboxamide as colorless crystals.
m.p. 188-189°C (recrystallized from ethyl acetate-isopropyl ether)
NMR(200MHz, $CDCl_3$) ppm: 2.44(3H,s), 4.61(2H,d,J=6.2Hz), 7.05(1H,d,J=5.3Hz), 7.15(2H,d,J=8.1Hz), 7.32(2H,d, J=8.1Hz), 7.43(1H,bt), 7.70(2H,s), 7.80(1H,s), 8.85(1H,d,J=5.3Hz), 9.56(1H,s).

(Step 4)

The compound as obtained in Step 3 was reacted with 4-amino-1-butanol and treated in the same manner as in Reference Example 5, to obtain the entitled compound as colorless crystals.
m.p. 128-131°C (recrystallized from ethyl acetate-isopropyl ether)
NMR(200MHz, $CDCl_3$) ppm: 1.45-1.70(2H,m), 1.75-2.05(2H,m), 2.31(3H,s), 3.65(2H,t,J=5.9Hz), 3.98(2H,m), 4.36(2H,d,J=6.0Hz), 7.00(1H,d,J=5.7Hz), 7.12(2H,d,J=8.1Hz), 7.18(2H,d,J=8.1Hz), 7.56(2H,s), 7.80(1H,s), 8.47(1H,d,J=5.7Hz), 9.41(1H,s).

Reference Example 12:

N-[3,5-Bis(trifluoromethyl)benzyl]-2-chloro-N-(2-hydroxyethyl)-4-phenyl-3-pyridinecarboxamide

(Step 1)

3,5-Bis(trifluoromethyl)benzyl bromide (1.1 ml) was added to a THF (30 ml) solution of 2-aminoethanol (3.6 ml) while cooling it with ice. The resulting mixture was stirred for 1 hour at room temperature, ethyl acetate (30 ml) was added thereto, and this was washed with water and a saturated, aqueous sodium chloride solution. The organic layer was dried and the solvent was removed by distillation. Thus, obtained was N-[3,5-bis(trifluoromethyl)benzyl]-N-(2-hydroxyethyl)amine as colorless crystals (1.38 g).
m.p. 107-108°C (recrystallized from ethanol-ethyl ether)
NMR(200MHz, $CDCl_3$) ppm: 2.83(2H,t,J=5.4Hz), 3.72(2H,t,J=5.4Hz), 3.96(2H,s), 7.78(1H,s), 7.82(2H,s).

(Step 2)

Thionyl chloride (0.7 ml) and DMF (catalytic amount) were added to a THF (10 ml) solution of 2-chloro-4-phenyl-3-pyridinecarboxylic acid (318 mg) and heated under reflux for 4 hours. The solvent was removed by distillation, and the residue was dissolved in THF (5 ml). The resulting solution was added to a mixture of N-[3,5-bis(trifluoromethyl)benzyl]-N-(2-hydroxyethyl)amine (391 mg) that had been obtained in Step 1, triethylamine (0.57 ml) and THF (10 ml), while cooling with ice, and then stirred for 2 hours at room temperature. The solvent was removed by distillation, and water was added to the residue, which was then extracted with ethyl acetate. The extract was washed with water and dried, and then the solvent was removed by distillation. The residue was separated and purified by column chromatography (hexane:ethyl acetate = 1:1) using silica gel, and the entitled compound was obtained as an oil (551 mg) (ratio of cis-trans isomers with respect to the amide bond = about 2:1).
NMR(200MHz, $CDCl_3$) ppm: 2.82-3.92(4H,m), 4.16(1Hx1/3,d,J=16.0Hz), 4.41(1Hx1/3,d,J=16.0Hz), 4.73(1Hx2/3,d,J=15.0Hz), 4.87(1Hx2/3,d,J=15.0Hz), 7.20-8.85(9H,m), 8.43(1H,m).

Reference Example 13:

(S)-N-[3,5-Bis(trifluoromethyl)benzyl]-7,8-dihydro-7-(3-hydroxy-2-methylpropyl)-5-(4-methylphenyl)-8-oxo-6-pyrido[3,4-b]pyridinecarboxamide

(S)-3-Amino-2-methyl-1-propanol (307 mg) was added to a THF (10 ml)-methanol (7.5 ml) solution of the compound (1.0 g) as obtained in Reference Example 3, and stirred for 14 hours at room temperature. Diluted hydrochloric acid was added to the reaction mixture, which was then extracted with ethyl acetate. The extract was washed with a saturated saline solution and then dried, and the solvent was removed by distillation. Acetonitrile (3 ml), toluene (21 ml) and DBU (0.42 ml) were added to the residue and heated under reflux for 1 hour. After cooled, the reaction mixture was diluted with ethyl acetate and then washed with water, diluted hydrochloric acid and a saturated saline solution in that order. The organic layer was dried, and the solvent was removed by distillation. Thus, the entitled compound was obtained as colorless crystals.
m.p. 123-125° C (after once melted, this again solidified), 215-216° C (re-melted) (recrystallized from ethyl acetate-isopropyl ether)
$[a]_D$: +11.1° (C=0.350,$CHCl_3$)
NMR(200MHz, $CDCl_3$) ppm: 0.79(3H,d,J=7.0Hz), 2.13(1H,m), 2.28(3H,s), 3.10-3.70(4H,m), 4.48(2H,d,J=6.2Hz), 7.00-7.25(4H,m), 7.43(1H,dd,J=8.4,4.2Hz), 7.59(1H,dd,J=8.4,1.6Hz), 7.69(2H,s), 7.79(1H,s), 8.38(1H,bt), 8.70(1H,dd,J=4.2,1.6Hz)

| Elemental Analysis for $C_{29}H_{25}N_3O_3F_6 \cdot 0.5H_2O$: | | | |
|---|---|---|---|
| Calcd.(%) | C, 59.39; | H, 4.47; | N, 7.16 |
| Found (%) | C, 59.64; | H, 4.31; | N, 7.01 |

Reference Example 14:

(R)-N-[3,5-Bis(trifluoromethyl)benzyl]-7,8-dihydro-7-(3-hydroxy-2-methylpropyl)-8-oxo-5-phenyl-6-pyrido[3,4-b] pyridinecarboxamide

The compound as obtained in Step 3 in Reference Example 9 was reacted with (R)-3-amino-2-methyl-1-propanol and treated in the same manner as in Reference Example 13, to obtain the entitled compound as colorless crystals.
m.p. 101-103°C (recrystallized from ethyl ether-isopropyl ether)
NMR(200MHz, $CDCl_3$) ppm: 0.77(3H,d,J=6.6Hz), 2.14(1H,m), 3.10-3.70(4H,m), 4.47(2H,d,J=5.8Hz), 7.1-7.4(5H,m), 7.45(1H,dd,J=8.4,4.2Hz), 7.60(1H,d,J=8.4Hz), 7.65(2H,s), 7.78(1H,s), 8.60(1H,bt), 8.69(1H,d,J=4.2Hz)
$[a]_D$: -5.4° (C=0.512,$CHCl_3$).

Reference Example 15:

(R)-N-[3,5-Bis(trifluoromethyl)benzyl]-7,8-dihydro-7-(3-hydroxy-2-methylpropyl)-5-(4-methylphenyl)-8-oxo-6-pyrido [3,4-b]pyridinecarboxamide

The compound as obtained in Reference Example 3 was reacted with (R)-3-amino-2-methyl-1-propanol and treated in the same manner as in Reference Example 13, to obtain the entitled compound as colorless crystals.
m.p. 123-125° C (after once melted, this again solidified), 215-216°C (re-melted) (recrystallized from ethyl acetate-isopropyl ether)
MMR(200MHz, $CDCl_3$) ppm: same as the spectrum of the compound of Reference Example 13.
$[a]_D$: -9.0° (C=0.346,$CHCl_3$).

Reference Example 16:

( +/-)-N-[3,5-Bis(trifluoromethyl)benzyl]-7,8-dihydro-7-(4-hydroxy-3-methylbutyl)-8-oxo-5-phenyl-6-pyrido[3,4-b] pyridinecarboxamide

The compound as obtained in Step 3 in Reference Example 9 was reacted with 4-amino-2-methyl-1-butanol and treated in the same manner as in Reference Example 13, to obtain the entitled compound as colorless crystals.
m.p. 217-219°C (recrystallized from ethyl acetate-isopropyl ether).

Reference Example 17:

(+/-)-N-[3,5-Bis(trifluoromethyl)benzyl]-7,8-dihydro-7-(4-hydroxy-3-methylbutyl)-5-(4-methylphenyl)-8-oxo-6-pyrido [3,4-b]pyridinecarboxamide

The compound as obtained in Reference Example 3 was reacted with 4-amino-2-methyl-1-butanol and treated in the same manner as in Reference Example 13, to obtain the entitled compound as colorless crystals.
m.p. 129-130° C (after once melted, this again solidified), 188-190°C (re-melted) (recrystallized from ethyl acetate-isopropyl ether)
NMR(200MHz, $CDCl_3$) ppm: 0.79(3H,d,J=6.6Hz), 1.4-1.8(3H,m), 2.28(3H,s), 3.03(1H,t,J=6.6Hz,-OH), 3.2-3.7(4H,m), 4.49(2H,d,J=5.8Hz), 7.0-7.3(4H,m), 7.30(1H,dd,J=8.4,4.4Hz), 7.53(1H,dd,J=8.4,1.4Hz), 7.68(2H,s), 7.78(1H,s), 8.48 (1H.t.J=6.0Hz), 8.61(1H,dd,J=4.4,1.4Hz).

Reference Example 18:

(R)-N-[3,5-Bis(trifluoromethyl)benzyl]-7,8-dihydro-7-(4-hydroxy-3-methylbutyl)-8-oxo-5-phenyl-6-pyrido[3,4-b] pyridinecarboxamide

The compound as obtained in Step 3 in Reference Example 9 was reacted with (R)-4-amino-2-methyl-1-butanol tetrahydropyranyl (THP) ether and treated in the same manner as in Reference Example 13, to obtain THP ether of the entitled compound as a pale orange oil. This compound was reacted with p-toluenesulfonic acid in methanol at room temperature to thereby remove the THP group, and the entitled compound was obtained as colorless crystals.
m.p. 213-215°C (recrystallized from ethyl acetate-ethyl ether)
NMR(200MHz, $CDCl_3$) ppm: same as the spectrum of the compound of Reference Example 16.
$[a]_D$: +1.0° (C=0.519,$CHCl_3$).

(R)-4-amino-2-methyl-1-butanol THP ether was prepared according to the following method.

Using methyl ester of (S)-(-)-3-hydroxy-2-methyl propionic acid as a starting material, (R)-4-hydroxy-3-methylbutane nitrile THP ether was prepared by reacting according to the methods described in references [Kenji Mori, Tetrahedron, Vol.39, 3107-3109 (1983) which descrobes a method for synthesizing enantiomers; H. Mattes et al., Journal of Medicinal Chemistry, Vol.30, 1948-1951, 1987]. [Colorless oill substances, NMR (200MHz, $CDCl_3$)ppm: 1.09 (3H ×1/2, d,J=6.8Hz), 1.10 (3H×1/2, d,j=6.8Hz), 1.5-1.9(6H,m), 2.1-2.6 (3H,m), 3.17-3.88(4H,m), 4.60(1H,b).]

Solutions (100ml) of ethyl ether of this compound (22.7g) was added to suspensions (200ml) of ethyl ether of hydrogenated lithium alminium (3.7g) at 0°C with stirring strongly and gradually. And, the reaction mixture was stirred for 1 hour at room temperature and was cooled in ice water again to which water (3ml), 15% of aqueous NaOH and water (10ml) was added with stirring. The resulting precipitates were separated by filtration with Celite and was washed with ehtyl acetate. The filtrated solution and washing solution were gethered and were washed with aqueous potassium carbonate and saturated saline and dried. The solvent was distilled off whereby (R)-4-amino-2-methyl-1-butanol THP ether was obtained as colorless oily substances (21.7g).
NMR(200MHz, $CDCl_3$)ppm: 0.94(3H×1/2, d, J=6.8Hz), 0.95(3H ×1/2, d, J=6.8Hz), 1.2-1.9(11H,m), 3.13-3.90(6H,m), 4.57(1H,b).

Reference Example 19:

(R)-N-[3,5-Bis(trifluoromethyl)benzyl]-7,8-dihydro-7-(4-hydroxy-3-methylbutyl)-5-(4-methylphenyl)-8-oxo-6-pyrido [3,4-b]pyridinecarboxamide

The compound as obtained in Reference Example 3 was reacted with (R)-4-amino-2-methyl-1-butanol THP ether and treated in the same manner as in Reference Example 13, to obtain THP ether of the entitled compound as a pale orange oil. This compound was reacted with p-toluenesulfonic acid in methanol at room temperature to thereby remove the THP group, and the entitled compound was obtained as colorless crystals.
m.p. 123-125° C (after once melted, this again solidified), 205-206°C (re-melted) (recrystallized from ethyl acetate-isopropyl ether)
NMR(200MHz, $CDCl_3$) ppm: same as the spectrum of the compound of Reference Example 17.
$[a]_D$: +1.2° (C=0.471,$CHCl_3$).

Reference Example 20:

(S)-N-[3,5-Bis(trifluoromethyl)benzyl]-7,8-dihydro-7-(4-hydroxy-3-methylbutyl)-8-oxo-5-phenyl-6-pyrido[3,4-b] pyridinecarboxamide

(S)-4-Amino-2-methyl-1-butanol THP ether was used in place of (R)-4-amino-2-methyl-1-butanol THP ether in Reference Example 18 and reacted and treated in the same manner as in Reference Example 18, to obtain the entitled compound as colorless crystals.
m.p. 213-214°C (recrystallized from ethyl acetate-ethyl ether)
NMR(200MHz, $CDCl_3$) ppm: same as the spectrum of the compound of Reference Example 16.
$[a]_D$: -1.5° (C=0.492,$CHCl_3$).

Reference Example 21:

(S)-N-[3,5-Bis(trifluoromethyl)benzyl]-7,8-dihydro-7-(4-hydroxy-3-methylbutyl)-5-(4-methylphenyl)-8-oxo-6-pyrido [3,4-b]pyridinecarboxamide

(S)-4-Amino-2-methyl-1-butanol THP ether was used in place of (R)-4-amino-2-methyl-1-butanol THP ether in Reference Example 19 and reacted and treated in the same manner as in Reference Example 19, to obtain the entitled compound as colorless crystals.
m.p. 213-215° C (after once melted, this again solidified), 207-208°C (re-melted) (recrystallized from ethyl acetate-isopropyl ether)
NMR(200MHz, $CDCl_3$) ppm: same as the spectrum of the compound of Reference Example 17.
$[a]_D$: -2.7° (C=0.391,$CHCl_3$).

Reference Example 22:

N-(2-Aminoethyl)-N-[3,5-bis(trifluoromethyl)benzyl]-2-chloro-4-phenyl-3-pyridinecarboxamide

(Step 1)

Thionyl chloride (0.15 ml) and DMF (catalytic amount) were added to a THF (5 ml) solution of 2-chloro-4-phenyl-3-pyridinecarboxylic acid (145 mg) and heated under reflux for 2 hours. The solvent was removed by distillation, and the residue was dissolved in THF (5 ml). The resulting solution was added to a mixture of N-[3,5-bis(trifluoromethyl) benzyl]-N'-tert-butoxycarbonylethylenediamine (240 mg), triethylamine (0.26 ml) and THF (10 ml), while cooling with ice, and stirred for 3 hours at room temperature. The N-[3,5-bis(trifluoromethyl)benzyl]-N'-tert-butoxycarbonylethylenediamine used herein was prepared as an oily compound, by reacting ethylenediamine with 3,5-bis(trifluoromethyl) benzyl methanesulfonate in THF to give an oily compound of N-[3,5-bis(trifluoromethyl)benzyl]ethylenediamine, followed by further reacting the compound with di-tert-butyl dicarbonate in the presence of triethylamine in THF.

The solvent was removed from the reaction mixture, and water was added to the residue, which was then extracted with ethyl acetate. The extract was washed with water and dried, and the solvent was removed by distillation. Thus, obtained was N-[3,5-bis(trifluoromethyl)benzyl]-N-[2-(tert-butoxycarbonylamino)ethyl]-2-chloro-4-phenyl-3-pyridinecarboxamide as a pale yellow oil.
NMR(200MHz, $CDCl_3$) ppm: 1.20-1.60(total 9H,m), 2.70-4.90(total 7H,m), 7.20-8.00(total 9H,m), 8.46(1H,d,J=5.2Hz).

(Step 2)

A 4N-HCl/ethyl acetate solution (10 ml) was added to the compound as obtained in Step 1 and stirred for 30 minutes at room temperature. The solvent was removed by distillation, and aqueous sodium hydrogencarbonate was added to the residue, which was then extracted with ethyl acetate. The extract was washed with water and then dried, and the solvent was removed by distillation. Thus, the entitled compound was obtained as a pale yellow oil (349 mg).
NMR(200MHz, $CDCl_3$) ppm: 2.30-3.70(4H,m), 4.15(1Hx2/5,d,J=16.2Hz), 4.38(1Hx2/5,d,J=16.2Hz), 4.65(1Hx3/5,d, J=15.2Hz), 4.84(1Hx3/5,d,J=15.2Hz), 7.20-7.60(6H,m), 7.65-7.80(6H,m), 8.47(1H,m).

Reference Example 23:

N-[3,5-Bis(trifluoromethyl)benzyl]-2-chloro-N-(3-hydroxypropyl)-4-phenyl-3-pyridinecarboxamide

(Step 1)

3-Amino-1-propanol was used in place of 2-aminoethanol in Step 1 in Reference Example 12, reacted and treated in the same manner as in Step 1 in Reference Example 12, to obtain N-[3,5-bis(trifluoromethyl)benzyl]-N-(3-hydroxypropyl)amine as colorless crystals.
m.p. 57-58°C (recrystallized from ethyl ether-hexane)
NMR(200MHz, $CDCl_3$) ppm: 1.77(2H,quintet,J=5.8Hz), 2.89(2H,t,J=5.8Hz), 3.82(2H,t,J=5.8Hz), 3.93(2H,s), 7.89(3H, s)

| Elemental Analysis for $C_{12}H_{13}NOF_6$: | | | |
|---|---|---|---|
| Calcd.(%) | C, 47.85; | H, 4.35; | N, 4.65 |

(continued)

| Elemental Analysis for $C_{12}H_{13}NOF_6$: | | | |
|---|---|---|---|
| Found (%) | C, 47.76; | H, 4.32; | N, 4.65 |

(Step 2)

The amine as obtained in Step 1 was used in place of N-[3,5-bis(trifluoromethyl)benzyl]-N-(2-hydroxyethyl) amine in Step 2 in Reference Example 12 , reacted and treated in the same manner as in Step 2 in Reference Example 12, to obtain the entitled compound as colorless crystals (the ratio of cis-trans isomers with respect to the amide bond was about 3:1).
m.p. 121-122°C (recrystallized from ethyl acetate-isopropyl ether)
NMR(200MHz, $CDCl_3$) ppm: 1.00-1.70(2H,m), 2.75-3.20(2H,m), 3.35-3.55(3H,m), 4.06(1Hx1/4,d,J=16.2Hz), 4.31 (1Hx1/4,d,J=16.2Hz), 4.65(1Hx3/4,d,J=15.2Hz), 4.76(1Hx3/4,d,J=15.2Hz), 7.20-7.55(6H,m), 7.72(2H,s), 7.80(1H,s), 8.47(1H,d,J=5.2Hz)

| Elemental Analysis for $C_{24}H_{19}N_2O_2F_6Cl$: | | | |
|---|---|---|---|
| Calcd.(%) | C, 55.77; | H, 3.71; | N, 5.42 |
| Found (%) | C, 55.65; | H, 3.70; | N, 5.57 |

Reference Example 24:

N-[3,5-Bis(trifluoromethyl)benzyl]-2-chloro-N-(2-hydroxyethyl)-5-methyl-4-phenyl-3-pyridinecarboxamide

2-Chloro-5-methyl-4-phenyl-3-pyridinecarboxylic acid was used in place of 2-chloro-4-phenyl-3-pyridinecarboxylic acid in Step 2 in Reference Example 12, reacted and treated in the same manner as in Step 2 in Reference Example 12, to obtain the entitled compound as colorless crystals.
m.p. 146-148°C (recrystallized from ethyl acetate-isopropyl ether)
NMR(200MHz, $CDCl_3$) ppm: 2.09(3H,s), 3.02(1H,dt,J=15.0,5.6Hz), 3.25(1H,dt,J=15.0,5.6Hz), 3.60(2H,m), 4.57(1H, d,J=15.2Hz), 4.79(1H,d,J=15.2Hz), 7.05-7.50(5H,m), 7.62(2H,s), 7.76(1H,s), 8.33(1H,s).

Reference Example 25:

N-[3,5-Bis(trifluoromethyl)benzyl]-2-chloro-N-(3-hydroxypropyl)-5-methyl-4-phenyl-3-pyridinecarboxamide

2-Chloro-5-methyl-4-phenyl-3-pyridinecarboxylic acid was used in place of 2-chloro-4-phenyl-3-pyridinecarboxylic acid in Step 2 in Reference Example 12, reacted with N-[3,5-bis(trifluoromethyl)benzyl]-N-(3-hydroxypropyl) amine that had been obtained in Step 1 in Reference Example 23, in place of N-[3,5-bis(trifluoromethyl)benzyl]-N-(2-hydroxyethyl)amine, and treated in the same manner as in Step 2 in Reference Example 12, to obtain the entitled compound as a pale yellow oil.
NMR(200MHz, $CDCl_3$) ppm: 1.10-1.80(2H,m), 2.06(3Hx1/2,s), 2.08(3Hx1/2,s), 2.80-3.30(3H,m), 3.35-3.70(1H,m), 4.08(1Hx1/2,d,J=16.4Hz), 4.39(1Hx1/2,d,J=15.0Hz), 4.47(1Hx1/2,d,J=16.4Hz), 4.70(1Hx1/2,d,J=15.0Hz), 6.90-7.62 (7H,m), 7.72(1Hx1/2,s), 7.77(1Hx1/2,s), 8.28(1Hx1/2,s), 8.31(1Hx1/2,s).

Reference Example 26:

(+/-)-N-[3,5-Bis(trifluoromethyl)benzyl]-7,8-dihydro-7-(2,3-dihydroxypropyl)-5-(4-methylphenyl)-8-oxo-6-pyrido[3,4-b] pyridinecarboxamide

The compound as obtained in Reference Example 3 was reacted with (+/-)-3-amino-1,2-propanediol and treated in the same manner as in Reference Example 13, to obtain the entitled compound as a pale yellow foam.
NMR(200MHz, $CDCl_3$) ppm: 2.20(3H,s), 3.50(2H,m), 4.02-4.30(5H,m), 4.75(1H,b), 5.35(1H,b), 6.92-7.46(6H,m), 7.55 (2H,s), 7.70(1H,s), 8.63(1H,m), 8.83(1H,b).

Reference Example 27:

N-Benzyl-8-oxo-5-phenyl-8H-pyrano[3,4-b]pyridine-6-carboxamide

The compound as obtained in Step 2 in Reference Example 9 was reacted with benzylamine and treated in the same manner as in Reference Example 3, to obtain the entitled compound as colorless crystals.
m.p. 188-189°C (recrystallized from acetone-ethyl ether)
NMR(200MHz, $CDCl_3$) ppm: 4.48(2H,d,J=5.4Hz), 7.2-7.4(8H,m), 7.49-7.65(5H,m), 8.95(1H,dd,J=4.4,2.0Hz)

Reference Example 28:

N-(3,4-Dichlorobenzyl)-8-oxo-5-phenyl-8H-pyrano[3,4-b]pyridine-6-carboxamide

The compound as obtained in Step 2 in Reference Example 9 was reacted with 3,4-dichlorobenzylamine and treated in the same manner as in Reference Example 3, to obtain the entitled compound as colorless crystals.
m.p. 198-200°C (recrystallized from acetone-ethyl ether)
NMR(200MHz, $CDCl_3$) ppm: 4.44(2H,d,J=6.0Hz), 7.10(1H,dd,J=8.2,2.0Hz), 7.25-7.70(10H,m), 8.96(1H,dd, J=4.3,1.7Hz)

Reference Example 29:

N-[3,5-Bis(trifluoromethyl)benzyl]-2-chloro-N-[(S)-3-hydroxy-2-methylpropyl]-5-methyl-4-phenyl-3-pyridinecarboxamide

The same process as in Step 2 in Reference Example 12 was repeated, except that 2-chloro-5-methyl-4-phenyl-3-pyridinecarboxylic acid was reacted with N-[3,5-bis(trifluoromethyl)benzyl]-N-[(S)-3-hydroxy-2-methylpropyl] amine [this was prepared by reacting 3,5-bis(trifluoromethyl)benzyl methanesulfonate with (S)-3-amino-2-methylpropanol in THF, and this is a colorless oily substance and was identified by NMR(200MHz, $CDCl_3$) ppm: 0.86(3H,d, J=6.8Hz), 1.98(1H,m), 2.63(1H,dd,J=9.4,11.8Hz), 2.70-2.90(3H,m), 3.56(1H,dd,J=8.6,10.6Hz), 3.71(1H,ddd, J=1.4,4.0,10.6Hz), 3.87(1H,d,J=13.8Hz), 3.98(1H,d,J=13.8Hz), 7.79(3H,s)]

In place of 2-chloro-4-phenyl-3-pyridinecarboxylic acid and N-[3,5-bis(trifluoromethyl)benzyl]-N-(2-hydroxyethyl)amine in Step 2 in Reference Example 12, 2-chloro-5-methyl-4-phenyl-3-pyridinecarboxylic acid was reacted with the above mentioned N-[3,5-bis(trifluoromethyl)benzyl]-N-[(s)-3-hydroxy-2-methylpropyl]amine and treated in the same manner as in Reference Example 12, to obtain the entitled compound as a colorless oily substance.
NMR(200MHz, $CDCl_3$) ppm: 0.60-0.82(3H,m), 1.50-2.00(1H,m), 2.00-2.15(3H,m), 2.15-3.92(4H,m), 4.05-4.92(2H,m), 7.00-7.85(8H,m), 8.34(1H,m)

Reference Example 30:

N-[3,5-Bis(trifluoromethyl)benzyl]-2-chloro-N-[(R)-3-hydroxy-2-methylpropyl]-5-methyl-4-phenyl-3-pyridinecarboxamide

N-[3,5-Bis(trifluoromethyl)benzyl]-N-[(R)-3-hydroxy-2-methylpropyl]amine was reacted and treated in the same manner as in Reference Exaple 29, in place of N-[3,5-bis(trifluoromethyl)benzyl]-N-[(S)-3-hydroxy-2-methy lpropyl]amine in Reference Example 29, to obtain the entitled compound as a colorless oily substance. The NMR spectrum (200MHz, $CDCl_3$) of the compound obtained herein was the same as that of the compound obtained in Reference Example 29.

Reference Example 31:

N-[3,5-Bis(trifluoromethyl)benzyl]-2-chloro-N-(2-hydroxyethyl)-6-methyl-4-phenyl-3-pyridinecarboxamide

(Step 1)

A mixture of ethyl 2-chloro-6-methyl-4-phenyl-3-pyridinecarboxylate (15.43 g), ethanol (70 ml) and aqueous 4N-sodium hydroxide solution (70 ml) was heated under reflux for 2.5 hours. The reaction mixture was concentrated, and the resulting concentrate was made acidic (pH 3) by adding hydrochloric acid thereto and then extracted with ethyl acetate. The extract was washed with saturated aqueous sodium chloride solution and dried, and the solvent was

removed by distillation. Thus, obtained was 2-chloro-6-methyl-4-phenyl-3-pyridinecarboxylic acid as colorless crystals (11.2 g).
m.p. 191-194°C (recrystallized from ethyl acetate-isopropyl ether)
NMR(200MHz, $CDCl_3$) ppm: 2.59(3H,s), 7.16(1H,s), 7.45(5H,s), 9.53(1H,b)

(Step 2)

In place of 2-chloro-4-phenyl-3-pyridinecarboxylic acid in Step 2 in Reference Example 12, 2-chloro-6-methyl-4-phenyl-3-pyridinecarboxylic acid (as obtained in the previous Step 1) was reacted and treated in the same manner as in Step 2 in Reference Example 12, to obtain the entitled compound as a pale-yellow, oily substance.
NMR(200MHz, $CDCl_3$) ppm: 1.95-3.80(4H,m), 2.58(3H,s), 4.15(1Hx2/5,d,J=16.2Hz), 4.41(1Hx2/5,d,J=16.2Hz), 4.75 (1Hx3/5,d,J=15.0Hz), 4.85(1Hx3/5,d,J=15.0Hz), 7.15(1Hx3/5,s), 7,17(1Hx2/5,d,J=15.0Hz), 7.23-7.58(5H,m), 7.74 (2H,s), 7.78(1H,s)

Reference Example 32:

N-[3,5-Bis(trifluoromethyl)benzyl]-2-chloro-N-(3-hydroxypropyl)-6-methyl-4-phenyl-3-pyridinecarboxamide

The same process as in Step 2 in Reference Example 12 was repeated, except that 2-chloro-6-methyl-4-phenyl-3-pyridinecarboxylic acid was reacted with N-[3,5-bis(trifluoromethyl)benzyl]-N-(3-hydroxypropyl)amine in place of reacting 2-chloro-4-phenyl-3-pyridinecarboxylic acid with N-[3,5-bis(trifluoromethyl)benzyl]-N-(2-hydroxyethyl)amine, to obtain the entitled compound as a pale-yellow, oily substance.
NMR(200MHz, $CDCl_3$) ppm: 1.15-1.65(2H,m), 2.59(3H,s), 2.75-3.20(2H,m), 4.06(1Hx2/5,d,J=15.4Hz), 4.31(1Hx2/5,d, J=15.4Hz), 4.65(1Hx3/5,d,J=15.2Hz), 4.74(1Hx3/5,d,J=15.2Hz), 7.16(1H,s), 7.20-7.60(5H,m), 7.72(2H,s), 7.78(1H,s)

Reference Example 33:

N-[3,5-Bis(trifluoromethyl)benzyl]-N-(2-hydroxyethyl)-5-methyl-2-methylaminocarbonyl-4-phenyl-3-pyridinecarboxamide

(Step 1)

A mixture of diethyl 5-methyl-4-phenyl-2,3-pyridinedicarboxylate (13.0 g) [this was prepared in accordance with the method described in Japanese Patent Laid-Open No. 62-106081, and this has a melting point of 73-74°C (after recrystallized from ethyl ether-isopropyl ether)], potassium hydroxide (20 g) and 70 %-ethanol (200 ml) was heated under reflux for 3 hours. The solvent was removed by distillation, and the residue was diluted with water and then washed with ethyl ether. The PH of the aqueous layer was adjusted to 2 - 3 by adding 2 N-hydrochloric acid, and then extracted with ethyl acetate. The extract was washed with saturated saline solution and dried, and the solvent was removed by distillation. Thus, obtained was 5-methyl-4-phenyl-2,3-pyridinedicarboxylic acid as pale yellow crystals (3.06 g).
m.p. 187-188°C (recrystallized from THF-ethyl ether)
NMR(200MHz, DMSO-$d_6$) ppm: 2.10(3H,s), 7.20-7.30(2H,m), 7.40-7.55(3H,m), 8.65(1H,s)

(Step 2)

The compound (2.9 g) as obtained in the previous Step 1 was heated in acetic anhydride (50 ml) under reflux for 2 hours. The solvent was removed by distillation, and ethanol (50 ml) was added to the residue and stirred for 4 hours at room temperature. Then, the solvent was removed by distillation, and the residue was dissolved in ethyl acetate. The resulting solution was washed with saturated saline solution and dried, and the solvent was removed by distillation. Thus, obtained was a mixture of 2-ethyl ester and 3-ethyl ester (about 3:2) of 5-methyl-4-phenyl-2,3-pyridinedicarboxylic acid as a pale-brown, oily substance (3.39 g).

(Step 3)

DMF (3 drops) and oxalyl chloride (2.0 ml) were added to a THF (30 ml) solution of the oily substance (1.94 g) as obtained in Step 2, and stirred for 30 minutes at room temperature. The solvent was removed by distillation, and the residue was dissolved in THF (40 ml). N-[3,5-Bis(trifluoromethyl)benzyl]-N-(2-hydroxyethyl)amine (2.2 g) and triethylamine (2.0 ml) were added to the resulting solution and stirred for 16 hours at room temperature. The reaction

mixture was diluted with ethyl acetate, then washed with water, diluted hydrochloric acid, aqueous potassium carbonate solution and saturated saline solution, and dried. The solvent was removed by distillation, and the residue was purified by column chromatography (hexane:ethyl acetate = 1:2) using silica gel to obtain N-[3,5-bis(trifluoromethyl)benzyl]-2-ethoxycarbonyl-N-(2-hydroxyethyl)-5-methyl-4-phenyl-3-pyridinecarboxamide as colorless crystals (1.31 g).
m.p. 138-139°C (recrystallized from ethyl acetate-isopropyl ether)
NMR(200MHz, $CDCl_3$) ppm: 1.45(3Hx1/4,t,J=7.1Hz), 1.46(3Hx3/4,t,J=7.1Hz), 2.18(3Hx3/4,s), 2.19(3Hx1/4,s), 2.82 (1H,m), 3.2-3.7(3H,m). 4.15-4.62(4H,m), 7.05-7.80(8H,m), 8.65(1Hx3/4,s), 8.68(1Hx1/4,s)

(Step 4)

40 %Methylamine-methanol solution (15 ml) was added to a THF (5 ml) solution of the compound (377 mg) as obtained in Step 3 and stirred for 16 hours at room temperature. The solvent was removed by distillation, and the entitled compound was obtained as a pale-yellow, oily substance (370 mg).
NMR(200MHz, $CDCl_3$) ppm: 2.12(3Hx2/3,s), 2.14(3Hx1/3,s), 2.83(1H,m), 3.03(3Hx1/3,d,J=5.2Hz), 3.04(3Hx2/3,d, J=4.8Hz), 3.25-3.80(3H,m), 4.30(1Hx2/3,d,J=15Hz), 4.36(1Hx1/3,d,J=15Hz), 4.59(1Hx1/3,d,J=15Hz), 4.86(1Hx2/3,d, J=15Hz), 7.0-7.9(8H,m), 8.02(1H,2/3,bd), 8.17(1Hx1/3,bd), 8.46(1H,s)

Reference Example 34:

N-[3,5-Bis(trifluoromethyl)benzyl]-N-(2-hydroxyethyl)-2-methylaminocarbnyl-4-phenyl-3-pyridinecarboxamide

(Step 1)

In place of diethyl 5-methyl-4-phenyl-2,3-pyridinedicarboxylate in Step 1 in Reference Example 33, diethyl 4-phenyl-2,3-pyridinedicarboxylate (see Japanese Patent Laid-Open No. 62-106081) was reacted and treated in the same manner as in Step 1 in Reference Exaple 33, to obtain 4-phenyl-2,3-pyridinedicarboxylic acid as pale yellow crystals.
m.p. 146-148°C (recrystallized from THF-isopropyl ether) NMR(200MHz, $CDCl_3$ + DMSO-$d_6$) ppm: 7.3-7.6(6H,m), 8.69 (1H,d,J=5.0Hz)

(Step 2)

The compound as obtained in Step 1 was reacted and treated in the same manner as in Step 2 in Reference Example 33, to obtain a mixture of 2-ethyl ester and 3-ethyl ester (about 3:2) of 4-phenyl-2,3-pyridinedicarboxylic acid as a pale-brown, oily substance.

(Step 3)

The oily substance as obtained in Step 2 was reacted and treated in the same manner as in Step 3 in Reference Example 33, to obtain N-[3,5-bis(trifluoromethyl)benzyl]-2-ethoxycarbonyl-N-(2-hydroxyethyl)-4-phenyl-3-pyridinecarboxamide as a pale-yellow, oily substance.
NMR(200MHz, $CDCl_3$) ppm: 1.48(3H,t,J=7.1Hz), 2.71(1H,m), 3.1-3.7(3H,m), 4.1-4.9(4H,m), 7.18-7.52(6H,m), 7.65-7.82(3H,m), 8.78(1Hx3.4,d,J=4.8Hz), 8.80(1Hx1/4,d,J=4.8Hz)

(Step 4)

The compound as obtained in Step 3 was reacted and treated in the same manner as in Step 4 in Reference Example 33, to obtain the entitled compound as a pale-yellow, oily substance.
NMR(200MHz, $CDCl_3$) ppm: 2.73(1H,m), 3.05(3Hx1/3,d,J=5.0Hz), 3.06(3Hx2/3,d,J=5.0Hz), 3.1-3.9(3H,m), 4.29 (1Hx1/3,d,J=16Hz), 4.52(1Hx2/3,d,J=15Hz), 4.54(1Hx1/3,d,J=16Hz), 4.93(1Hx2/3,d,J=15Hz), 7.0-7.9(9H,m), 7.95 (1Hx2/3,bd), 8.19(1Hx1/3,bd), 8.59(1H,d,J=5.2Hz)

Reference Example 35:

N-Benzyl-2-ethoxycarbonyl-N-(2-hydroxyethyl)-5-methyl-4-phenyl-3-pyridinecarboxamide

The oily substance as obtained in Step 2 in Reference Example 33 was reacted with N-benzyl-N-(2-hydroxyethyl)amine and treated in the same manner as in Step 3 in Reference Example 33, to obtain the entitled compound as

a pale-yellow, oily substance.
NMR(200MHz, $CDCl_3$) ppm: 1.44(3Hx1/4,t,J=7.2Hz), 1.46(3Hx3/4,t,J=7.1Hz), 2.15(3Hx3/4,s), 2.19(3Hx1/4,s), 2.6-3.7 (4H,m), 3.96(1Hx3/4,d,J=15Hz), 4.00(1Hx1/4,d,J=15Hz), 4.4-4.6(2H+1Hx1/4,m), 5.37(1Hx3/4,d,J=15Hz), 6.48 (2Hx3/4,m), 6.82(2Hx1/4,m), 7.0-7.6(8H,m), 8.65(1Hx3.4,s), 8.66(1Hx1/4,s)

Reference Example 36:

N-[3,5-Bis(trifluoromethyl)benzyl]-N-(3-hydroxypropyl)-5-methyl-2-methylaminocarbonyl-4-phenyl-3-pyridinecarboxamide

(Step 1)

The oily substance as obtained in Step 2 in Reference Example 33 was reacted with N-[3,5-bis(trifluoromethyl) benzyl]-N-(3-hydroxypropyl)amine and treated in the same manner as in Step 3 in Reference Example 33 to obtain N-[3,5-bis(trifluoromethyl)benzyl]-2-ethoxycarbonyl-N-(3-hydroxypropyl)-5-methyl-4-phenyl-3-pyridinecarboxamide as a pale-yellow, oily substance.
NMR(200MHz, $CDCl_3$) ppm: 1.44(3Hx1/4,t,J=7.1Hz), 1.45(3Hx3/4,t,J=7.1Hz), 1.60(2H,m), 2.17(3Hx3/4,s), 2.18 (3Hx1/4,s), 2.7-3.7(4H,m), 3.96(1Hx1/4,d,J=16Hz), 4.35-4.60(3H+1Hx3/4,m), 7.10-7.80(8H,m), 8.64(1Hx3/4,s), 8.68 (1Hx1/4,s)

(Step 2)

The compound as obtained in Step 1 was reacted and treated in the same manner as in Step 4 in Reference Example 33 to obtain the entitled compound as a pale-yellow, oily substance.
NMR(200MHz, $CDCl_3$) ppm: 1.3-1.9(2H,m), 2.11(3Hx3/5,s), 2.14(3Hx2/5,s), 2.7-3.8(4H,m), 3.02(3Hx3/5,d,J=5.2Hz), 3.03(3Hx2/5,d,J=5.2Hz), 4.04(1Hx2/5,d,J=16Hz), 4.28(1Hx3/5,d,J=15Hz), 4.46(1Hx2/5,d,J=16Hz), 4.82(1Hx3/5,d, J=15Hz), 7.0-7.6(5H,m), 7.63(2H3/5,s), 7.67(2Hx2/5,s), 7.73(1H,s), 7.96(1Hx3/5,bd), 8.06(1Hx2/5,bd), 8.45 (1Hx3/5,s), 8.48(1Hx2/5,s)

The compounds as described in Reference Example 37 - 45 were obtained as pale yellow oily substances using 2-chloro-4-phenyl-3-pyridinecarboxylic acid and N-substituted-N-(substituted)benzylamines {N-(2-hydroxyethyl) -N-(3,4,5-trimethoxybenzyl)amine,N-(3,4-dichlorobenzyl)-N-(2-hydroxyethyl)amine,N-(3,4-dimethoxybenzyl)-N-(2-hydroxyethyl)amine, N-benzyl-N-[2-hydroxyethyl]amine, N-(2-hydroxypropyl)-N-(3,4,5-trimethoxybenzyl)amine, N-benzyl-N-[(S)-3-hydroxy-2-methylpropyl]amine, N-benzyl-N-[(R)-3-hydroxy-2-methylpropyl]amine, N-[3,5-bistrifluoromethyl)benzyl]-N-[(S)-3-hydroxy-2-methylpropyl]amine and N-[3,5-(bistrifluoromethyl)benzyl]-N-[(R)-3-hydroxy-2-methylpropyl]amine, respectively} by substantially the same reaction and work-up as Reference Example 12-Step 2. The physico-chemical data are described below.

Reference Example 37:

2-Chloro-N-(2-hydroxyethyl)-4-phenyl-N-(3,4,5-trimethoxybenzyl)-3-pyridinecarboxamide
NMR(200MHz, $CDCl_3$) ppm: 2.05-2.50(2H,m), 2.80-4.00(12H,m), 4.00-4.40(1Hx3/2,m), 4.93(1Hx1/2,d,J=14.2Hz), 6.22(2Hx1/2,s), 6.55(2Hx1/2,s), 7.25-7.70(6H,m), 8.42(1Hx1/2,d,J=6.2Hz), 8.48(1Hx1/2,d,J=5.8Hz) (a 1:1 mixture of the amide rotamers).

Reference Example 38:

2-Chloro-N-(3,4-dichlorobenzyl)-N-(2-hydroxyethyl)-4-phenyl-3-pyridinecarboxamide
NMR(200MHz, $CDCl_3$) ppm: 1.80-3.85(5H,m), 3.96(1Hx4/9,d,J=16.0Hz), 4.24(1Hx4/9,d,J=16.0Hz), 4.44(1Hx5/9,d, J=15.2Hz), 4.92(1Hx5/9,d,J=15.2Hz), 6.50-6.85(2H,m), 7.10-7.70(7H,m), 8.46(1H,m) (a 5:4 mixture of the amide rotamers).

Reference Example 39:

2-Chloro-N-(3,4-dimethoxybenzyl)-N-(2-hydroxyethyl)-4-phenyl-3-pyridinecarboxamide
NMR(200MHz, $CDCl_3$) ppm: 2.70-4.30(12H,m), 4.53(1Hx1/2,d,J=14.8Hz), 4.74(1Hx1/2,d,J=14.8Hz), 6.30-7.00(3H, m), 7.20-7.65(6H,m), 8.39(1Hx1/2,d,J=5.0Hz), 8.46(1Hx1/2,d,J=5.2Hz) (a 1:1 mixture of the amide rotamers).

Reference Example 40:

N-Benzyl-2-chloro-N-(2-hydroxyethyl)-4-phenyl-3-pyridinecarboxamide
NMR(200MHz, $CDCl_3$) ppm: 2.27(1Hx1/2,b), 2.60(1Hx1/2,b), 2.75-3.15(1H,m), 3.25-3.65(3H,m), 3.90(1Hx1/2, d, J=15.4Hz), 4.26(1Hx1/2,d,J=15.4Hz), 4.49(1Hx1/2, d,J=15.0Hz), 4.95(1Hx1/2,d,J=15.0Hz), 6.74(2Hx1/2,m), 6.92 (2Hx1/2,m), 7.10-7.65(9H,m), 8.42(1H,m) (a 1:1 mixture of the amide rotamers).

Reference Example 41

2-Chloro-N-(3-hydroxypropyl)-4-phenyl-N-(3,4,5-trimethoxybenzyl)-3-pyridinecarboxamide
NMR(200MHz, $CDCl_3$) ppm: 1.10-2.30(3H,m), 2.70-4.30(14H+1Hx3/7,m), 4.88(1Hx4/7,d,J=14.8Hz), 6.18(2Hx4/7,s), 6.52(2Hx3/7,s), 7.20-7.60(6H,m), 8.47(1H,m) (a 4:3 mixture of the amide rotamers).

Reference Example 42:

N-Benzyl-2-chloro-N-[(S)-3-hydroxy-2-methylpropyl]-4-phenyl-3-pyridinecarboxamide
NMR(200MHz, $CDCl_3$) ppm: 0.50-0.85(3H,m), 1.40-1.85(1H,m), 2.20-3.75(5H,m), 3.80-5.15(2H,m), 6.60-7.65(11H, m), 8.42(1H,m) (a 2:1 mixture of the amide rotamers).

Reference Example 43:

N-Benzyl-2-chloro-N-[(R)-3-hydroxy-2-methylpropyl]-4-phenyl-3-pyridinecarboxamide
MR(200MHz, $CDCl_3$) ppm: same as the spectrum of the compound of Reference Example 42.

Reference Example 44:

N-[3,5-Bis(trifluoromethyl)benzyl]-2-chloro-N-[(S)-3 -hydroxy-2-methylpropyl]-4-phenyl-3-pyridinecarboxamide
NMR(200MHz, $CDCl_3$) ppm: 0.53(3Hx1/4,d,J=7.0Hz), 0.63(3Hx1/4,d,J=7.0Hz), 0.75(3Hx1/4,d,J=6.8Hz), 0.81 (3Hx1/4,d,J=6.8Hz), 1.50-1.90(1H,m), 2.42-3.80(5H,m), 4.00-4.95(2H,m), 7.10-7.90(9H,m), 8.42(1H,m) (a 1:1 mixture of the amide rotamers).

Reference Example 45:

N-[3,5-Bis(trifluoromethyl)benzyl]-2-chloro-N-[(R)-3-hydroxy-2-methylpropyl]-4-phenyl-3-pyridinecarboxamide
NMR(200MHz, $CDCl_3$) ppm: same as the spectrum of the compound of Reference Example 44.

Reference Example 46:

N-Benzyl-7,8-dihydro-7-(4-hydroxybutyl)-5-(4-methylphenyl)-8-oxo-6-pyrido[3,4-b]pyridinecarboxamide

(Step 1)

Using the compound as obtained in Reference Example 2 - Step 2 and benzylamine, substantially the same reaction and work-up as Reference Example 2 - Step 4 was conducted to give N-benzyl-5-(4-methylphenyl)-8-oxo-8H-pyrano[3,4-b]pyridine-6-carboxamide as colorless crystals.
m.p. 208-209°C (recrystallized from acetone-isopropyl ether)
NMR(200MHz, $CDCl_3$) ppm: 2.45(3H,s), 4.48(2H,d,J=5.6Hz), 7.10-7.40(10H,m), 7.58(2H,m), 8.94(1H,dd, J=3.6&2.2Hz).

(Step 2)

Using the compound as obtained in Step 1 and 4-amino-1-butanol, substantially the same reaction and work-up as Reference Example 13 was conducted to give the entitled compound as colorless crystals.
m.p. 205-207°C (recrystallized from acetone-isopropyl ether)
NMR(200MHz, $CDCl_3$) ppm: 1.48(2H,m), 1.83 (2H,m), 2.45(3H,s), 2.86(1H,b), 3.57(2H,t,J=5.9Hz), 3.85(2H,m), 4.34 (2H,d,J=6.0Hz), 6.8-7.1(2H,m), 7.10-7.35(8H,m), 7.50(1H,m), 7.55(1H,dd,J=8.4&1.4Hz), 8.60(1H,dd,J=4.0&1.4Hz).

Reference Example 47:

(R)-N-Benzyl-7,8-dihydro-7-(4-hydroxy-3-methylbutyl)-5-(4-methylphenyl)-8-oxo-6-pyrido[3,4-b]pyridinecarboxamide

Starting from the compound as obtained in Reference Example 46 - Step 1 and THP-ether of (R)-4-amino-2-methyl-1-butanol, substantially the same reaction and work-up as Reference Example 19 was conducted to give the entitled compound as colorless crystals.
m.p. 226-227°C (recrystallized from acetone-ethyl ether) NMR(200MHz, $CDCl_3$) ppm: 0.81(3H,d,J=6.6Hz), 1.5-2.0(3H, m), 2.44(3H,s), 3.20-3.55(3H,m), 3.93(2H,m), 4.31(2H,d,J=5.4Hz), 6.75-6.90(2H,m), 7.1-7.3(8H,m), 7.39(1H,dd, J=8.2&4.2Hz), 7.61(1H,d,J=8.2Hz), 8.68(1H,d,J=4.2Hz).

Reference Example 48:

(S)-N-Benzyl-7,8-dihydro-7-(4-hydroxy-3-methylbutyl)-5-(4-methylphenyl)-8-oxo-6-pyrido[3,4-b]pyridinecarboxamide

Starting from the compound as obtained in Reference Example 46 - Step 1 and THP-ether of (S)-4-amino-2-methyl-1-butanol, substantially the same reaction and work-up as Reference Example 19 was conducted to give the entitled compound as colorless crystals.
m.p. 226-227°C (recrystallized from acetone-ethyl ether)
NMR(200MHz, $CDCl_3$) ppm: same as the spectrum of the compound of Reference Example 47.

Reference Example 49:

(R)-7,8-Dihydro-7-(4-hydroxy-3-methylbutyl)-5-(4-methylphenyl)-8-oxo-N-(3,4,5-trimethoxybenzyl)-6-pyrido[3,4-b] pyridinecarboxamide

(Step 1)

Using the compound as obtained in Reference Example 2 - Step 2 and 3,4, 5-trimethoxybenzylamine, substantially the same reaction and work-up as Reference Example 2 - Step 4 was conducted to give 5-(4-methylphenyl)-8-oxo-N-(3,4,5-trimethoxybenzyl)-8H-pyrano[3,4-b]pyridine-6-carboxamide as colorless crystals.
m.p. 195-196°C (recrystallized from acetone-isopropyl ether)
NMR(200MHz, $CDCl_3$) ppm: 2.45(3H,s), 3.84(3H,s), 3.85(6H,s), 4.40(2H,d,J=5.8Hz), 6.50(2H,s), 7.17(2H,d,J=8.0Hz), 7.27(1H,b), 7.32(2H,d,J=8.0Hz), 7.58(2H,m), 8.94(1H,dd,J=4.0&2.2Hz).

(Step 2)

Starting from the compound as obtained in Step 1 and THP-ether of (R)-4-amino-2-methyl-1-butanol, substantially the same reaction and work-up as Reference Example 19 was conducted to give the entitled compound as colorless crystals.
m.p. 194-195°C (recrystallized from acetone-ethyl ether)
NMR(200MHz, $CDCl_3$) ppm: 0.84(3H,d,J=6.8Hz), 1.5-2.0(3H,m), 2.38(3H,s), 3.2-3.6(3H,m), 3.65-3.95(2H,m), 3.80 (6H,s), 3.82(3H,s), 4.23(2H,d,J=6.0Hz), 6.40(2H,s), 7.05-7.40(4H,m), 7.32(1H,dd,J=8.2&4.2Hz), 7.56(1H,dd, J=8.2&1.6Hz), 7.80(1H,m), 8.63(1H,dd,J=4.2&1.6Hz).

Reference Example 50:

(S)-7,8-Dihydro-7-(4-hydroxy-3-methylbutyl)-5-(4-methylphenyl)-8-oxo-N-(3,4,5-trimethoxybenzyl)-6-pyrido[3,4-b] pyridinecarboxamide

Starting from the compound as obtained in Reference Example 49 - Step 1 and THP-ether of (S)-4-amino-2-methyl-1-butanol, substantially the same reaction and work-up as Reference Example 19 was conducted to give the entitled compound as colorless crystals.
m.p. 194-195°C (recrystallized from acetone-ethyl ether)
NMR(200MHz, $CDCl_3$) ppm: same as the spectrum of the compound of Reference Example 49.

Reference Example 51:

(R)-N-(3,5-Dimethoxybenzyl)-7,8-Dihydro-7-(4-hydroxy-3-methylbutyl)-5-(4-methylphenyl)-8-oxo-6-pyrido[3,4-b]pyridinecarboxamide

(Step 1)

Using the compound as obtained in Reference Example 2 - Step 2 and 3,5-dimethoxybenzylamine, substantially the same reaction and work-up as Reference Example 2 - Step 4 was conducted to give N-(3,5-dimethoxybenzyl)-5-(4-methylphenyl)-8-oxo-8H-pyrano[3,4-b]pyridine-6-carboxamide as colorless crystals.
m.p. 154-155°C (recrystallized from ethyl acetate-isopropyl ether)
NMR(200MHz, $CDCl_3$) ppm: 2.45(3H,s), 3.78(6H,s), 4.41(2H,d,J=5.4Hz), 6.41(3H,m), 7.17(2H,d,J=8.0Hz), 7.23(1H, b), 7.33(2H,d,J=8.0Hz), 7.58(2H,m), 8.94(1H,dd,J=4.0&2.2Hz).

(Step 2)

Starting from the compound as obtained in Step 1 and THP-ether of (R)-4-amino-2-methyl-1-butanol, substantially the same reaction and work-up as Reference Example 19 was conducted to give the entitled compound as colorless crystals.
m.p. 169-172°C (recrystallized from acetone-isopropyl ether)
NMR(200MHz, $CDCl_3$) ppm: 0.85(3H,d,J=6.8Hz), 1.62(1H,m), 1.79(2H,m), 2.40(3H,s), 3.11(1H,b), 3.25-3.60(2H,m), 3.76(6H,s), 3.86(2H,m), 4.23(2H,d,J=5.6Hz), 6.25(2H,d,J=2.2Hz), 6.35(1H,t,J=2.2Hz), 7.15-7.35(4H,m), 7.30(1H,dd, J=8.4&4.2Hz), 7.44(1H,m), 7.56(1H,dd,J=8.4&1.6Hz), 8.65(1H,dd,J=4.2&1.6Hz).

The compounds as described in Reference Example 52 - 54 and 144 were obtained as pale yellow oily substances using 2-chloro-4-(4-methylphenyl)-3-pyridinecarboxylic acid [prepared from 2-cyano-3-methyl-3-(4-methylphenyl)propenoic acid ethyl ester by condensation with N,N-dimethylacetamide dimethyl acetal, followed by cyclization using hydrogen chloride and alkaline hydrolysis of the ester group: m.p. 205-208 °C (decomposed)] and N-substituted-N-(substituted)benzylamines {i.e.,N-benzyl-N-(2-hydroxyethyl)amine, N-[3,5-bis(trifluoromethyl)benzyl]-N-(2-hydroxyethyl)amine, N-benzyl-N-[(S)-3-hydroxy-2-methylpropyl]amine, and N-[3,5-bis(trifluoromethyl)benzyl]3-N-[(S)-3-hydroxy-2-methylpropyl]amine, respectively} by substantially the same reaction and work-up as Reference Example 12-Step 2. The physico-chemical data are described below.

Reference Example 52:

N-Benzyl-2-chloro-N-(2-hydroxyethyl)-4-(4-methylphenyl)-3-pyridinecarboxamide
NMR(200MHz, $CDCl_3$) ppm: 2.43(3Hx1/2,s), 2.46(3Hx1/2,s), 2.70-3.80(total 4H,m), 3.90(1Hx1/2,d,J=15.4Hz), 4.24 (1Hx1/2,d,J=15.4Hz), 4.51(1Hx1/2,d,J=15.2Hz), 4.94(1Hx1/2,d,J=15.2Hz), 6.74(1H,m), 6.97(1H,m), 7.10-7.55(8H,m), 8.40(1H,m) (a1 : 1 mixture of the amide rotamers).

Reference Example 53:

N-[3,5-Bis(trifluoromethyl)benzyl]-2-chloro-N-(2-hydroxyethyl)-4-(4-methylphenyl)-3-pyridinecarboxamide
NMR(200MHz, $CDCl_3$) ppm: 2.36(3Hx7/11,s), 2.44(3Hx4/11,s), 2.80-3.80(total 4H,m), 4.16(1Hx4/11,d,J=16.2Hz), 4.41 (1Hx4/11,d,J=16.2Hz), 4.77(1Hx7/11,d,J=15.0Hz), 4.90(1Hx7/11,d,J=15.0Hz), 7.10-7.50(6H,m), 7.76(2H,m), 8.42(1H, m) (a 7 : 4 mixture of the amide rotamers).

Reference Example 54:

N-Benzyl-2-chloro-N-[(S)-3-hydroxy-2-methylpropyl]4-(4-methylphenyl)3-pyridinecarboxamide
NMR(200MHz, $CDCl_3$) ppm: 0.59(3Hx1/4,d,J=7.0Hz), 0.66(3Hx1/4,d,J=7.0Hz), 0.77(3Hx1/4,d,J=3.8Hz), 0.80 (3Hx1/4,d,J=3.8Hz), 1.40-1.90(1H,m), 2.30-2.50(3H,m), 2.50-3.80(total 5H,m), 3.80-4.42(2Hx3/4,m), 5.05(2Hx1/4,m), 6.60-7.50(total 10H,m), 8.40(1H,m) (a 1 : 1 mixture of the amide rotamers).

Reference Example 55:

7-[3,5-Bis(trifluoromethyl)benzyl]-6,7,8,9-tetrahydro-5-(4-methylphenyl)-6,11-dioxo-11H-pyrazino[2,1-g][1,7]naphthyridine

A mixture of the compound (200 mg) as obtained in Reference Example 1, triethylamine (0.20 ml), methanesulfonyl chloride (0.10 ml) and dichloromethane (10 ml) was stirred at 0° C for 2 hours. Ethyl acetate was added to the reaction mixture, which was then washed with water and dried. The solvent was evaporated to give N-[3,5-bis(trifluoromethyl)benzyl]-7,8-dihydro-7-(2-methanesulfonyloxyethyl)-5-(4-methylphenyl)-8-oxo-6-pyrido[3,4-b]pyridinecarboxamide. This compound was dissolved in DMF (5 ml), and sodium hydride (60 % oily) (30 mg) was added thereto and stirred for 1.5 hours at room temperature. The reaction mixture was diluted with ethyl acetate, washed successively with water, diluted hydrochloric acid and water, and dried. After the solvent was removed by distillation, the entitled compound was obtained as colorless crystals (109 mg).
m.p. 270-271°C (recrystallized from ethyl acetate-ethyl ether)
NMR(200MHz, $CDCl_3$)ppm:2.46(3H,s), 3.67(2H,t like,J=5.4Hz), 4.51(2H,t like,J=5.4Hz), 4.81(2H,s), 7.13(2H,d, J=8.1Hz), 7.33(2H,d,J=8.1Hz), 7.52(1H,dd,J=8.4,4.4Hz), 7.64(1H,dd,J=8.4,1.6Hz), 7.70(2H,s), 7.84(1H,s), 8.97(1H, dd,J=4.4,1.6Hz)

| Elemental Analysis for $C_{27}H_{19}N_3O_2F_6$: | | | |
|---|---|---|---|
| Calcd.(%) | C, 61.02; | H, 3.60; | N, 7.91 |
| Found (%) | C, 61.07; | H, 3.50; | N, 7.85 |

Reference Example 56:

7-[3,5-Bis(trifluoromethyl)benzyl]-6,7,8,9,10,12-hexahydro-5-(4-methylphenyl)-6,12-dioxo[1,4]diazepino[2,1-g][1,7]naphthyridine

A mixture of N-[3,5-bis(trifluoromethyl)benzyl]-7-(3-chloropropyl)-7,8-dihydro-5-(4-methylphenyl)-8-oxo-6-pyrido[3,4-b]pyridinecarboxamide (66 mg) obtained in Reference Example 2, sodium hydride (60 % oily) (84 mg) and THF (3 ml) was stirred at room temperature for 14 hours. 2 N-HCl was added to the reaction mixture, which was then made basic with aqueous potassium carbonate and thereafter extracted with ethyl acetate. The extract was washed with water and dried, and the solvent was removed by distillation. Thus, the entitled compound was obtained as colorless crystals (35 mg).
m.p. 194-195°C (recrystallized from ethyl acetate-isopropyl ether)
NMR(200MHz, $CDCl_3$) ppm: 2.16(2H,m), 2.42(3H,s), 3.25-3.70(3H,m), 4.12(1H,d,J=15Hz), 5.34(1H,d,J=15Hz), 5.52 (1H,m), 6.93(1H,d,J=8.2Hz), 7.20(1H,d,J=8.2Hz), 7.30-7.45(2H,m), 7.51(1H,dd,J=8.4,4.4Hz), 7.62(2H,s), 7.70(1H,dd, J=8.4,1.6Hz), 7.84(1H,s), 8.93(1H,dd,J=4.4,1.6Hz)

| Elemental Analysis for $C_{28}H_{21}N_3O_2F_6$: | | | |
|---|---|---|---|
| Calcd.(%) | C, 61.65; | H, 3.88; | N, 7.70 |
| Found (%) | C, 61.29; | H, 4.06; | N, 7.61 |

Reference Example 57:

7-[3,5-Bis(trifluoromethyl)benzyl]-6,7,8,9,10,11-hexahydro-5-(4-methylphenyl)-6,13-dioxo-13H-[1,4]diazocino[2,1-g][1,7]naphthyridine

The compound as obtained in Reference Example 5 was reacted and treated in the same manner as in Reference Example 55 to obtain the entitled compound as colorless crystals.
m.p. 192-193°C (recrystallized from ethyl acetate-isopropyl ether)
NMR(200MHz, $CDCl_3$) ppm: 1.7-2.5(4H,m), 2.37(3H,s), 3.25(1H,m), 3.40-3.72(2H,m), 4.01(1H,d,J=15Hz), 5.13(1H, dd,J=14,5.4Hz), 5.46(1H,d,J=15Hz), 6.85(1H,d,J=7.9Hz), 7.05(1H,d,J=7.9Hz), 7.26(1H,d,J=7.8Hz), 7.34(1H,d, J=7.8Hz), 7.42-7.60(2H,m), 7.47(2H,s), 7.81(1H,s), 8.92(1H,m)

Reference Example 58:

6,7,8,9,10,12-Hexahydro-7-(2-methoxybenzyl)-5-(4-methylphenyl)-6,12-dioxo[1,4]diazepino[2,1-g][1,7]naphthyridine

The compound as obtained in Reference Example 6 was reacted and treated in the same manner as in Reference Example 55 to obtain the entitled compound as colorless crystals.
m.p. 264-266°C (recrystallized from ethyl acetate-ethyl ether)
NMR(200MHz, $CDCl_3$) ppm: 1.7-2.1(2H,m), 2.43(3H,s), 3.25-3.52(3H,m), 3.84(3H,s), 4.67(2H,s), 5.39(1H,dd, J=14,5.8Hz), 6.85-7.00(3H,m), 7.10-7.22(2H,m), 7.22-7.44(3H,m), 7.48(1H,dd,J=8.4,4.4Hz), 7.72(1H,dd, J=8.4,1.4Hz), 8.90(1H,dd,J=4.4,1.4Hz)

Reference Example 59:

6,7,8,9,10,11-Hexahydro-7-(2-methoxybenzyl)-5-(4-methylphenyl)-6,13-dioxo-13H-[1,4]diazocino[2,1-g][1,7] naphthyridine

The compound as obtained in Reference Example 7 was reacted and treated in the same manner as in Reference Example 55 to obtain the entitled compound as colorless crystals.
m.p. 235-235°C (recrystallized from ethyl acetate)
NMR(200MHz, $CDCl_3$) ppm: 1.6-2.3(4H,m), 2.46(3H,s), 3.15-3.30(1H,m), 3.38-3.65(2H,m), 3.80(3H,s), 4.24(1H,d, J=15Hz), 5.04(1H,d,J=15Hz), 5.13(1H,dd,J=15,6.4Hz), 6.25(1H,dd,J=7.6,1.4Hz), 6.63(1H,dt,$J_d$=0.5Hz,$J_t$=7.6Hz), 6.82(1H,d,J=7.4Hz), 6.96(1H,dd,J=7.6,2.0Hz), 7.11-7.34(3H,m), 7.38-7.47(1H,m), 7.47(1H,dd,J=8.3,4.3Hz), 7.62(1H, dd,J=8.3,1.7Hz), 8.90(1H,dd,J=4.3,1.7Hz)

Reference Example 60:

7-[3,5-Bis(trifluoromethyl)benzyl]-6,7,8,9,10,11,12,14-octahydro-5-(4-methylphenyl)-6,14-dioxo[1,4]diazonino[2,1-g] [1,7]naphthyridine

The compound as obtained in Reference Example 8 was reacted and treated in the same manner as in Reference Example 55 to obtain the entitled compound as colorless crystals.
m.p. 177-179°C (recrystallized from ethyl acetate-isopropyl ether)
NMR(200MHz, $CDCl_3$) ppm: 1.45-1.95(4H,m), 2.10(2H,m), 2.33(3H,s), 3.06-3.24(1H,m), 3.32-3.56(2H,m), 3.86(1H,d, J=15Hz), 4.95(1H,dt,$J_d$=15Hz,$J_t$=4.8Hz), 5.38(1H,d,J=15Hz), 6.86(1H,dd,J=8.0,1.5Hz), 7.00(1H,d,J=8.0Hz), 7.17(1H, d,J=8.2Hz), 7.29(1H,dd,J=8.2,1.5Hz), 7.40-7.54(2H,m), 7.44(2H,s), 7.79(1H,s), 8.89(1H,dd,J=3.8,2.0Hz)

Reference Example 61:

7-[3,5-Bis(trifluoromethyl)benzyl]-6,7,8,9,10,12-hexahydro-6,12-dioxo-5-phenyl[1,4]diazepino[2,1-g][1,7] naphthyridine

The compound as obtained in Reference Example 9 was reacted and treated in the same manner as in Reference Example 55 to obtain the entitled compound as colorless crystals.
m.p. 244-245°C (recrystallized from ethyl acetate-THF-ethyl)
NMR(200MHz, $CDCl_3$) ppm: 2.00-2.25(2H,m), 3.25-3.70(3H,m), 4.15(1H,d,J=15Hz), 5.30(1H,d,J=15Hz), 5.52(1H,m), 7.05(1H,d,J=7.4Hz), 7.3-7.7(6H,m), 7.62(2H,s), 7.84(1H,s), 8.93(1H,dd,J=4.2,1.6Hz)

Reference Example 62:

7-[3,5-Bis(trifluoromethyl)benzyl]-6,7,8,9,10,11-hexahydro-6,13-dioxo-5-phenyl-13H-[1,4]diazocino[2,1-g][1,7] naphthyridine

The compound as obtained in Reference Example 10 was reacted and treated in the same manner as in Reference Example 55 to obtain the entitled compound as colorless crystals.
m.p. 205-206°C (recrystallized from ethyl acetate-ethyl ether)
NMR(200MHz, $CDCl_3$) ppm: 1.70-2.35(4H,m), 3.18-3.36(1H,m), 3.4-3.7(2H,m), 3.98(1H,d,J=15Hz), 5.14(1H,dd, J=14,5.8Hz), 5.43(1H,d,J=15Hz), 6.94(1H,d,J=7.3Hz), 7.19(1H,t,J=7.3Hz), 7.3-7.6(5H,m), 7.44(2H,s), 7.79(1H,s), 8.91(1H,dd,J=4.0,1.8Hz)

Reference Example 63:

7-[3,5-Bis(trifluoromethyl)benzyl]-6,7,8,9,10,11-hexahydro-5-(4-methylphenyl)-6,13-dioxo-13H-[1,4]diazocino[1,2-b][2,7]naphthyridine

The compound as obtained in Reference Example 11 was reacted and treated in the same manner as in Reference Example 55 to obtain the entitled compound as colorless crystals.
m.p. 231-233°C (recrystallized from THF-isopropyl ether)
NMR(200MHz, $CDCl_3$) ppm: 1.7-2.3(4H,m), 2.37(3H,s), 3.2-3.7(3H,m), 4.00(1H,d.J=15Hz), 5.05(1H,dd,J=15,6.2Hz), 5.44(1H,d,J=15Hz), 6.83(1H,dd,J=7.8,1.6Hz), 6.98(1H,d,J=5.4Hz), 7.04(1H,d,J=7.8Hz), 7.25(1H,d,J=7.8Hz), 7.33 (1H,dd,J=7.8,1.6Hz), 7.46(2H,s), 7.81(1H,s), 8.64(1H,d,J=5.4Hz), 9.68(1H,s)

Reference Example 64:

7-[3,5-Bis(trifluoromethyl)benzyl]-1,2,3,4,6,7,8,9,10,11-decahydro-2-methyl-5-(4-methylphenyl)-6,13-dioxo-13H-[1,4]diazocino[1,2-b][2,7]naphthyridine

A mixture of the compound (250 mg) as obtained in Reference Example 63, iodomethane (3 ml) and ethyl acetate (6 ml) was heated under reflux for 1.5 hours. After the solvent was removed by distillation, the residue was dissolved in methanol (15 ml). Sodium borohydride (50 mg) was added to the resulting solution at 0°C with stirring, and the mixture was then further stirred at 0°C for one hour and thereafter concentrated. Ethyl acetate was added to the resulting concentrate, which was then washed with water and dried. Then, the solvent was removed by distillation. The residue was dissolved in methanol (15 ml), and 10 % palladium-carbon (50 % hydrous) (100 mg) was added thereto and stirred in a hydrogen atmosphere at room temperature for 3 hours. The catalyst was removed by filtration, and the solvent was removed from the filtrate by distillation. The residue was subjected to column chromatography (ethyl acetate ® ethyl acetate:methanol = 4:1) using silica gel, and the entitled compound was obtained as pale yellow crystals (150 mg).
m.p. 233-235°C (recrystallized from THF-ethyl acetate-isopropyl ether)
NMR(200MHz, $CDCl_3$) ppm: 1.7-2.6(8H,m), 2.31(3H,s), 2.47(3H,s), 3.1-3.8(5H,m), 3.95(1H,d,J=15Hz), 4.93(1H,dd, J=14,6.2Hz), 5.41(1H,d,J=15Hz), 6.72(1H,d,J=7.8Hz), 6.98(1H,d,J=7.8Hz), 7.19(2H,s), 7.42(2H,s), 7.78(1H,s)

Reference Example 65:

4-[3,5-Bis(trifluoromethyl)benzyl]-2,3,4,5-tetrahydro-5-oxo-6-phenylpyrido[3,2-f][1,4]oxazepine

Sodium hydride (60 % oily) (60 mg) was added to a THF (15 ml) solution of N-[3,5-bis(trifluoromethyl)benzyl]-2-chloro-N-(2-hydroxyeth yl)-4-phenyl-3-pyridinecarboxamide (Reference Example 12) (348 mg) and the mixture was stirred for 2 hours while heating under reflux. Ethyl acetate was added to the reaction mixture, which was then washed with water and dried. After the solvent was removed by distillation, the entitled compound was obtained as colorless crystals (278 mg).
m.p. 200-201°C (recrystallized from ethanol-hexane)
NMR(200MHz, $CDCl_3$) ppm: 3.70(2H,t,J=5.8Hz), 4.47(2H,t,J=5.8Hz), 4.88(2H,s), 7.24(1H,d,J=5.2Hz), 7.25-7.55(5H, m), 7.80(2H,s), 7.86(1H,s), 8.44(1H,d,J=5.2Hz)
EI-MS m/z: 466 (M+) $[(C_{23}H_{16}N_2O_2F_6)^+]$

Reference Example 66

(9R)-7-[3,5-Bis(trifluoromethyl)benzyl]-6,7,8,9,10,12-hexahydro-9-methyl-5-(4-methylphenyl)-6,12-dioxo[1,4]diazepino[2,1-g][1,7]naphthyridine

A mixture of the compound (700 mg) as obtained in Reference Example 13, triethylamine (0.41 ml), methanesulfonyl chloride (0.224 ml) and THF (15 ml) was stirred at room temperature for 30 minutes, and a saturated aqueous sodium hydrogencarbonate solution (15 ml) was added thereto and again stirred for 30 minutes at room temperature. The reaction mixture was extracted with ethyl acetate, the extract was washed with diluted hydrochloric acid and a saturated aqueous sodium chloride solution and dried, and the solvent was removed by distillation. The residue was dissolved in THF (15 ml), and then sodium hydride (60 % oily) (76 mg) was added thereto and stirred at room temperature for 1.5 hours. The reaction mixture was diluted with ethyl acetate, washed with diluted hydrochloric acid, aqueous sodium carbonate and a saturated aqueous sodium chloride solution and then dried, and the solvent

was removed by distillation. The residue was subjected to column chromatography (ethyl acetate:methanol = 9:1) using silica gel, and the entitled compound was obtained as colorless crystals (408 mg).
m.p. 179-180°C (recrystallized from ethyl acetate-isopropyl ether)
NMR(200MHz, $CDCl_3$) ppm: 1.05(2Hx2/3,d,J=7.0Hz), 1.22(3Hx1/3,d,J=7.0Hz), 2.39(3Hx1/3,s), 2.42(3Hx2/3,s), 2.52 (1H,m), 3.0-3.3(2H,m), 3.48(1Hx2/3,dd,J=14,4.6Hz), 3.71(1Hx1/3,dd,J=16,5.2Hz), 4.06(1Hx1/3,d,J=15Hz), 4.12 (1Hx2/3,d,J=15Hz), 5.28-5.65(2H,m), 6.83(1Hx1/3,d,J=7.4Hz), 6.96(1Hx2/3,d,J=7.6Hz), 7.09(1Hx1/3,d,J=7.4Hz), 7.20(1Hx2/3,d,J=7.6Hz), 7.35(2H,m), 7.42-7.75(4H,m), 7.83(1H,s), 8.92(1H,d,J=3.6Hz)

| Elemental Analysis for $C_{29}H_{23}N_3O_2F_6$: | | | |
|---|---|---|---|
| Calcd.(%) | C, 62.25; | H, 4.14; | N, 7.51 |
| Found (%) | C, 62.00; | H, 4.08; | N, 7.24 |

$[a]_D$ : -60.2° (c=0.348, MeOH)

Reference Example 67:

(9S)-7-[3,5-Bis(trifluoromethyl)benzyl]-6,7,8,9,10,12-hexahydro-9-methyl-6,12-dioxo-5-phenyl[1,4]diazepino[2,1-g][1,7]naphthyridine

The compound as obtained in Reference Example 14 was reacted and treated in the same manner as in Reference Example 66 to obtain the entitled compound as colorless crystals.
m.p. 150-152°C (recrystallized from ethyl acetate-isopropyl ether)
NMR(200MHz, $CDCl_3$) ppm: 1.06(3Hx2/3,d,J=7.0Hz), 1.21(3Hx1/3,d,J=7.0Hz), 2.50(1H,m), 3.05-3.30(2H,m), 3.49 (1Hx2/3,dd,J=14,4.6Hz), 3.72(1Hx1/3,dd,J=16,5.4Hz), 4.07(1Hx1/3,d,J=15Hz), 4.14(1Hx2/3,d,J=15Hz), 5.25-5.62 (2H,m), 6.94(1Hx1/3,d,J=7.6Hz), 7.08(1Hx2/3,d,J=7.4Hz), 7.2-7.7(8H,m), 7.83(1H,s), 8.93(1H,dd,J=4.3,1.7Hz)

| Elemental Analysis for $C_{28}H_{21}N_3O_2F_6$: | | | |
|---|---|---|---|
| Calcd.(%) | C, 61.65; | H, 3.88; | N, 7.70 |
| Found (%) | C, 61.33; | H, 3.89; | N, 7.51 |

$[a]_D$ : +69.8° (c=0.353, MeOH)

Reference Example 68:

(9S)-7-[3,5-Bis(trifluoromethyl)benzyl]-6,7,8,9,10,12-hexahydro-9-methyl-5-(4-methylphenyl)-6,12-dioxo[1,4]diazepino[2,1-g][1,7]naphthyridine

The compound as obtained in Reference Example 15 was reacted and treated in the same manner as in Reference Example 66 to obtain the entitled compound as colorless crystals.
m.p. 179-180°C (recrystallized from ethyl acetate-isopropyl ether)
NMR (200 MHz, $CDCl_3$) ppm: Same as the spectrum of the compound of Reference Example 66

| Elemental Analysis for $C_{29}H_{23}N_3O_2F_6$: | | | |
|---|---|---|---|
| Calcd.(%) | C, 62.25; | H, 4.14; | N, 7.51 |
| Found (%) | C, 61.94; | H, 4.16; | N, 7.24 |

$[a]_D$ : +58.2° (c=0.353, MeOH)

Reference Example 69:

(+/-)-7-[3,5-Bis(trifluoromethyl)benzyl]-6,7,8,9,10,11-hexahydro-9-methyl-6,13-dioxo-5-phenyl-13H-[1,4]diazocino[2,1-g][1,7]naphthyridine

Methanesulfonyl chloride (0.29 ml) was added to a THF (15 ml) solution of the compound (830 mg) as obtained in Reference Example 16 and triethylamine (0.56 ml) with stirring and cooling with ice. The resulting mixture was stirred

for 50 minutes, while still cooling with ice, and then a saturated aqueous sodium hydrogencarbonate solution (15 ml) was added thereto and again stirred for 40 minutes at room temperature. The reaction mixture was extracted with ethyl acetate. The extract was washed with diluted hydrochloric acid and a saturated aqueous sodium chloride solution and dried, and then the solvent was removed by distillation. The residue was dissolved in THF (25 ml), and sodium hydride (60 % oily) (90 mg) was added thereto and stirred for one hour with heating under reflux. The reaction mixture was diluted with ethyl acetate, washed with diluted hydrochloric acid, aqueous sodium carbonate and a saturated sodium chloride solution, and then dried. After the solvent was removed by distillation, the entitled compound was obtained as colorless crystals (460 mg).
m.p. 257-258°C (recrystallized from ethyl acetate-ethyl ether)
NMR(200MHz, $CDCl_3$) ppm: 0.92(3H,d,J=6.6Hz), 1.73(1H,m), 1.95-2.40(2H,m), 2.98(1H,d,J=15Hz), 3.30-3.65(2H,m), 3.97(1H,d,J=15Hz), 5.11(1H,dd,J=14,5.9Hz), 5.43(1H,d,J=15Hz), 6.93(1H,d,J=7.6Hz), 7.19(1H,dd,J=7.6,7.0Hz), 7.3-7.6(7H,m), 7.81(1H,s), 8.91(1H,dd,J=4.0,2.0Hz)

| Elemental Analysis for $C_{29}H_{23}N_3O_2F_6$: | | | |
|---|---|---|---|
| Calcd.(%) | C, 62.25; | H, 4.14; | N, 7.51 |
| Found (%) | C, 61.93; | H, 4.05; | N, 7.57 |

Reference Example 70:

(+/-)-7-[3,5-Bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine

The compound as obtained in Reference Example 17 was reacted and treated in the same manner as in Reference Example 69 to obtain the entitled compound as colorless crystals.
m.p. 280-281°C (recrystallized from ethyl acetate-THF-isopropyl ether)
NMR(200MHz, $CDCl_3$) ppm: 0.91(3H,d,J=6.8Hz), 1.73(1H,m), 1.95-2.40(2H,m), 2.37(3H,s), 2.97(1H,d,J=15Hz), 3.35-3.62(2H,m), 3.99(1H,d,J=15Hz), 5.10(1H,dd,J=14,5.3Hz), 5.46(1H,d,J=15Hz), 6.83(1H,dd,J=7.8,1.6Hz), 7.05(1H,d,J=7.8Hz), 7.25(1H,d,J=7.8Hz), 7.34(1H,dd,J=7.8,1.6Hz), 7.46(1H,dd,J=8.4,4.2Hz), 7.47(2H,s), 7.55(1H,dd, J=8.4,1.8Hz), 7.81(1H,s), 8.91(1H,dd,J=4.2,1.8Hz)

| Elemental Analysis for $C_{30}H_{25}N_3O_2F_6$: | | | |
|---|---|---|---|
| Calcd.(%) | C, 62.83; | H, 4.39; | N, 7.33 |
| Found (%) | C, 62.61; | H, 4.21; | N, 7.12. |

Reference Example 71:

(9R)-7-[3,5-Bis(trifluoromethyl)benzyl]-6,7,8,9,10,11-hexahydro-9-methyl-6,13-dioxo-5-phenyl-13H-[1,4]diazocino[2,1-g] [1,7]naphthyridine

The compound as obtained in Reference Example 18 was reacted and treated in the same manner as in Reference Example 69 to obtain the entitled compound as colorless crystals.
m.p. 245-247°C (recrystallized from ethyl acetate-isopropyl ether)
NMR (200 MHz, $CDCl_3$) ppm: Same as the spectrum of the compound of Reference Example 69
$[a]_D$ : +133.8° (c=0.51 , MeOH)

| Elemental Analysis for $C_{29}H_{23}N_3O_2F_6$: | | | |
|---|---|---|---|
| Calcd.(%) | C, 62.25; | H, 4.14; | N, 7.51 |
| Found (%) | C, 62.13; | H, 4.13; | N, 7.40 |

Reference Example 72:

(9R)-7-[3,5-Bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine

The compound as obtained in Reference Example 19 was reacted and treated in the same manner as in

Reference Example 69 to obtain the entitled compound as colorless crystals.
m.p. 226-228°C (recrystallized from ethyl acetate-isopropyl ether)
NMR (200 MHz, $CDCl_3$) ppm: Same as the spectrum of the compound of Reference Example 70
$[a]_D$ : +109.4° (c=0.541, MeOH).

| Elemental Analysis for $C_{30}H_{25}N_3O_2F_6$: | | | |
|---|---|---|---|
| Calcd.(%) | C, 62.83; | H, 4.39; | N, 7.33 |
| Found (%) | C, 62.55; | H, 4.56; | N, 7.10 |

Reference Example 73:

(9S)-7-[3,5-Bis(trifluoromethyl)benzyl]-6,7,8,9,10,11-hexahydro-9-methyl-6,13-dioxo-5-phenyl-13H-[1,4]diazocino [2,1-g] [1,7]naphthyridine

The compound as obtained in Reference Example 20 was reacted and treated in the same manner as in Reference Example 69 to obtain the entitled compound as colorless crystals.
m.p. 242-244°C (recrystallized from ethyl acetate-isopropyl ether)
NMR (200 MHz, $CDCl_3$) ppm: Same as the spectrum of the compound of Reference Example 69
$[a]_D$ : -130.4° (c=0.496, MeOH).

| Elemental Analysis for $C_{29}H_{23}N_3O_2F_6$: | | | |
|---|---|---|---|
| Calcd.(%) | C, 62.25; | H, 4.14; | N, 7.51 |
| Found (%) | C, 62.07; | H, 4.15; | N, 7.36 |

Reference Example 74:

(9S)-7-[3,5-Bis(trifluoromethyl)benzyl]-6,7,8,9,10,11-hexahydro-9-methyl-5-(4-methylphenyl)-6,13-dioxo-13H-[1,4] diazocino[2,1-g][1,7]naphthyridine

The compound as obtained in Reference Example 21 was reacted and treated in the same manner as in Reference Example 69 to obtain the entitled compound as colorless crystals.
m.p. 227-228°C (recrystallized from ethyl acetate-isopropyl ether)
NMR (200 MHz, $CDCl_3$) ppm: Same as the spectrum of the compound of Reference Example 70
$[a]_D$ : -107.1° (c=0.521, MeOH).

| Elemental Analysis for $C_{30}H_{25}N_3O_2F_6$: | | | |
|---|---|---|---|
| Calcd.(%) | C, 62.83; | H, 4.39; | N, 7.33 |
| Found (%) | C, 62.55; | H, 4.40; | N, 7.13 |

Reference Example 75:

4-[3,5-Bis(trifluoromethyl)benzyl]-2,3,4,5-tetrahydro-5-oxo-6-phenyl-lH-pyrido[2,3-e][1,4]diazepine

A mixture of the compound (370 mg) as obtained in Reference Example 22, anhydrous potassium carbonate (200 mg) and xylene (10 ml) was stirred for 9 hours with heating under reflux. After the reaction mixture was cooled, water was added thereto. Then, the mixture was extracted with ethyl acetate. The extract was washed with water and dried, and the solvent was removed by distillation. Thus, the entitled compound was obtained as colorless crystals.
m.p. 242-243°C (recrystallized from ethyl acetate-isopropyl ether)
NMR(200MHz, $CDCl_3$) ppm: 3.60-3.80(4H,m), 4.81(2H,s), 4.86(1H,s), 6.87(1H,d,J=5.2Hz), 7.30-7.50(6H,m), 7.79(2H, s), 7.85(1H,s), 8.21(1H,d,J=5.2Hz)

| Elemental Analysis for $C_{23}H_{17}N_3OF_6$: | | | |
|---|---|---|---|
| Calcd.(%) | C, 59.36; | H, 3.68; | N, 9.03 |
| Found (%) | C, 59.24; | H, 3.66; | N, 9.06 |

EI-MS m/z: 465 ($M^+$) [$(C_{23}H_{17}N_3OF_6)^+$]

Reference Example 76:

5-[3,5-Bis(trifluoromethyl)benzyl]-2,3,4,5-tetrahydro-6-oxo-7-phenyl-6H-pyrido[2,3-b][1,5]oxazocine

The compound as obtained in Reference Example 23 was reacted and treated in the same manner as in Reference Example 65 to obtain the entitled compound as colorless crystals.
m.p. 188-189°C (recrystallized from ethyl acetate-isopropyl ether)
NMR(200MHz, $CDCl_3$) ppm: 1.65-1.88(1H,m), 2.18-2.45(1H,m), 3.36(1H,dd,J=15.2Hz), 3.73(1H,m), 4.17(1H,d, J=15.2Hz), 4.32(1H,dt,J=12.6,3.6Hz), 4.67(1H,ddd,J=12.6,5.6,2.4Hz), 5.50(1H,d,J=15.2Hz), 7.16(1H,d,J=5.2Hz), 7.20-7.45(5H,m), 7.71(2H,s), 7.83(1H,s), 8.41(1H,d,J=5.2Hz)

| Elemental Analysis for $C_{24}H_{18}N_2O_2F_6$: | | | |
|---|---|---|---|
| Calcd.(%) | C, 60.00; | H, 3.78; | N, 5.83 |
| Found (%) | C, 59.92; | H, 3.76; | N, 5.89 |

Reference Example 77:

4-[3,5-Bis(trifluoromethyl)benzyl]-2,3,4,5-tetrahydro-7-methyl-5-oxo-6-phenylpyrido[3,2-f][1,4]oxazepine

The compound as obtained in Reference Example 24 was reacted and treated in the same manner as in Reference Example 65 to obtain the entitled compound as colorless crystals.
m.p. 179-181°C (recrystallized from ethyl acetate-isopropyl ether)
NMR(200MHz, $CDCl_3$) ppm: 2.13(3H,s), 3.57(2H,t,J=5.8Hz), 4.42(2H,t,J=5.8Hz), 4.80(2H,s), 7.16(2H,m), 7.47(3H,m), 7.65(2H,s), 7.81(1H,s), 8.32(1H,s)

Reference Example 78:

5-[3,5-Bis(trifluoromethyl)benzyl]-2,3,4,5-tetrahydro-8-methyl-6-oxo-7-phenyl-6H-pyrido[2,3-b][1,5]oxazocine

The compound as obtained in Reference Example 25 was reacted and treated in the same manner as in Reference Example 65 to obtain the entitled compound as colorless crystals.
m.p. 180-182°C (recrystallized from ethyl acetate-isopropyl ether)
NMR(200MHz, $CDCl_3$) ppm: 1.71(1H,m), 2.07(3H,m), 2.28(1H,m), 3.24(1H,dd,J=15.2,3.8Hz), 3.64(1H,dd, J=15.2,12.0Hz), 4.05(1H,d,J=15.6Hz), 4.27(1H,dt,J=12.6,3.8Hz), 4.63(1H,ddd,J=12.6,5.4,2.0Hz), 5.45(1H,d, J=15.6Hz), 7.38(5H,m), 7.54(2H,s), 7.78(1H,s), 8.29(1H,s)

Reference Example 79:

(+/-)-7-[3,5-Bis(trifluoromethyl)benzyl]-6,7,8,9,10,12-hexahydro-9-hydroxy-5-(4-methylphenyl)-6,12-dioxo[1,4] diazepino [2,1-g][1,7]naphthyridine

The compound as obtained in Reference Example 26 was reacted and treated in the same manner as in Reference Example 56 to obtain the entitled compound as colorless crystals.
m.p. 282-283°C (recrystallized from acetone-ethyl ether) NMR(200MHz, $CDCl_3$) ppm: 2.43(3H,s), 3.35-3.63(3H,m), 4.02(1Hx3/8,d,J=3.5Hz,-OH), 4.21(1Hx3/8,d,J=15Hz), 4.30(1Hx5/8,d,J=3.5Hz,-OH), 4.38(1Hx5/8,d,J=15Hz), 4.60 (1H,m), 5.24(1Hx3/8,d,J=15Hz), 5.61(1Hx5/8,d,J=15Hz), 5.68(1H,m), 6.92(1H,t-like,J=3.8Hz), 7.19-7.86(8H,m), 8.95 (1H,d,J=4Hz)

| Elemental Analysis for $C_{28}H_{21}N_3O_3F_6 \cdot 1/4H_2O$: | | | |
|---|---|---|---|
| Calcd.(%) | C, 59.42; | H, 3.83; | N, 7.42 |
| Found (%) | C, 59.45; | H, 3.74; | N, 7.39 |

EI-MS m/z: 561 ($M^+$) [$(C_{28}H_{21}N_3O_3F_6)^+$]

Reference Example 80:

7-Benzyl-6,7,8,9,10,12-hexahydro-6,12-dioxo-5-phenyl[1,4]diazepino[2,1-g][1,7]naphthyridine

7-Benzyl-7,8-dihydro-7-(3-hydroxypropyl)-8-oxo-5-phenyl-6-pyrido[3,4-b]pyridinecarboxamide (this was obtained by reacting the compound as obtained in Reference Example 27 with 3-amino-1-propanol and treated in the same manner as in Reference Example 13) was reacted and treated in the same manner as in Reference Example 12 to obtain the entitled compound as colorless crystals.
m.p. 210-212°C (recrystallized from ethyl acetate-ethyl ether)
NMR(200MHz, $CDCl_3$) ppm: 1.7-2.2(2H,m), 3.2-3.6(3H,m), 4.30(1H,d,J=14Hz), 4.89(1H,d,J=14Hz), 5.43(1H,dd, J=14,5.7Hz), 7.0-7.7(11H,m), 7.70(1H,dd,J=8,4,1.6Hz), 8.92(1H,dd,J=4.4,1.6Hz)

Reference Example 81:

7-Benzyl-6,7,8,9,10,11-hexahydro-6,13-dioxo-5-phenyl-13H-[1,4]diazocino[2,1-g][1,7]naphthyridine

7-Benzyl-7,8-dihydro-7-(5-hydroxybutyl)-8-oxo-5-phenyl-6-pyrido[3,4-b]pyridinecarboxamide (this was obtained by reacting the compound as obtained in Reference Example 27 with 4-amino-1-butanol and treated in the same manner as in Reference Example 16) was reacted and treated in the same manner as in Reference Example 69 to obtain the entitled compound as colorless crystals.
m.p. 243-244°C (recrystallized from acetone-ethyl ether)
NMR(200MHz, $CDCl_3$) ppm: 1.6-2.3(4H,m), 3.15(1H,m), 3.35-3.65(2H,m), 3,76(1H,d,J=15Hz), 5.15(1H,dd, J=14,5,7Hz), 5.42(1H,d,J=15Hz), 6.64(2H,d,J=6.2Hz), 7.0-7.3(4H,m), 7.3-7.7(6H,m), 8.91(1H,dd,J=4.2,1.8Hz)

Reference Example 82:

7-Benzyl-6,7,8,9,10,11,12,14-octahydro-6,14-dioxo-5-phenyl-[1,4]diazonino[2,1-g][1,7]naphthyridine

7-Benzyl-7,8-dihydro-7-(5-hydroxypentyl)-8-oxo-5-phenyl-6-pyrido[3,4-b]pyridinecarboxamide (this was obtained by reacting the compound as obtained in Reference Example 27 with 5-amino-1-pentanol and treated in the same manner as in Reference Example 16) was reacted and treated in the same manner as in Reference Example 69 to obtain the entitled compound as colorless crystals.
m.p. 224-226°C (recrystallized from ethyl acetate-ethyl ether)
NMR(200MHz, $CDCl_3$) ppm: 1.3-1.9(4H,m), 2.09(2H,m), 2.85-3.05(1H,m), 3.15-3.40(1H,m), 3.50(1H,dt,$J_d$=15Hz, $J_t$=6.4Hz). 3.64(1H,d,J=15Hz), 4.97(1H,dt,$J_d$=15Hz,$J_t$=4.8Hz), 5.48(1H,d,J=15Hz), 6.43(2H,d,J=7.2Hz), 7.05-7.25 (4H,m), 7.3-7.7(6H,m), 8.91(1H,dd,J=4.2,1.8Hz)

Reference Example 83:

7-(3,4-Dichlorbenzyl)-6,7,8,9,10,12-hexahydro-6,12-dioxo-5-phenyl[1,4]diazepino[2,1-g][1,7]naphthyridine

7-(3,4-Dichlorobenzyl)-7,8-dihydro-7-(3-hydroxypropyl)-8-oxo-5-phenyl-6-pyrido[3,4-b]pyridinecarboxamide (this was obtained by reacting the compound as obtained in Reference Example 28 with 3-amino-1-propanol and treated in the same manner as in Reference Example 13) was reacted and treated in the same manner as in Reference Example 66 to obtain the entitled compound as colorless crystals.
m.p. 224-226°C (recrystallized from ethyl acetate-ethyl ether)
NMR(200MHz, $CDCl_3$) ppm: 1.9-2.3(2H,m), 3.2-3.6(3H,m), 4.01(1H,d,J=15Hz), 5.05(1H,d,J=15Hz), 5.49(1H,dd, J=13,5.0Hz), 6.9-7.1(2H,m), 7.25(1H,m), 7.38(1H,d,J=8.6Hz), 7.3-7.8(6H,m), 8.93(1H,d,J=4.0Hz)

Reference Example 84:

7-(3,4-Dichlorbenzyl)-6,7,8,9,10,11-hexahydro-6,13-dioxo-5-phenyl-13H-[1,4]diazocino[2,1-g][1,7]naphthyridine

7-(3,4-Dichlorobenzyl)-7,8-dihydro-7-(5-hydroxybutyl)-8-oxo-5-phenyl-6-pyrido[3,4-b]pyridinecarboxamide (this was obtained by reacting the compound as obtained in Reference Example 28 with 4-amino-1-butanol and treated in the same manner as in Reference Example 16) was reacted and treated in the same manner as in Reference Example 15 to obtain the entitled compound as colorless crystals.
m.p. 236-238°C (recrystallized from acetone-ethyl ether)

NMR(200MHz, $CDCl_3$) ppm: 1.7-2.3(4H,m), 3.14(1H,m), 3.39-3.60(2H,m), 3.70(1H,d,J=15Hz), 5.14(1H,dd, J=15,5.9Hz), 5.35(1H,d,J=15Hz), 6.35(1H,dd,J=8.4,2.0Hz), 7.02(2H,m), 7.18(1H,d,J=8.4Hz), 7.3-7.6(6H,m), 8.91(1H, dd,J=4.0,1.8Hz)

Reference Example 85:

(S)-5-[3,5-Bis(trifluoromethyl)benzyl]-2,3,4,5-tetrahydro-3,8-dimethyl-6-oxo-7-phenyl-6H-pyrido[2,3-b][1,5]oxazocine

The compound as obtained in Reference Example 29 was reacted and treated in the same manner as in Reference Example 65 to obtain the entitled compound as colorless crystals.
m.p. 147-148° C (recrystallized from ethyl acetate-hexane)
NMR(200MHz, $CDCl_3$) ppm: 0.83(3H,d,J=7.4Hz), 2.07(3H,s), 2.39(1H,m), 2.97(1H,d,J=15.4Hz), 3.48(1H,m), 3.87(1H, dd,J=10.4,12.4Hz), 4.06(1H,d,J=15.6Hz), 4.59(1H,dd,J=5.2,12.4Hz),5.44(1H,d,J=15.4Hz),7.37(2H,s), 7.53(2H,s), 7.78(1H,s), 8.29(1H,s)

| Elemental Analysis for $C_{26}H_{22}N_2O_2F_6$: | | | |
|---|---|---|---|
| Calcd.(%) | C 61.42, | H 4.36, | N 5.51 |
| Found (%) | C 61.30, | H 4.52, | N 5.70 |

$[a]_D^{20}$: -106.8° (C = 0.257, $CHCl_3$)

Reference Example 86:

(R)-5-[3,5-Bis(trifluoromethyl)benzyl]-2,3,4,5-tetrahydro-3,8-dimethyl-6-oxo-7-phenyl-6H-pyrido[2,3-b][1,5]oxazocine

The compound as obtained in Reference Example 30 was reacted and treated in the same manner as in Reference Example 65 to obtain the entitled compound as colorless crystals.
m.p. 147-149° C (recrystallized from ethyl acetate-hexane)
NMR(200MHz, $CDCl_3$) ppm: Same as the spectrum of the compound of Reference Example 85

| Elemental Analysis for $C_{26}H_{22}N_2O_2F_6$: | | | |
|---|---|---|---|
| Calcd.(%) | C 61.42, | H 4.36, | N 5.51 |
| Found (%) | C 61.26, | H 4.33, | N 5.69 |

$[a]_D^{20}$: +102.5° (C = 0.573, $CHCl_3$)

Reference Example 87:

4-[3,5-Bis(trifluoromethyl)benzyl]-2,3,4,5-tetrahydro-8-methyl-5-oxo-6-phenylpyrido[3,2-f][1,4]oxazepine

The compound as obtained in Reference Example 31 was reacted and treated in the same manner as in Reference Example 65 to obtain the entitled compound as colorless crystals.
m.p. 151-153°C (recrystallized from ethyl acetate-isopropyl ether)
NMR(200MHz, $CDCl_3$) ppm: 2.58(3H,s), 3.69(2H,t,J=5.4Hz), 4.47(2H,d,J=5.4Hz), 4.87(2H,s), 7.11(1H,s), 7.17-7.56 (5H,m), 7.80(2H,s), 7.86(1H,s)

| Elemental Analysis for $C_{24}H_{18}N_2O_2F_6 \cdot 1/4\ H_2O$: | | | |
|---|---|---|---|
| Calcd.(%) | C 59.44, | H 3.85, | N 5.78 |
| Found (%) | C 59.42, | H 3.82, | N 5.84 |

Reference Example 88:

5-[3,5-Bis(trifluoromethyl)benzyl]-2,3,4,5-tetrahydro-9-methyl-6-oxo-7-phenyl-6H-pyrido[2,3-b][1,5]oxazocine

The compound as obtained in Reference Example 32 was reacted and treated in the same manner as in

Reference Example 65 to obtain the entitled compound as colorless crystals.
m.p. 164-165°C (recrystallized from ethyl acetate-isopropyl ether)
NMR(200MHz, $CDCl_3$) ppm: 1.79(1H,m), 2.30(1H,m), 2.56(3H,s), 3.35(1H,m), 3.77(1H,m), 4.14(1H,d.J=15.2Hz), 4.31 (1H,m), 4.65(1H,m), 5.49(1H,d,J=15.2Hz), 7.02(1H,s), 7.20-7.50(5H,m), 7.72(2H,s), 7.83(1H,s).

| Elemental Analysis for $C_{25}H_{20}N_2O_2F_6$: | | | |
|---|---|---|---|
| Calcd.(%) | C 60.73, | H 4.08, | N 5.68 |
| Found (%) | C 60.43, | H 4.04, | N 5.74 |

Reference Example 89:

4-[3,5-Bis(trifluoromethyl)benzyl]-2,3,4,5-tetrahydro-8-methyl-5-oxo-6-phenylpyrido[3,2-f][1,4]oxazepine 9-oxide

m-Chloroperbenzoic acid (870 mg) was added to a chloroform (30 ml) solution of the compound (1.20 g) as obtained in Reference Example 87 and stirred for 20 hours at room temperature. The solvent was removed by distillation, and aqueous potassium carbonate solution was added to the residue, which was then extracted with ethyl acetate. The extract was washed with aqueous potassium carbonate solution and dried, and the solvent was removed by distillation. Thus, the entitled compound was obtained as colorless crystals (1.10 g).
m.p. 181-183°C (recrystallized from TFH-isopropyl ether)
NMR(200MHz, $CDCl_3$) ppm: 2.62(3H,s), 3.72(2H,m), 4.65(2H,m), 4.89(2H,s), 7.18(1H,s), 7.20-7.50(5H,m), 7.79(2H, s), 7.87(1H,s)

| Elemental Analysis for $C_{24}H_{18}N_2O_3F_6 \cdot 1/2\ H_2O$: | | | |
|---|---|---|---|
| Calcd.(%) | C 57.03, | H 3.79, | N 5.54 |
| Found (%) | C 57.15, | H 3.77, | N 5.16 |

Reference Example 90:

5-[3,5-Bis(trifluoromethyl)benzyl]-2,3,4,5-tetrahydro-9-methyl-6-oxo-7-phenyl-6H-pyrido[2,3-b][1,5]oxazocine 10-oxide

The compound as obtained in Reference Example 88 was reacted and treated in the same manner as in Reference Example 89 to obtain the entitled compound as colorless crystals (727 mg).
m.p. 116-118°C (recrystallized from ethanol-ethyl ether)
NMR(200MHz, $CDCl_3$) ppm: 1.60-1.82(1H,m), 2.42(1H,m), 2.61(3H,s), 3.43(1H,dd,J=6.0,17.0Hz), 3.81(1H,m), 4.18 (1H,d,J=15.4Hz), 4.25(1H,m), 4.78(1H,dd,J=5.2,12.6Hz), 5.52(1H,d,J=15.4Hz), 7.16(1H,s), 7.18-7.50(5H,m), 7.72 (2H,s), 7.84(1H,s)

| Elemental Analysis for $C_{25}H_{20}N_2O_3 \cdot 1/2\ H_2O$: | | | |
|---|---|---|---|
| Calcd.(%) | C 58.31, | H 4.01, N | 5.44 |
| Found (%) | C 58.17, | H 4.38, N | 5.31 |

Reference Example 91:

8-Acetoxymethyl-4-[3,5-bis(trifluoromethyl)benzyl]-2,3,4,5-tetrahydro-5-oxo-6-phenylpyrido[3,2-f][1,4]oxazepine

A mixture of the compound (939 mg) as obtained in Reference Example 89 and acetic anhydride (25 ml) was heated under reflux for 20 minutes. The solvent was removed by distillation, and aqueous potassium carbonate solution was added to the residue, which was then extracted with ethyl acetate. The extract was washed with water and dried, and the solvent was removed by distillation. Thus, the entitled compound was obtained as colorless crystals (740 mg).
m.p. 122-124°C (recrystallized from ethyl acetate-isopropyl ether)
NMR(200MHz, $CDCl_3$) ppm: 2.18(3H,s), 3.71(2H,t,J=5.6Hz), 4.50(2H,t,J=5.6Hz), 4.88(2H,s), 5.21(2H,s), 7.18-7.50 (6H,m), 7.79(2H,s), 7.87(1H,s)

| Elemental Analysis for $C_{26}H_{20}N_2O_4F_6$: | | | |
|---|---|---|---|
| Calcd.(%) | C 58.00, | H 3.74, | N 5.20 |
| Found (%) | C 57.60, | H 4.02, | N 5.09 |

Reference Example 92:

9-Acetoxymethyl-5-[3,5-bis(trifluoromethyl)benzyl]-2,3,4,5-tetrahydro-6-oxo-7-phenyl-6H-pyrido[2,3-b][1,5]oxazocine

The compound as obtained in Reference Example 90 was reacted and treated in the same manner as in Reference Example 91 to obtain the entitled compound as colorless crystals (479 mg).
m.p. 156-157°C (recrystallized from ethyl acetate-isopropyl ether)
NMR(200MHz, $CDCl_3$) ppm: 1.60-1.95(1H,m), 2.00-2.20(1H,m), 2.17(3H,s), 336(1H,m), 3,75(1H,m), 4.14(1H,d, J=15.2Hz), 4.31(1H,m), 4.61(1H,m), 5.20(2H,s), 5.48(1H,d,J=15.2Hz), 7.18(1H,s), 7.20-7.50(5H,m), 7.70(2H,s), 7.83 (1H,s)

| Elemental Analysis for $C_{27}H_{22}N_2O_4F_6$: | | | |
|---|---|---|---|
| Calcd.(%) | C 58.70, | H 4.01, | N 5.07 |
| Found (%) | C 58.81, | H 4.11, | N 5.17 |

Reference Example 93:

4-[3,5-Bis(trifluoromethyl)benzyl]-8-chloromethyl-2,3,4,5-tetrahydro-5-oxo-6-phenylpyrido[3,2-f][1,4]oxazepine

Phosphorus oxychloride (1.24 ml) and triethylamine (1.85 ml) were dropwise added at the same time to a dichloromethane (100 ml) solution of the compound (4.40 g) as obtained in Reference Example 89, while stirring at room temperature. The resulting mixture was heated under reflux for 1 hour, and then the solvent was removed by distillation. Aqueous potassium carbonate solution was added to the residue, which was then extracted with ethyl acetate-THF. The extract was washed with water and dried, and the solvent was removed by distillation. Thus, the entitled compound was obtained as colorless crystals (1.44 g).
m.p. 183-184°C (recrystallized from ethyl acetate-isopropyl ether)
NMR(200MHz, $CDCl_3$) ppm: 3.73(2H,t,J=5.4Hz), 4.51(2H,t,J=5.4Hz), 4.66(2H,s), 4.89(2H,s), 7.27(1H,s), 7.30-7.55 (5H,m), 7.81(2H,s), 7.88(1H,s)

| Elemental Analysis for $C_{24}H_{17}N_2O_2F_6Cl$: | | | |
|---|---|---|---|
| Calcd.(%) | C 55.99, | H 3.33, | N 5.44 |
| Found (%) | C 55.75, | H 3.53, | N 5.27 |

Reference Example 94:

4-[3,5-Bis(trifluoromethyl)benzyl]-2,3,4,5-tetrahydro-8-methoxymethyl-5-oxo-6-phenylpyrido[3,2-f][1,4]oxazepine

A mixture of the compound (151 mg) as obtained in Reference Example 93, THF (2 ml), methanol (1 ml) and 28 % sodium methoxide-methanol solution (1 ml) was stirred for 2 hours at room temperature. The solvent was removed by distillation, and water was added to the residue, which was then extracted with ethyl acetate. The extract was washed with water and dried, and the solvent was removed by distillation. Thus, the entitled compound was obtained as colorless crystals (118 mg).
m.p. 139-140°C (recrystallized from ethyl acetate-isopropyl ether)
NMR(200MHz, $CDCl_3$) ppm: 3.51(3H,s), 3.71(2H,t,J=5.6Hz), 4.49(2H,t,J=5.6Hz), 4.58(2H,s), 4.89(2H,s), 7.27(1H,s), 7.30-7.52(5H,m), 7.81(2H,s), 7.87(1H,s)

| Elemental Analysis for $C_{25}H_{20}N_2O_3F_6$: | | | |
|---|---|---|---|
| Calcd.(%) | C 58.83, | H 3.95, | N 5.49 |
| Found (%) | C 58.73, | H 3.95, | N 5.57 |

Reference Example 95:

4-[3,5-Bis(trifluoromethyl)benzyl]-2,3,4,5-tetrahydro-8-(1-methylethyl)-5-oxo-6-phenylpyrido[3,2-f][1,4]oxazepine

A mixture of the compound (150 mg) as obtained in Reference Example 93, THF (1 ml), isopropanol (10 ml) and sodium hydride (60 % oily) (120 mg) was stirred for 3 hours at room temperature. The solvent was removed by distillation, and water was added to the residue, which was then extracted with ethyl acetate. The extract was washed with water and dried, and the solvent was removed by distillation. The residue was purified by column chromatography (hexane:ethyl acetate = 1:1) using silica gel to obtain the entitled compound as colorless crystals (74 mg).
m.p. 134-136°C (recrystallized from ethyl acetate-hexane)
NMR(200MHz, $CDCl_3$) ppm: 1.26(6H,d,J=6.0Hz), 3.60-3.90(3H,m), 4.48(2H,t,J=5.4Hz), 4.63(2H,s), 4.89(2H,s), 7.27 (1H,s), 7.30-7.55(5H,m), 7.81(2H,s), 7.87(1H,s)

| Elemental Analysis for $C_{27}H_{24}N_2O_3F_6$: | | | |
|---|---|---|---|
| Calcd.(%) | C 60.22, | H 4.49, | N 5.20 |
| Found (%) | C 60.00, | H 4.61, | N 5.07 |

Reference Example 96:

4-[3,5-Bis(trifluoromethyl)benzyl]-2,3,4,5-tetrahydro-8-methylthiomethyl-5-oxo-6-phenylpyrido[3,2-f][1,4]oxazepine

A mixture of the compound (125 mg) as obtained in Reference Example 93, methanol (5 ml) and aqueous 15 % sodium methylmercaptan solution (1 ml) was stirred for 10 minutes at room temperature. The solvent was removed by distillation, and water was added to the residue, which was then extracted with ethyl acetate. The extract was washed with water and dried, and the solvent was removed by distillation. Thus, the entitled compound was obtained as colorless crystals (103 mg).
m.p. 165-166°C (recrystallized from ethyl acetate-isopropyl ether)
NMR(200MHz, $CDCl_3$) ppm: 2.14(3H,s), 3.72(2H,t,J=5.4Hz), 3.79(2H,s), 4.49(2H,t,J=5.4Hz), 4.89(2H,s), 7.30-7.50 (5H,m), 7.34(1H,s), 7.81(2H,s), 7.87(1H,s)

| Elemental Analysis for $C_{25}H_{20}N_2O_2SF_6 \cdot 1/6\ H_2O$: | | | |
|---|---|---|---|
| Calcd.(%) | C 56.71, | H 3.87, | N 5.29 |
| Found (%) | C 56.67, | H 3.87, | N 5.23 |

Reference Example 97:

8-Aminomethyl-4-[3,5-bis(trifluoromethyl)benzyl]-2,3,4,5-tetrahydro-5-oxo-6-phenylpyrido[3,2-f][1,4]oxazepine

A mixture of the compound (500 mg) as obtained in Reference Example 93, THF (10 ml) and aqueous 25 % ammonia (10 ml) was heated in a sealed tube at 120°C for 1 hour. After cooled, the solvent was removed by distillation, and water was added to the residue, which was then extracted with ethyl acetate. The extract was washed with water and dried, and the solvent was removed by distillation. Thus, the entitled compound was obtained as colorless crystals (443 mg).
m.p. 188-191°C (recrystallized from THF-ethyl ether)
NMR(200MHz, $CDCl_3$) ppm: 3.71(2H,t,J=5.6Hz), 4.00(2H,s), 4.50(2H,t,J=5.6Hz), 4.89(2H,s), 7.20-7.60(6H,m), 7.81 (2H,s), 7.87(1H,s)

| Elemental Analysis for $C_{24}H_{19}N_3O_2F_6$: | | | |
|---|---|---|---|
| Calcd.(%) | C 58.19, | H 3.87, | N 8.48 |
| Found (%) | C 58.36, | H 3.81, | N 8.00 |

Reference Example 98:

4-[3,5-Bis(trifluoromethyl)benzyl]-2,3,4,5-tetrahydro-8-methylaminomethyl-5-oxo-6-phenylpyrido[3,2-f][1,4]oxazepine

A mixture of the compound (150 mg) as obtained in Reference Example 93 and 40 % methylamine-methanol solution was stirred for 30 minutes at room temperature. The solvent was removed by distillation, and water was added to the residue, which was then extracted with ethyl acetate. The extract was washed with water and dried, and the solvent was removed by distillation. Thus, the entitled compound was obtained as colorless crystals (115 mg).
m.p. 152-154°C (recrystallized from ethyl acetate-isopropyl ether)
NMR(200MHz, $CDCl_3$) ppm: 2.50(3H,s), 3.70(2H,t,J=5.6Hz), 3.89(2H,s), 4.48(2H,t,J=5.6Hz), 4.88(2H,s), 7.22-7.50 (6H,m), 7.80(2H,s), 7.86(1H,s)

| Elemental Analysis for $C_{25}H_{21}N_3O_2F_6$: | | | |
|---|---|---|---|
| Calcd.(%) | C 58.94, | H 4.15, | N 8.25 |
| Found (%) | C 58.71, | H 4.25, | N 8.35 |

Reference Example 99:

4-[3,5-Bis(trifluoromethyl)benzyl]-8-dimethylaminomethyl-2,3,4,5-tetrahydro-5-oxo-6-phenylpyrido[3,2-f][1,4] oxazepine

Dimethylamine (1 ml) was added to a THF (3 ml) solutin of the compound (150 mg) as obtained in Reference Example 93, and then stirred for 30 minutes at room temperature. The solvent was removed by distillation, and water was added to the residue, which was then extracted with ethyl acetate. The extract was washed with water and dried, and the solvent was removed by distillation. Thus, the entitled compound was obtained as colorless crystals (128 mg).
m.p. 186-188°C (recrystallized from ethyl acetate-isopropyl ether)
NMR(200MHz, $CDCl_3$) ppm: 2.33(6H,s), 3.60(2H,s), 3.71(2H,t,J=5.6Hz), 4.49(2H,t,J=5.6Hz), 4.89(2H,s), 7.26(1H,s), 7.30-7.50(5H,m), 7.81(2H,s), 7.86(1H,s)

| Elemental Analysis for $C_{26}H_{23}N_3O_2F_6$: | | | |
|---|---|---|---|
| Calcd.(%) | C 59.66, | H 4.43, | N 8.03 |
| Found (%) | C 59.43. | H 4.49. | N 7.84 |

Reference Example 100:

4-[3,5-Bis(trifluoromethyl)benzyl]-8-cyclopropylaminomethyl-2,3,4,5-tetrahydro-5-oxo-6-phenylpyrido[3,2-f][1,4] oxazepine

Cyclopropylamine (0.5 ml) was added to a THF (10 ml) solutin of the compound (155 mg) as obtained in Reference Example 93, and then heated under reflux for 15 hours. The solvent was removed by distillation, and water was added to the residue, which was then extracted with ethyl acetate. The extract was washed with water and dried, and the solvent was removed by distillation. The residue was subjected to column chromatography (ethyl acetate: methanol = 9:1) using silica gel, to thereby separate and purify the product. Thus, the entitled compound was obtained as colorless crystals (127 mg).
m.p. 129-131°C (recrystallized from ethyl acetate-hexane)
NMR(200MHz, $CDCl_3$) ppm: 0.44(4H,m), 2.19(1H,m), 3.69(2H,t,J=5.6Hz), 3.97(2H,s), 4.48(2H,t,J=5.6Hz), 4.87(2H, s), 7.25(1H,s), 7.26-7.55(5H,m), 7.79(2H,s), 7.86(1H,s)

| Elemental Analysis for $C_{27}H_{23}N_3O_2F_6 \cdot 1/6\ H_2O$: | | | |
|---|---|---|---|
| Calcd.(%) | C 60.22, | H 4.37, | N 7.80 |
| Found (%) | C 59.98, | H 4.40, | N 7.85 |

Reference Example 101:

4-[3,5-Bis(trifluoromethyl)benzyl]-2,3,4,5-tetrahydro-8-(N-methylpiperazinomethyl)-5-oxo-6-phenylpyrido[3,2-f][1,4] oxazepine

N-Methylpiperazine (1 ml) was added to a THF (1 ml) solutin of the compound (150 mg) as obtained in Reference Example 93, and then stirred for 15 hours at room temperature. The solvent was removed by distillation, and water was added to the residue, which was then extracted with ethyl acetate. The extract was washed with water and dried, and the solvent was removed by distillation. Thus, the entitled compound was obtained as colorless crystals (105 mg).
m.p. 181-182°C (recrystallized from ethyl acetate-isopropyl ether)
NMR(200MHz, $CDCl_3$) ppm: 2.30(3H,s), 2.48(4H,br), 2.59(4H,br), 3.68(2H,s), 3.71(2H,t,J=5.6Hz), 4.48(2H,t,J=5.6Hz), 4.89(2H,s), 7.27(1H,s), 7.30-7.50(5H,m), 7.81(2H,m), 7.87(1H,s)

| Elemental Analysis for $C_{26}H_{28}N_4O_2F_6$: | | | |
|---|---|---|---|
| Calcd.(%) | C 60.20, | H 4.88, | N 9.68 |
| Found (%) | C 59.96, | H 5.00, | N 9.51 |

Reference Example 102:

8-Acetylaminomethyl-4-[3,5-bis(trifluoromethyl)benzyl]-2,3,4,5-tetrahydro-5-oxo-6-phenylpyrido[3,2-f][1,4]oxazepine

Acetic anhydride (1 ml) was added to a pyridine (3 ml) solutin of the compound (150 mg) as obtained in Reference Example 97, and then stirred for 20 minutes at room temperature. The solvent was removed by distillation, and ethyl acetate was added to the residue. The resulting mixture was washed with 1 N-hydrochloric acid and water and dried, and then the solvent was removed by distillation. Thus, the entitled compound was obtained as colorless crystals (113 mg).
m.p. 223-224°C (recrystallized from THF-ethyl ether)
NMR(200MHz, $CDCl_3$) ppm: 2.07(3H,s), 3.72(2H,t,J=5.4Hz), 4.49(2H,t,J=5.4Hz), 4.56(2H,d,J=5.4Hz), 4.88(2H,s), 6.62(1H,br), 7.21(1H,s), 7.22-7.55(5H,m), 7.80(2H,s), 7.87(1H,s)

| Elemental Analysis for $C_{26}H_{21}N_3O_3F_6$: | | | |
|---|---|---|---|
| Calcd.(%) | C 58.10, | H 3.94, | N 7.82 |
| Found (%) | C 58.06, | H 3.97, | N 7.99 |

Reference Example 103:

4-[3,5-Bis(trifluoromethyl)benzyl]-2,3,4,5-tetrahydro-8-methanesulfonylaminomethyl-5-oxo-6-phenylpyrido[3,2-f][1,4] oxazepine

Triethylamine (0.085 ml) and methanesulfonyl chloride (0.050 ml) were added to a THF (5 ml) solutin of the compound (150 mg) as obtained in Reference Example 97, and then stirred for 1 hour at room temperature. The solvent was removed by distillation, and ethyl acetate was added to the residue. The resulting mixture was washed with aqueous potassium carbonate solution and water and dried, and then the solvent was removed by distillation. Thus , the entitled compound was obtained as colorless crystals (108 mg).
m.p. 194-195°C (recrystallized from THF-isopropyl ether)
NMR(200MHz, $CDCl_3$) ppm: 2.99(3H,s), 3.72(2H,t,J=5.4Hz), 4.44(2H,d,J=6.0Hz), 4.48(2H,t,J=5.4Hz), 4.88(2H,s), 5.55(1H,t,J=6.0Hz), 7.26(1H,s), 7.27-7.50(5H,m), 7.80(2H,s), 7.87(1H,s)

| Elemental Analysis for $C_{25}H_{21}N_3O_4SF_6 \cdot 1/2\ H_2O$: | | | |
|---|---|---|---|
| Calcd.(%) | C 51.55, | H 3.80, | N 7.21 |
| Found (%) | C 51.43, | H 3.78, | N 7.07 |

Reference Example 104:

6-[3,5-Bis(trifluoromethyl)benzyl]-5,6,7,8,9,10-hexahydro-3,9-dimethyl-5,10-dioxo-4-phenylpyrido[2,3-f][1,4] diazocine

Triethylamine (0.42 ml) and methanesulfonyl chloride (0.24 ml) were added to a THF (15 ml) solutin of the compound (370 mg) as obtained in Reference Example 33, and then stirred for 1 hour at room temperature. Aqueous saturated sodium hydrogencarbonate solution (15 ml) was added to the reaction mixture and further stirred for 40 minutes at room temperature. Then, the mixture was extracted with ethyl acetate. The extract was washed with diluted hydrochloric acid and saturated saline solution and dried, and then the solvent was removed by distillation. The residue was dissolved in THF (30 ml), and sodium hydride (60 % oily) (84 mg) was added thereto and heated under reflux for 40 minutes. The resulting reaction mixture was diluted with ethyl acetate, then washed with diluted hydrochloric acid, aqueous sodium carbonate solution and saturated saline solution, and thereafter the solvent was removed by distillation. Thus, the entitled compound was obtained as colorless crystals (213 mg).
m.p. 203-205°C (recrystallized from ethyl acetate-isopropyl ether)
NMR(200MHz, $CDCl_3$) ppm: 1.72(1H,dd,J=15,7.2Hz), 2.18(3H,s), 2.75(1H,m), 3.04(3H,s), 3.54(3H,m), 4.09(1H,dd, J=14,7.2Hz), 7.2-7.6(5H,m), 7.48(2H,s), 7.74(1H,s), 8.69(1H,s)

| Elemental Analysis for $C_{26}H_{21}N_3O_2F_6 \cdot 0.2\ H_2O$: | | | |
|---|---|---|---|
| Calcd.(%) | C 59.48, | H 4.11, | N 8.00 |
| Found (%) | C 59.39, | H 4.13, | N 7.83 |

EI-MS m/z: 521 (M+) [$(C_{26}H_{21}N_3O_2F_6)^+$]

Reference Example 105:

6-[3,5-Bis(trifluoromethyl)benzyl]-5,6,7,8,9,10-hexahydro-9-methyl-5,10-dioxo-4-phenylpyrido[2,3-f][1,4]diazocine

The compound as obtained in Reference Example 34 was reacted and treated in the same manner as in Reference Example 104 to obtain the entitled compound as colorless crystals.
m.p. 167-169°C (recrystallized from ethyl acetate-isopropyl ether)
NMR(200MHz, $CDCl_3$) ppm: 2.05(1H,m), 2.87(1H,m), 3.10(3H,s), 3.36(1H,d,J=14Hz), 3.48(1H,d,J=14Hz), 3.97(1H, m), 4.26(1H,dd,J=15,7.1Hz), 7.35(3H,m), 7.53(5H,m), 7.71(1H,s), 8.81(1H,d,J=5.0Hz)

| Elemental Analysis for $C_{25}H_{19}N_3O_2F_6$: | | | |
|---|---|---|---|
| Calcd.(%) | C 59.18, | H 3.77, | N 8.28 |
| Found (%) | C 58.90, | H 3.81, | N 8.05 |

Reference Example 106:

6-Benzyl-5,6,7,8,9,10-hexahydro-3,9-dimethyl-5,10-dioxo-4-phenylpyrido[2,3-f][1,4]diazocine

N-Benzyl-N-(2-hydroxyethyl)-5-methyl-2-methylaminocarbonyl-4-phenyl-3-pyridinecarboxamide (this was prepared by reacting the compound as obtained in Reference Example 35 with methylamine and treated in the same manner as in Step 4 in Reference Example 33) was reacted and treated in the same manner as in Reference Example 104 to obtain the entitled compound as colorless crystals.
m.p. 183-184°C (recrystallized from ethyl acetate-isopropyl ether)
NMR(200MHz, $CDCl_3$) ppm: 1.46(1H,dd,J=15,8.1Hz), 2.17(3H,s), 2.69(1H,m), 3.02(3H,s), 3.27(1H,d,J=13Hz), 3.44 (1H,d,J=13Hz), 3.56(1H,m), 4.00(1H,m), 7.01(2H,m), 7.2-7.6(8H,m), 8.68(1H,s)

| Elemental Analysis for $C_{24}H_{23}N_3O_2$: | | | |
|---|---|---|---|
| Calcd.(%) | C 74.78, | H 6.01, | N 10.09 |
| Found (%) | C 74.52, | H 6.13, | N 10.82 |

Reference Example 107:

6-[3,5-Bis(trifluoromethyl)benzyl]-9-ethyl-5,6,7,8,9,10-hexahydro-3-methyl-5,10-dioxo-4-phenylpyrido[2,3-f][1,4] diazocine

N-[3,5-Bis(trifluoromethyl)benzyl]-2-ethylaminocarbonyl-N-(2-hydroxyethyl)-5-methyl-4-phenyl-3-pyridinecarboxamide (this was prepared by reacting the compound as obtained in Step 3 in Reference Example 33 with ethylamine and treated in the same manner as in Step 4 in Reference Example 33) was reacted and treated in the same manner as in Reference Example 104 to obtain the entitled compound as colorless crystals.
m.p. 228-229°C (recrystallized from ethyl acetate-isopropyl ether)
NMR(200MHz, $CDCl_3$) ppm: 1.33(3H,t,J=7.0Hz), 1.51(1H,dd,J=15,7.6Hz), 2.18(3H,s), 2.72(1H,m), 3.39(1H,m), 3.42 (1H,d,J=14Hz), 3.57(1H,d,J=14Hz), 3,57(1H,m), 3.77(1H,m), 4.03(1H,dd,J=15,7.6Hz), 7.2-7.6(5H,m), 7.48(2H,s), 7.74(1H,s), 8.69(1H,s)

| Elemental Analysis for $C_{27}H_{23}N_3O_2F_6$: | | | |
|---|---|---|---|
| Calcd.(%) | C 60.56, | H 4.33, | N 7.85 |
| Found (%) | C 60.28, | H 4.51, | N 7.65 |

Reference Example 108:

6-[3,5-Bis(trifluoromethyl)benzyl]-6,7,8,9,10,11-hexahydro-3,10-dimethyl-5,11-dioxo-4-phenyl-5H-pyrido[2,3-g][1,5] diazonine

The compound as obtained in Reference Example 36 was reacted and treated in the same manner as in Reference Example 104, to obtain the entitled compound as colorless crystals.
m.p. 247-249°C (recrystallized from THF-ethyl acetate-isopropyl ether)
NMR(200MHz, $CDCl_3$) ppm: 1.2-1.4(1H,m), 1.8-2.3(1H,m), 2.15(3H,s), 3.0-3.6(4H,m), 3.04(3H,s), 3.91(1H,d,J=15Hz), 5.32(1H,d,J=15Hz), 7.0-7.5(7H,m), 7.75(1H,s), 8.59(1H,s)

| Elemental Analysis for $C_{27}H_{23}N_3O_2F_6$: | | | |
|---|---|---|---|
| Calcd.(%) | C 60.56, | H 4.33, | N 7.85 |
| Found (%) | C 60.41, | H 4.46, | N 7.87 |

EI-MS m/z: 535 ($M^+$) [$(C_{27}H_{23}N_3O_2F_6)^+$]

Reference Example 109:

4-[3,5-Bis(trifluoromethyl)benzyl]-8-hydroxymethyl-2,3,4,5-tetrahydro-5-oxo-6-phenylpyrido[3,2-f][1,4]oxazepine

A mixture of 8-acetoxymethyl-4-[3,5-bis(trifluoromethyl)benzyl]-2,3,4,5-tetrahydro-5-oxo-6-phenylpyrido [3,2-f][1,4]oxazepine (Reference Example 91) (4,51 g), ethanol (50 ml), and 4N-NaOH (50 ml) was stirred for 1.5 hours at room temperature. After evaporation of the solvent, water was added to the residue. The pH of the mixture was adjusted to ca.8 using dilute hydrochloric acid, and extracted with ethyl acetate. The extract was washed with water, dried and evaporated to give the entitled compound as colorless crystals (4.10 g).
m.p. 199-201°C (recrystallized from ethyl acetate-isopropyl ether)
NMR(200MHz, $CDCl_3$) ppm: 3.10(1H,b), 3.71(2H,t,J=5.6Hz), 4.50(2H,t,J=5.6Hz), 4.78(2H,s), 4.88(2H,s), 7.20-7.50 (6H,m), 7.80(2H,m), 7.87(1H,s).

Reference Example 110:

4-[3,5-Bis(trifluoromethyl)benzyl]-2,3,4,5-tetrahydro-5-oxo-6-phenylpyrido[3,2-f][1,4]oxazepine-8-carboxylic acid

A mixture of 4-[3,5-bis(trifluoromethyl)benzyl]-8-hydroxymethyl-2,3,4,5-tetrahydro-5-oxo-6-phenylpyrido [3,2-f][1,4]oxazepine (Reference Example 109) (3.49 g), 2N-NaOH (100 ml) and pottasium permanganate (2.22 g) was stirred for 45 hours at room temperature. To the reaction mixture was added saturated aqueous sodium thiosulfate (10 ml). After the pH of the mixture was adjusted to ca.3 using hydrochloric acid. The mixture was extracted with ethyl

acetate-THF (1:2). The extract was washed with aqueous sodium chloride solution, dried and evaporated to give the entitled compound as colorless crystals (2.74 g).
m.p. 119-123°C (recrystallized from methanol-ethyl ether)
NMR(200MHz, DMSO-$d_6$) ppm: 3.94(2H,b), 4.46(2H,b), 4.91(2H,s), 7.25-7.55(5H,m), 7.90(1H,s), 8.06(2H,s), 8.12(1H, s).

Reference Example 111:

4-[3,5-Bis(trifluoromethyl)benzyl]-2,3,4,5-tetrahydro-5-oxo-6-phenylpyrido[3,2-f][1,4]oxazepine-8-carboxamide

A mixture of 4-[3,5-bis(trifluoromethyl)benzyl]-2,3,4,5-tetrahydro-5-oxo-6-phenylpyrido[3,2-f][1,4]oxazepine-8-carboxylic acid (Reference Example 110) (220 mg), THF (15 ml), DMF (catalytic amount) and thionyl chloride (0.087 ml) was stirred for 2.5 hours with heating under reflux. The solvent was evaporated, and the residue was dissolved in THF (10 ml). To the solution was added aqueous ammonia (2 ml). After being stirred for 1 hour at room temperature, the mixture was concentrated. To the concentrate was added water, and the mixture was extracted with ethyl acetate. The extract was washed with water, dried and evaporated to give the entitled compound as colorless crystals (163 mg).
m.p. 221-222°C (recrystallized from ethyl acetate-isopropyl ether)
NMR(200MHz, $CDCl_3$) ppm: 3.74(2H,t,J=5.6Hz), 4.50(2H,t,J=5.6Hz), 4.92(2H,s), 5.80(1H,b), 7.30-7.55(6H,m), 7.83 (2H,s), 7.89(1H,s), 8.20(1H,s).

The compounds of Reference Examples 112 to 117 were similarly prepared by reaction and work-up as described in Reference Example 111 using 4-[3,5-bis(trifluoromethyl)benzyl]-2,3,4,5-tetrahydro-5-oxo-6-phenylpyrido [3,2-f][1,4]oxazepine-8-carboxylic acid (Reference Example 110) (via the acid chloride) and substituted amines (methylamine, dimethylamine, n-butylamine, piperidine, morpholine, and 1-methylpiperazine). The physico-chemical data are described below.

Reference Example 112:

4-[3,5-Bis(trifluoromethyl)benzyl]-N-methyl-2,3,4,5-tetrahydro-5-oxo-6-phenylpyrido[3,2-f][1,4]oxazepine-8-carboxamide
m.p. 145°C (decomposed) (recrystallized from THF-ethyl ether)
NMR(200MHz, $CDCl_3$) ppm: 3.04(3H,d,J=5.2Hz), 3.73(2H,t,J=5.4Hz), 4.48(2H,t,J=5.4Hz), 4.90(2H,s), 7.25-7.60(5H, m), 7.65-7.95(1H,b), 7.81(2H,s), 7.87(1H,s), 8.17(1H,s).

Reference Example 113:

4-[3,5-Bis(trifluoromethyl)benzyl]-N,N-dimethyl-2,3,4,5-tetrahydro-5-oxo-6-phenylpyrido[3,2-f][1,4]oxazepine-8-carboxamide
m.p. 235-236°C (recrystallized from ethyl acetate-isopropyl ether)
NMR(200MHz, $CDCl_3$) ppm: 3.11(3H,s), 3.15(3H,s), 3.72(2H,t,J=5.6Hz), 4.47(2H,t,J=5.6Hz), 4.90(2H,s), 7.25-7.50 (5H,m), 7.60(1H,s), 7.82(2H,s), 7.88(1H,s).

Reference Example 114:

4-[3,5-Bis(trifluoromethyl)benzyl]-N-n-butyl-2,3,4,5-tetrahydro-5-oxo-6-phenylpyrido[3,2-f][1,4]oxazepine-8-carboxamide
m.p. 194-196°C (recrystallized from ethyl acetate-isopropyl ether)
NMR(200MHz, $CDCl_3$) ppm: 0.96(3H,t,J=7.2Hz), 1.20-1.80(6H,m), 4.78(2H,m), 3.73(2H,t,J=5.6Hz), 4.49(2H,t, J=5.6Hz), 4.91(2H,s), 7.30-7.58(5H,m), 7.82(2H,s), 7.88(1H,s), 8.18(1H,s).

Reference Example 115:

4-[3,5-Bis(trifluoromethyl)benzyl]-2,3,4,5-tetrahydro-5-oxo-6-phenyl-8-piperidinocarbonylpyrido[3,2-f][1,4] oxazepine
m.p. 218-220°C (recrystallized from THF-isopropyl ether)
NMR(200MHz, $CDCl_3$) ppm: 1.60(2H,b), 1.69(4H,b), 3.44(2H,t,J=5.6Hz), 3.72(4H,m), 4.46(2H,t,J=5.6Hz), 4.89(2H,s), 7.20-7.60(6H,m), 7.81(2H,s), 7.87(1H,s).

Reference Example 116:

4-[3,5-Bis(trifluoromethyl)benzyl]-2,3,4,5-tetrahydro-8-morpholinocarbonyl-5-oxo-6-phenylpyrido[3,2-f][1,4] oxazepine
m.p. 265-266°C (recrystallized from ethyl acetate-isopropyl ether)
NMR(200MHz, $CDCl_3$) ppm: 3.55-3.90(10H,m), 4.46(2H,t,J=5.6Hz), 4.89(2H,s), 7.25-7.52(5H,m), 7.59(1H,s), 7.81 (2H,s), 7.88(1H,s).

Reference Example 117:

4-[3,5-Bis(trifluoromethyl)benzyl]-2,3,4,5-tetrahydro-8-[1-(4-methylpiperazinyl)carbonyl]-5-oxo-6-phenyl-pyrido[3,2-f][1,4]oxazepine-8-carboxamide m.p. 196-198°C (recrystallized from THF-isopropyl ether) NMR(200MHz, $CDCl_3$)ppm: 2.35(3H,s), 2.45(2H,m), 2.54(2H,m), 3.61(2H,m), 3.72(2H,t,J=5.6Hz), 3.84(2H,m), 4.46(2H,t,J=5.6Hz), 4.89(2H,s), 7.25-7.50(5H,m), 7.54(1H,s), 7.81(2H,s), 7.88(1H,s).

The compounds as described in Reference Example 118 - 126 were obtained as colorless crystals from the compounds of Reference Example 37 - 45, respectively, by substantially the same reaction and work-up as Reference Example 65 (i.e. , by cyclization in the presence of sodium hydride in THF). The physico-chemical data are described below.

Reference Example 118:

2,3,4,5-Tetrahydro-5-oxo-6-phenyl-4-(3,4,5-trimethoxybenzyl)pyrido[3,2-f][1,4]oxazepine
m.p. 177-179°C (recrystallized from acetone-ethyl ether)
NMR(200MHz, $CDCl_3$) ppm: 3.70(2H,t,J=5.6Hz), 3.85(6H,s), 3.87(3H,s), 4.34(2H,t,J=5.6Hz), 4.72(2H,s), 6.60(2H,s), 7.24(1H,d,J=5.2Hz), 7.30-7.55(5H,m), 8.42(1H,d,J=5.2Hz).

Reference Example 119:

4-(3,4-Dichlorobenzyl)-2,3,4,5-tetrahydro-5-oxo-6-phenylpyrido[3,2-f][1,4]oxazepine
m.p. 189-192°C (recrystallized from THF-ethyl ether)
NMR(200MHz, $CDCl_3$) ppm: 3.67(2H,t,J=5.4Hz), 4.42(2H,t,J=5.4Hz), 4.71(2H,s), 7.10-7.70(9H,m), 8.43(1H,d, J=5.2Hz).

Reference Example 120:

4-(3,4-Dimethoxybenzyl)-2,3,4,5-tetrahydro-5-oxo-6-phenylpyrido[3,2-f][1,4]oxazepine
m.p. 175-176°C (recrystallized from THF-ethyl ether)
NMR(200MHz, $CDCl_3$) ppm: 3.67(2H,t,J=5.4Hz), 3.85(3H,s), 3.91(3H,s), 4.29(2H,t,J=5.4Hz), 4.72(2H,s), 6.80-7.00 (3H,m), 7.22(1H,d,J=5.2Hz), 7.30-7.50(5H,m), 8.40(1H,d,J=5.2Hz).

Reference Example 121:

4-Benzyl-2,3,4,5-tetrahydro-5-oxo-6-phenylpyrido[3,2-f][1,4]oxazepine
m.p. 209-211°C (recrystallized from methanol-ethyl ether) NMR(200MHz, $CDCl_3$) ppm: 3.64(2H,t,J=5.6Hz), 4.33(2H, t,J=5.6Hz), 4.77(2H,s), 7.22(1H,d,J=5.2Hz), 7.30-7.55(5H,m), 8.39(1H,d,J=5.2Hz).

Reference Example 122:

2,3,4,5-Tetrahydro-6-oxo-7-phenyl-5-(3,4,5-trimethoxybenzyl)-6H-pyrido[2,3-b][1,5]oxazocine
m.p. 155-156°C (recrystallized from ethyl acetate-ethyl ether)
NMR(200MHz, $CDCl_3$) ppm: 1.65-1.85(1H,m), 2.29(1H,m), 3.40-3.75(2H,m), 3.77(6H,s), 3.87(3H,s), 4.07(1H,d, J=14.2Hz), 4.27(1H,m), 4.66(1H,m), 5.22(1H,d,J=14.2Hz), 6.53(2H,s), 7.15(1H,d,J=5.2Hz), 7.35(5H,m), 8.40(1H,d, J=5.2Hz).

Reference Example 123:

(S)-5-Benzyl-2,3,4,5-tetrahydro-3-methyl-6-oxo-7-phenyl-6H-pyrido[2,3-b][1,5]oxazocine
m.p. 139-141°C (recrystallized from ethyl acetate-isopropyl ether)

NMR(200MHz, $CDCl_3$) ppm: 0.84(3H,d,J=7.0Hz), 2.43(1H,m), 3.12(1H,d,J=14.8Hz), 3.39(1H,dd,J=15.4&10.2Hz), 3.72-4.00(2H,m), 4.60(1H,dd,J=12.4&5.2Hz), 5.51(1H,d,J=14.8Hz), 7.16(1H,d,J=5.0Hz), 7.20-7.50(10H,m), 8.39(1H, d,J=5.0Hz).

Reference Example 124:

(R)-5-Benzyl-2,3,4,5-tetrahydro-3-methyl-6-oxo-7-phenyl-6H-pyrido[2,3-b][1,5]oxazocine
m.p. 139-140°C (recrystallized from ethyl acetate-isopropyl ether)
NMR(200MHz, $CDCl_3$) ppm: the same as the spectrum of the compound of Reference Example 123.

Reference Example 125:

(S)-5-[3,5-Bis(trifluoromethyl)benzyl]-2,3,4,5-tetrahydro-3-methyl-6-oxo-7-phenyl-6H-pyrido[2,3-b][1,5]oxazocine
m.p. 142-143°C (recrystallized from ethyl acetate-isopropyl ether)
NMR(200MHz, $CDCl_3$) ppm: 0.87(3H,d,J=7.0Hz), 2.46(1H,m), 3.10(1H,d,J=15.4Hz), 3.59(1H,dd,J=15.0&10.6Hz), 3.92(1H,dd,J=12.6&10.4Hz), 4.20(1H,d,J=15.4Hz), 4.63(1H,dd,J=12.6&5.2Hz), 5.50(1H,d,J=15.4Hz), 7.18(1H,d, J=5.0Hz), 7.20-7.50(5H,m), 7.72(2H,s), 7.84(1H,s), 8.43(1H,d,J=5.0Hz).

Reference Example 126:

(R)-5-[3,5-Bis(trifluoromethyl)benzyl]-2,3,4,5-tetrahydro-3-methyl-6-oxo-7-phenyl-6H-pyrido[2,3-b][1,5]oxazocine
m.p. 142-143°C (recrystallized from ethyl acetate-isopropyl ether)
MMR(200MHz, $CDCl_3$) ppm: same as the spectrum of the compound of Reference Example 125.

Reference Example 127:

7-Benzyl-6,7,8,9,10,11-hexahydro-5-(4-methylphenyl)-6,13-dioxo-13H-[1,4]diazocino[2,1-g][1,7]naphthyridine

The compound as obtained in Reference Example 46 was reacted and treated in the same manner as Reference Example 69.
m.p. 239-241°C (recrystallized from ethyl acetate-isopropyl ether)
NMR(200MHz, $CDCl_3$) ppm: 1.6-2.1(4H,m), 2.50(3H,s), 3.14(1H,dd,J=15&3.8Hz), 3.3-3.7(2H,m), 3.77(1H,d,J=15Hz). 5.14(1H,dd,J=14&5.9Hz), 5.42(1H,d,J=15Hz), 6.67(2H,d,J=7.0Hz), 6.92(1H,dd,J=7.6&1.8Hz), 7.1-7.5(6H,m), 7.46 (1H,dd,J=8.4&4.4Hz), 7.60(1H,dd,J=8.4&1.8Hz), 8.90(1H,dd,J=4.4&1.8Hz).

Reference Example 128:

(9R)-7-Benzyl-6,7,8,9,10,11-hexahydro-9-methyl-5-(4-meihylphenyl)-6,13-dioxo-13H-[1,4] diazocino[2,1-g][1,7] naphthyridine

The compound as obtained in Reference Example 47 was reacted and treated in the same manner as Reference Example 69.
m.p. 218-220°C (recrystallized from ethyl acetate-isopropyl ether)
NMR(200MHz, $CDCl_3$) ppm: 0.85(3H,d,J=7.0Hz), 1.50-1.75(1H,m), 1.90-2.35(2H,m), 2.50(3H,s), 2.89(1H,d,J=15Hz), 3.26(1H,dd,J=14&10Hz), 3.59(1H,dd,J=14&11Hz), 3.75(1H,d,J=15Hz), 5.10(1H,dd,J=14&6.1Hz), 5.42(1H,d,J=15Hz), 6.69(2H,d,J=6.8Hz), 6.91(1H,dd,J=7.8&1.8Hz), 7.1-7.5(6H,m), 7.46(1H,dd,J=8.4&4.2Hz), 7.60(1H,dd,J=8.4&1.8Hz), 8.90(1H,dd,J=4.2&1.8Hz).

Reference Example 129:

(9S)-7-Benzyl-6,7,8,9,10,11-hexahydro-9-methyl-5-(4-methylphenyl)-6,13-dioxo-13H-[1,4]diazocino[2,1-g][1,7] naphthyridine

The compound as obtained in Reference Example 47 was reacted and treated in the same manner as Reference Example 69.
m.p. 218-220°C (recrystallized from ethyl acetate-isopropyl ether)

NMR(200MHz, $CDCl_3$) ppm: same as the spectrum of the compound of Reference Example 74.

Reference Example 130:

(9R)-6,7,8,9,10,11-Hexahydro-9-methyl-5-(4-methylphenyl)-6,13-dioxo-7-(3,4,5-trimethoxybenzyl)-13H-[1,4]diazocino[2,1-g][1,7]naphthyridine

The compound as obtained in Reference Example 49 was reacted and treated in the same manner as Reference Example 69.
A white powder.
NMR(200MHz, $CDCl_3$) ppm: 0.90(3H,d,J=6.6Hz), 1.5-1.8(1H,m), 1.9-2.5(2H,m), 2.41(3H,s), 3.11(1H,d,J=15Hz), 3.35(1H,dd,J=15&11Hz), 3.56(1H,dd,J=14&11Hz), 3.7-3.9(1H,m), 3.75(6H,s), 3.87(3H,s), 5.07(1H,dd,J=14&5.9Hz), 5.19(1H,d,J=15Hz), 6.30(2H,s), 6.77(1H,d,J=8.0Hz), 6.97(1H,d,J=8.0Hz), 7.29(1H,d,J=8.2Hz), 7.37(1H,d,J=8.2Hz), 7.45(1H,dd,J=8.4&4.0Hz), 7.58(1H,dd,J=8.4&1.4Hz), 8.89(1H,dd,J=4.0&1.4Hz).

Reference Example 131:

(9S)-6,7,8,9,10,11-Hexahydro-9-methyl-5-(4-methylphenyl)-6,13-dioxo-7-(3,4,5-trimethoxybenzyl)-13H-[1,4]diazocino[2,1-g][1,7]naphthyridine

The compound as obtained in Reference Example 50 was reacted and treated in the same manner as Reference Example 69.
A white powder.
NMR(200MHz, $CDCl_3$) ppm: same as the spectrum of the compound of Reference Example 76.

Reference Example 132:

(9R)-7-(3,5-Dimethoxybenzyl)-6,7,8,9,10,11-hexahydro-9-methyl-5-(4-methylphenyl)-6,13-dioxo-13H-[1,4]diazocino[2,1-g][1,7]naphthyridine

The compound as obtained in Reference Example 51 was reacted and treated in the same manner as Reference Example 69.
m.p. 206-208°C (recrystallized from ethanol-isopropyl ether)
NMR(200MHz, $CDCl_3$) ppm: 0.87(3H,d,J=7.0Hz), 1.67(1H,m), 1.9-2.4(2H,m), 2.42(3H,s), 3.05(1H,d,J=15Hz), 3.24-3.40(1H,m), 3.45-3.85(2H,m), 3.74(6H,s), 5.08(1H,dd,J=14&5.8Hz), 5.26(1H,d,J=14Hz), 6.12(2H,d,J=2.0Hz), 6.38(1H,t,J=2.0Hz), 6.84(1H,d,J=7.0Hz), 7.09(1H,d,J=7.0Hz), 7.29(1H,d,J=9.2Hz), 7.38(1H,d,J=9.2Hz), 7.46(1H,dd,J=8.2&4.2Hz), 7.62(1H,dd,J=8.2&1.6Hz), 8.89(1H,dd,J=4.2&1.6Hz).
The compounds as described in Reference Example 133 - 136 were obtained as colorless crystals from the compounds of Reference Example 52 - 54 and 144, respectively, by substantially the same reaction and work-up as Reference Example 65 (i.e., by cyclization in the presence of sodium hydride in THF). The physico-chemical data are described below.

Reference Example 133:

4-Benzyl-2,3,4,5-tetrahydro-5-oxo-6-(4-methylphenyl)pyrido[3,2-f][1,4]oxazepine
m.p. 203-204° C (recrystallized from methanol-ethyl ether)
NMR(200MHz, $CDCl_3$) ppm: 2.41(3H,s), 3.64(2H,t,J=5.4Hz), 4.32(2H,t,J=5.4Hz), 4.78(2H,s), 7.21 (1H,d,J=5.2Hz), 7.25(4H,s), 7.38(5H,s), 8.37(1H,d,J=5.2Hz).

Reference Example 134:

4-[3,5-Bis(trifluoromethyl)benzyl]2,3,4,5-tetrahydro-5-oxo-6-(4-methylphenyl)pyrido[3,2-f][1,4]oxazepine
m.p. 212-213 °C (recrystallized from acetone-isopropyl ether)
NMR(200MHz, $CDCl_3$) ppm: 2.40(3H,s), 3.70(2H,t,J=5.6Hz), 4.47(2H,t,J=5.6Hz), 4.89(2H,s), 7.24(total 5H,m), 7.81(2H,s), 7.87(1H,s), 8.41(1H,d,J=5.2Hz).

Reference Example 135:

(S)-5-Benzyl-2,3,4,5-tetrahydro-3-methyl-7-(4-methylphenyl)-6-oxo-6H-pyrido[2,3-b][1,5]oxazocine
m.p. 148-149 °C (recrystallized from acetone-ethyl ether)
NMR(200MHz, $CDCl_3$) ppm: 0.83(3H,d,J=7.4Hz), 2.30-2.60(1H,b), 2.42(3H,s), 3.11(1H,d,J=15.4Hz), 3.40(1H,dd, J=15.4,10.4Hz), 3.75-4.00(2H,m), 4.59(1H,dd,J=12.4,4.8Hz), 5.50(1H,d,J=15.0Hz), 7.15(1H,d,J=4.8Hz), 7.20-7.40 (total 9H,m), 8.37(1H,d,J=4.8Hz).

Reference Example 136:

(S)-5-[3,5-Bis(trifluoromethyl)benzyl]2,3,4,5-tetrahydro-3-methyl-7-(4-methylphenyl)-6-oxo-6H-pyrido[2,3-b][1,5]oxazocine
m.p. 159-160 °C (recrystallized from acetone-isopropyl ether)
NMR(200MHz, $CDCl_3$) ppm: 0.86(3H,d,J=7.0Hz), 2.20-2.60(1H,b), 2.37(3H,s), 3.09(1H,d,J=15.4Hz), 3.58(1H,dd, J=15.4,10.4Hz), 3.89(1H,t,J=11.6Hz), 4.18(1H,d,J=15.4Hz), 4.62(1H,dd,J=12.2,5.2Hz), 5.53(1H,d,J=15.4Hz), 7.17 (total 5H,m), 7.72(2H,s), 7.84(1H,s), 8.40(1H,d,J=5.2Hz).

Reference Example 137

(9R)-7-[3, 5-bis(trifluoromethyl)benzyl]-6,7,8,9,10,11-hexahydro-5-(4-hydroxymethylphenyl)-9-methyl-6,13-dioxo-13H-[1,4]diazosino[2,1-g][1,7]napththylidine

Streptomyces subrutilus IFO 13388 was cultured at 28 °C for 14 days on an agar slant, pH 7.3, containing 0.4 % yeast extract, 1 % malt extract, 0.4 % glucose and 2 % agar (ISP Medium No. 2). Separately, a medium containing 0.5 % glucose, 5 % dextrin, 3.5 % soy bean meal and 0.7 % calcium carbonate was prepared, and 40 ml of the medium was introduced into 200 ml Erlenmeyer flask and sterilized with steam at 120 °C for 20 minutes. The cultured microorganism was inoculated via a loop of platinum into this medium and cultured at 28 °C for 48 hours under shaking. 1 ml of the resulting culture was transferred to each of 13 flasks containing the same medium and cultured at 28 °C for 48 hours under shaking. A DMSO solution (5.2 ml) of (9 R)-7-[3, 5-bis(trifluoromethyl)benzyl]-6, 7, 8, 9, 10, 11-hexahydro-9-methyl-5-(4-methylphenyl)-6, 13-dioxo-13H-[1, 4] diazosino [2, 1-g] [1, 7] napththylidine (104 mg) obtained in Reference Example 72 was added in an amount of 0.4 ml to each culture and reacted at 28 °C for 48 hours under shaking. After reaction, each culture was adjusted to pH 4 with 2 N sulfuric acid and extracted with 500 ml ethyl acetate.

The extract was concentrated, and the concentrate was separated and purified by column chromatography on silica gel (ethyl acetate-methanol = 9 : 1) whereby the title compound was obtained as colorless crystal (30 mg).
Melting point: 240-241 °C (recrystallized from ethyl acetate-ethyl ether).
NMR (200 MHz, $CDCl_3$) ppm: 0.91 (3H, d, J = 6.6 Hz), 1.5 - 1.9 (2H, m), 1.95 - 2.40 (2H, m), 2.98 (1H, d, J = 15 Hz), 3.35 - 3.65 (2H, m), 4.02 (1H, d, J = 15 Hz), 4.75 (2H, s), 5.10 (1H, dd, J = 15, 5.5 Hz), 5.46 (1H, d, J = 15 Hz), 6.97 (1H, d, J = 8.0 Hz), 7.26 (1H, d, J = 8.0 Hz), 7.4 - 7.6 (6H, m), 7.81 (1H, s), 8.93 (1H, dd, J = 4.0, 1.8 Hz)
Elementary analysis: $C_{30}H_{25}N_3O_3F_6$
Theoretical (%): C 61.12, H 4.27, N 7.13
Found (%): C 61.15, H 4.21, N 7.03
EI-MS m/z: 589($M^+$)[$(C_{30}H_{25}N_3O_3F_6)^+$].

Reference Example 138

(9R)-7-[3,5-bis(trifluoromethyl)benzyl]-5-(4-formylphenyl)-6,7,8,9,10,11-hexahydro-9-methyl-6,13-dioxo-13H-[1,4]diazosino[2,1-g][1,7]napththylidine

Streptomyces tanashiensis subsp. cephalomyceticus IFO 13929 was cultured at 28 °C for 14 days on an agar slant, pH 7.3, containing 0.4 % yeast extract, 1 % malt extract, 0.4 % glucose and 2 % agar (ISP Medium No. 2). Separately, a medium containing 0.5 % glucose, 5 % dextrin, 3.5 % soy bean meal and 0.7 % calcium carbonate was prepared, and 40 ml of the medium was introduced into 200 ml Erlenmeyer flask and sterilized with steam at 120 °C for 20 minutes. The cultured microorganism was inoculated via a loop of platinum into this medium and cultured at 28 °C for 48 hours under shaking. 1 ml of the resulting culture was transferred to each of 25 flasks containing the same medium and cultured at 28 °C for 48 hours under shaking. A DMSO solution (10 ml) of (9R)-7-[3, 5-bis(trifluoromethyl) benzyl]-6,7,8,9,10,11-hexahydro-9-methyl-5-(4-methylphenyl)-6,13-dioxo-13H-[1,4]diazosino[2,1-g][1,7]napththylidine (200 mg) obtained in Reference Example 72 was added in an amount of 0.4 ml to each culture and reacted at 28 °C for 48 hours under shaking. After reaction, each culture was adjusted to pH 4 with 2 N sulfuric acid and extracted

with 1 L ethyl acetate.

The extract was concentrated, and the concentrate was separated and purified by column chromatography on silica gel (ethyl acetate-methanol = 9 : 1) whereby colorless crystals containing the title compound were obtained. The structure of this product was determined by comparing its NMR spectrum with that of the product separately prepared in Reference Example 139.

Reference Example 139

(9R)-7-[3,5-bis(trifluoromethyl)benzyl]-5-(4-formylphenyl)-6,7,8,9,10,11-hexahydro-9-methyl-6,13-dioxo-13H-[1,4]diazosino[2,1-g][1,7]napththylidine

A mixture of (9R)-7-[3,5-bis(trifluoromethyl)benzyl]-6,7,8,9,10,11-hexahydro-5-(4-hydroxymethylphenyl)-9-methyl-6,13-dioxo-13H-[1,4]diazosino[2,1-g][1,7]napththylidine (109 mg) obtained in Reference Example 137, manganese dioxide (1.3 g) and dichloromethane (13 ml) was stirred at room temperature for 2 hours and the resulting precipitates were separated by filtration with Celite. The filtrate was concentrated whereby the title compound was obtained as colorless crystal (70 mg).
Melting point: 213-214 °C (recrystallized from ethyl acetate-ethyl ether).

NMR (200 MHz, $CDCl_3$) ppm: 0.95 (3H, d, J = 6.6 Hz), 1.60 - 1.85 (1H, m), 1.95 - 2.40 (2H, m), 3.05 (1H, d, J = 15 Hz), 3.35 - 3.65 (2H, m), 4.00 (1H, d, J = 15 Hz), 5.11 (1H, dd, J = 14, 5.7 Hz), 5.38 (1H, d, J = 15 Hz), 7.12 (1H, d, J = 8.4 Hz), 7.40 - 7.55 (4H, m), 7.60 - 7.75 (2H, m), 7.78 (1H, s), 7.96 (1H, d, J = 8.0 Hz), 8.94 (1H, m), 10.03 (1H, s)
Analyzed value of element: $C_{30}H_{23}N_3O_3F_6$
Calculated value (%): C 61.33, H 3.95, N 7.15
Measured value (%): C 61.25, H 4.04, N 7.11
EI-MS m/z: 587($M^+$)[$(C_{30}H_{23}N_3O_3F_6)^+$].

Reference Example 140

(9R)-7-[3,5-bis(trifluoromethyl)benzyl]-5-(4-carboxyphenyl)-6,7,8,9,10,11-hexahydro-9-methyl-6,13-dioxo-13H-[1,4]diazosino[2,1-g][1,7]napththylidine

Streptomyces lavenduligriseus IFO 13405 was cultured at 28 °C for 14 days on an agar slant, pH 7.3, containing 0.4 % yeast extract, 1 % malt extract, 0.4 % glucose and 2 % agar (ISP Medium No.2). Separately, a medium containing 0.5 % glucose, 5 % dextrin, 3.5 % soy bean meal and 0.7 % calcium carbonate was prepared, and 40 ml of the medium was introduced into 200 ml Erlenmeyer flask and sterilized with steam at 120 °C for 20 minutes. The cultured microorganism was inoculated via a loop of platinum into this medium and cultured at 28 °C for 48 hours under shaking. 1 ml of the resulting culture was transferred to each of 25 flasks containing the same medium and cultured at 28 °C for 48 hours under shaking. A DMSO solution (10 ml) of (9R)-7-[3,5-bis(trifluoromethyl)benzyl]-6,7,8,9,10,11-hexahydro-9-methyl-5-(4-methylphenyl)-6,13-dioxo-13H-[1,4]diazosino[2,1-g][1,7]napththylidine (200 mg) obtained in Reference Example 72 was added in an amount of 0.4 ml to each culture and reacted at 28 °C for 48 hours under shaking. After reaction, each culture was adjusted to pH 4 with 2 N sulfuric acid and extracted with 1 L ethyl acetate.

The extract was concentrated, and the concentrate was extracted with 1 N aqueous sodium hydroxide. The extract was adjusted to pH 3-4 with 2 N hydrochloric acid and extracted with ethyl acetate. The extract was washed with saturated saline and dried, and the solvent was distilled off. The residue was separated and purified by column chromatography on silica gel (ethyl acetate-methanol = 9 : 1) whereby the title compound was obtained as colorless crystal (58 mg).
Melting point: 300-301 °C (recrystallized from ethyl acetate-ethyl ether).

NMR (200 MHz, $CDCl_3$) ppm: 0.96 (3H, d, J = 6.6 Hz), 1.76 (1H, m), 1.95 - 2.40 (2H, m), 3.09 (1H, d, J = 15 Hz), 3.40 - 3.65 (2H, m), 4.02 (1H, d, J = 15 Hz), 5.12 (1H, dd, J = 14, 5.6 Hz), 5.36 (1H, d, J = 15 Hz), 7.04 (1H, dd, J = 8.0, 1.6 Hz), 7.45 - 7.65 (5H, m), 7.83 (1H, s), 7.91 (1H, d, J = 8.4 Hz), 8.20 (1H, dd, J = 8.4, 1.8 Hz), 8.97 (1H, dd, J = 3.6, 2.2 Hz)
EI-MS m/z: 603($M^+$)[$(C_{30}H_{23}N_3O_4F_6)^+$].

Reference Example 141

(9R)-7-[3,5-bis(trifluoromethyl)benzyl]-6,7,8,9,10,11-hexahydro-9-methyl-5-(4-methylphenyl)-6,13-dioxo-13H-[1,4] diazosino[2,1-g][1,7]napththylidine N-oxide

A mixture of (9R)-7-[3,5-bis(trifluoromethyl)benzyl]-6,7,8,9,10,11-hexahydro-9-methyl-5-(4-methylphenyl)-6,13-dioxo-13H-[1, 4]diazosino[2, 1-g][1, 7]napththylidine (1.60 g) obtained in Reference Example 72, m-chloroperbenzoic acid (1.2 g) and dichloromethane (20 ml) was stirred at room temperature for 3 hours, and the solvent was distilled off. The residue was dissolved in ethyl acetate, washed with aqueous potassium carbonate and saturated saline, and dried. The solvent was distilled off whereby the title compound was obtained as pale yellow crystal (0.73 g).
Melting point: 237-239 °C (recrystallized from ethyl acetate-ethyl ether).

NMR (200 MHz, $CDCl_3$) ppm: 0.91 (3H, d, J = 6.6 Hz), 1.73 (1H, m), 1.9 - 2.4 (2H, m), 2.36 (3H, s), 2.97 (1H, d, J = 15 Hz), 3.3 - 3.5 (2H, m), 3.97 (1H, d, J = 15 Hz), 4.97 (1H, dd, J = 14, 5.2 Hz), 5.41 (1H, d, J = 15 Hz), 6.82 (2H, m), 7.04 (1H, d, J = 7.4 Hz), 7.15 - 7.35 (3H, m), 7.44 (2H, s), 7.81 (1H, s), 8.31 (1H, dd, J = 5.4, 0.8 Hz)
Analyzed value of element: $C_{30}H_{25}N_3O_3F_6$
Calculated value (%): C 61.12, H 4.27, N 7.13
Measured value (%): C 60.77, H 4.55, N 7.00

Reference Example 142

(9R)-7-[3,5-bis(trifluoromethyl)benzyl]-6,7,8,9,10,11-hexahydro-5-(4-hydroxymethylphenyl)-9-methyl-6,13-dioxo-13H-[1,4]diazosino[2,1-g][1,7]napththylidine N-oxide

A mixture of (9R)-7-[3,5-bis(trifluoromethyl)benzyl]-6,7,8,9,10,11-hexahydro-5-(4-hydroxymethylphenyl)-9-methyl-6,13-dioxo-13H-[1,4]diazosino[2,1-g][1,7]napththylidine (100 mg) obtained in Reference Example 137, 4-dimethylaminopyridine (catalystamount), acetic anhydride (0.1 ml) and pyridine (3 ml) was stirred at room temperature for 16 hours, and the solvent was distilled off. After ethyl acetate was added, the residue was washed with dilute hydrochloric acid and saturated saline and dried, and the solvent was distilled off, and the residue was dissolved in dichloromethane (10 ml). m-Chloroperbenzoic acid (102 mg) was added to this solution, and the mixture was stirred at room temperature for 1 hour. The reaction solution was diluted with dichloromethane, washed with 0.1 N aqueous sodium hydroxide and dried, and the solvent was distilled off. Methanol (10 ml) and 1 N aqueous sodium hydroxide (2 ml) were added to the residue, then the mixture was stirred at room temperature for 30 minutes, water was added to it, and the product was extracted with ethyl acetate. The extract was washed with saturated saline and dried, and the solvent was distilled off whereby the title compound was obtained as pale yellow crystal (28 mg).
Melting point: 240-243 °C (recrystallized from ethyl acetate-ethyl ether).

NMR (200 MHz, $CDCl_3$) ppm: 0.91 (3H, d, J = 6.2 Hz), 1.5 - 2.4 (4H, m), 2.98 (1H, d, J = 15 Hz), 3.44 (2H, m), 4.00 (1H, d, J = 15 Hz), 4.74 (2H, s), 4.98 (1H, m), 5.40 (1H, d, J = 15 Hz), 6.88 (1H, m), 7.26 (1H, d, J = 7.4 Hz), 7.45 (6H, m), 7.81 (1H, s), 8.32 (1H, m)

Reference Example 143

(9R)-7-[3,5-bis(trifluoromethyl)benzyl]-5-(4-carboxyphenyl)-6,7,8,9,10,11-hexahydro-9-methyl-6,13-dioxo-13H-[1,4] diazosino[2,1-g][1,7]napththylidine N-oxide

Ethyl ether (10 ml) containing diazomethane (prepared using N-nitrosomethyl urea and potassium hydroxide) was added to a THF solution (20 ml) of (9R)-7-[3,5-bis(trifluoromethyl)benzyl]-5-(4-carboxyphenyl)-6,7,8,9,10,11-hexahydro-9-methyl-6,13-dioxo-13H-[1,4]diazosino[2,1-g][1,7]napththylidine (200 mg) obtained in Reference Example 140, and the mixture was stirred at room temperature for 30 minutes. After the solvent was distilled off, the residue was dissolved in dichloromethane (20 ml). m-Chloroperbenzoic acid (0.6 g) was added little by little to this solution, and the mixture was stirred at room temperature for 2 hours. The reaction solution was diluted with dichloromethane, washed with 1 N aqueous sodium hydroxide and water and dried, and the solvent was distilled off. Methanol (20 ml) and 1 N aqueous sodium hydroxide (5 ml) were added to the residue, and the mixture was heated for 30 minutes under reflux. After the solvent was distilled off, water was added to the residue, then water was added to the residue, and the extract was adjusted to pH 2-3 with 2 N hydrochloric acid. This solution was extracted with ethyl acetate, and the extract was washed with saturated saline and dried, and the solvent was distilled off whereby the title compound was obtained as pale yellow crystal (88 mg).

NMR (200 MHz, $CDCl_3$) ppm: 0.95 (3H, d, J = 6.6 Hz), 1.75 (1H, m), 1.9 - 2.4 (2H, m), 3.08 (1H, d, J = 15 Hz), 3.47 (2H, m), 4.00 (1H, d, J = 15 Hz), 5.00 (1H, dd, J = 14, 6.0 Hz), 5.29 (1H, d, J = 15 Hz), 6.80 (1H, d, J = 8.4 Hz),

7.02 (1H, d, J = 7.3 Hz), 7.28 (1H, dd, J = 7.0, 6.6 Hz), 7.46 (2H, s), 7.54 (1H, d, J = 7.0 Hz), 7.81 (1H, s), 7.88 (1H, d, J = 7.3 Hz), 8.16 (1H, d, J = 8.4 Hz), 8.39 (1H, d, J = 6.6 Hz).

Reference Example 144

N-[3,5-bis(trifluoromethyl)benzyl]-2-chloro-N-[(S)-3-hydroxy-2-methylpropyl]-4-(4-methylphenyl)-3-pyridine carboxamide

NMR (200 MHz, $CDCl_3$) ppm: 0.54 (3H × 1/4, d, J = 7.0 Hz), 0.63 (3H × 1/4, d, J = 7.0 Hz), 0.79 (3H × 1/4, d, J = 7.0 Hz), 0.84 (3H × 1/4, d, J = 7.0 Hz), 1.50 - 1.90 (1H, m), 2.25 - 2.45 (3H, m), 2.45 - 3.90 (total 5H, m), 4.05 - 4.45 (1H, m), 4.50 - 4.95 (1H, m), 7.00 - 7.20 (1H, m), 7.20 - 7.50 (total 5H, m), 7.70 - 7.85 (2H, m), 8.42 (1H, m). (Mixture of amide rotational isomers (1 : 1)).

Reference Example 145

(9R)-[10,10,11,11-$^2H_4$]-7-[3,5-bis(trifluromethyl)benzyl]-8,9,10,11-tetrahydro-9-methyl-5-(4-methylphenyl)-7H-[1,4] diazosino[2,1-g][1,7]naphthylidine-6, 13-dione ($d_4$ derivative of the compound in Reference Example 72)

(Step 1)

(S)-(+)-3-hydroxy-2-methyl propionic acid methyl ester

An ethyl ether solution (60 ml) of THP-ether (39.5 g) (prepared according to a method described in the literature [Joji Mori, Tetrahedron, 39, 3107-3109 (1983): a process of synthesizing enantiomers is described]) was added gradually to a suspension of deuterated lithium aluminum (5.80 g) in ethyl ether (200 ml) at 0 °C under vigorous stirring. Then, the reaction mixture was stirred at room temperature for 1 hour and cooled again on ice-cold water, and a mixed solution of 1 N aqueous sodium hydroxide (24 ml) and THF (24 ml) was added to it under stirring. The resulting precipitates were removed by filtration through celite, and the filtrate was dried, and the solvent was distilled off whereby (S)-[1, 1-$^2H_2$]-2-methyl-1,3-propanediol mono-THP ether was obtained as colorless oily matter (35.6 g).

NMR (200 MHz, $CDCl_3$) ppm: 0.90 (3H × 1/2, d, J = 7.0 Hz), 0.91 (3H × 1/2, d, J = 7.0 Hz), 1.4 - 1.9 (6H, m), 2.01 (1H, m), 2.74 (1H, bs), 3.3 - 3.6 (3H, m), 3.89 (1H, m), 4.58 (1H, b).

(Step 2)

p-Toluenesulfonyl chloride (39 g) was added under cooling and stirring to a pyridine solution (150 ml) of the compound (35.6 g) obtained in Step 1. This mixture was stirred at room temperature overnight, then diluted with ethyl ether, washed with water, dilute hydrochloric acid and saline and dried, and the solvent was distilled off. The residue was dissolved in DMSO (150 ml), and sodium cyanide (13 g) was added to it, and the mixture was stirred at room temperature for 6 hours. After dilution with hexane, the solution was washed with water and dried, and the solvent was distilled off whereby (R)-[2,2-$^2H_2$]-4-hydroxy-3-methylbutanenitrile THP ether was obtained as pale yellow oily matter (19.1 g).

NMR (200 MHz, $CDCl_3$) ppm: 1.09 (3H × 1/2, d, J = 6.0 Hz), 1.10 (3H × 1/2, d, J = 6.8 Hz), 1.5 - 1.9 (6H, m), 2.16 (1H, m), 3.16 - 3.88 (4H, m), 4.60 (1H, b).

(Step 3)

An ethyl ether solution (80 ml) of the compound (19.1 g) obtained in Step 2 was added gradually at 0 °C under vigorous stirring to a suspension of deuterated lithium aluminum (4.85 g) in ethyl ether (200 ml). Then, the reaction mixture was stirred at room temperature for 1 hour and cooled again on ice-cold water, and a mixed solution of 1 N aqueous sodium hydroxide (20 ml) and THF (20 ml) was added to it under stirring. The resulting precipitates were removed by filtration through Celite, and the filtrate was dried and the solvent was distilled off whereby (R)-[3, 3, 4, 4-$^2H_4$]-4-amino-2-methyl-1-butanol THP ether was obtained as pale yellow oily matter (19.3 g).

NMR (200 MHz, $CDCl_3$) ppm: 0.93 (3H × 1/2, d, J = 6.8 Hz), 0.94 (3H × 1/2, d, J = 6.8 Hz), 1.2 - 1.9 (9H, m), 3.13 - 3.27 (1H, m), 3.45 - 3.64 (2H, m), 3.86 (1H, m), 4.57 (1H, b).

(Step 4)

The compound obtained in Step 3 and the compound obtained in Reference Example 3 were reacted and

treated in the same manner as in Reference Example 5 whereby (R)-N-[3,5-(bistrifluoromethyl)benzyl]-7,8-dihydro-7-([1,1,2,2-$^2H_4$]-4-hydroxy-3-methylbutyl)-5-(4-methylphenyl)-8-oxo-1,7-naphthylidine-6-carboxamide was obtained as colorless crystal.
Melting point: 205-207 °C (recrystallized from ethyl acetate-ethyl ether).
NMR (200 MHz, $CDCl_3$) ppm: 0.78 (3H, d, J = 7.0 Hz), 1.55 (1H, m), 2.28 (3H, s), 3.14 (1H, b, OH), 3.2 - 3.5 (2H, m), 4.50 (2H, d, J = 6.0 Hz), 7.0 - 7.3 (4H, m), 7.29 (1H, dd, J = 8.4, 4.4 Hz), 7.54 (1H, dd, J = 8.4, 1.6 Hz), 7.69 (2H, s), 7.78 (1H, s), 8.55 (1H, t, J = 6.0 Hz), 8.61 (1H, dd, J = 4.4, 1.6 Hz).
Analyzed value of element: $C_{30}H_{23}D_4F_6N_3O_3 \cdot 1/2H_2O$
Calculated value (%): C 59.60, H 4.00, D 1.33, N 6.95
Measured value (%): C 59.94, H 3.82, D 1.29, N 7.13.

(Step 5)

The compound obtained in Step 4 was reacted and treated in the same manner as in Reference Example 69 whereby the title compound was obtained as colorless crystal.
Melting point: 227-229 °C (recrystallized from ethyl acetate-methanol-ethyl ether).
NMR (200 MHz, $CDCl_3$) ppm: 0.91 (3H, d, J = 7.0 Hz), 2.08 (1H, m), 2.37 (3H, s), 2.97 (1H, dd, J = 15, 1.4 Hz), 3.45 (1H, dd, J = 15, 11 Hz), 3.99 (1H, d, J = 15 Hz), 5.46 (1H, d, J = 15 Hz), 6.83 (1H, dd, J = 7.8, 1.8 Hz), 7.05 (1H, d, J = 7.8 Hz), 7.26 (1H, d, J = 7.8 Hz), 7.34 (1H, dd, J = 7.8, 1.8 Hz), 7.47 (1H, dd, J = 8.6, 4.4 Hz), 7.48 (2H, s), 7.56 (1H, dd, J = 8.6, 1.8 Hz), 7.82 (1H, s), 8.91 (1H, dd, J = 4.1, 1.8 Hz).
Analyzed value of element: $C_{30}H_{21}D_4F_6N_3O_2$
Calculated value (%): C 62.39, H 3.66, D 1.39, N 7.28
Measured value (%): C 62.41, H 3.65, D 1.35, N, 7.21.
EI-MS m/z: 577($M^+$), 558, 350, 313
$[\alpha]_D$: +116.6° (c = 0.541, MeOH).

Reference Example 146

(9R)-[10,10,11,11-$^2H_4$]-7-[3,5-bis(trifluoromethyl)benzyl]-8,9,10,11-tetrahydro-5-(4-hydroxymethylphenyl)-9-methyl-7H-[1,4]diazosino[2,1-g][1,7]napththylidine-6,13-dione ($d_4$ derivative of the compound of Reference Example 137)

The compound obtained in Reference Example 145 was reacted and treated in the same manner as in Reference Example 137 whereby the title compound was obtained as colorless crystal.
Melting point: 241-242 °C (recrystallized from ethyl acetate-methanol-ethyl ether).
NMR (200 MHz, $CDCl_3$) ppm: 0.91 (3H, d, J = 7.0 Hz), 1.85 (1H, m), 2.09 (1H, m), 2.98 (1H, dd, J = 15, 1.4 Hz), 3.47 (1H, dd, J = 15, 11 Hz), 4.02 (1H, d, J = 15 Hz), 4.75 (2H, d, J = 4.4 Hz), 5.45 (1H, d, J = 15 Hz), 6.97 (1H, d, J = 8.0 Hz), 7.25 (1H, d, J = 8.0 Hz), 7.4 - 7.6 (6H, m), 7.81 (1H, s), 8.91 (1H, dd, J = 4.0, 2.2 Hz).
Analyzed value of element: $C_{30}H_{21}D_4F_6N_3O_3 \cdot 1/2H_2O$
Calculated value (%): C 59.80, H 3.68, D 1.34, N 6.97
Measured value (%): C 59.76, H 3.78, D 1.40, N 6.73.
EI-MS m/z: 593($M^+$), 554, 519, 366, 313
$[\alpha]_D$: +94.2° (c = 0.538, MeOH).

Reference Example 147

(9R)-[10,10,11,11-$^2H_4$]-7-[3,5-bis(trifluoromethyl)benzyl]-5-(4-formylphenyl)-8,9,10,11-tetrahydro-9-methyl-7H-[1,4] diazosino[2,1-g][1,7]napththylidine-6,13-dione ($d_4$ derivative of the compound in Reference Example 138)

The compound obtained in Reference Example 146 was reacted and treated in the same manner as in Reference Example 138 or 139 whereby the title compound was obtained as colorless crystal.
Melting point: 215-216 °C (recrystallized from ethyl acetate-ethyl ether).
NMR (200 MHz, $CDCl_3$) ppm: 0.95 (3H, d, J = 7.0 Hz), 2.10 (1H, m), 3.05 (1H, dd, J = 15, 1.4 Hz), 3.47 (1H, dd, J = 15, 11 Hz), 4.00 (1H, d, J = 15 Hz), 5.39 (1H, d, J = 15 Hz), 7.12 (1H, dd, J = 8.0, 1.8 Hz), 7.40 - 7.55 (4H, m), 7.62 - 7.74 (2H, m), 7.79 (1H, s), 7.97 (1H, dd, J = 8.0, 1.8 Hz), 8.94 (1H, dd, J = 4.0, 1.8 Hz), 10.04 (1H, s).
Analyzed value of element: $C_{30}H_{19}D_4F_6N_3O_3$
Calculated value (%): C 60.91, H 3.24, D 1.36, N 7.10
Measured value (%): C 60.89, H 3.31, D 1.36, N 6.99.
EI-MS m/z: 591($M^+$), 572, 517, 364, 313

$[\alpha]_D$: +106.1° (c = 0.510, MeOH).

Reference Example 148

(9R)-[10,10,11,11-$^2H_4$]-7-[3,5-bis(trifluoromethyl)benzyl]-5-(4-carboxyphenyl)-8,9,10,11-tetrahydro-9-methyl-7H-[1,4] diazosino[2,1-g][1,7]napththylidine-6,13-dione ($d_4$ derivative of the compound in Reference Example 140)

Potassium permanganate (1.7 g) was added little by little to a mixture of the compound (2.0 g) obtained in Reference Example 146, t-butanol (80 ml) and 0.3 N aqueous sodium hydroxide (55 ml) at 0 °C under stirring. The mixture was stirred at room temperature for 1 hour, and ethanol (10 ml) was added to it, and the mixture was stirred at room temperature for 30 minutes. The resulting precipitates were removed by filtration through Celite and washed with ethanol. The filtrate and the wash were combined, then the solvent was distilled off, and the residue was dissolved in 1 N aqueous sodium hydroxide. This solution was washed with a mixed solution of ethyl ether and THF, then acidified with 2 N hydrochloric acid, and extracted with ethyl acetate. The extract was washed with saline and dried, and the solvent was distilled off whereby the title compound was obtained as colorless crystal (1.71 g).
Melting point: 302-303 °C (recrystallized from ethyl acetate-methanol-ethyl ether).
NMR (200 MHz, $CDCl_3$) ppm: 0.95 (3H, d, J = 6.6 Hz), 2.09 (1H, m), 3.09 (1H, d, J = 14 Hz), 3.49 (1H, dd, J = 14, 9.4 Hz), 4.02 (1H, d, J = 15 Hz), 5.36 (1H, d, J = 15 Hz), 7.04 (1H, d, J = 8.0 Hz), 7.50 (4H, m), 7.59 (1H, d, J = 8.4 Hz), 7.82 (1H, s), 7.91 (1H, d, J = 8.4 Hz), 8.20 (1H, d, J = 8.0 Hz), 8.97 (1H, dd, J = 3.6, 2.2 Hz).
Analyzed value of element: $C_{30}H_{19}D_4F_6N_3O_4 \cdot 1/2H_2O$
Calculated value (%): C 58.44, H 3.27, D 1.31, N 6.82
Measured value (%): C 58.47, H 3.21, D 1.32, N 6.89.
EI-MS m/z: 607($M^+$), 568, 533, 380, 313
$[\alpha]_D$: +123.2° (c = 0.545, MeOH).

Reference Example 149

(R)-N-[3,5-di(benzyloxy)benzyl]-7,8-dihydro-7-(4-hydroxy-3-methylbutyl)-5-(4-methylphenyl)-8-oxo-6-pyrido[3,4-b] pyridine carboxamide

(Step 1)

The compound obtained in Step 2 in Reference Example 2 and 3, 5-di(benzyloxy)benzylamine were reacted and treated in the same manner as in Step 4 in Reference Example 2 whereby N-[3,5-di(benzyloxy)benzyl]-5-(4-methylphenyl)-8-oxo-8H-pyrano[3,4-b]pyridine-6-carboxamide was obtained as colorless crystal.
Melting point: 177-179 °C (recrystallized from ethyl acetate-ethyl ether).
NMR (200 MHz, $CDCl_3$) ppm: 2.43 (3H, s), 4.40 (2H, d, J = 5.8 Hz), 5.01 (4H, s), 6.51 (3H, m), 7.1 - 7.5 (15H, m), 7.57 (2H, m), 8.94 (1H, dd, J = 3.6, 2.2 Hz)
Analyzed value of element: $C_{37}H_{30}N_2O_5$
Calculated value (%): C 76.27, H 5.19, N 4.81
Measured value (%): C 76.36, H 5.11, N 4.78.

(Step 2)

The compound obtained in Step 1 and (R)-4-amino-2-methyl-1-butanol THP ether were reacted and treated in the same manner as in Reference Example 19 whereby the title compound was obtained as colorless crystal.
Melting point: 168-170 °C (recrystallized from ethyl acetate-ethyl ether).
NMR (200 MHz, $CDCl_3$) ppm: 0.83 (3H, d, J = 6.6 Hz), 1.5 - 1.9 (3H, m), 2.30 (3H, s), 2.99 (1H, m), 3.2 - 3.6 (2H, m), 3.75 - 3.95 (2H, m), 4.21 (2H, d, J = 6.0 Hz), 4.98 (4H, s), 6.33 (2H, d, J = 1.8 Hz), 6.50 (1H, bt), 7.1 - 7.5 (16H, m), 7.58 (1H, d, J = 8.4 Hz), 8.67 (1H, dd, J = 4.4, 1.2 Hz)
Analyzed value of element: $C_{42}H_{41}N_3O_5 \cdot 0.3H_2O$
Calculated value (%): C 74.93, H 6.23, N 6.24
Measured value (%): C 74.80, H 6.25, N 6.28.

Reference Example 150

(R)-N-(3,5-dimethoxybenzyl)-7,8-dihydro-7-(4-hydroxy-3-methylbutyl)-8-oxo-5-phenyl-6-pyrido[3,4-b]pyridine carboxamide

(Step 1)

The compound obtained in Step 2 in Reference Example 9 and 3, 5-dimethoxybenzylamine were reacted and treated in the same manner as in Step 4 in Reference Example 2 whereby N-(3,5-dimethoxybenzyl)-8-oxo-5-phenyl-8H-pyrano[3,4-b]pyridine-6-carboxamide was obtained as colorless crystal.
Melting point: 126-127 °C (recrystallized from ethyl acetate-ethyl ether).
NMR (200 MHz, $CDCl_3$) ppm: 3.78 (6H, s), 4.40 (2H, d, J = 5.8 Hz), 6.41 (3H, m), 7.20 - 7.35 (3H, m), 7.45 - 7.65 (5H, m), 8.95 (1H, dd, J = 4.0, 1.4 Hz)
Analyzed value of element: $C_{24}H_{20}N_2O_5$
Calculated value (%): C 69.22, H 4.84, N 6.73
Measured value (%): C 69.27, H 4.72, N 6.83.

(Step 2)

The compound obtained in Step 1 and (R)-4-amino-2-methyl-1-butanol THP ether were reacted and treated in the same manner as in Example 19 whereby the title compound was obtained as colorless crystal.
Melting point: 150-151 °C (recrystallized from ethyl acetate-ethyl ether).
NMR (200 MHz, $CDCl_3$) ppm: 0.83 (3H, d, J = 6.6 Hz), 1.5 - 1.9 (3H, m), 3.09 (1H, m), 3.2 - 3.6 (2H, m), 3.6 - 3.9 (2H, m), 3.76 (6H, s), 4.20 (2H, d, J = 5.8 Hz), 6.27 (2H, d, J = 2.2 Hz), 6.35 (1H, t, J = 2.2 Hz), 7.2 - 7.7 (8H, m), 8.61 (1H, dd, J = 4.4, 1.4 Hz)
Analyzed value of element: $C_{29}H_{31}N_3O_5$
Calculated value(%): C 69.44, H 6.23, N 8.38
Measured value(%): C 69.11, H 6.04, N 8.51.

Reference Example 151

(R)-N-[3, 5-bis(trifluoromethyl)benzyl)-5-(4-chlorophenyl)-7,8-dihydro-7-(4-hydroxy-3-methylbutyl)-8-oxo-6-pyrido [3,4-b]pyridine carboxamide

(Step 1)

In place of 3-(4-methylbenzoyl)-2-pyridinecarboxylic acid in Step 1 in Reference Example 2, 3-(4-chlorobenzoyl)-2-pyridinecarboxylic acid was reacted and treated in the same manner as in Steps 1 and 2 in Reference Example 2 whereby 5-(4-chlorophenyl)-8-oxo-8 H-pyrano [3, 4-b] pyridine-6-carboxylic acid was obtained as colorless crystal.
NMR (200 MHz, $CDCl_3$ + DMSO-$d_6$) ppm: 7.24 (2H, d, J = 8.0 Hz), 7.47 (2H, d, J = 8.0 Hz), 7.50 (1H, d, J = 8.0 Hz), 7.63 (1H, dd, J = 8.0, 4.4 Hz), 8.96 (1H, d, J = 4.4 Hz).

(Step 2)

The compound obtained in Step 1 and 3,5-bis(trifluoromethyl) benzylamine were reacted and treated in the same manner as in Step 4 in Reference Example 2 whereby N-[3,5-bis(trifluoromethyl)benzyl]-5-(4-chlorophenyl)-8-oxo-8H-pyrano[3,4-b]pyridine-6-carboxamide was obtained as colorless crystal.
Melting point: 217-219 °C (recrystallized from ethyl acetate-isopropyl ether).
NMR (200 MHz, $CDCl_3$) ppm: 4.63 (2H, d, J = 6.2 Hz), 7.23 (2H, d, J = 8.2 Hz), 7.51 (2H, d, J = 8.2 Hz), 7.53 (1H, dd, J = 8.4, 1.6 Hz), 7.65 (1H, dd, J = 8.4, 4.4 Hz), 7.6 - 7.8 (1H, m), 7.73 (2H, s), 7.80 (1H, s), 8.98 (1H, dd, J = 4.4, 1.6 Hz).

(Step 3)

The compound obtained in Step 2 and (R)-4-amino-2-methyl-1-butanol THP ether were reacted and treated in the same manner as in Reference Example 19 whereby the title compound was obtained as colorless crystal.
Melting point: 259-261 °C (recrystallized from ethyl acetate-isopropyl ether).
NMR (200 MHz, $CDCl_3$) ppm: 0.78 (3H, d, J = 6.2 Hz), 1.4 - 1.8 (3H, m), 3.02 (1H, m), 3.2 - 3.7 (4H, m), 4.48

(2H, m), 7.1 - 7.4 (5H, m), 7.46 (1H, d, J = 8.0 Hz), 7.72 (2H, s), 7.85 (1H, s), 8.60 (2H, m).

Reference Example 152

(R)-N-[3, 5-bis(trifluoromethyl)benzyl]-5-(3,4-dichlorophenyl)-7,8-dihydro-7-(4-hydroxy-3-methylbutyl)-8-oxo-6-pyrido [3,4-b]pyridine carboxamide

(Step 1)

In place of 3-(4-methylbenzoyl)-2-pyridinecarboxylic acid in Step 1 in Reference Example 2,3-(3,4-dichlorobenzoyl)-2-pyridinecarboxylic acid was reacted and treated in the same manner as in Steps 1 and 2 in Reference Example 2 whereby 5-(3,4-dichlorophenyl)-8-oxo-8H-pyrano[3,4-b]pyridine-6-carboxylic acid was obtained as colorless crystal.

NMR (200 MHz, DMSO-$d_6$) ppm: 7.34 (1H, dd, J = 8.2, 1.8 Hz), 7.50 (1H, dd, J = 8.4, 1.4 Hz), 7.66 (1H, d, J = 1.8 Hz), 7.72 - 7.85 (2H, m), 8.96 (1H, dd, J = 4.4, 1.4 Hz).

(Step 2)

The compound obtained in Step 1 and 3,5-bis(trifluoromethyl) benzylamine were reacted and treated in the same manner as in Step 4 in Reference Example 2 whereby N-[3,5-bis(trifluoromethyl)benzyl]-5-(3,4-dichlorophenyl)-8-oxo-8H-pyrano[3,4-b]pyridine-6-carboxamide was obtained as colorless crystal.
Melting point: 220-221 °C (recrystallized from ethyl acetate-isopropyl ether).

NMR (200 MHz, $CDCl_3$) ppm: 4.64 (2H, d, J = 6.2Hz), 7.15 (1H, dd, J = 8.4, 1.8 Hz), 7.39 (1H, d, J = 1.8 Hz), 7.5 - 7.8 (4H, m), 7.75 (2H, s), 7.81 (1H, s), 8.99 (1H, dd, J = 4.4, 1.4 Hz).

(Step 3)

The compound obtained in Step 2 and (R)-4-amino-2-methyl-1-butanol THP ether were reacted and treated in the same manner as in Reference Example 19 whereby the title compound was obtained. This compound was subjected without purification to the reaction in Example 7.

Reference Example 153

(R)-5-(3,4-dichlorophenyl)-N-(3,5-dimethoxybenzyl)-7,8-dihydro-7-(4-hydroxy-3-methylbutyl)-8-oxo-6-pyrido [3,4-b] pyridine carboxamide

(Step 1)

The compound obtained in Step 1 in Reference Example 152 and 3, 5-dimethoxybenzylamine were reacted and treated in the same manner as in Step 4 in Reference Example 2 whereby 5-(3,4-dichlorophenyl)-N-(3,5-dimethoxybenzyl)-8-oxo-8H-pyrano[3,4-b]pyridine-6-carboxamide was obtained as colorless crystal.
Melting point: 220-221 °C (recrystallized from ethyl acetate-ethyl ether).

NMR (200 MHz, $CDCl_3$) ppm: 3.78 (6H, s), 4.41 (2H, d, J = 6.0 Hz), 6.38 - 6.48 (3H, m), 7.15 (1H, dd, J = 8.4, 1.8 Hz), 7.34 - 7.82 (5H, m), 8.96 (1H, dd, J = 4.2, 1.6 Hz) Analyzed value of element: $C_{24}H_{18}N_2O_5Cl_2$
Calculated value(%): C 59.40, H 3.74, N 5.77
Measured value(%): C 59.13, H 3.81, N 5.77.

(Step 2)

The compound obtained in Step 1 and (R)-4-amino-2-methyl-1-butanol THP ether were reacted and treated in the same manner as in Reference Example 19 whereby the title compound was obtained. This compound was subjected without purification to the reaction in Example 8.

Reference Example 154

(R)-N-(3,5-dimethylbenzyl)-7,8-dihydro-7-(4-hydroxy-3-methylbutyl)-5-(4-methylphenyl)-8-oxo-6-pyrido[3,4-b] pyridine carboxamide

(Step 1)

The compound obtained in Step 2 in Reference Example 2 and 3, 5-dimethylbenzylamine were reacted and treated in the same manner as in Step 4 in Reference Example 2 whereby N-(3,5-dimethylbenzyl)-5-(4-methylphenyl)-8-oxo-8H-pyrano[3,4-b]pyridine-6-carboxamide was obtained as colorless crystal.
Melting point: 201-202 °C (recrystallized from ethyl acetate-isopropyl ether).
NMR (200 MHz, $CDCl_3$) ppm: 2.30 (6H, s), 2.45 (3H, s), 4.39 (2H, d, J = 5.8 Hz), 6.88 (2H, s), 6.93 (1H, s), 7.17 (2H, d, J = 8.0 Hz), 7.19 (1H, m), 7.33 (2H, d, J = 8.0 Hz), 7.57 (2H, m), 8.93 (1H, dd, J = 4.0, 2.2 Hz)
Analyzed value of element: $C_{25}H_{22}N_2O_3$
Calculated value (%): C 75.36, H 5.57, N 7.03
Measured value (%): C 74.93, H 5.58, N 7.00.

(Step 2)

The compound obtained in Step 1 and (R)-4-amino-2-methyl-1-butanol THP ether were reacted and treated in the same manner as in Reference Example 19 whereby the title compound was obtained as colorless crystal.
Melting point: 193-194 °C (recrystallized from ethyl acetate-isopropyl ether).
NMR (200 MHz, $CDCl_3$) ppm: 0.86 (3H, d, J = 6.6 Hz), 1.5 - 2.0 (3H, m), 2.25 (6H, s), 2.42 (3H, s), 3.14 (1H, m), 3.2 - 3.4 (2H, m), 3.89 (2H, m), 4.20 (2H, d, J = 5.4 Hz), 6.61 (2H, s), 6.87 (1H, s), 7.1 - 7.4 (6H, m), 7.58 (1H, dd, J = 8.4, 1.4 Hz), 8.62 (1H, dd, J = 4.4, 1.4 Hz)
Analyzed value of element: $C_{30}H_{33}N_3O_3 \cdot 0.3\ H_2O$
Calculated value (%): C 73.69, H 6.93, N 8.59
Measured value (%): C 73.85, H 6.95, N 8.52.

Reference Example 155

(R)-N-(3,5-dichlorobenzyl)-7,8-dihydro-7-(4-hydroxy-3-methylbutyl)-5-(4-methylphenyl)-8-oxo-6-pyrido[3,4-b]pyridine carboxamide

(Step 1)

The compound obtained in Step 2 in Reference Example 2 and 3, 5-dichlorobenzylamine were reacted and treated in the same manner as in Step 4 in Reference Example 2 whereby N-(3,5-dichlorobenzyl)-5-(4-methylphenyl)-8-oxo-8H-pyrano[3,4-b]pyridine-6-carboxamide was obtained as colorless crystal.
Melting point: 232-233 °C (recrystallized from THF-isopropyl ether).
NMR (200 MHz, $CDCl_3$) ppm: 2.45 (3H, s), 4.44 (2H, d, J = 6.4 Hz), 7.1 - 7.3 (5H, m), 7.34 (2H, d, J = 7.6 Hz), 7.43 (1H, bt), 7.59 (2H, m), 8.95 (1H, dd, J = 4.0, 2.2 Hz)
Analyzed value of element: $C_{23}H_{16}N_2O_3Cl_2$
Calculated value (%): C 62.89, H 3.67, N 6.38
Measured value (%): C 62.62, H 3.70, N 6.36.

(Step 2)

The compound obtained in Step 1 and (R)-4-amino-2-methyl-1-butanol THP ether were reacted and treated in the same manner as in Reference Example 19 whereby the title compound was obtained as colorless crystal.
Melting point: 123-125 °C (recrystallized from ethyl acetate-isopropyl ether).
NMR (200 MHz, $CDCl_3$) ppm: 0.81 (3H, d, J = 7.0 Hz), 1.5 - 2.0 (3H, m), 2.39 (3H, s), 3.17 (1H, m), 3.2 - 3.8 (4H, m), 4.34 (2H, d, J = 6.2 Hz), 6.95 (2H, d, J = 2.0 Hz), 7.1 - 7.4 (5H, m), 7.31 (1H, dd, J = 8.2, 4.4 Hz), 7.56 (1H, dd, J = 8.2, 1.6 Hz), 8.31 (1H, bt), 8.62 (1H, dd, J = 4.4, 1.6 Hz)
Analyzed value of element: $C_{28}H_{27}N_3O_3Cl_2$
Calculated value (%): C 64.13, H 5.19, N 8.01
Measured value (%): C 63.82, H 5.01, N 7.96.

Reference Example 156

(R)-5-(3,4-dichlorophenyl)-N-(3,5-dimethylbenzyl)-7,8-dihydro-7-(4-hydroxy-3-methylbutyl)-8-oxo-6-pyrido[3,4-b] pyridine carboxamide

(Step 1)

The compound obtained in Step 1 in Reference Example 152 and 3,5-dimethylbenzylamine were reacted and treated in the same manner as in Step 4 in Reference Example 2 whereby 5-(3,4-dichlorophenyl)-N-(3,5-dimethylphenyl)-8-oxo-8H-pyrano[3,4-b]pyridine-6-carboxamide was obtained as colorless crystal.
Melting point: 210-211 °C (recrystallized from ethyl acetate-isopropyl ether).
NMR (200 MHz, $CDCl_3$) ppm: 2.30 (6H, s), 4.40 (2H, d, J = 5.6 Hz), 6.89 (2H, s), 6.94 (1H, s), 7.16 (1H, dd, J = 8.2, 2.2 Hz), 7.30 (1H, bt), 7.39 (1H, d, J = 2.2 Hz), 7.50 (1H, dd, J = 8.4, 1.6 Hz), 7.60 (1H, d, J = 8.2 Hz), 7.65 (1H, dd, J = 8.4, 4.4 Hz), 8.97 (1H, dd, J = 4.4, 1.6 Hz) Analyzed value of element: $C_{24}H_{18}N_2O_3Cl_2 \cdot 0.2H_2O$
Calculated value (%): C 63.09, H 4.06, N 6.13
Measured value (%): C 63.13, H 3.94, N 6.14.

(Step 2)

The compound obtained in Step 1 and (R)-4-amino-2-methyl-1-butanol THP ether were reacted and treated in the same manner as in Reference Example 19 whereby the title compound was obtained as colorless foam.
NMR (200 MHz, $CDCl_3$) ppm: 0.86 (3H, d, J = 5.8 Hz), 1.4 - 1.9 (3H, m), 2.29 (6H, s), 2.90 (1H, m), 3.25 - 3.85 (4H, m), 4.29 (2H, d, J = 5.6 Hz), 6.72 (2H, s), 6.93 (1H, s), 7.25 - 7.55 (5H, m), 7.75 (1H, m), 8.60 (1H, m).

Reference Example 157

(R)-N-(3,5-dimethoxybenzyl)-5-(4-fluorophenyl)-7,8-dihydro-7-(4-hydroxy-3-methylbutyl)-8-oxo-6-pyrido[3,4-b] pyridine carboxamide

(Step 1)

In place of 3-(4-methylbenzoyl)-2-pyridinecarboxylic acid in Step 1 in Reference Example 2,3-(4-fluorobenzoyl)-2-pyridinecarboxylic acid was reacted and treated in the same manner as in Steps 1 and 2 in Reference Example 2 whereby 5-(4-fluorophenyl)-8-oxo-8 H-pyrano[3,4-b]pyridine-6-carboxylic acid was obtained as colorless crystal.
NMR (200 MHz, DMSO-$d_6$) ppm: 7.25 - 7.50 (5H, m), 7.81 (1H, dd, J = 8.4, 4.4 Hz), 8.95 (1H, dd, J = 4.4, 1.4 Hz).

(Step 2)

The compound obtained in Step 1 and 3,5-dimethoxybenzylamine were reacted and treated in the same manner as in Step 4 in Reference Example 2 whereby N-(3,5-dimethoxybenzyl)-5-(4-fluorophenyl)-8-oxo-8H-pyrano [3,4-b]pyridine-6-carboxamide was obtained as colorless crystal.
Melting point: 193-194 °C (recrystallized from ethyl acetate-isopropyl ether).
NMR (200 MHz, $CDCl_3$) ppm: 3.78 (6H, s), 4.41 (2H, d, J = 6.0 Hz), 6.42 (3H, m), 7.2 - 7.4 (5H, m), 7.52 (1H. dd, J = 8.4, 1.6 Hz), 7.64 (1H, dd, J = 8.4, 4.4 Hz), 8.97 (1H, dd, J = 4.4, 1.6 Hz)
Analyzed value of element: $C_{24}H_{19}N_2O_5F$
Calculated value (%): C 66.36, H 4.41, N 6.45
Measured value (%): C 66.07, H 4.55, N 6.27.

(Step 3)

The compound obtained in Step 2 and (R)-4-amino-2-methyl-1-butanol THP ether were reacted and treated in the same manner as in Reference Example 19 whereby the title compound was obtained as colorless crystal.
Melting point: 170-171 °C (recrystallized from ethyl acetate-isopropyl ether).
NMR (200 MHz, $CDCl_3$) ppm: 0.82 (3H, d, J = 6.6 Hz), 1.5 - 1.9 (3H, m), 3.06 (1H, m), 3.2 - 3.6 (2H, m), 3.65 - 3.85 (2H, m), 3.78 (6H, s), 4.27 (2H, d, J = 6.2 Hz), 6.26 (2H, d, J = 2.2 Hz), 6.36 (1H, t, J =2.2 Hz), 7.07 (2H, t, J = 8.6 Hz), 7.28 (1H, dd, J = 8.4, 4.0 Hz), 7.35 - 7.50 (2H, m), 7.46 (1H, dd, J = 8.4, 1.4 Hz), 7.91 (1H, bt), 8.59 (1H, dd, J = 4.0, 1.4 Hz)
Analyzed value of element: $C_{29}H_{30}N_3O_5F$

Calculated value (%): C 67.04, H 5.82, N 8.09
Measured value (%): C 66.87, H 5.73, N 8.04.

Reference Example 158

(R)-5-(4-chlorophenyl)-N-(3,5-dimethoxybenzyl)-7,8-dihydro-7-(4-hydroxy-3-methylbutyl)-8-oxo-6-pyrido[3,4-b] pyridine carboxamide

(Step 1)

The compound obtained in Step 1 in Reference Example 151 and 3, 5-dimethoxybenzylamine were reacted and treated in the same manner as in Step 4 in Reference Example 2 whereby 5-(4-chlorophenyl)-N-(3,5-dimethoxybenzyl)-8-oxo-8 H-pyrano[3,4-b]pyridine-6-carboxamide was obtained as colorless crystal.
Melting point: 179-180 °C (recrystallized from ethyl acetate-isopropyl ether).
NMR (200 MHz, $CDCl_3$) ppm: 3.78 (6H, s), 4.41 (2H, d, J = 5.8 Hz), 6.42 (3H, m), 7.23 (2H, d, J = 8.4 Hz), 7.34 (1H, bt), 7.51 (2H, d, J = 8.4 Hz), 7.52 (1H, dd, J = 8.2, 1.6 Hz), 7.63 (1H, dd, J = 8.2, 4.4 Hz), 8.97 (1H, dd, J = 4.4, 1.6 Hz)
Analyzed value of element: $C_{24}H_{19}N_2O_5Cl$
Calculated value (%): C 63.93, H 4.25, N 6.21
Measured value (%): C 63.70, H 4.37, N 6.11.

(Step 2)

The compound obtained in Step 1 and (R)-4-amino-2-methyl-1-butanol THP ether were reacted and treated in the same manner as in Reference Example 19 whereby the title compound was obtained as colorless crystal.
Melting point: 181-182 °C (recrystallized from ethyl acetate-ethyl ether-isopropyl ether).
NMR (200 MHz, $CDCl_3$) ppm: 0.83 (3H, d, J = 6.6 Hz), 1.4 - 1.8 (3H, m), 2.98 (1H, m), 3.2 - 3.4 (2H, m), 3.60 - 3.85 (2H, m), 3.78 (6H, s), 4.27 (2H, d, J = 5.8 Hz), 6.29 (2H, d, J = 2.2 Hz), 6.38 (1H, t, J = 2.2 Hz), 7.22 - 7.45 (5H, m), 7.45 (1H, dd, J = 8.4, 1.8 Hz), 7.82 (1H, bt), 8.60 (1H, dd, J = 4.0, 1.8 Hz)
Analyzed value of element: $C_{29}H_{30}N_3O_5Cl$
Calculated value (%): C 64.98, H 5.64, N 7.84
Measured value (%): C 64.79, H 5.58, N 7.73.

Reference Example 159

(R)-N-[3,5-bis(trifluoromethyl)benzyl]-5-(4-fluorophenyl)-7,8-dihydro-7-(4-hydroxy-3-methylbutyl)-8-oxo-6-pyrido[3, 4-b]pyridine carboxamide

(Step 1)

The compound obtained in Step 1 in Reference Example 157 and 3, 5-bis(trifluoromethyl)benzylamine were reacted and treated in the same manner as in Step 4 in Reference Example 2 whereby N-[3,5-bis(trifluoromethyl) benzyl]-5-(4-fluorophenyl)-8-oxo-8H-pyrano [3,4-b] pyridine-6-carboxamide was obtained as colorless crystal.
Melting point: 166-167 °C (recrystallized from ethyl acetate-isopropyl ether).
NMR (200 MHz, $CDCl_3$) ppm: 4.63 (2H, d, J = 6.2 Hz), 7.1 - 7.3 (4H, m), 7.54 (1H, dd, J = 8.4, 1.6 Hz), 7.6 - 7.8 (1H, m), 7.65 (1H, dd, J = 8.4, 4.4 Hz), 7.73 (2H, s), 7.80 (1H, s), 8.98 (1H, dd, J = 4.4, 1.6 Hz)
Analyzed value of element: $C_{24}H_{13}N_2O_3F_7$
Calculated value (%): C 56.48, H 2.57, N 5.49
Measured value (%): C 56.52, H 2.68, N 5.47.

(Step 2)

The compound obtained in Step 1 and (R)-4-amino-2-methyl-1-butanol THP ether were reacted and treated in the same manner as in Reference Example 19 whereby the title compound was obtained as colorless crystal.
Melting point: 212-213 °C (recrystallized from ethyl acetate-isopropyl ether).
NMR (200 MHz, $CDCl_3$) ppm: 0.77 (3H, d, J = 7.0 Hz), 1.4 - 1.8 (3H, m), 3.1 - 3.7 (5H, m), 4.51 (2H, m), 6.84 - 7.00 (2H, m), 7.25 - 7.48 (3H, m), 7.48 (1H, dd, J = 8.4, 1.8 Hz), 7.69 (2H, s), 7.82 (1H, s), 8.60 (1H, dd, J = 4.2, 1.8 Hz), 8.76 (1H, bt)
Analyzed value of element: $C_{29}H_{24}N_3O_3F_7$

Calculated value (%): C 58.49, H 4.06, N 7.06
Measured value (%): C 58.28, H 4.06, N 7.02.

Reference Example 160

(±)-N-[3,5-bis(trifluoromethyl)benzyl]-7,8-dihydro-7-(4-hydroxy-3-ethylbutyl)-5-(4-methylphenyl-8-oxo-6-pyrido[3,4-b]pyridine carboxamide

The compound obtained in Reference Example 3 and (±)-4-amino-2-ethyl-1-butanol were reacted in the same manner as in Reference Example 5 whereby the title compound was obtained as colorless oily matter.

NMR (200 MHz, $CDCl_3$) ppm: 0.86 (3H, t, J = 7.0 Hz), 1.0 - 1.4 (3H, m), 1.5 - 1.9 (2H, m), 2.28 (3H, s), 3.0 - 3.6 (5H, m), 4.50 (2H, d, J = 6.2 Hz), 7.07 (2H, d, J = 8.6 Hz), 7.1 - 7.3 (2H, m), 7.31 (1H, dd, J = 8.0, 4.2 Hz), 7.55 (1H, dd, J = 8.0, 1.4 Hz), 7.68 (2H, s), 7.78 (1H, s), 8.43 (1H, bt), 8.65 (1H, m).

Reference Example 161

(±)-N-[3,5-bis(trifluoromethyl)benzyl]-7,8-dihydro-7-[4-hydroxy-3-(1-methylethyl)butyl)-5-(4-methylphenyl)-8-oxo-6-pyrido[3,4-b]pyridine carboxamide

The compound obtained in Reference Example 3 and (±)-4-amino-2-(1-methylethyl)-1-butanol were reacted in the same manner as in Reference Example 5 whereby the title compound was obtained as colorless oily matter.

NMR (200 MHz, $CDCl_3$) ppm: 0.82 (6H, d, J = 6.6 Hz), 1.3 - 2.0 (4H, m), 2.27 (3H, s), 3.30 (1H, m), 3.52 (4H, m), 4.50 (2H, d, J = 5.8 Hz), 7.07 (2H, d, J = 8.6 Hz), 7.20 - 7.35 (3H, m), 7.54 (1H, dd, J = 8.4, 1.6 Hz), 7.67 (2H, s), 7.78 (1H, s), 8.51 (1H, bt), 8.63 (1H, dd, J = 4.4, 1.6 Hz).

Reference Example 162

(±)-5-(3,4-dichlorophenyl)-N-(3,5-dimethoxybenzyl)-7, 8-dihydro-7-(4-hydroxy-3-ethylbutyl)-8-oxo-6-pyrido[3,4-b]pyridine carboxamide

The compound obtained in Step 1 in Reference Example 153 and (±)-4-amino-2-ethyl-1-butanol were reacted in the same manner as in Reference Example 5 whereby the title compound was obtained as colorless oily matter. This compound was subjected without purification to the reaction in Example 17.

Reference Example 163

(±)-5-(3,4-dichlorophenyl)-N-(3,5-dimethoxybenzyl)-7,8-dihydro-7-[4-hydroxy-3-(1-methylethyl)butyl]-8-oxo-6-pyrido[3,4-b]pyridine carboxamide

The compound obtained in Step 1 in Reference Example 153 and (±)-4-amino-2-(1-methylethyl)-1-butanol were reacted in the same manner as in Reference Example 5 whereby the title compound was obtained as colorless oily matter. This compound was subjected without purification to the reaction in Example 18.

Reference Example 164

(±)-7-[3,5-bis(trifluoromethyl)benzyl]-6,7,8,9,10,11-hexahydro-10-methyl-5-(4-methylphenyl)-6,13-dioxo-13H-[1,4]diazosino[2,1-g][1,7]napththylidine

(Step 1)

The compound obtained in Reference Example 3 and (±)-4-amino-3-methyl-1-butanol were reacted and treated in the same manner as in Reference Example 5 whereby (±)-N-[3,5-bis(trifluoromethyl)benzyl]-7,8-dihydro-7-(4-hydroxy-2-methylbutyl)-5-(4-methylphenyl)-8-oxo-6-pyrido[3,4-b]pyridine carboxamide was obtained as colorless foam.

NMR (200 MHz, $CDCl_3$) ppm: 0.81 (3H, d, J = 7.0 Hz), 1.3 - 2.4 (3H, m), 2.30 (3H, s), 2.95 (1H, m), 3.4 - 3.9 (4H, m), 4.46 (2H, m), 7.0 - 7.4 (5H, m), 7.52 (1H, d, J = 7.0 Hz), 7.71 (2H, s), 7.78 (1H, s), 8.56 (1H, bt), 8.64 (1H, d, J = 3.0 Hz).

(Step 2)

The compound obtained in Step 1 was reacted and treated in the same manner as in Reference Example 69 whereby the title compound was obtained as colorless powder.

NMR (200 MHz, $CDCl_3$) ppm: 1.18 (3H, d, J = 7.0 Hz), 1.4 - 2.5 (3H, m), 2.37 (3H, s), 3.1 - 3.8 (3H, m), 4.02 (1H, d, J = 15 Hz), 4.81 (1H, d, J = 14 Hz), 5.46 (1H, d, J = 15 Hz), 6.84 (1H, d, J = 7.2 Hz), 7.06 (1H, d, J = 7.2 Hz), 7.2 - 7.6 (4H, m), 7.48 (2H, s), 7.81 (1H, s), 8.90 (1H, m).

Example 1

(9R)-7-[3,5-di(benzyloxy)benzyl]-6,7,8,9,10,11-hexahydro-9-methyl-5-(4-methylphenyl)-6,13-dioxo-13 H-[1,4] diazosino[2,1-g][1, 7] napththylidine

The compound obtained in Reference Example 149 was reacted and treated in the same manner as in Reference Example 69 whereby the title compound was obtained as colorless powder.

NMR (200 MHz, $CDCl_3$) ppm: 0.82 (3H, d, J = 6.6 Hz), 1.65 (1H, m), 1.8 - 2.4 (2H, m), 2.15 (3H, s), 3.02 (1H, d, J = 15 Hz), 3.32 (1H, dd, J = 15, 10 Hz), 3.52 (1H, dd, J = 14, 10 Hz), 3.81 (1H, d, J = 14 Hz), 4.97 (4H, s), 5.04 (1H, dd, J = 14, 5.6 Hz), 5.24 (1H, d, J = 14 Hz), 6.28 (2H, d, J = 2.2 Hz), 6.55 (1H, bt), 6.86 (1H, d, J = 8.0 Hz), 7.03 (1H, d, J = 8.0 Hz), 7.2 - 7.5 (13H, m), 7.60 (1H, dd, J = 8.4, 1.2 Hz), 8.88 (1H, dd, J = 4.4, 1.2 Hz).

Analyzed value of element: $C_{42}H_{39}N_3O_4 \cdot 0.5H_2O$

Calculated value (%): C 76.57, H 6.12, N 6.38

Measured value (%): C 76.19, H 6.33, N 6.25.

Example 2

(9R)-7-(3,5-dihydroxybenzyl)-6,7,8,9,10,11-hexahydro-9-methyl-5-(4-methylphenyl)-6,13-dioxo-13 H-[1,4]diazosino [2,1-g][1, 7] napththylidine

A mixture of the compound (2.3 g) obtained in Example 1, 10 % palladium-carbon (50 % water content) (3.0 g) and methanol (70 ml) was heated under reflux in a hydrogen atmosphere for 15 hours. After the catalyst was removed by filtration through Celite, the filtrate was concentrated whereby the title compound was obtained as colorless powder (1.08 g).

NMR (200 MHz, DMSO-$d_6$) ppm: 0.78 (3H, d, J = 6.2 Hz), 1.1 - 1.6 (1H, m), 1.9 - 2.5 (2H, m), 2.38 (3H, s), 2.90 (1H, d, J = 15 Hz), 3.0 - 3.6 (2H, m), 3.87 (1H, d, J = 14 Hz), 4.7 - 4.9 (1H, m), 4.84 (1H, d, J = 14 Hz), 5.88 (2H, d, J = 1.8 Hz), 6.17 (1H, bt), 6.9 - 7.3 (4H, m), 7.5 - 7.7 (2H, m), 8.84 (1H, m), 9.21 (2H, s).

Example 3

(9R)-7-(3,5-diethoxybenzyl)-6,7,8,9,10,11-hexahydro-9-methyl-5-(4-methylphenyl)-6,13-dioxo-13H-[1,4]diazosino [2,1-g][1, 7]napththylidine

A mixture of the compound (200 mg) obtained in Example 2, sodium hydride (60 %, oily) (70 mg) and DMF (7 ml) was stirred at room temperature for 30 minutes , then ethyl iodide was added to it under cooling on ice, and the mixture was stirred at room temperature for 1 hour. The reaction solution was diluted with ethyl acetate and washed successively with water, dilute hydrochloric acid, saturated aqueous sodium hydrogen carbonate and saturated saline. After the organic layer was dried, the solvent was distilled off, and the residue was purified by silica gel column chromatography (ethyl acetate : methanol = 9 : 1) whereby the title compound was obtained as colorless powder (66.5 mg).

NMR (200 MHz, $CDCl_3$) ppm: 0.86 (3H, d, J = 6.6 Hz), 1.43 (6H, t, J = 6.9 Hz), 1.66 (1H, m), 1.9 - 2.4 (2H, m), 2.42 (3H, s), 3.06 (1H, d, J = 15 Hz), 3.33 (1H, dd, J = 15, 11 Hz), 3.56 (1H, dd, J = 14, 11 Hz), 3.81 (1H, d, J = 14 Hz), 3.95 (4H, q, J = 6.9 Hz), 5.06 (1H, dd, J = 14, 5.6 Hz), 5.23 (1H, d, J = 14 Hz), 6.17 (2H, d, J = 2.2 Hz), 6.38 (1H, t, J = 2.2 Hz), 6.88 (1H, dd, J = 7.8, 1.6 Hz), 7.15 (1H, d, J = 7.8 Hz), 7.29 (1H, d, J = 7.8 Hz), 7.36 (1H, dd, J = 7.8, 1.6 Hz), 7.46 (1H, dd, J = 8.4, 4.4 Hz), 7.63 (1H, dd, J = 8.4, 1.4 Hz), 8.90 (1H, dd, J = 4.4, 1.4 Hz).

Example 4

(9R)-7-[3,5-di(1-methylethyloxy)benzyl)-6,7,8,9,10,11-hexahydro-9-methyl-5-(4-methylphenyl)-6,13-dioxo-13H-[1,4]diazosino[2,1-g][1,7]napththylidine

The compound obtained in Example 2 and 2-iodopropane were reacted and treated in the same manner as in Example 3 whereby the title compound was obtained as colorless powder.

NMR (200 MHz, $CDCl_3$) ppm: 0.84 (3H, d, J = 7.0 Hz), 1.34 (12H, d, J = 6.0 Hz), 1.65 (1H, m), 1.9 - 2.4 (2H, m), 2.44 (3H, s), 3.06 (1H, d, J = 15 Hz), 3.35 (1H, dd, J = 15, 10 Hz), 3.55 (1H, dd, J = 14, 11 Hz), 3.88 (1H, d, J = 14 Hz), 4.48 (2H, m), 5.05 (1H, dd, J = 14, 5.7 Hz), 5.19 (1H, d, J = 14 Hz), 6.18 (2H, d, J = 2.2 Hz), 6.37 (1H, t, J = 2.2 Hz), 6.95 (1H, d, J = 7.4 Hz), 7.23 (1H, d, J = 7.6 Hz), 7.29 (1H, d, J = 7.6 Hz), 7.36 (1H, d, J = 7.4 Hz), 7.47 (1H, dd, J = 8.4, 4.2 Hz), 7.65 (1H, dd, J = 8.4, 1.6 Hz), 8.90 (1H, dd, J = 4.2, 1.6 Hz).

Example 5

(9R)-7-(3,5-dimethoxybenzyl)-6,7,8,9,10,11-hexahydro-9-methyl-6,13-dioxo-5-phenyl-13H-[1,4]diazosino[2,1-g][1,7]napththylidine

The compound obtained in Reference Example 150 was reacted and treated in the same manner as in Reference Example 69 whereby the title compound was obtained as colorless crystal.
Melting point: 199-201 °C (recrystallized from ethyl acetate-ethyl ether).

NMR (200 MHz, $CDCl_3$) ppm: 0.87 (3H, d, J = 7.0 Hz), 1.70 (1H, m), 1.90 - 2.29 (2H, m), 3.05 (1H, d, J = 15 Hz), 3.33 (1H, dd, J = 15, 10 Hz), 3.56 (1H, dd, J = 14, 11 Hz), 3.73 (6H, s), 3.80 (1H, d, J = 14 Hz), 5.08 (1H, dd, J = 14, 5.7 Hz), 5.23 (1H, d, J = 14 Hz), 6.12 (2H, d, J = 2.2 Hz), 6.37 (1H, t, J = 2.2 Hz), 6.97 (1H, d, J = 7.2 Hz), 7.23 - 7.55 (5H, m), 7.59 (1H, dd, J = 8.2, 1.4 Hz), 8.90 (1H, dd, J = 4.2, 1.4 Hz)
Analyzed value of element: $C_{29}H_{29}N_3O_4$
Calculated value (%): C 72.03, H 6.04, N 8.69
Measured value (%): C 71.94, H 6.09, N 8.93.

Example 6

(9R)-7-[3,5-bis(trifluoromethyl)benzyl]-5-(4-chlorophenyl)-6,7,8,9,10,11-hexahydro-9-methyl-6,13-dioxo-13H-[1,4]diazosino[2,1-g][1,7]napththylidine

The compound obtained in Reference Example 151 was reacted and treated in the same manner as in Reference Example 69 whereby the title compound was obtained as colorless crystal.
Melting point: 226-227 °C (recrystallized from ethyl acetate-ethyl ether).

NMR (200 MHz, $CDCl_3$) ppm: 0.92 (3H, d, J = 6.6 Hz), 1.73 (1H, m), 1.9 - 2.4 (2H, m), 3.02 (1H, d, J = 16 Hz), 3.35 - 3.65 (2H, m), 4.01 (1H, d, J = 15 Hz), 5.10 (1H, dd, J = 14, 5.2 Hz), 5.39 (1H, d, J = 15 Hz), 6.88 (1H, d, J = 8.4 Hz), 7.19 (1H, d, J = 8.4 Hz), 7.4 - 7.6 (6H, m), 7.85 (1H, s), 8.93 (1H, t, J = 3.0 Hz).

Example 7

(9R)-7-[3,5-bis(trifluoromethyl)benzyl]-5-(3,4-dichlorophenyl)-(6,7,8,9,10,11-hexahydro-9-methyl-6,13-dioxo-13H-[1,4]diazosino[2,1-g][1,7]napththylidine

The compound obtained in Reference Example 152 was reacted and treated in the same manner as in Reference Example 69 whereby the title compound was obtained as colorless crystal.
Melting point: 280-282 °C (recrystallized from ethyl acetate-isopropyl ether).

NMR (200 MHz, $CDCl_3$) ppm: 0.93 (3H × 5/9, d, J = 6.6 Hz), 0.96 (3H × 4/9, d, J = 6.2 Hz), 1.67 (1H, m), 1.9 - 2.4 (2H, m), 3.02 (1H × 5/9, d, J = 1.5 Hz), 3.13 (1H × 4/9, d, J = 15 Hz), 3.35 - 3.65 (2H, m), 4.04 (1H × 5/9, d, J = 15 Hz), 4.05 (1H × 4/9, d, J = 15 Hz), 5.00 - 5.18 (1H, m), 5.26 (1 × 4/9, d, J = 15 Hz), 5.41 (1H × 5/9, d, J = 15 Hz), 6.79 (1H × 5/9, dd, J = 8.2, 2.0 Hz), 6.98 (1H × 4/9, d, J = 2.0 Hz), 7.25 - 7.65 (6H, m), 7.85 (1H, s), 8.94 (1H, m).

Example 8

(9R)-7-(3,5-dimethoxybenzyl)-5-(3,4-dichlorophenyl)-6,7,8,9,10,11-hexahydro-9-methyl-6,13-dioxo-13H-[1,4] diazosino[2,1-g][1,7]napththylidine

The compound obtained in Reference Example 153 was reacted and treated in the same manner as in Reference Example 69 whereby the title compound was obtained as colorless crystal.
Melting point: 207-208 °C (recrystallized from ethyl acetate-ethyl ether).
NMR (200 MHz, $CDCl_3$) ppm: 0.88 (3H × 1/2, d, J = 6.8 Hz), 0.89 (3H × 1/2, d, J = 6.8 Hz), 1.65 (1H, m), 1.9 - 2.4 (2H, m), 3.00 - 3.38 (2H, m), 3.44 - 3.90 (2H, m), 3.75 (3H, s), 3.77 (3H, s), 5.07 (1H, dd, J = 14, 6.2 Hz), 5.25 (1H, dd, J = 15, 7.4 Hz), 5.99 (1H, d, J = 2.2 Hz), 6.12 (1H, d, J = 2.2 Hz), 6.36 (1H × 1/2, t, J = 2.2 Hz), 6.40 (1H × 1/2, t, J = 2.2 Hz), 6.79 (1H × 1/2, dd, J = 8.0, 2.2 Hz), 7.11 (1H × 1/2, d, J = 1.8 Hz), 7.25 - 7.65 (4H, m), 8.91 (1H, m).
Analyzed value of element: $C_{29}H_{27}N_3O_4Cl_2$
Calculated value (%): C 63.05, H 4.93, N 7.61
Measured value (%): C 62.73, H 5.07, N 7.64.

Example 9

(9R)-7-(3,5-dimethylbenzyl)-6,7,8,9,10,11-hexahydro-9-methyl-5-(4-methylphenyl)-6,13-dioxo-13H-[1,4]diazosino [2,1-g][1,7]napththylidine

The compound obtained in Reference Example 154 was reacted and treated in the same manner as in Reference Example 69 whereby the title compound was obtained as colorless crystal.
Melting point: 200-202 °C (recrystallized from THF-isopropyl ether).
NMR (200 MHz, $CDCl_3$) ppm: 0.85 (3H, d, J = 6.6 Hz), 1.67 (1H, m), 1.9 - 2.4 (2H, m), 2.25 (6H, s), 2.44 (3H, s), 2.97 (1H, d, J = 15 Hz), 3.27 (1H, dd, J = 15, 10 Hz), 3.58 (1H, dd, J = 14, 11 Hz), 3.73 (1H, d, J = 15 Hz), 5.08 (1H, dd, J = 14, 5.7 Hz), 5.38 (1H, d, J = 15 Hz), 6.50 (2H, s), 6.92 (2H, m), 7.15 (1H, d, J = 8.0 Hz), 7.35 (1H, d, J = 7.6 Hz), 7.45 (1H, d, J = 7.6 Hz), 7.46 (1H, dd, J = 8.2, 4.2 Hz), 7.58 (1H, dd, J = 8.2, 1.6 Hz), 8.90 (1H, dd, J = 4.2, 1.6 Hz)
Analyzed value of element: $C_{30}H_{31}N_3O_2$
Calculated value (%): C 77.39, H 6.71, N 9.03
Measured value (%): C 77.01, H 6.75, N 8.95.

Example 10

(9R)-7-(3,5-dichlorobenzyl)-6,7,8,9,10,11-hexahydro-9-methyl-5-(4-methylphenyl)-6,13-dioxo-13H-[1,4]diazosino [2,1-g][1,7]napththylidine

The compound obtained in Reference Example 155 was reacted and treated in the same manner as in Reference Example 69 whereby the title compound was obtained as colorless crystal.
Melting point: 139-141 °C (recrystallized from ethyl acetate-ethyl ether).
NMR (200 MHz, $CDCl_3$) ppm: 0.90 (3H, d, J = 6.6 Hz), 1.70 (1H, m), 1.9 - 2.4 (2H, m), 2.43 (3H, s), 2.95 (1H, d, J = 15 Hz), 3.38 (1H, dd, J = 15, 11 Hz), 3.52 (1H, dd, J = 15, 11 Hz), 3.74 (1H, d, J = 15 Hz), 5.08 (1H, dd, J = 15, 5.6 Hz), 5.39 (1H, d, J = 15 Hz), 6.79 (2H, d, J = 1.8 Hz), 6.88 (1H, dd, J = 7.6, 1.8 Hz), 7.21 (1H, d, J = 7.6 Hz), 7.29 (1H, s), 7.31 (1H, d, J = 7.6 Hz), 7.38 (1H, dd, J = 7.6, 1.8 Hz), 7.47 (1H, dd, J = 8.4, 4.4 Hz), 7.59 (1H, dd, J = 8.4, 1.8 Hz), 8.90 (1H, dd, J = 4.4, 1.8 Hz)
Analyzed value of element: $C_{28}H_{25}N_3O_2Cl_2 \cdot 0.3H_2O$
Calculated value (%): C 65.71, H 5.04, N 8.21
Measured value (%): C 65.40, H 4.90, N 8.17.

Example 11

(9R)-5-(3,4-dichlorophenyl)-7-(3,5-dimethylbenzyl)-6,7,8,9,10,11-hexahydro-9-methyl-6,13-dioxo-13H-[1,4]diazosino [2,1-g][1,7]napththylidine

The compound obtained in Reference Example 156 was reacted and treated in the same manner as in Reference Example 69 whereby the title compound was obtained as colorless powder.
Melting point: 137-139 °C.
NMR (200 MHz, $CDCl_3$) ppm: 0.86 (3H × 1/2, d, J = 6.2 Hz), 0.89 (3H × 1/2, d, J = 6.0 Hz), 1.5 - 2.3 (3H, m),

2.25 (3H, s), 2'.28 (3H, s), 2.90 - 3.84 (4H, m), 5.07 (1H, dd, J = 14, 5.8 Hz), 5.31 (1H, dd, J = 14, 9.2 Hz), 6.50 (1H, s), 6.57 (1H, s), 6.78 (1H × 1/2, dd, J = 8.0, 1.8 Hz), 6.93 (1H, d, J = 6.0 Hz), 7.12 (1H × 1/2, d, J = 1.8 Hz), 7.30 (1H, d, J = 8.8 Hz), 7.4 - 7.7 (3H, m), 8.92 (1H, m)
Analyzed value of element: $C_{29}H_{27}N_3O_2Cl_2 \cdot 0.3H_2O$
Calculated value (%): C 66.24, H 5.29, N 7.99
Measured value (%): C 66.21, H 5.49, N 7.70.

Example 12

(9R)-7-(3,5-dimethoxybenzyl)-5-(4-fluorophenyl)-6,7,8,9,10,11-hexahydro-9-methyl-6,13-dioxo-13H-[1,4]diazosino[2,1-g][1,7]napththylidine

The compound obtained in Reference Example 157 was reacted and treated in the same manner as in Reference Example 69 whereby the title compound was obtained as colorless crystal.
Melting point: 192-193 °C (recrystallized from ethyl acetate-ethyl ether).
NMR (200 MHz, $CDCl_3$) ppm: 0.88 (3H, d, J = 6.8 Hz), 1.5 - 1.8 (1H, m), 1.9 - 2.4 (2H, m), 3.03 (1H, d, J = 15 Hz), 3.36 (1H, dd, J = 15, 10 Hz), 3.56 (1H, dd, J = 14, 11 Hz), 3.75 (1H, d, J = 15 Hz), 3.76 (6H, s), 5.08 (1H, dd, J = 14, 5.6 Hz), 5.27 (1H, d, J = 15 Hz), 6.03 (2H, d, J = 2.2 Hz), 6.37 (1H, t, J = 2.2 Hz), 6.9 - 7.1 (2H, m), 7.19 (1H, m), 7.45 - 7.60 (3H, m), 8.91 (1H, dd, J = 4.2, 2.0 Hz) $[a]_D$ = +109.4° (c = 0.497%, methanol)
Analyzed value of element: $C_{29}H_{28}N_3O_4F$
Calculated value (%): C 69.45, H 5.63, N 8.38
Measured value (%): C 69.32, H 5.57, N 8.31.

Example 13

(9R)-5-(4-chlorophenyl)-7-(3,5-dimethoxybenzyl)-6,7,8,9,10,11-hexahydro-9-methyl-6,13-dioxo-13H-[1,4]diazosino[2,1-g][1,7]napththylidine

The compound obtained in Reference Example 158 was reacted and treated in the same manner as in Reference Example 69 whereby the title compound was obtained as colorless crystal.
Melting point: 229-230 °C (recrystallized from ethyl acetate-ethyl ether)
NMR (200 MHz, $CDCl_3$) ppm: 0.88 (3H, d, J = 7.0 Hz), 1.5 - 1.8 (1H, m), 1.9 - 2.4 (2H, m), 3.06 (1H, d, J = 15 Hz), 3.29 (1H, dd, J = 15, 9.8 Hz), 3.56 (1H, dd, J = 14, 11 Hz), 3.76 (1H, d, J = 15 Hz), 3.77 (6H, s), 5.07 (1H, dd, J = 14, 5.6 Hz), 5.27 (1H, d, J = 15 Hz), 6.08 (2H, d, J = 2.2 Hz), 6.39 (1H, t, J = 2.2 Hz), 6.91 (1H, d, J = 8.4 Hz), 7.28 (1H, d, J = 8.4 Hz), 7.46 (2H, m), 7.48 (1H, dd, J = 8.4, 4.0 Hz), 7.55 (1H, dd, J = 8.4, 1.8 Hz), 8.91 (1H, dd, J = 4.0, 1.8 Hz)
Analyzed value of element: $C_{29}H_{28}N_3O_4Cl \cdot 0.2H_2O$
Calculated value (%): C 66.78, H 5.49, N 7.99
Measured value (%): C 66.78, H 5.54, N 7.88.

Example 14

(9R)-7-[3,5-bis(trifluoromethyl)benzyl]-5-(4-fluorophenyl)-6,7,8,9,10,11-hexahydro-9-methyl-6,13-dioxo-13H-[1,4]diazosino[2,1-g][1,7]napththylidine

The compound obtained in Reference Example 159 was reacted and treated in the same manner as in Reference Example 69 whereby the title compound was obtained as colorless crystal.
Melting point: 234-236 °C (recrystallized from ethyl acetate-ethyl ether).
NMR (200 MHz, $CDCl_3$) ppm: 0.93 (3H, d, J = 6.8 Hz), 1.73 (1H, m), 2.0 - 2.4 (2H, m), 3.00 (1H, d, J = 15 Hz), 3.34 - 3.62 (2H, m), 3.97 (1H, d, J = 15 Hz), 5.10 (1H, dd, J = 15, 5.2 Hz), 5.42 (1H, d, J = 15 Hz), 6.85 - 6.95 (2H, m), 7.13 (1H, dt, $J_d$ = 2.2, $J_t$ = 9.0 Hz), 7.40 - 7.52 (3H, m), 7.45 (2H, s), 7.83 (1H, s), 8.92 (1H, t, J = 2.9 Hz) Analyzed value of element: $C_{29}H_{22}N_3O_2F$
Calculated value (%): C 60.31, H 3.84, N 7.28
Measured value (%): C 60.43, H 3.98, N 7.13.

Example 15

(±)-7-[3, 5-bis(trifluoromethyl)benzyl]-9-ethyl-6,7,8,9,10,11-hexahydro-5-(4-methylphenyl)-6,13-dioxo-13H-[1,4] diazosino[2,1-g][1,7]napththylidine

The compound obtained in Reference Example 160 was reacted and treated in the same manner as in Reference Example 69 whereby the title compound was obtained as colorless crystal.
Melting point: 258-260 °C (recrystallized from ethyl acetate-isopropyl ether).

NMR (200 MHz, $CDCl_3$) ppm: 0.90 (3H, t, J = 7.1 Hz), 1.0 - 1.4 (2H, m), 1.5 - 1.9 (2H, m), 2.28 (1H, m), 2.39 (3H, s), 3.04 (1H, d, J = 15 Hz), 3.39 (1H, dd, J = 15, 9.6 Hz), 3.52 (1H, dd, J = 15, 11 Hz), 3.97 (1H, d, J = 15 Hz), 5.11 (1H, dd, J = 15, 6.6 Hz), 5.48 (1H, d, J = 15 Hz), 6.82 (1H, d, J = 7.6 Hz), 7.06 (1H, d, J = 7.6 Hz), 7.2 - 7.6 (4H, m), 7.48 (2H, s), 7.82 (1H, s), 8.91 (1H, dd, J = 4.0, 1.8 Hz)
Analyzed value of element: $C_{31}H_{27}N_3O_2F_6$
Calculated value (%): C 63.37, H 4.63, N 7.15
Measured value (%): C 63.24, H 4.67, N 7.29.

Example 16

(±)-7-[3, 5-bis(trifluoromethyl)benzyl]-6,7,8,9,10,11-hexahydro-9-(1-methylethyl)-5-(4-methylphenyl)-6,13-dioxo-13H-[1,4]diazosino[2,1-g][1,7]napththylidine

The compound obtained in Reference Example 161 was reacted and treated in the same manner as in Reference Example 69 whereby the title compound was obtained as colorless crystal.
Melting point: 228-229 °C (recrystallized from ethyl acetate-isopropyl ether).

NMR (200 MHz, $CDCl_3$) ppm: 0.82 (3H, d, J = 6.6 Hz), 0.87 (3H, d, J = 7.0 Hz), 1.5 - 2.4 (4H, m), 2.39 (3H, s), 3.06 (1H, d, J = 15 Hz), 3.38 (1H, dd, J = 15, 9.0 Hz), 3.50 (1H, dd, J = 14, 10 Hz), 3.95 (1H, d, J = 15 Hz), 5.14 (1H, dd, J = 14, 5.6 Hz), 5.49 (1H, d, J = 15 Hz), 6.83 (1H, dd, J = 7.8, 1.4 Hz), 7.07 (1H, d, J = 7.8 Hz), 7.28 (1H, d, J = 8.0 Hz), 7.35 (1H, dd, J = 8.0, 1.4 Hz), 7.47 (1H, dd, J = 8.4, 4.4 Hz), 7.48 (2H, s), 7.56 (1H, dd, J = 8.4, 1.8 Hz), 7.82 (1H, s), 8.91 (1H, dd, J = 4.4, 1.8 Hz)
Analyzed value of element: $C_{32}H_{29}N_3O_2F_6$
Calculated value (%): C 63.89, H 4.86, N 6.98
Measured value (%): C 63.82, H 4.70, N 7.13.

Example 17

(±)-5-(3,4-dichlorophenyl)-7-(3,5-dimethoxybenzyl)-9-ethyl-6,7,8,9,10,11-hexahydro-6,13-dioxo-13H-[1,4]diazosino [2,1-g][1,7]napththylidine

The compound obtained in Reference Example 162 was reacted and treated in the same manner as in Reference Example 69 whereby the title compound was obtained as colorless crystal.
Melting point: 260-262 °C (recrystallized from ethyl acetate-isopropyl ether).

NMR (200 MHz, $CDCl_3$) ppm: 0.85 - 1.00 (3H, m) , 1.0 - 1.4 (2H, m), 1.5 - 1.9 (2H, m), 2.23 (1H, m), 3.19 (2H, m), 3.47 - 3.80 (2H, m), 3.75 (3H, s), 3.77 (3H, s), 5.09 (1H, dd, J = 14, 6.2 Hz), 5.28 (1H, dd, J = 14, 7.6 Hz), 5.99 (1H, d, J = 2.2 Hz), 6.13 (1H, d, J = 2.2 Hz), 6.36 (1H × 1/2, t, J = 2.2 Hz), 6.40 (1H × 1/2, t, J = 2.2 Hz), 6.76 (1H × 1/2, dd, J = 8.2, 2.2 Hz), 7.09 (1H × 1/2, d, J = 2.2 Hz), 7.25 - 7.62 (4H, m), 8.91 (1H, m)
Analyzed value of element: $C_{30}H_{29}N_3O_4Cl_2$
Calculated value (%): C 63.61, H 5.16, N 7.42
Measured value (%): C 63.20, H 5.15, N 7.58.

Example 18

(±)-5-(3,4-dichlorophenyl)-7-(3,5-dimethoxybenzyl)-6,7,8,9,10,11-hexahydro-9-(1-methylethyl)-6,13-dioxo-13H-[1,4] diazosino[2,1-g][1,7]napththylidine

The compound obtained in Reference Example 163 was reacted and treated in the same manner as in Reference Example 69 whereby the title compound was obtained as colorless crystal.
Melting point: 202-205 °C (recrystallized from ethyl acetate-isopropyl ether).

NMR (200 MHz, $CDCl_3$) ppm: 0.85 (6H, m), 1.4 - 2.0 (3H, m), 2.15 (1H, m), 3.18 (2H, m), 3.4 - 3.8 (2H, m), 3.75

(3H, s), 3.77 (3H, s), 5.11 (1H, dd, J = 14, 6.4 Hz), 5.28 (1H, dd, J = 14, 6.2 Hz), 5.9 - 6.8 (3.5H, m), 7.0 - 7.6 (4.5H, m), 8.91 (1H, m).

Example 19

| | | |
|---|---|---|
| (1) | Compound in Reference Example 72 | 10.0 mg |
| (2) | Lactose | 60.0 mg |
| (3) | Corn starch | 35.0 mg |
| (4) | Hydroxypropylmethyl cellulose | 3.0 mg |
| (5) | Magnesium stearate | 2.0 mg |

A mixture consisting of 10.0 mg of the compound obtained in Reference Example 72, 60.0 mg of lactose and 35.0 mg of corn starch was granulated with 0.03 ml of 10 weight-% aqueous hydroxypropylmethyl cellulose (3.0 mg of hydroxypropylmethyl cellulose), dried at 40 °C and passed through a screen. The resulting granules were mixed with 2.0 mg of magnesium stearate and compressed. The resulting raw tablets were coated with a sugar coating consisting of an aqueous suspension of sucrose, titanium dioxide, talk and gum arabic. The coated tablets were lubricated with molasses to give coated tablets.

Example 20

| | | |
|---|---|---|
| (1) | Compound in Reference Example 72 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound obtained in Reference Example 72 and 3.0 mg of magnesium stearate were granulated with 0.07 ml of an aqueous solution of water-soluble starch (7.0 mg of soluble starch), then dried and mixed with 70.0 mg of lactose and 50.0 mg of corn starch. The mixture was compressed to give tablets.

Example 21

| | | |
|---|---|---|
| (1) | Compound in Example 12 | 10.0 mg |
| (2) | Lactose | 60.0 mg |
| (3) | Corn starch | 35.0 mg |
| (4) | Hydroxypropylmethyl cellulose | 3.0 mg |
| (5) | Magnesium stearate | 2.0 mg |

A mixture consisting of 10.0 mg of the compound obtained in Example 12, 60.0 mg of lactose and 35.0 mg of corn starch was granulated with 0.03 ml of 10 weight-% aqueous hydroxypropylmethyl cellulose (3.0 mg of hydroxypropylmethyl cellulose), dried at 40 °C and passed through a screen. The resulting granules were mixed with 2.0 mg magnesium stearate and compressed. The resulting raw tablets were coated with a sugar coating consisting of an aqueous suspension of sucrose, titanium dioxide, talk and gum arabic. The coated tablets were lubricated with molasses to give coated tablets.

Example 22

| | | |
|---|---|---|
| (1) | Compound in Example 12 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound obtained in Example 12 and 3.0 mg of magnesium stearate were granulated with 0.07 ml of an aqueous solution of water-soluble starch (7.0 mg of soluble starch), then dried and mixed with 70.0 mg of lactose and 50.0 mg of corn starch. The mixture was compressed to give tablets.

Experimental Example 1

Radioligand receptor binding inhibitory activity Binding inhibitory activity using receptor from human lymphoblast cells (IM-9)

The method of M.A. Cascieri et al. "Molecular Pharmacology 42, p.458 (1992)" was modified and used. The receptor was prepared from human lymphoblast cells (IM-9). IM-9 cells (2 x $10^5$ cells/ml) were inoculated and incubated for 3 days (one liter), which was then subjected to centrifuge for 5 minutes at 500xg to obtain cell pellets. The pellets were washed once with phosphate buffer (Flow Laboratories, CAT. No. 28-103-05), which were then crushed using Polytron.homogenizer "Kinematika, Germany" in 30 ml of 50 mM Tris-HCl buffer (pH 7.4) containing 120 mM sodium chloride, 5mM potassium chloride, 2 mg/ml chymostatin, 40 mg/ml bacitracin, 5 mg/ml phosphoramidon, 0.5 mM phenylmethyl sulfonyl fluoride, 1 mM ethylenediamine tetra-acetic acid, which was subjected to centrifuge at 40,000xg for 20 minutes. The residue was washed twice with 30 ml of the above-mentioned buffer, which was then preserved frozen (-80°C) as a specimen of the receptor.

The specimen was suspended in a reaction buffer (50 mM Tri-HCl buffer (pH 7.4), 0.02% bovine serum albumin, 1 mM phenylmethylsulfonyl fluoride, 2 mg/chymostatin, 40 mg/ml bacitracin, 3 mM manganese chloride) and 100 ul portion was the suspension was used in the reaction. After addition of the sample and $^{125}$I-BHSP (0.46 KBq), the reaction was allowed to proceed in 0.2 ml of reaction buffer at 25° C for 30 minutes. The amount of nonspecific binding was determined by adding substance P at a final concentration of 2 x $10^{-6}$M. After the reaction, using a cell harvester (290 PHD, Cambridge Technology, Inc, U.S.A.), rapid filtration was carried out through a glass filter (GF/B, Whatman, U.S.A.) to stop the reaction. After washing three times with 250 ul of 50 mM of Tris-HCl buffer (pH 7.4) containing 0.02% bovine serum albumin, the radioactivity remaining on the filter was determined with a gamma counter. Before use, the filter was immersed in 0.1% polyethyleneimine for 24 hours and air-dried. The antagonistic activity of each test compounds obtained in Reference Examples and Examples, in terms of the concentration necessary to cause 50% inhibition ($IC_{50}$) under the above-described conditions, was expressed in nM [Table 1]. (Radioligand means substance P labelled with $^{125}$I.)

[Table 1]

| Reference Example No. | $IC_{50}$ value (nM) |
|---|---|
| 56 | 0.28 |
| 57 | 0.76 |
| 62 | 1.2 |
| 64 | 0.66 |
| 67 | 0.17 |
| 68 | 0.28 |
| 69 | 0.88 |
| 70 | 0.17 |
| 71 | 0.23 |
| 72 | 0.43 |
| 77 | 1.1 |
| 78 | 1.6 |

[Table 1] (continued)

| Reference Example No. | $IC_{50}$ value (nM) |
|---|---|
| 79 | 0.1 |
| 85 | 0.36 |
| 89 | 0.44 |
| 91 | 0.28 |
| 92 | 0.74 |
| 93 | 0.42 |
| 94 | 0.17 |
| 99 | 0.12 |
| 101 | 0.2 |
| 137 | 0.84 |
| Example No. | $IC_{50}$ value (nM) |
| 3 | 0.69 |
| 4 | 0.17 |
| 5 | 0.48 |
| 6 | 0.13 |
| 7 | 0.83 |
| 8 | 0.05 |
| 9 | 0.86 |
| 10 | 1.0 |
| 11 | 0.81 |
| 12 | 1.5 |
| 13 | 1.1 |
| 14 | 0.37 |
| 15 | 1.1 |

From the Table 1, it is understood that the heterocyclic compounds (I) or salts thereof have an excellent substance P receptor antagonistic effect.

Experimental Example 2

Binding inhibitory activity toward human $NK_2$ receptor

First strand cDNA synthesized by reverse transcription at 48 C° for 1 h from 2 mg of human stomach poly $A^+$ RNA (Clontech Laboratories, Inc., USA) with Superscript RNase $H^-$ reverse transcriptase (GIBCO BRL Life Technologies, Inc., USA) and a gene specific 3'-primer (5'CTAACCCCTACCTCCCAACACTGCCACATTGGG-3') which was designed according to the published nucleotide sequence coding for human $NK_2$ receptor reported by A. Graham, B. Hopkins, S. J. Powell, P. Danks, and I. Briggs [Biochemical and Biophysical Research Communications 177, pp 8-16 (1991)]. Polymerase chain reaction (PCR) was performed at 95 °C for 1 min, 55 °C for 2 min, 72°C for 3 min for 50 cycles using taq DNA polymerase (Takara Shuzoh, Shiga, Japan), the above mentioned 3'-primer and a gene specific 5'-primer (5'-GAGCCAGGTCCTTTGTTCCAGACCCAGAAGCAG-3') which was also designed according to the published nucleotide sequence of human $NK_2$ receptor cCNA reported by A. Graham et al. described above. The resultant PCR product, 1.3 kilobase-pair DNA fragment was cloned into the Hincll site of pBluescript II $SK^+$ (Stratagene, USA).

The identity of the obtained clone was confirmed by nucleotide sequence analysis. In order to obtain an expression vector, the 1.3 kilobase-pair DNA fragment of human $NK_2$ receptor cDNA was placed downstream of the SRa promoter [Y. Takebe, M. Seiki, J. Fujisawa, P. Hoy, K. Yokota, K. Arai, M. Yoshida, and N. Arai "Molecular and Cellular Biology 8, p 466-472(1988)"].

COS-7 cells were cultured in DMEM medium (ICN Biomedicals, Inc., USA) supplemented with 10 % fetal bovine serum at density of 3 x $10^6$ per 175-$cm^2$ flask (Nunc, Denmark) for 1 day. The cells were transfected with 30 mg of the above mentioned expression vector and 150 mg of transfectam (BioSepra, Inc., USA) at 37 ° C for 5 h. After 3 days, cells were washed with phosphate buffer (ICN Biomedicals, Inc., Cat. No. 2810305, USA) containing 0.1 % ethylenediamine tetra-acetic acid, detached from a flask and centrifuged at 170 x g for 5 min to obtain cell pellets. The

cell pellets were suspended in 50 mM Tris-HCl buffer (pH 7.4) (containing 120 mM sodium chloride, 5 mM potssium chloride, 2 mg/ml chymostatin, 40 mg/ml bacitracin, 5 mg/ml phosphoramidon, 0.5 mM phenylmethylsulfonyl fluoride and 1 mM ethylenediamine tetra-acetic acid), and were then disrupted by a Physicotron handy micro homogenizer NS-310E (Nichi-on-i Rikakiki Seisakusho, Chiba, Japan), which was subjected to centrifuge at 40,000 x g for 60 min. The resulting pellet was washed twice with the above mentioned buffer, which was then preserved frozen at -80 °C as a specimen of the receptor.

The specimen was suspended in reaction buffer (50 mM Tris-HCl buffer (pH 7.4) containing 0.02 % bovine serum albumin, 1 mM phenylmethylsulfonyl fluoride, 2 mg/ml chymostatin, 40 mg/ml bacitracin and 3 mM manganese chloride) to give a protein concentration of 0.6 mg/ml, and 0.1 ml of the portion of the suspension was employed in the reaction. After addition of a test compound and (2-[$^{125}$I]iodohistidyl$^1$)Neurokinin A (Amersham, UK) (74 TBq/mmol, 1.48 kBq, final concentration of 0.1 nM) to a final volume of 0.2 ml, the mixture was incubated at room temperature for 60 min in a 96-well plate. Then the mixture was filtered through a glass fiber filter (UniFilter-96, GF/B, Packard Instrument, Inc., USA) under reduced pressure on a Filter Mate Cell Harvester (Packard Instrument, Inc. , USA). After washing the filter three times with 0.3 ml of the above mentioned reaction buffer, the radioactivity remaining on the filter was determined on a TopCound Micro Scintillation Counter (Packard Instrument, Inc., USA). The nonspecific binding was defined as the binding activity in the presence of 10 x $10^{-6}$ M of neurokinin A (Peptide Instituted, Osaka, Japan). The filters were presoaked overnight in 0.5 % bovine serum albumin. The antagonistic activity of test compounds obtained in Reference Examples was expressed in nM in terms of the concentration necessary to cause 50 % inhibition ($IC_{50}$) under the above described conditions [Table 2].

[Table 2]

| Reference Example No. | $IC_{50}$ value (nM) |
|---|---|
| 76 | 7.5 |
| 81 | 6.2 |
| 87 | 9.5 |

From Table 2, it is apparent that the heterocyclic compounds (I) or salts thereof have excellent inhibitory activity toward human $NK_2$ receptor.

Experimental Example 3

Inhibitory effect on plasma extravasation induced by capsaicin in trachea of guinea pigs

Guinea pigs (Hartley type white male guinea pige), (n=6) were anesthetized with 35 mg/kg of pentobarbital injected intraperitoneally (i.p.), then test compounds were administered intravenously (i.v.). After 5 minutes, a mixed solution of capsaicin (150 mg/kg) and Evans'blue dye (20 mg/kg) was administered intravenously to cause reaction. Ten minutes later, test animals were sacrificed by cutting the aorta, then perfused through pulmonary artery with 50 ml of physiological saline. The trachea was excised, and its wet weight was measure. The trachea was incubated at room temperature in 1 ml of acetone-0.3 % sodium sulfate (7:3) overnight and Evans' blue dye was extracted from the trachea. The extract solution was centrifuged at 2800 rpm for 5 minutes. The amount of Evans' blue dye in the supernatant was quantified by measuring absorbance at 620 mm.

The reaction of accelerating the permeability of blood vessels is expressed in terms of the amount of Evans' blue (µg) per trachea unit weight (g) and the effect of drug was evaluated by calculating the degree of inhibition (% inhibition) according to the following equation:

$$\% \text{ Inhibition} = \left( 1 = \frac{\text{blood - vessel permeability in a drug administration group - blood - vessel permeability in a capsaicin - untreated group}}{\text{blood - vessel permeability in a control group - blood - vessel permeability in a capsaicin - untreated group}} \right) \times 100$$

[Table 3]

| Test Compound (Reference Example No) | Dosage (i.v.) μg/kg | ($ID_{50}$;μg/kg, i.v.) | Degree of Inhibition (%) |
|---|---|---|---|
| 56 | | ( 7.2) | |
| 57 | | ( 4.2) | |
| 60 | | (11) | |
| 61 | | ( 2.6) | |
| 62 | | ( 3.2) | |
| 66 | | (41) | |
| 67 | | (1.6) | |
| 68 | | ( 3.2) | |
| 69 | 10 | | 68.4*** |
| 70 | 10 | | 60.5*** |
| 71 | | ( 1.9) | |
| 72 | | ( 2.6) | |
| 76 | 100 | | 41.2* |
| 77 | 100 | | 64.0* |
| 78 | 100 | | 53.1* |
| 137 | | ( 2.5) | |

a) Dunnett's test: * $p<0.05$, ** $p<0.01$, *** $p<0.001$
b) $ID_{50}$ values are given in parentheses.

From Table 3, it is apparent that the heterocyclic compounds (I) or salts thereof have excellent inhibitory action on the plasma extravasation induced by capsaicin.

INDUSTRIAL APPLICABILITY

The heterocyclic compounds (I) or salts thereof have a tachykinin receptor antagonistic activity in vitro, and are usable as safe medicines for preventing and treating depression, anxiety, manic-depressive illness or psychopathy in mammals.

## Claims

**1.** A pharmaceutical composition for preventing or treating depression, anxiety, manic-depressive illness or psychopathy which comprises a compound represented by the formula:

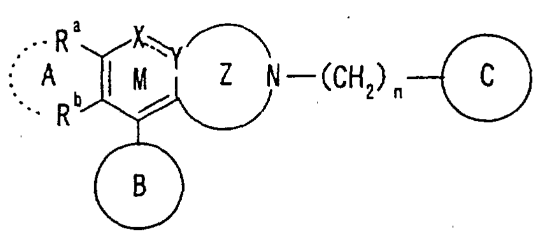

wherein ring M is a heterocyclic ring wherein

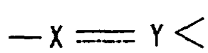

is one of -N=C<, -CO-N< or -CS-N<;

$R^a$ and $R^b$ are bonded to each other to form Ring A, or they are the same or different and represent, independently, a hydrogen atom or a substituent on the Ring M;

Ring A and Ring B represent, independently, an optionally substituted homocyclic or heterocyclic ring, with the proviso that at least one of them is an optionally substituted heterocyclic ring;
Ring C is an optionally substituted homocyclic or heterocyclic ring;
Ring Z is an optionally substituted nitrogen-containing heterocyclic ring; and
n is an integer from 1 to 6, or a salt thereof.

**2.** The pharmaceutical composition as claimed in claim 1, wherein $R^a$ and $R^b$ are the same or different and represent, independently, a hydrogen atom or a substituent selected from the group consisting of

(1) a halogen atom,
(2) a $C_{1-6}$ alkyl group optionally having from 1 to 5 substituents selected from the group consisting of

(a) a hydroxy group,
(b) a $C_{1-6}$ alkoxy group,
(c) a $C_{1-6}$ alkylthio group,
(d) an amino group,
(e) a $C_{1-7}$ acylamino group,
(f) a carboxyl group,
(g) a nitro group,
(h) a mono- or di-$C_{1-6}$ alkylamino group,
(i) a mono- or di-$C_{3-8}$ cycloalkylamino group,
(j) a $C_{6-10}$ arylamino group,
(k) a 5-membered to 9-membered cyclicamino group which may have 1 to 3 hetero atoms selected from the group consisting of nitrogen, oxygen and sulfur atoms in addition to the nitrogen atom in the amino group and which may be substituted by $C_{1-6}$ alkyl group,
(l) a 5-membered or 6-membered aromatic heterocyclic group having from 1 to 3 hetero atoms selected from the group consisting of nitrogen, oxygen and sulfur atoms in addition to carbon atoms,
(m) a 5-membered to 9-membered non-aromatic heterocyclic ring having from 1 to 3 hetero atoms selected from the group consisting of nitrogen, oxygen and sulfur atoms in addition to carbon atoms,
(n) a $C_{1-4}$ alkylsulfonylamino group,
(o) a $C_{1-6}$ alkyl-carbonyloxy group and
(p) a halogen atom,

(3) an optionally halogenated $C_{1-6}$ alkoxy group,
(4) an optionally halogenated $C_{1-6}$ alkylthio group,
(5) a $C_{3-10}$ cycloalkyl group,
(6) a $C_{6-10}$ aryl group,
(7) a $C_{1-7}$ acylamino group,
(8) a $C_{1-3}$ acyloxy group,
(9) a hydroxy group,
(10) a nitro group,
(11) a cyano group,
(12) an amino group,
(13) a mono- or di-$C_{1-6}$ alkylamino group,
(14) a 5-membered to 9-membered cyclicamino group which may have 1 to 3 hetero atoms selected from the group consisting of nitrogen, oxygen and sulfur atoms in addition to the nitrogen atom in the amino group and which may be substituted by $C_{1-6}$ alkyl group,
(15) a $C_{1-6}$ alkyl-carbonylamino group,
(16) a $C_{1-6}$ alkyl-sulfonylamino group,
(17) a $C_{1-6}$ alkoxy-carbonyl group,
(18) a carboxyl group,
(19) a $C_{1-6}$ alkyl-carbonyl group,
(20) a carbamoyl group,
(21) a mono- or di-$C_{1-6}$ alkylcarbamoyl group and
(22) $C_{1-6}$ alkylsulfonyl group, or

$R^a$ and $R^b$ are bonded to each other to form Ring A, and the Ring A is

(i) a 5-membered to 9-membered aromatic heterocycle having from 1 to 3 hetero atoms selected from the group consisting of nitrogen, oxygen and sulfur atoms, in addition to carbon atoms,
(ii) a 5-membered to 9-membered non-aromatic heterocyclic group having from 1 to 3 hetero atoms selected from the group consisting of nitrogen, oxygen and sulfur atoms, in addition to carbon atoms, or
(iii) a 3-membered to 10-membered cyclic hydrocarbon each of which may have 1 to 4 substituents selected from

(1) a halogen atom,
(2) a $C_{1-6}$ alkyl group optionally having from 1 to 5 substituents selected from the group consisting of

(a) a hydroxy group,
(b) an amino group,
(c) a carboxyl group,
(d) a nitro group,
(e) a mono- or di-$C_{1-6}$ alkylamino group,
(f) a $C_{1-6}$ alkyl-carbonyloxy group and
(g) a halogen atom,

(3) an optionally halogenated $C_{1-6}$ alkoxy group,
(4) an optionally halogenated $C_{1-6}$ alkylthio group,
(5) a $C_{6-10}$ aryl group,
(6) a $C_{1-7}$ acylamino group,
(7) a $C_{1-3}$ acyloxy group,
(8) a hydroxy group,
(9) a nitro group,
(10) a cyano group,
(11) an amino group,
(12) a mono- or di-$C_{1-6}$ alkylamino group,
(13) a 5-membered to 9-membered cyclicamino group which may have 1 to 3 hetero atoms selected from the group consisting of nitrogen, oxygen and sulfur atoms in addition to the nitrogen atom in the amino group,
(14) a $C_{1-6}$ alkyl-carbonylamino group,
(15) a $C_{1-6}$ alkyl-sulfonylamino group,
(16) a $C_{1-6}$ alkoxy-carbonyl group,
(17) a carboxyl group,
(18) a $C_{1-6}$ alkyl-carbonyl group,
(19) a carbamoyl group,
(20) a mono- or di-$C_{1-6}$ alkylcarbamoyl group,
(21) a $C_{1-6}$ alkylsulfonyl group, or
(22) an oxo group;

the Ring B is a

(i) 5-membered to 9-membered aromatic heterocycle having from 1 to 3 hetero atoms selected from the group consisting of nitrogen, nitrogen, oxygen and sulfur atoms, in addition to carbon atoms,
(ii) a 5-membered to 9-membered non-aromatic heterocyclic group having from 1 to 3 hetero atoms selected from the group consisting of nitrogen, oxygen and sulfur atoms, in addition to carbon atoms, or
(iii) a 3-membered to 10-membered cyclic hydrocarbon group each of which may have 1 to 4 substituents selected from the group consisting of

(1) a halogen atom,
(2) a $C_{1-6}$ alkyl group optionally having from 1 to 5 substituents selected from the group consisting of

(a) a hydroxy group,
(b) an amino group,
(c) a carboxyl group,
(d) a nitro group,
(e) a mono- or di-$C_{1-6}$ alkylamino group,

(f) a $C_{1-6}$ alkyl-carbonyloxy group and
(g) a halogen atom,

(3) an optionally halogenated $C_{1-6}$ alkoxy group,
(4) an optionally halogenated $C_{1-6}$ alkylthio group,
(5) a $C_{6-10}$ aryl group,
(6) a $C_{1-7}$ acylamino group,
(7) a $C_{1-3}$ acyloxy group,
(8) a hydroxy group,
(9) a nitro group,
(10) a cyano group,
(11) an amino group,
(12) a mono- or di-$C_{1-6}$ alkylamino group,
(13) a 5-membered to 9-membered cyclicamino group which may have 1 to 3 hetero atoms selected from the group consisting of nitrogen, oxygen and sulfur atoms in addition to the nitrogen atom in the amino group,
(14) a $C_{1-6}$ alkyl-carbonylamino group,
(15) a $C_{1-6}$ alkyl-sulfonylamino group,
(16) a $C_{1-6}$ alkoxy-carbonyl group,
(17) a carboxyl group,
(18) a $C_{1-6}$ alkyl-carbonyl group,
(19) a carbamoyl group,
(20) a mono- or di-$C_{1-6}$ alkylcarbamoyl group,
(21) a $C_{1-6}$ alkylsulfonyl group, and
(22) an oxo group;

the Ring C is

(i) a 5-membered to 9-membered heterocycle which may have 1 to 3 hetero atoms selected from the group consisting of nitrogen, oxygen and sulfur atoms which optionally having 1 to 5 substituents selected from the group consisting of

(1) a halogen atom,
(2) an optionally halogenated $C_{1-10}$ alkyl group,
(3) an amino-substituted $C_{1-4}$ alkyl group,
(4) a mono- or di-$C_{1-4}$ alkylamino-substituted $C_{1-4}$ alkyl group,
(5) a carboxyl-substituted $C_{1-4}$ alkyl group,
(6) a $C_{1-4}$ alkoxy-carbonyl-substituted $C_{1-4}$ alkyl group,
(7) a hydroxy-substituted $C_{1-4}$ alkyl group,
(8) a $C_{3-10}$ cycloalkyl group,
(9) a nitro group,
(10) a cyano group,
(11) a hydroxy group,
(12) an optionally-halogenated $C_{1-10}$ alkoxy group,
(13) an optionally-halogenated $C_{1-4}$ alkylthio group,
(14) an amino group,
(15) a mono- or di-$C_{1-4}$ alkylamino group,
(16) a 5-membered to 9-membered cyclic amino group optionally having 1 to 3 hetero atoms selected from the group consisting of nitrogen, oxygen and sulfur atoms in addition to the nitrogen atom in the amino group,
(17) a $C_{1-4}$ alkyl-carbonylamino group,
(18) an aminocarbonyloxy group,
(19) a mono- or di-$C_{1-4}$ alkylaminocarbonyloxy group,
(20) a $C_{1-4}$ alkylsulfonylamino group,
(21) a $C_{1-4}$ alkoxy-carbonyl group,
(22) an aralkyloxycarbonyl group,
(23) a carboxyl group,
(24) a $C_{1-6}$ alkyl-carbonyl group,

(25) a $C_{3-6}$ cycloalkyl-carbonyl group,
(26) a carbamoyl group,
(27) a mono- or di-$C_{1-4}$ alkylcarbamoyl group,
(28) a $C_{1-6}$ alkylsulfonyl group and
(29) a 5-membered or 6-membered aromatic monocyclic heterocyclic group having 1 to 4 hetero atoms selected from the group consisting of nitrogen, oxygen and sulfur atoms in addition to carbon atoms, which may have 1 to 3 substituents selected from an optionally halogenated $C_{1-4}$ alkyl;, or

(ii) a 3-membered to 10-membered cyclic hydrocarbon, optionally having 1 to 5 substituents selected from the group consisting of

(1) a halogen atom,
(2) an optionally halogenated $C_{1-10}$ alkyl group,
(3) an amino-substituted $C_{1-4}$ alkyl group,
(4) a mono- or di-$C_{1-4}$ alkylamino-substituted $C_{1-4}$ alkyl group,
(5) a carboxyl-substituted $C_{1-4}$ alkyl group,
(6) a hydroxy-substituted $C_{1-4}$ alkyl group,
(7) a $C_{1-4}$ alkoxy-carbonyl-substituted $C_{1-4}$ alkyl group,
(8) a $C_{3-10}$ cycloalkyl group,
(9) a nitro group,
(10) a cyano group,
(11) a hydroxy group,
(12) an optionally-halogenated $C_{1-10}$ alkoxy group,
(13) an optionally-halogenated $C_{1-4}$ alkylthio group,
(14) an amino group,
(15) a mono- or di-$C_{1-4}$ alkylamino group,
(16) a 5-membered to 9-membered cyclic amino group optionally having 1 to 3 hetero atoms selected from the group consisting of nitrogen, oxygen and sulfur atoms in addition to the nitrogen atom in the amino group,
(17) a $C_{1-4}$ alkyl-carbonylamino group,
(18) an aminocarbonyloxy group,
(19) a mono- or di-$C_{1-4}$ alkylaminocarbonyloxy group,
(20) a $C_{1-4}$ alkylsulfonylamino group,
(21) a $C_{1-4}$ alkoxy-carbonyl group,
(22) an aralkyloxycarbonyl group,
(23) a carboxyl group,
(24) a $C_{1-6}$ alkyl-carbonyl group,
(25) a $C_{3-6}$ cycloalkyl-carbonyl group,
(26) a carbamoyl group,
(27) a mono- or di-$C_{1-4}$ alkylcarbamoyl group,
(28) a $C_{1-6}$ alkylsulfonyl group and
(29) a 5-membered or 6-membered aromatic monocyclic heterocyclic group having 1 to 4 hetero atoms selected from the group consisting of nitrogen, oxygen and sulfur atoms in addition to carbon atoms, which may have 1 to 3 substituents selected from an optionally halogenated $C_{1-4}$ alkyl;

the Ring Z is a 5-membered to 12-membered heterocycle optionally having at least one hetero atom selected from the group consisting of nitrogen, oxygen and sulfur atoms, in addition to Y and the nitrogen atom already on Ring Z, and optionally having 1 to 5 substituents selected from the group consisting of

(1) a $C_{1-6}$ alkyl group,
(2) a $C_{2-6}$ alkenyl group,
(3) a $C_{2-6}$ alkynyl group,
(4) a $C_{3-8}$ cycloalkyl group,
(5) a $C_{3-8}$ cycloalkyl-$C_{1-4}$ alkyl group,
(6) a $C_{6-14}$ aryl group,
(7) a nitro group,
(8) a cyano group,
(9) a hydroxy group,

(10) a $C_{1-4}$ alkoxy group,
(11) a $C_{1-4}$ alkylthio group,
(12) a amino group,
(13) a mono- or di-$C_{1-4}$ alkylamino group,
(14) a 5-membered to 9-membered cyclic amino group optionally having 1 to 3 hetero atoms selected from the group consisting of nitrogen, oxygen and sulfur atoms in addition to the nitrogen atom in the amino group,
(15) a $C_{1-4}$ alkyl-carbonylamino group,
(16) a $C_{1-4}$ alkylsulfonylamino group,
(17) a $C_{1-4}$ alkoxy-carbonyl group,
(18) a carboxyl group,
(19) a $C_{1-6}$ alkyl-carbonyl group,
(20) a carbamoyl group,
(21) a mono- or di-$C_{1-4}$ alkylcarbamoyl group,
(22) a $C_{1-6}$ alkylsulfonyl group,
(23) an oxo group, and
(24) a thioxo group.

**3.** The pharmaceutical composition as claimed in claim 1, wherein $R^a$ and $R^b$ are the same or different and represent, independently, a hydrogen atom or a substituent selected from the group consisting of

(1) a halogen atom,
(2) a $C_{1-6}$ alkyl group optionally having from 1 to 5 substituents selected from the group consisting of

(a) a hydroxy group,
(b) a $C_{1-6}$ alkoxy group,
(c) a $C_{1-6}$ alkylthio group,
(d) an amino group,
(e) a $C_{1-7}$ acylamino group,
(f) a mono- or di-$C_{1-6}$ alkylamino group,
(g) a mono- or di-$C_{3-8}$ cycloalkylamino group,
(h) a 5-membered to 9-membered cyclicamino group which may have 1 to 3 hetero atoms selected from the group consisting nitrogen, oxygen and sulfur atoms in addition to the nitrogen atom in the amino group and,
(i) a $C_{1-4}$ alkylsulfonylamino group,
(j) a $C_{1-6}$ alkyl-carbonyloxy group and
(k) a halogen atom,

(3) a 5-membered to 9-membered cyclicamino group which may have 1 to 3 hetero atoms selected from the group consisting of oxygen and sulfur atoms in addition to the nitrogen atom in the amino group and which may be substituted by $C_{1-6}$ alkyl group,
(4) a carboxyl group,
(5) a carbamoyl group,
(6) a mono- or di-$C_{1-6}$ alkylcarbamoyl group; or

$R^a$ and $R^b$ are bonded to each other to form Ring A, and the Ring A is a 5-membered to 9-membered aromatic heterocyclic ring having from 1 to 3 hetero atoms selected from the group consisting of nitrogen, oxygen and sulfur atoms in addition to carbon atoms, which may be substituted by $C_{1-6}$ alkyl group; the Ring B is a $C_{6-14}$ aryl group which may be substituted by substituents selected from the group consisting of (i) a $C_{1-6}$ alkyl group optionally substituted by a hydroxy group, (ii) a $C_{1-6}$ alkylcarbonyl group and (iii) a carboxyl group; the Ring C is a $C_{6-14}$ aryl group which may be substituted by 1 to 3 substituents selected from the group consisting of (i) a halogen atom, (ii) an optionally halogenated $C_{1-10}$ alkyl group and (iii) a $C_{1-10}$ alkoxy group;
the Ring Z is a 5-membered to 12-membered heterocyclic ring optionally having at least one hetero atom selected from the group consisting of nitrogen, oxygen and sulfur atoms in addition to Y and the nitrogen atom, which may be substituted by 1 to 3 substituents selected from the group consisting of (i) a $C_{1-6}$ alkyl group, (ii) a hydroxy group and (iii) an oxo group;

**4.** The pharmaceutical composition as claimed in claim 1, which comprises a compound represented by the formula:

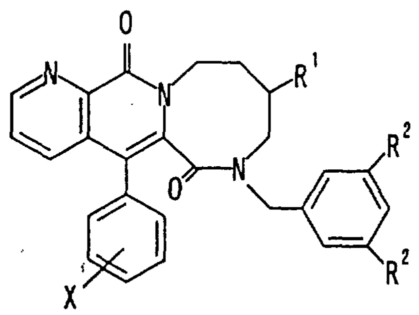

wherein $R^1$ is a $C_{1-6}$ alkyl group, $R^2$ is a $C_{1-6}$ alkoxy group, optionally halogenated $C_{1-6}$ alkyl group, a halogen atom, a hydroxy group or $C_{7-15}$ aralkyloxy group, X is 1 to 5 groups selected from the group consisting of a hydrogen atom, a $C_{1-6}$ alkyl group and a halogen atom, provided that (1) when $R^1$ is a methyl group and $R^2$ is s trifluoromethyl group, X is a halogen atom, and (2) when $R^1$ is a methyl group and $R^2$ is a methoxy group, X is a hydrogen atom or a halogen atom.

**5.** The pharmaceutical composition as claimed in claim 1, which comprises (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine or a salt thereof.

**6.** The pharmaceutical composition as claimed in claim 1, which comprises (9R)-7-(3,5-dimethoxybenzyl)-6,7,8,9,10,11-hexahydro-9-methyl-5-(4-methylphenyl)-6,13-dioxo-13H-[1,4]diazocino[2,1-g][1,7]naphthyridine or a salt thereof.

**7.** The pharmaceutical composition as claimed in claim 1, which comprises (9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine or a salt thereof.

**8.** (95)-7-[3,5-bis(trifluoromethyl)benzyl]-6,7,8,9,10,12-hexahydro-9-methyl-5-(4-methyl)phenyl-6,12-dioxo-[1,4]diazepino[2,1-g][1,7]naphthyridine or a salt thereof.

**9.** A compound represented by the formula:

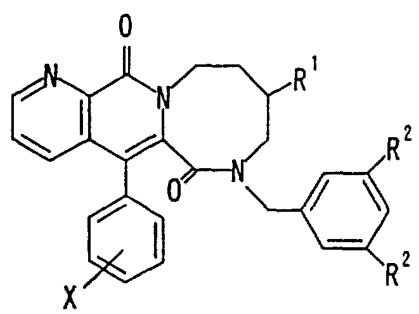

wherein $R^1$ is a $C_{1-6}$ alkyl group, $R^2$ is a $C_{1-6}$ alkoxy group, optionally halogenated $C_{1-6}$ alkyl group, a halogen atom, a hydroxy group or a $C_{7-15}$ aralkyloxy group, X is 1 to 5 groups selected from the group consisting of a hydrogen atom, a $C_{1-6}$ alkyl group and a halogen atom, provided that (1) when $R^1$ is a methyl group and $R^2$ is s trifluoromethyl group, X is a halogen atom and (2) when $R^1$ is a methyl group and $R^2$ is a methoxy group, X is a hydrogen atom or a halogen atom, or a salt thereof.

**10.** A compound as claimed in claim 9, wherein $R^1$ is a $C_{1-3}$ alkyl group, $R^2$ is a $C_{1-3}$ alkoxy group, optionally halogenated $C_{1-3}$ alkyl group, a halogen atom, a hydroxy group or a benzyloxy group, X is a hydrogen atom, or 1 or 2 groups selected from the group consisting of a $C_{1-3}$ alkyl group and a halogen atom.

**11.** A compound as claimed in claim 9, wherein $R^1$ is a methyl group group, $R^2$ is a methoxy group, an ethoxy group, an isopropoxy group, a methyl group, a trifluoromethyl group, a chlorine atom, a hydroxy group or a benzyloxy

group, X is a hydrogen atom, a methyl group, or 1 or 2 chlorine or fluorine atoms.

**12.** A compound as claimed in claim 9 which is (9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine.

**13.** A pro-drug of a compound as claimed in claim 9.

**14.** A pharmaceutical composition which comprises a compound as claimed in claim 9.

**15.** A composition for antagonizing a tachykinin receptor which comprises a compound as claimed in claim 9.

**16.** A composition for antagonizing a Substance P receptor which comprises a compound as claimed in claim 9.

**17.** A composition for antagonizing a neurokinin A receptor which comprises a compound as claimed in claim 9.

**18.** A pharmaceutical composition for preventing or treating disorders of micturition which comprises a compound as claimed in claim 9.

**19.** A pharmaceutical composition for preventing or treating disorders of asthma, rheumatoid arthritis, osteoarthritis, pain, cough, irritable bowel syndrome or emesis, which comprises a compound as claimed in claim 9.

**20.** Use of a compound represented by the formula:

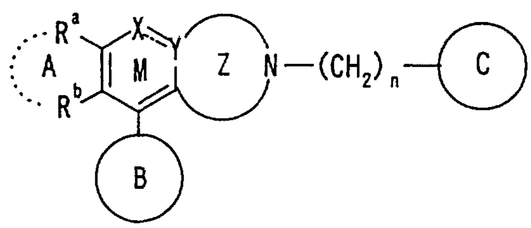

wherein ring M is a heterocyclic ring wherein

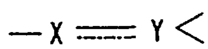

is one of -N=C<, -CO-N< or -CS-N<;

$R^a$ and $R^b$ are bonded to each other to form Ring A, or they are the same or different and represent, independently, a hydrogen atom or a substituent on the Ring M;
Ring A and Ring B represent, independently, an optionally substituted homocyclic or heterocyclic ring, with the proviso that at least one of them is an optionally substituted heterocyclic ring;
Ring C is an optionally substituted homocyclic or heterocyclic ring;
Ring Z is an optionally substituted nitrogen-containing heterocyclic ring; and
n is an integer from 1 to 6, or a salt thereof for manufacturing a composition for preventing or treating depression, anxiety, manic-depressive illness or psychopathy.

**21.** A method for preventing or treating disorders of micturition in mammals which comprises administrating to a subject in need an effective amount of a compound represented by the formula:

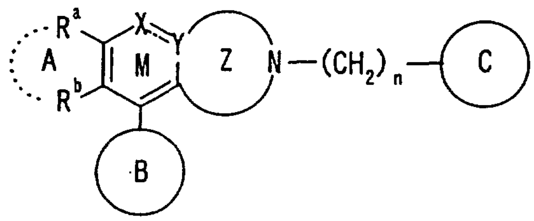

wherein ring M is a heterocyclic ring wherein

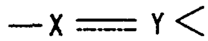

is one of -N=C<, -CO-N< or -CS-N<;

$R^a$ and $R^b$ are bonded to each other to form Ring A, or they are the same or different and represent, independently, a hydrogen atom or a substituent on the Ring M;
Ring A and Ring B represent, independently, an optionally substituted homocyclic or heterocyclic ring, with the proviso that at least one of them is an optionally substituted heterocyclic ring;
Ring C is an optionally substituted homocyclic or heterocyclic ring;
Ring Z is an optionally substituted nitrogen-containing heterocyclic ring; and
n is an integer from 1 to 6, or a salt thereof.

**22.** Use of a compound as claimed in claim 9 for manufacturing a composition for antagonizing a tachykinin receptor.

**23.** Use of a compound as claimed in claim 9 for manufacturing a pharmaceutical composition for antagonizing a Substance P receptor.

**24.** Use of a compound as claimed in claim 9 for manufacturing a pharmaceutical composition for antagonizing a neurokinin A receptor.

**25.** Use of a compound as claimed in claim 9 for manufacturing a pharmaceutical composition for treating disorders of micturition.

**26.** Use of a compound as claimed in claim 9 for manufacturing a pharmaceutical composition for treating disorders of asthma, rheumatoid arthritis, osteoarthritis, pain, cough, irritable bowel syndrome or emesis, which comprises a compound as claimed in claim 9.

**27.** A method for antagonizing a tachykinin receptor in mammals which comprises administrating to a subject in need, an effective amount of a compound as claimed in claim 9.

**28.** A method for antagonizing a Substance P receptor in mammals which comprises administrating to a subject in need an effective amount of a compound as claimed in claim 9.

**29.** A method for antagonizing a neurokinin A receptor in mammals which comprises administrating to a subject in need an effective amount of a compound as claimed in claim 9.

**30.** A method for preventing or treating disorders of micturition in mammals which comprises administrating to a subject in need an effective amount of a compound as claimed in claim 9.

**31.** A method for preventing or treating disorders of asthma, rheumatoid arthritis, osteoarthritis, pain, cough, irritable bowel syndrome or emesis in mammals which comprises administrating to a subject in need an effective amount of a compound as claimed in claim 9.